# EUROPEAN PATENT SPECIFICATION

(11) **EP 3 887 372 B1**
(45) Date of publication and mention of the grant of the patent: **12.10.2022**
(21) Application number: 19817162.1
(22) Date of filing: 25.11.2019
(51) Int. Cl.: C07D 471/04, C07D 487/04, C07D 519/00, A61P 11/00, A61P 31/12, A61K 31/437, A61K 31/5025

(54) **FURTHER HETEROAROMATIC COMPOUNDS HAVING ACTIVITY AGAINST RSV**
HETEROAROMATISCHE VERBINDUNGEN MIT ANTI-RSV AKTIVITÄT
COMPOSES HETEROAROMATIQUES AYANT UNE ACTIVITE CONTRE LE RSV

(30) Priority: 26.11.2018 EP 18208276
(43) Date of publication of application: 06.10.2021
(73) Proprietor: Janssen Sciences Ireland Unlimited Company, Ringaskiddy, Co Cork (IE)
(72) Inventor: GUILLEMONT, Jérôme, Émile, Georges, 92787 Issy-les-Moulineaux Cedex 9 (FR); LANCOIS, David, Francis, Alain, 92787 Issy-les-Moulineaux Cedex 9 (FR); CHAO, Sovy, 92787 Issy-les-Moulineaux Cedex 9 (FR); ANGIBAUD, Patrick, René, 92787 Issy-les Moulineaux Cedex 9 (FR); MERCEY, Guillaume, Jean, Maurice, 92787 Issy-les Moulineaux Cedex 9 (FR); RABOISSON, Pierre, Jean-Marie, Bernard, 2340 Beerse (BE); MICHAUT, Antoine, Benjamin, 67405 Illkirch Cedex (FR); RIGAUX, Peter, 2340 Beerse (BE)
(74) Representative: Verberckmoes, Filip Gerard
(86) International application number: PCT/EP2019/082404
(87) International publication number: WO 2020/109224

(56) References cited:
- WO-A1-2016/091774
- WO-A1-2016/174079
- US-A1- 2013 164 280

## Description

### Field of the Invention

The invention concerns compounds having antiviral activity, in particular having an inhibitory activity on the replication of the respiratory syncytial virus (RSV). The invention further concerns pharmaceutical compositions comprising these compounds and the compounds for use in the treatment or prevention of respiratory syncytial virus infection. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### Background

Human RSV or Respiratory Syncytial Virus is a large RNA virus, member of the family of *Pneumoviridae*, genus *Orthopneumovirus* together with bovine RSV virus. Human RSV is responsible for a spectrum of respiratory tract diseases in people of all ages throughout the world. It is the major cause of lower respiratory tract illness during infancy and childhood. Over half of all infants encounter RSV in their first year of life, and almost all within their first two years. The infection in young children can cause lung damage that persists for years and may contribute to chronic lung disease in later life (chronic wheezing, asthma). Older children and adults often suffer from a (bad) common cold upon RSV infection. In old age, susceptibility again increases, and RSV has been implicated in a number of outbreaks of pneumonia in the aged resulting in significant mortality.

Infection with a virus from a given subgroup does not protect against a subsequent infection with an RSV isolate from the same subgroup in the following winter season. Re-infection with RSV is thus common, despite the existence of only two subtypes, A and B.

Today only three drugs have been approved for use against RSV infection. A first one is ribavirin, a nucleoside analogue that provides an aerosol treatment for serious RSV infection in hospitalized children. The aerosol route of administration, the toxicity (risk of teratogenicity), the cost and the highly variable efficacy limit its use. Synagis^{®} (palivizumab a monoclonal antibody, is used for passive immunoprophylaxis . Athough the benefit of Synagis^{®} has been demonstrated, the treatment is expensive, requires parenteral administration and is restricted to children at risk for developing severe pathology.

Clearly there is a need for an efficacious non-toxic and easy to administer drug against RSV replication. It would be particularly preferred to provide drugs against RSV replication that could be administered perorally.

Compounds that exhibit anti-RSV activity are disclosed in WO-2016/174079.

### Detailed description of the Invention

The present invention relates to compounds of formula (I) 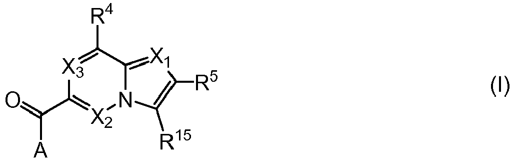 including any stereochemically isomeric form thereof, wherein
A is 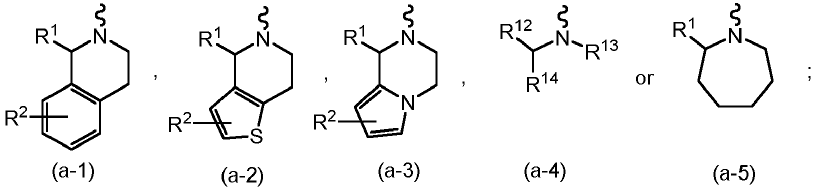 R⁵ is 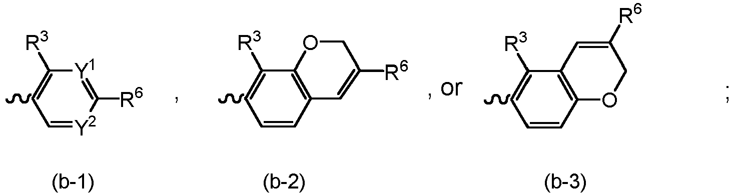
X₁, X₂, and X₃ are selected from X₁ is N, X₂ is CH, and X₃ is CH;
   or X₁ is N, X₂ is N, and X₃ is CH,
   or X₁ is N, X₂ is CH, and X₃ is N,
   or X₁ is CH, X₂ is CH, and X₃ is CH, and
   or X₁ is CH, X₂ is N, and X₃ is CH,
   wherein each CH is optionally substituted with halo or
   C₁₋₄alkyl;
Y¹ and Y² are each independently selected from CH, CF and N;
R¹ is CH₃ or CH₂CH₃;
R² is hydrogen, halo or C₁₋₄alkyl;
R¹² is C₁₋₂alkyl;
R¹³ and R¹⁴ are each independently selected from C₁₋₆alkyl;
R¹⁵ is hydrogen or C₁₋₄alkyl;
R³ is halo;
R⁴ is C₁₋₆alkyl; C₃₋₆cycloalkyl; di(C₁₋₄alkyl)amino, pyrrolidinyl, Heteroaryl¹; phenyl; phenyl substituted with 1, 2 or 3 substituents each individually selected from halo, hydroxy, cyano, C₁₋₄alkyl, polyhaloC₁₋₄alkyl, and C₁₋₄alkyloxy;
R⁶ is a substituent selected from substituent (a), (b), (c), (d) or (e); wherein
   (a) is -(CO)-OH, -(CO)-NR⁷R⁸, or -NR⁷R⁸;
   (b) is Heteroaryl²;
   (c) is C₂₋₆alkenyl substituted with one or two substituents selected from C₁₋₆alkyl, -(CO)-OH or -(CO)-NR⁸R⁹; or
   (d) is -NR⁸-(CO)-Heterocycle wherein said Heterocycle is substituted with one, two or three substituents each independently selected from halo, hydroxy, C₁₋₄alkyl of C₁₋₄alkyloxy; or
   (e) is C₃₋₆cycloalkyl or Heterocycle, wherein said C₃₋₆cycloalkyl and Heterocycle is substituted with one, two or three substituents each independently selected from C₁₋₆alkyl;
      C₁₋₆alkyl substituted with one, two or three substituents each independently selected from halo, hydroxy, hydroxycarbonyl, and aminocarbonyl;
      hydroxy;
      halo;
      -(CO)-OH;
      -(CO)-NR¹⁰R¹¹;
      -(CO)-NR⁸-SO₂-R⁹;
      -NR⁸R⁹;
      -NR⁸-(CO)-C₁-₄alkyl;
      -NR⁸-(CO)-C₃₋₆cycloalkyl;
      -NR⁸-SO₂-R⁹;
      -SO₂-NR¹⁰R¹¹; or
      -SO₂-NR₈-(CO)-R⁹;
      wherein
      R⁷ is hydrogen, C₁₋₄alkyl, hydroxyC₁₋₄alkyl or dihydroxyC₁₋₄alkyl;
      each R⁸ is independently selected from hydrogen, C₁₋₄alkyl, or hydroxyC₁₋₄alkyl;
      R⁹ is C₁₋₄alkyl, polyhaloC₁₋₄alkyl, or C₃₋₆cycloalkyl;
      R¹⁰ and R¹¹ are each indepently selected from hydrogen; C₁₋₄alkyl; polyhaloC₁₋₄alkyl; C₃₋₆cycloalkyl; C₃₋₆cycloalkyl substituted with C₁₋₄alkyl; or C₁₋₄alkyl substituted with hydroxy or cyano;

Heterocycle is azetidinyl, pyrrolodinyl, piperidinyl, or homopiperidinyl;

Heteroaryl¹ is thienyl, pyridinyl or pyrimidinyl, wherein each Heteroaryl¹ is optionally substituted with one or two substituents each independently selected from C₁₋₄alkyl, halo, amino, and aminocarbonyl;

Heteroaryl² is pyrrolyl, pyrazolyl or thiazolyl; wherein each Heteroaryl² is optionally substituted with one or two substituents each independently selected from C₁₋₄alkyl, halo, -(CO)-OR⁷ or -(CO)-NR⁸R⁹;
or a pharmaceutically acceptable acid addition salt thereof.

As used in the foregoing definitions:
- halo is generic to fluoro, chloro, bromo and iodo;
- C₁₋₄alkyl defines straight and branched chain saturated hydrocarbon radicals having from 1 to 4 carbon atoms such as, for example, methyl, ethyl, propyl, butyl, 1-methylethyl, 2-methylpropyl and the like;
- C₁₋₆alkyl is meant to include C₁₋₄alkyl and the higher homologues thereof having 5 or 6 carbon atoms, such as, for example, 2 methylbutyl, pentyl, hexyl and the like;
- C₂₋₆alkenyl defines bivalent straight or branched chain hydrocarbon radicals containing from 2 to 6 carbon atoms such as, for example, 1,2-ethanediyl, 1,3-propanediyl, 1,4-butanediyl, 1,5-pentanediyl, 1,6-hexanediyl, and the branched isomers thereof;
- C₃₋₆cycloalkyl is generic to cyclopropyl, cyclobutyl, cyclopentyl, and cyclohexyl;
- polyhaloC₁₋₄alkyl is defined as polyhalosubstituted C₁₋₄alkyl, in particular C₁₋₄alkyl (as hereinabove defined) substituted with 2 to 6 halogen atoms such as difluoromethyl, trifluoromethyl, trifluoroethyl, and the like;
- -(CO)- or (CO) means carbonyl.

The term "compounds of the invention" as used herein, is meant to include the compounds of formula (I), and the salts and solvates thereof.

As used herein, any chemical formula with bonds shown only as solid lines and not as solid wedged or hashed wedged bonds, or otherwise indicated as having a particular configuration (e.g. R, S) around one or more atoms, contemplates each possible stereoisomer, or mixture of two or more stereoisomers.

Hereinbefore and hereinafter, the terms "compound of formula (I)" and "intermediates of synthesis of formula (I)" are meant to include the stereoisomers thereof and the tautomeric forms thereof.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers. Enantiomers are stereoisomers that are non-superimposable mirror images of each other. A 1:1 mixture of a pair of enantiomers is a racemate or racemic mixture. Diastereomers (or diastereoisomers) are stereoisomers that are not enantiomers, i.e. they are not related as mirror images. If a compound contains a double bond, the substituents may be in the E or the Z configuration. Substituents on bivalent cyclic (partially) saturated radicals may have either the cis- or trans-configuration; for example, if a compound contains a disubstituted cycloalkyl group, the substituents may be in the cis or trans configuration.

The term "stereoisomers" also includes any rotamers, also called conformational isomers, the compounds of formula (I) may form.

Therefore, the invention includes enantiomers, diastereomers, racemates, E isomers, Z isomers, cis isomers, trans isomers, rotamers, and mixtures thereof, whenever chemically possible.

The meaning of all those terms, i.e. enantiomers, diastereomers, racemates, E isomers, Z isomers, cis isomers, trans isomers and mixtures thereof are known to the skilled person.

The absolute configuration is specified according to the Cahn-Ingold-Prelog system. The configuration at an asymmetric atom is specified by either R or S. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of formula (I) is for instance specified as (R), this means that the compound is substantially free of the (S) isomer; when a compound of formula (I) is for instance specified as E, this means that the compound is substantially free of the Z isomer; when a compound of formula (I) is for instance specified as cis, this means that the compound is substantially free of the trans isomer.

Some of the compounds according to formula (I) may also exist in their tautomeric form. Such forms in so far as they may exist, although not explicitly indicated in the above formula (I) are intended to be included within the scope of the present invention.

It follows that a single compound may exist in both stereoisomeric and tautomeric form.

Atropisomers (or atropoisomers) are stereoisomers which have a particular spatial configuration, resulting from a restricted rotation about a single bond, due to large steric hindrance. All atropisomeric forms of the compounds of Formula (I) are intended to be included within the scope of the present invention.

The pharmaceutically acceptable acid addition salts as mentioned hereinabove are meant to comprise the therapeutically active non-toxic acid addition salt forms that the compounds of formula (I) are able to form. These pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (*i*.*e*. ethanedioic), malonic, succinic (*i*.*e*. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

The compounds of formula (I) may exist in both unsolvated and solvated forms. The term 'solvate' is used herein to describe a molecular association comprising a compound of the invention and one or more pharmaceutically acceptable solvent molecules, e.g. water or ethanol. The term 'hydrate' is used when said solvent is water.

For the avoidance of doubt, compounds of formula (I) may contain the stated atoms in any of their natural or non-natural isotopic forms. In this respect, embodiments of the invention that may be mentioned include those in which (a) the compound of formula (I) is not isotopically enriched or labelled with respect to any atoms of the compound; and (b) the compound of formula (I) is isotopically enriched or labelled with respect to one or more atoms of the compound. Compounds of formula (I) that are isotopically enriched or labelled (with respect to one or more atoms of the compound) with one or more stable isotopes include, for example, compounds of formula (I) that are isotopically enriched or labelled with one or more atoms such as deuterium, ¹³C, ¹⁴C, ¹⁴N, ¹⁵O or the like.

A first group of compounds are compounds of formula (I) wherein radical A is of formula (a-1).

A second group of compounds are compounds of formula (I) wherein radical A is of formula (a-2).

A third group of compounds are compounds of formula (I) wherein radical A is of formula (a-5).

A fourth group of compounds are compounds of formula (I) wherein R⁵ is of formula (b-1) wherein Y¹ and Y² are CH.

A fifth group of compounds are compounds of formula (I) wherein R⁵ is of formula (b-2).

A sixth group of compounds are compounds of formula (I) wherein R⁵ is of formula (b-3).

A seventh group of compounds are compounds of formula (I) R¹ is CH₃.

An eight group of compounds are compounds of formula (I) wherein radical A is of formula (a-2).

A ninth group of compounds are compounds of formula (I) wherein R⁴ is C₃₋₆cycloalkyl.

A 10^{th} group of compounds are compounds of formula (I) wherein R⁶ is Heterocycle having one, two or three substituents as defined in (e).

A 11^{th} group of compounds are compounds of formula (I) wherein X₁ is N, X₂ is CH, and X₃ is CH.

A 12^{th} group of compounds are compounds of formula (I) wherein X₁ is N, X₂ is N, and X₃ is CH.

A 13^{th} group of compounds are compounds of formula (I) wherein X₁ is N, X₂ is CH, and X₃ is N.

Interesting compounds of formula (I) are those compounds of formula (I) wherein one or more of the following restrictions apply :
a) A is a radical of formula (a-1) wherein R¹ is CH₃; or
b) A is a radical of formula (a-2) wherein R¹ is CH₃; or
c) A is a radical of formula (a-5) wherein R¹ is CH₃; or
d) R² is hydrogen; or
e) R³ is fluoro; or
f) R⁴ is C₃₋₆cycloalkyl, in particular cyclopropyl; or
g) R⁴ is C₁₋₄alkyl, in particular ethyl; or
h) R⁴ is Heteroaryl¹ wherein Heteroaryl¹ is pyridinyl; or
i) R⁵ is of formula (b-1) wherein Y¹ and Y² are CH and R³ is halo, in particular R³ is fluoro; and
j) R⁶ is C₃₋₆cycloalkyl or pyrrolidinyl, wherein said C₃₋₆cycloalkyl or pyrrolidinyl are substituted with one or two substituents each independently selected from OH, -(CO)-OH or -(CO)-NR¹⁰R¹¹.

In general compounds of formula (I) can be prepared by reacting an intermediate of formula (II) with an alkylboronate intermediate of formula (III) in at least one reaction-inert solvent and optionally in the presence of at least one transition metal coupling reagent and/or at least one suitable ligand, the said process further optionally comprising converting a compound of formula (I) into an addition salt thereof. Suitable metal coupling reagents and/or suitable ligands for this reaction are, e.g. palladium compounds such as palladium tetra(triphenylphosphine), tris(dibenzylidene-acetone dipalladium, 2,2'-bis(diphenylphosphino)-1,1'-binaphtyl and the like.

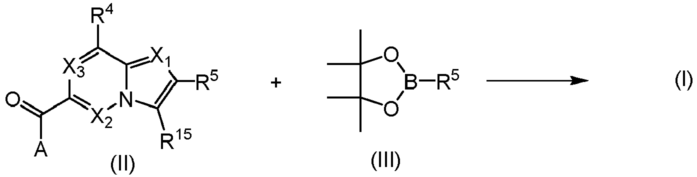

Compounds of formula (I-a), defined as compounds of formula (I) wherein R⁵ is of formula (b-1), can also be prepared by reacting an intermediate of formula (IV) with either an intermediate of formula (V), (VI) or (VII) in a reaction-inert solvent and optionally in the presence of at least one transition metal coupling reagent and/or at least one suitable ligand, the said process further optionally comprising converting a compound of formula (I) into an addition salt thereof.

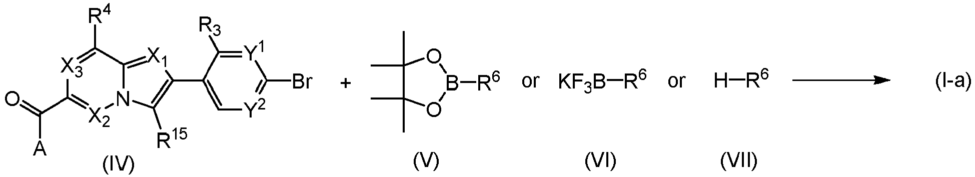

Other synthetic pathways for preparing compounds of formula (I) have been described in the experimental party as general methods of preparation and specific working examples.

The compounds of formula (I) may further be prepared by converting compounds of formula (I) into each other according to art-known group transformation reactions.

The starting materials and some of the intermediates are known compounds and are commercially available or may be prepared according to conventional reaction procedures generally known in the art.

The compounds of formula (I) as prepared in the hereinabove described processes may be synthesized in the form of racemic mixtures of enantiomers which can be separated from one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I) show antiviral properties. Viral infections treatable using the compounds and methods of the present invention include those infections brought on by ortho- and paramyxoviruses and in particular by human and bovine respiratory syncytial virus (RSV). A number of the compounds of this invention moreover are active against mutated strains of RSV. Additionally, many of the compounds of this invention show a favorable pharmacokinetic profile and have attractive properties in terms of bioavailabilty, including an acceptable half-life, AUC and peak values and lacking unfavourable phenomena such as insufficient quick onset and tissue retention.

The *in vitro* antiviral activity against RSV of the present compounds was tested in a test as described in the experimental part of the description, and may also be demonstrated in a virus yield reduction assay. The *in vivo* antiviral activity against RSV of the present compounds may be demonstrated in a test model using cotton rats as described in Wyde et al. in Antiviral Research, 38, p. 31 - 42 (1998).

The compounds of formula (I) show antiviral properties. Viral infections preventable or treatable using the compounds and methods of the present invention include those infections brought on by *Pneumoviridae* and in particular by human and bovine respiratory syncytial virus (RSV).

Therefore the present compounds of formula (I), or a pharmaceutically acceptable acid addition salt thereof, may be used as a medicine, in particular may be used as a medicine for the treatment or prevention of infections brought on by *Pneumoviridae* and in particular by human and bovine respiratory syncytial virus (RSV).

The present invention also provides the use of a compound of formula (I) or a pharmaceutically acceptable salt thereof for the manufacture of a medicament for the treatment or prevention of infections brought on by *Pneumoviridae* and in particular by human and bovine respiratory syncytial virus (RSV).

In other aspects, provided are methods of treating a respiratory syncytial virus (RSV) infection in an individual in need thereof comprising administering to the individual a therapeutically effective amount of a compound of formula (I) provided herein, or a pharmaceutically acceptable salt thereof, or a pharmaceutical composition comprising a therapeutically effective amount of a compound of formula (I) provided herein, or a pharmaceutically acceptable salt thereof. In some embodiments, the individual has one or more symptoms of an RSV infection. In some embodiments, the RSV is RSV Type A. In some embodiments, the RSV is RSV Type B.

Also provided are methods of ameliorating one or more symptoms of an RSV infection in an individual in need thereof comprising administering to the individual a therapeutically effective amount of a compound of formula (I) provided herein, or a pharmaceutically acceptable salt thereof, or a pharmaceutical composition comprising a therapeutically effective amount of a compound of formula (I) provided herein, or a pharmaceutically acceptable salt thereof. In some embodiments, the symptom is one or more of : coughing, sneezing, runny nose, sore throat, fever, decrease of appetite, irritability, decreased activity, apnea, and wheezing. In some embodiments, the individual has a lower respiratory tract infection. In some embodiments, the individual has bronchiolitis, pneumonia, or croup. In some embodiments, the individual has been diagnosed with an RSV infection. In some embodiments, the RSV is RSV Type A. In some embodiments, the RSV is RSV Type B. In some embodiments, the RSV infection has been confirmed by a laboratory test. In some embodiments, the method further comprises obtaining the results of an RSV detecting laboratory test. In some embodiments, the laboratory test comprises detecting RSV in a nasal sample.

Also provided are methods of preventing an RSV infection in an individual at risk of developing an RSV infection comprising administering to the individual a prophylactically effective amount of a compound of formula (I) provided herein, or a pharmaceutically acceptable salt thereof, or a pharmaceutical composition comprising a prophylactically effective amount of a compound of formula (I) provided herein, or a pharmaceutically acceptable salt thereof. In some embodiments, the individual is between 0 and about 2 years of age. In some embodiments, the individual was born prematurely. In other embodiments, the individual is greater than 65 years of age. In some embodiments, the individual is immunocompromised.

As used herein, "treatment" or "treating" is an approach for obtaining beneficial or desired results including clinical results. For example, beneficial or desired results in treating a viral infection include, but are not limited to, one or more of the following: eliminating or lessening the severity of one or more symptoms resulting from the viral infection (such as but not limited to coughing, sneezing, runny nose, sore throat, fever, decrease of appetite, irritability, decreased activity, apnea, and wheezing), increasing the quality of life of those suffering from the viral infection, decreasing the dose of other medications required to treat the viral infection, delaying the progression of the viral infection, and/or prolonging survival of an individual.

As used herein, "preventing" a viral infection is an approach for eliminating or reducing the risk of developing a viral infection or delaying the onset of a viral infection, including biochemical, histological and/or behavioral symptoms of a viral infection. Prevention may be in the context of an individual at risk of developing the viral infection, such as where the "at risk" individual does not develop the viral infection over a period of time, such as during a viral season or during a period of exposure to the virus, which may be days to weeks to months. An individual "at risk" of developing a viral infection is an individual with one or more risk factors for developing the viral infection but who has not been diagnosed with and does not display symptoms consistent with a viral infection. Risk factors for developing an RSV infection include but are not limited to an individual's age (young children under age 5 such as children between about 0 and about 2 years of age, including infants, and individuals greater than 65 years of age), premature birth, co-morbidities associated with RSV and individuals who are immune-compromised.

As used herein, a "therapeutically effective dosage" or "therapeutically effective amount" of compound or salt thereof or pharmaceutical composition is an amount sufficient to produce a desired therapeutic outcome. A therapeutically effective amount or a therapeutically effective dosage can be administered in one or more administrations. A therapeutically effective amount or dosage may be considered in the context of administering one or more therapeutic agents (e.g., a compound, or pharmaceutically acceptable salt thereof), and a single agent may be considered to be given in a therapeutically effective amount if, in conjunction with one or more other agents, a desired therapeutic outcome is achieved. Suitable doses of any of the co-administered compounds may optionally be lowered due to the combined action (e.g., additive or synergistic effects) of the compounds.

As used herein, a "prophylactically effective dosage" or "prophylactically effective amount" is an amount sufficient to effect the preventative result of eliminating or reducing the risk of developing a viral infection or delaying the onset of a viral infection, including biochemical, histological and/or behavioral symptoms of a viral infection. A prophylactically effective amount or a prophylactically effective dosage can be administered in one or more administrations and over a period of time in which such prevention is desired. Additionally, the present invention provides pharmaceutical compositions comprising at least one pharmaceutically acceptable carrier and a therapeutically effective amount of a compound of formula (I).

Also provided are pharmaceutical compositions comprising a pharmaceutically acceptable carrier, a therapeutically active amount of a compound of formula (I), and another antiviral agent, in particular an RSV inhibiting compound.

Also, the combination of another antiviral agent and a compound of formula (I) can be used as a medicine. Thus, the present invention also relates to a product containing (a) a compound of formula (I), and (b) another antiviral compound, as a combined preparation for simultaneous, separate or sequential use in antiviral treatment. The different drugs may be combined in a single preparation together with pharmaceutically acceptable carriers. Other antiviral compounds (b) to be combined with a compound of formula (I) for use in the treatment of RSV are RSV fusion inhibitors or RSV polymerase inhibitors.

In order to prepare the pharmaceutical compositions of this invention, an effective amount of the particular compound, in free base form or acid addition salt form, as the active ingredient is combined in intimate admixture with at least one pharmaceutically acceptable carrier, which carrier may take a wide variety of forms depending on the form of preparation desired for administration. These pharmaceutical compositions are desirably in unitary dosage form suitable, preferably, for oral administration, rectal administration, percutaneous administration, parenteral or intramuscular injection.

For example in preparing the compositions in oral dosage form, any of the usual liquid pharmaceutical carriers may be employed, such as for instance water, glycols, oils, alcohols and the like in the case of oral liquid preparations such as suspensions, syrups, elixirs and solutions; or solid pharmaceutical carriers such as starches, sugars, kaolin, lubricants, binders, disintegrating agents and the like in the case of powders, pills, capsules and tablets. Because of their easy administration, tablets and capsules represent the most advantageous oral dosage unit form, in which case solid pharmaceutical carriers are obviously employed. For parenteral injection compositions, the pharmaceutical carrier will mainly comprise sterile water, although other ingredients may be included in order to improve solubility of the active ingredient. Injectable solutions may be prepared for instance by using a pharmaceutical carrier comprising a saline solution, a glucose solution or a mixture of both. Injectable suspensions may also be prepared by using appropriate liquid carriers, suspending agents and the like. In compositions suitable for percutaneous administration, the pharmaceutical carrier may optionally comprise a penetration enhancing agent and/or a suitable wetting agent, optionally combined with minor proportions of suitable additives which do not cause a significant deleterious effect to the skin. Said additives may be selected in order to facilitate administration of the active ingredient to the skin and/or be helpful for preparing the desired compositions. These topical compositions may be administered in various ways, e.g., as a transdermal patch, a spot-on or an ointment. Addition salts of the compounds of formula (I), due to their increased water solubility over the corresponding base form, are obviously more suitable in the preparation of aqueous compositions.

It is especially advantageous to formulate the pharmaceutical compositions of the invention in dosage unit form for ease of administration and uniformity of dosage. "Dosage unit form" as used herein refers to physically discrete units suitable as unitary dosages, each unit containing a predetermined amount of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

For oral administration, the pharmaceutical compositions of the present invention may take the form of solid dose forms, for example, tablets (both swallowable and chewable forms), capsules or gelcaps, prepared by conventional means with pharmaceutically acceptable excipients and carriers such as binding agents, fillers, lubricants, disintegrating agents, wetting agents and the like. Such tablets may also be coated by methods well known in the art.

Liquid preparations for oral administration may take the form of e.g. solutions, syrups or suspensions, or they may be formulated as a dry product for admixture with water and/or another suitable liquid carrier before use. Such liquid preparations may be prepared by conventional means, optionally with other pharmaceutically acceptable additives such as suspending agents, emulsifying agents, non-aqueous carriers, sweeteners, flavours, masking agents and preservatives.

The compounds of formula (I) may be formulated for parenteral administration by injection, conveniently intravenous, intramuscular or subcutaneous injection, for example by bolus injection or continuous intravenous infusion. Formulations for injection may be presented in unit dosage form, e.g. in ampoules or multi-dose containers, including an added preservative. They may take such forms as suspensions, solutions or emulsions in oily or aqueous vehicles, and may contain formulating agents such as isotonizing, suspending, stabilizing and/or dispersing agents. Alternatively, the active ingredient may be present in powder form for mixing with a suitable vehicle, e.g. sterile pyrogen free water, before use.

The compounds of formula (I) may also be formulated in rectal compositions such as suppositories or retention enemas, e.g. containing conventional suppository bases such as cocoa butter and/or other glycerides.

In general, it is contemplated that an antivirally effective daily amount would be from 0.01 mg/kg to 500 mg/kg body weight, more preferably from 0.1 mg/kg to 50 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention. The effective daily amount ranges mentioned hereinabove are therefore only guidelines.

Also, the combination of another antiviral agent and a compound of formula (I) can be used as a medicine. Thus, the present invention also relates to a product containing (a) a compound of formula (I), and (b) another antiviral compound, as a combined preparation for simultaneous, separate or sequential use in antiviral treatment. The different drugs may be combined in a single preparation together with pharmaceutically acceptable carriers. For instance, the compounds of the present invention may be combined with interferon-beta or tumor necrosis factor-alpha in order to treat or prevent RSV infections. Other antiviral compounds (b) to be combined with a compound of formula (I) for use in the treatment of RSV are RSV fusion inhibitors or RSV polymerase inhibitors. Specific antiviral compounds for combination with any of the compounds of formula (I) that are useful in the treatment of RSV are the RSV inhibiting compounds selected from ribavirin, lumicitabine, presatovir, nirsevimab, sisunatovir, ziresovir, ALX-0171, MDT-637, BTA-9881, BMS-433771, YM-543403, A-60444, TMC-353121, RFI-641, CL-387626, MBX-300, EDP-938, 3-({5-chloro-1-[3-(methyl-sulfonyl)propyl]-1*H-*benzimidazol-2-yl}methyl)-1-cyclopropyl-1,3-dihydro-2H-imidazo[4,5-c]pyridin-2-one, 3-[[7-chloro-3-(2-ethylsulfonyl-ethyl)imidazo[1,2-a]pyridin-2-yl]methyl]-1-cyclopropyl-imidazo[4,5-c]pyridin-2-one, and 3-({5-chloro-1-[3-(methyl-sulfonyl)propyl]-1*H*indol-2-yl}methyl)-1-(2,2,2-trifluoroethyl)-1,3-dihydro-2H-imidazo[4,5-c]pyridin-2-one.

### Experimental part

### A. Abbreviations

| | |
|---|---|
| µw | Microwave |
| AcOH | Acetic acid |
| NaOAc | Sodium acetate |
| aq. | Aqueous |
| br | Broad |
| cataCXium^{®} A | Di(1-adamantyl)-*n*-butylphosphine CAS [321921-71-5] |
| CDI | 1,1'-Carbonyldiimidazole CAS [530-62-1] |
| d | Doublet |
| DCM | Dichloromethane |
| DIPEA | *N*,*N*-Diisopropylethylamine |
| DMF | Dimethylformamide |
| DMSO | Dimethyl sulfoxide |
| EtOAc | Ethyl acetate |
| h | Hour |
| HATU | 1-[Bis(dimethylamino)methylene]-1*H*-1,2,3-triazolo[4,5-*b*]pyridinium 3-oxid hexafluorophosphate CAS [148893-10-1] |
| LiHMDS | Lithium bis(trimethylsilyl)amide |
| m | Multiplet |
| m/z | Mass-to-charge ratio |
| MeCN / ACN | Acetonitrile |
| Me-THF | 2-Methyltetrahydrofuran - CAS [96-47-9] |
| min | Minute(s) |
| NBS | *N*-Bromosuccinimide - CAS [128-08-5] |
| NMP | *N*-Methyl-2-pyrrolidone |
| | CAS [872-50-4] |
| NMR | Nuclear Magnetic Resonance |
| o/n | Overnight |
| Pd(OAc)₂ | Palladium (II) acetate - CAS [3375-31-3] |
| PdCl₂(dppf) | [1,1'-Bis(diphenylphosphino)ferrocene]dichloropalladium(II) CAS [72287-26-4] |
| PdCl₂(dppf).DC M | [1,1'-Bis(diphenylphosphino)ferrocene]dichloropalladium(II), complex with dichloromethane - CAS [95464-05-4] |
| dppf | 1,1'-Bis(diphenylphosphino)ferrocene |
| ppm | Parts per million |
| PTAT | Trimethylphenylammonium tribromide - CAS [4207-56-1] |
| q | Quartet |
| quin | Quintuplet |
| Pd₂(dba)₃ | Tris(dibenzylideneacetone)dipalladium(0) |
| rt | Room temperature |
| RuPhos | 2-Dicyclohexylphosphino-2',6'-diisopropoxybiphenyl CAS [787618-22-8] |
| s | Singulet |
| sext | Sextuplet |
| t | Triplet |
| *t*-BuOK | Potassium *tert*-butoxide |
| TED/DABCO | 1,4-Diazabicyclo[2.2.2]octane - CAS [280-57-9] |
| THF | Tetrahydrofuran |
| XantPhos | 4,5-Bis(diphenylphosphino)-9,9-dimethylxanthene CAS [161265-03-8] |
| Δ | Heat |
| RuPhos PdG3 | (2-Dicyclohexylphosphino-2',6'-diisopropoxy-1,1'-biphenyl)[2-(2'-amino-1,1'-biphenyl)]palladium(II) methanesulfonate |
| EDCI HCl | N-Ethyl-N'-(3-dimethylaminopropyl)carbodiimide hydrochloride |
| HOBt H₂O | 1-Hydroxybenzotriazole hydrate |
| DavePhos | 2-Dicyclohexylphosphino-2'-(*N*,*N*-dimethylamino)biphenyl |
| µw | Microwave |
| AcOH | Acetic acid |
| NaOAc | Sodium acetate |
| aq. | Aqueous |
| br | Broad |
| cataCXium^{®} A | Di(1-adamantyl)-*n*-butylphosphine - CAS [321921-71-5] |
| CDI | 1,1'-Carbonyldiimidazole - CAS [530-62-1] |
| d | Doublet |
| DCM | Dichloromethane |
| DIPEA | *N*,*N*-Diisopropylethylamine |
| DMF | Dimethylformamide |

The stereochemical configuration for some compounds has been designated as R^{∗} or S^{∗} (or ^{∗}R or ^{∗}S) when the absolute stereochemistry is undetermined (even if the bonds are drawn stereospecifically) although the compound itself has been isolated as a single stereoisomer and is enantiomerically pure. This means that the absolute stereoconfiguration of the stereocentre indicated by ^{∗} is undetermined (even if the bonds are drawn stereospecifically) although the compound is enantiomerically pure at the indicated centre.

### B. Compound synthesis

### Part 1 : Imidazo[1,2-a]pyridines

### Synthesis of Intermediates A3 and A6:

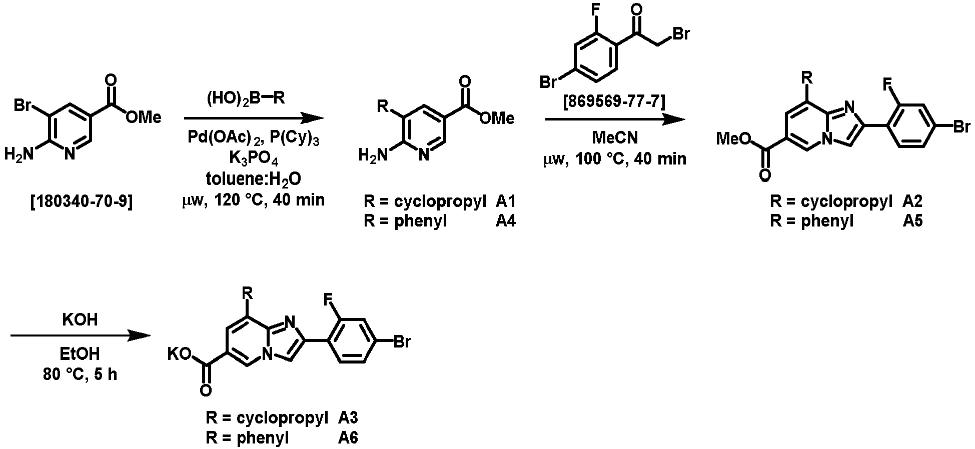

### Intermediate A1

### Methyl 6-amino-5-cyclopropylpyridine-3-carboxylate

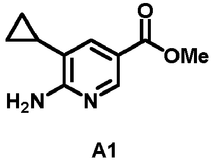

A mixture of methyl 6-amino-5-bromonicotinate [180340-70-9] (6.00 g, 26.0 mmol), cyclopropylboronic acid [411235-57-9] (3.35 g, 39.0 mmol) and potassium phosphate tribasic (18.7 g, 88.3 mmol) in toluene (56 mL) and H₂O (10 mL) was purged with nitrogen for 5 min. Tricyclohexylphosphine (728 mg, 2.60 mmol) and palladium acetate (583 mg, 2.60 mmol) were added and the mixture was purged with nitrogen for 2 min. The reaction mixture was heated at 120°C using a single mode microwave (Biotage^{®} Initiator EXP 60) with a power output ranging from 0 to 400 W for 40 min. The reaction mixture was filtered through a pad of Celite^{®} and washed with EtOAc and H₂O. The layers were separated, and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 120 g, 30 µm, liquid injection (DCM), mobile phase gradient: heptane / EtOAc from 90:10 to 60:40) to afford intermediate **A1** (3.3 g, 66%) as a yellow solid.

### Intermediate A2

### Methyl 2-(4-bromo-2-fluorophenyl)-8-cyclopropylimidazo[1,2-a]pyridine-6-carboxylate

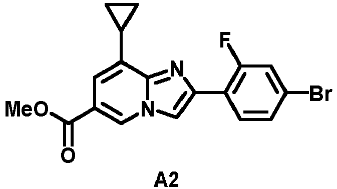

A mixture of intermediate **A1** (1.00 g, 5.20 mmol) and 4-bromo-2-fluorophenacyl bromide [869569-77-7] (1.85 g, 6.24 mmol) in MeCN (15 mL) was heated at 120°C using a single mode microwave (Anton Paar Monowave^{®} 300) with a power output ranging from 0 to 850 W for 40 min. The mixture was cooled to 0°C. The solid was filtered off and dried under vacuum to afford intermediate **A2** (1.38 g, 68%) as a beige solid.

### Intermediate A3

### Potassium 2-(4-bromo-2-fluorophenyl)-8-cyclopropylimidazo[1,2-a]pyridine-6-carboxylate

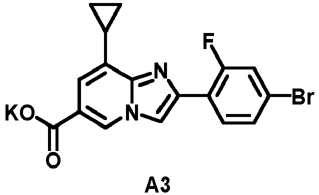

A mixture of intermediate **A2** (1.00 g, 2.57 mmol) and potassium hydroxide (577 mg, 10.3 mmol) in EtOH (40 mL) was stirred at 80°C for 5 h. The mixture was cooled to 0°C. The precipitate was filtered off and dried under vacuum to afford intermediate **A3** (0.85 g, 80%) as a beige solid.

### Synthesis of Intermediate A6 :

### Intermediate A4

### Methyl 6-amino-5-phenylpyridine-3-carboxylate

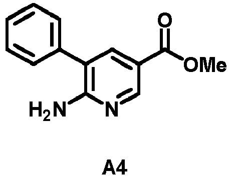

A mixture of methyl 6-amino-5-bromonicotinate [180340-70-9] (1.50 g, 6.49 mmol), phenylboronic acid [98-80-6] (871 mg, 7.14 mmol) and potassium phosphate tribasic (4.69 g, 22.1 mmol) in toluene (14 mL) and H₂O (2.5 mL) was purged with nitrogen for 5 min. Tricyclohexylphosphine (182 mg, 0.65 mmol) and palladium acetate (146 mg, 0.65 mmol) were added. The reaction mixture was purged with nitrogen for 2 min and heated at 120°C using a single mode microwave (Anton Paar Monowave^{®} 300) with a power output ranging from 0 to 850 W for 40 min. The reaction mixture was filtered through a pad of Celite^{®} and washed with EtOAc and H₂O. The layers were separated, and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 40 g, 30 µm, liquid injection (DCM), mobile phase gradient: heptane / EtOAc from 90:10 to 70:30) to afford intermediate **A4** (1.3 g, 88%) as a yellow solid.

### Intermediate A5

### Methyl 2-(4-bromo-2-fluorophenyl)-8-phenylimidazo[1,2-a]pyridine-6-carboxylate

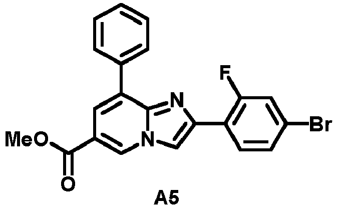

A mixture of intermediate **A4** (1.10 g, 4.82 mmol) and 4-bromo-2-fluorophenacyl bromide [869569-77-7] (1.71 g, 5.78 mmol) in MeCN (15 mL) was heated at 120°C using a single mode microwave (Anton Paar Monowave^{®} 300) with a power output ranging from 0 to 850 W for 40 min. The mixture was cooled to 0°C. The solid was filtered off and dried under vacuum to afford intermediate **A5** (0.9 g, 44%) as a white solid.

### Intermediate A6

### Potassium 2-(4-bromo-2-fluorophenyl)-8-phenylimidazo[1,2-a]pyridine-6-carboxylate

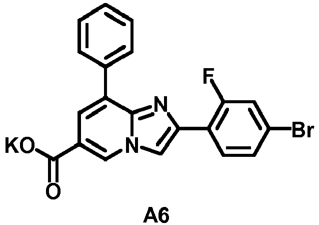

A mixture of intermediate **A5** (0.90 g, 2.12 mmol) and potassium hydroxide (475 mg, 8.47 mmol) in EtOH (30 mL) was stirred at 80°C for 5 h. The mixture was cooled to 0°C. The precipitate was filtered off and dried under vacuum to afford intermediate **A6** (578 mg, 61%) as a white solid.

### Synthesis of Intermediate A9

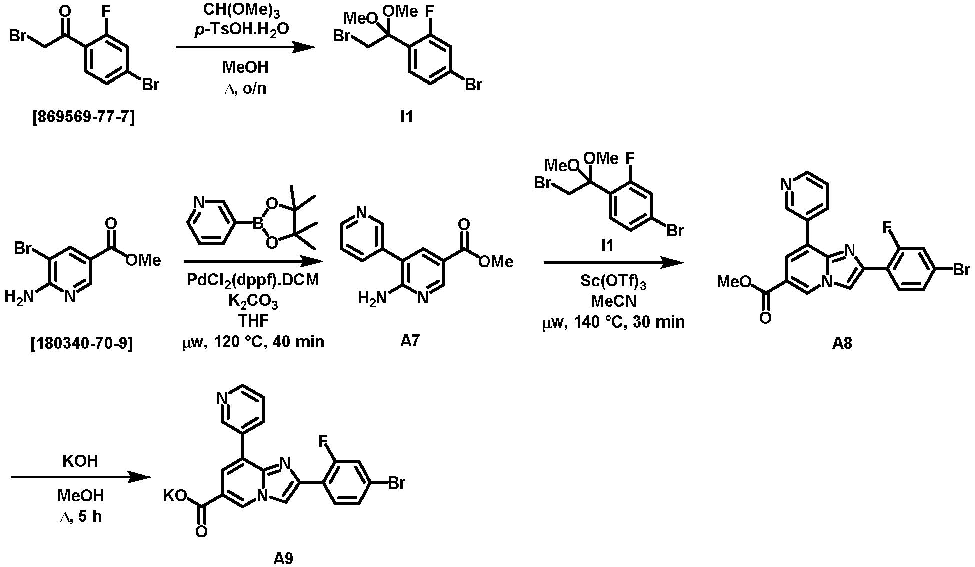

### Intermediate I1

### 4-Bromo-1-(2-bromo-1,1-dimethoxyethyl)-2-fluorobenzene

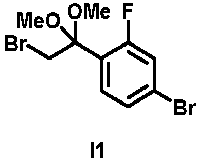

A mixture of 4-bromo-2-fluorophenacyl bromide [869569-77-7] (1.50 g, 5.07 mmol), trimethyl orthoformate (1.11 mL, 10.1 mmol) and *p*-toluenesulfonic acid monohydrate (48.3 mg, 0.25 mmol) in MeOH (45 mL) was stirred under reflux overnight. The solvent was evaporated in vacuo. The residue was diluted with DCM and H₂O. The organic phase was separated (hydrophobic frit) and evaporated to dryness to afford intermediate **I1** (1.14 g, 98%) as a colorless oil.

### Intermediate A7

### Methyl 6-amino-5 -(pyridin-3 -yl)pyridine-3 -carboxylate

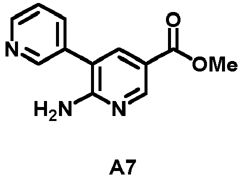

A mixture of methyl 6-amino 5-bromonicotinate [180340-70-9] (1.00 g, 4.33 mmol), 3-(4,4,5,5-tetramethyl-1,3,2-dioxaborolan-2-yl)pyridine [329214-79-1] (1.33 g, 6.49 mmol) and potassium carbonate (2.0 M in H₂O, 6.50 mL, 13.0 mmol) was purged with nitrogen for 5 min. [1,1'-Bis(diphenylphosphino)ferrocene]dichloro-palladium(II), dichloromethane complex (353 mg, 0.43 mmol) was added and the reaction mixture was purged again with nitrogen for 2 min. The reaction mixture was heated at 120°C using a single mode microwave (Anton Paar Monowave^{®} 300) with a power output ranging from 0 to 850 W for 40 min. The reaction mixture was filtered through a pad of Celite^{®} and washed with EtOAc and H₂O. The layers were separated, and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 40 g, 30 µm, dry loading (Celite^{®}), mobile phase gradient: DCM / MeOH from 100:0 to 97:3) to afford intermediate A7 (0.75 g, 76%) as a grey solid.

### Intermediate A8

### Methyl 2-(4-bromo-2-fluorophenyl)-8-(pyridin-3-yl)imidazo[1,2-a]pyridine-6-carboxylate

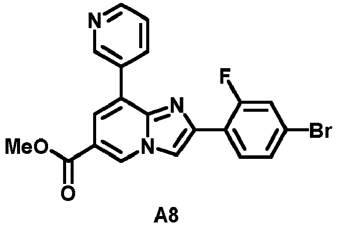

A mixture of intermediate **A7** (0.34 g, 1.48 mmol), intermediate **I1** (609 mg, 1.78 mmol) and scandium trifluoromethanesulfonate (36.5 mg, 74.2 µmol) in MeCN (7.4 mL) was heated at 140°C using a single mode microwave (Anton Paar Monowave^{®} 300) with a power output ranging from 0 to 850 W for 30 min. The solid was filtered off, washed with EtOAc and dried under vacuum. The residue was purified by flash chromatography over silica gel (Puriflash Interchim^{®} 24 g, 30 µm, dry loading (Celite^{®}), mobile phase gradient: DCM / MeOH from 100:0 to 98:2) to afford intermediate **A8** (0.18 g, 28%) as a beige solid.

### Intermediate A9

### Potassium 2-(4-bromo-2-fluorophenyl)-8-(pyridin-3-yl)imidazo[1,2-a]pyridine-6-carboxylate

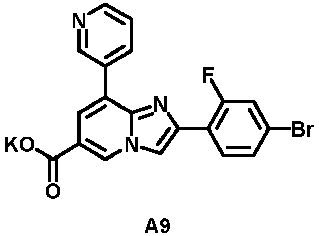

A mixture of intermediate **A8** (0.60 g, 1.41 mmol) and potassium hydroxide (316 mg, 5.63 mmol) in MeOH (20 mL) was stirred under reflux for 5 h. The mixture was acidified with acetic acid until pH 5. The precipitate was filtered off, washed with MeOH and H₂O and dried under vacuum to afford intermediate **A9** (0.35 g, 55%) as a beige solid.

### Synthesis of Intermediate A13:

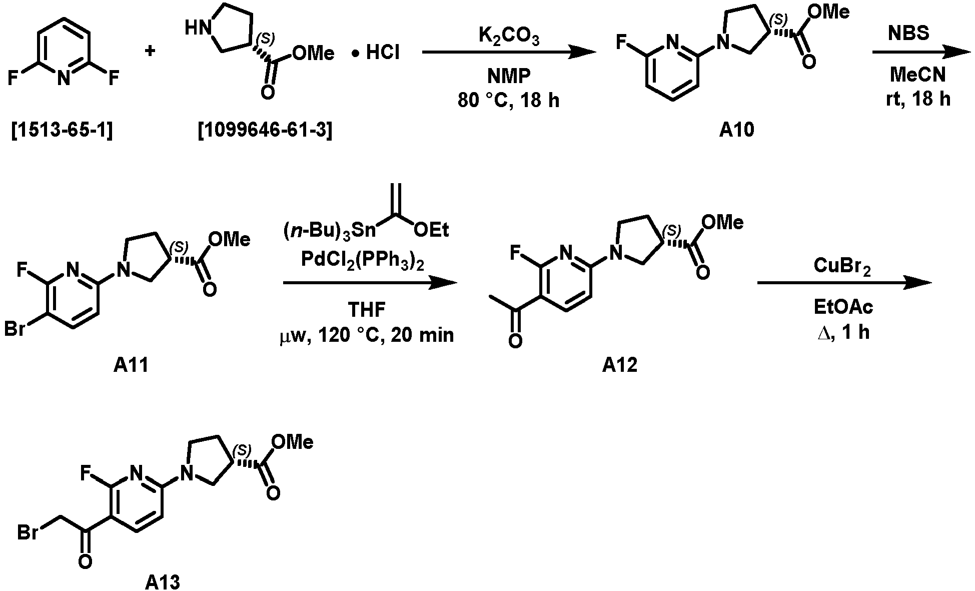

### Intermediate A10

### Methyl (3S)-1-(6-fluoropyridin-2-yl)pyrrolidine-3-carboxylate

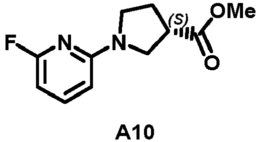

A mixture of 2,6-difluropyridine [1513-65-1] (1 mL, 11.0 mmol), (*S*)-methyl pyrrolidine-3-carboxylate hydrochloride [1099646-61-3] (2.00 g, 12.1 mmol) and potassium carbonate (4.57 g, 33.1 mmol) in NMP (50 mL) was stirred at 80°C for 18 h. The reaction mixture was diluted with EtOAc and H₂O. The layers were separated, and the aqueous phase was extracted with EtOAc. The combined organic extracts were washed with H₂O (4 times), dried over MgSO₄, filtered and evaporated to dryness to afford intermediate **A10** (2.22 g, 90%) as a colorless oil.

### Intermediate A11

### Methyl (3S)-1-(5-bromo-6-fluoropyridin-2-yl)pyrrolidine-3-carboxylate

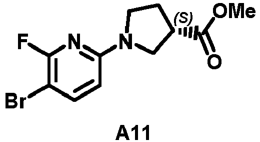

A mixture of intermediate **A10** (2.20 g, 9.81 mmol) and NBS (2.10 g, 11.8 mmol) in MeCN (49 mL) was stirred at rt for 18 h. The reaction was quenched with a saturated aqueous solution of Na₂S₂O₃. The mixture was extracted with DCM. The combined organic extracts were washed with brine, dried over MgSO₄, filtered and concentrated to dryness. The crude mixture was purified by flash chromatography over silica gel (Puriflash Interchim^{®} 40 g, 30 µm, liquid injection (DCM), mobile phase gradient: heptane / EtOAc from 100:0 to 80:20) to give intermediate **A11** (2.1 g, 71%) as a white solid.

### Intermediate A12

### Methyl (3S)-1-(5-acetyl-6-fluoropyridin-2-yl)pyrrolidine-3-carboxylate

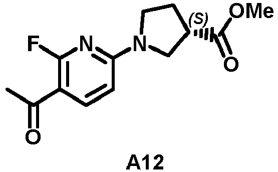

A solution of intermediate **A11** (1.00 g, 3.30 mmol) in THF (14 mL) was purged with nitrogen for 10 min. Tributyl(1-ethoxyvinyl)tin (2.23 mL, 6.60 mmol) and bis(triphenylphosphine)palladium(II) dichloride (232 mg, 0.33 mmol) were added. The reaction mixture was heated at 120°C using a single mode microwave (Anton Paar Monowave^{®} 300) with a power output ranging from 0 to 850 W for 20 min. A 3N aqueous solution of HCl (15 mL) was added and the resulting mixture was stirred at rt for 15 min. The layers were separated. The aqueous phase was extracted with EtOAc. The combined organic extracts were washed with H₂O and brine, dried over MgSO₄, filtered and evaporated to dryness. The crude mixture was purified by flash chromatography over silica gel (Puriflash Interchim^{®} 40 g, 30 µm, dry loading (Celite^{®}), mobile phase gradient: heptane / EtOAc from 100:0 to 70:30) to afford intermediate **A12** (0.71 g, 80%) as a yellow oil.

### Intermediate A13

### Methyl (3S)-1-[5-(2-bromoacetyl)-6-fluoropyridin-2-yl]pyrrolidine-3-carboxylate

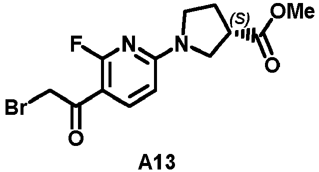

To a solution of copper(II) bromide (1.18 g, 5.27 mmol) in EtOAc (7 mL) was added a solution of intermediate **A12** (0.70 g, 2.64 mmol) in EtOAc (3 mL) dropwise. The reaction mixture was stirred under reflux for 1h. The solid was filtered out. The filtrate was washed with a saturated aqueous solution of NaHCO₃ and brine. The organic phase was dried over MgSO₄, filtered and concentrated in vacuo. The crude mixture was purified by flash chromatography over silica gel (Puriflash Interchim^{®} 40 g, 30 µm, mobile phase gradient: heptane / EtOAc from 90:10 to 60:40) to afford intermediate **A13** (0.68 g, 75%) as a yellow oil.

### Intermediate B1

### (1R)-2-[2-(4-Bromo-2-fluorophenyl)-8-cyclopropylimidazo[1,2-a]pyridine-6-carbonyl]-1-methyl-1,2,3,4-tetrahydroisoquinoline

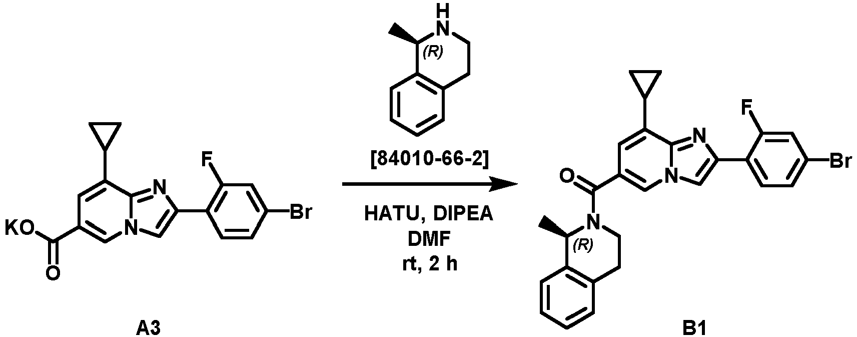

To a mixture of intermediate **A3** (0.77 g, 1.86 mmol) and (1*R*)-1-methyl-1,2,3,4-tetrahydroisoquinoline [84010-66-2] (329 mg, 2.24 mmol) in DMF (21.5 mL) were added DIPEA (0.98 mL, 5.59 mmol) and HATU (921 mg, 2.42 mmol). The reaction mixture was stirred at rt for 2 h. The mixture was poured out slowly into water and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 80 g, 30 µm, mobile phase gradient: heptane / EtOAc, from 90:10 to 70:30) to afford intermediate **B1** (0.63 g, 67%) as a white solid.

### Intermediate B2

### 2-(4-Bromo-2-fluorophenyl)-8-cyclopropyl-6-[(4R^{∗})-4-methyl-4,5,6,7-tetrahydro-thieno[3,2-c]pyridme-5-carbonyl]imidazo[1,2-a]pyridine

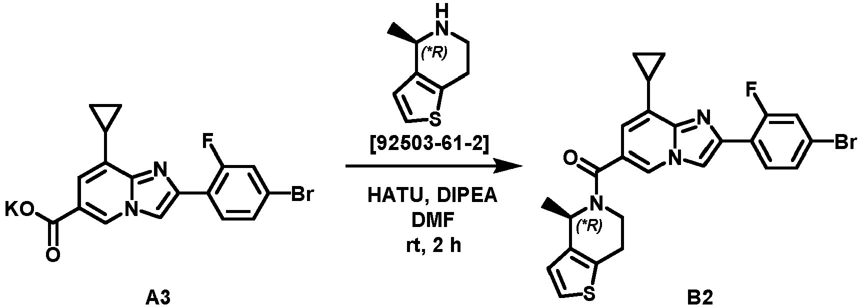

To a mixture of intermediate **A3** (0.27 g, 0.72 mmol) and 4-(*R*^{∗})-methyl-4,5,6,7-tetrahydrothieno[3,2-c]pyridine [92503-61-2] (0.13 g, 0.86 mmol) in DMF (5 mL) were added DIPEA (0.38 mL, 2.16 mmol) and HATU (0.36 g, 0.94 mmol). The reaction mixture was stirred at rt for 2 h. The mixture was poured out slowly into water and the aqueous phase was extracted with EtOAc. The combined organic extracts were washed with brine, dried over MgSO₄, filtered and evaporated to dryness.The crude mixture was purified by flash chromatography over silica gel (Puriflash Interchim^{®} 25 g, 30 µm, mobile phase gradient: heptane / EtOAc from 100:0 to 80:20). The good fractions were collected and the solvent was evaporated to afford intermediate **B2** (0.26 g, 71%) as a beige solid.

### Intermediate B3

### (2R)-1-[2-(4-Bromo-2-fluorophenyl)-8-cyclopropylimidazo[1,2-a]pyridine-6-carbonyl]-2-methylazepane

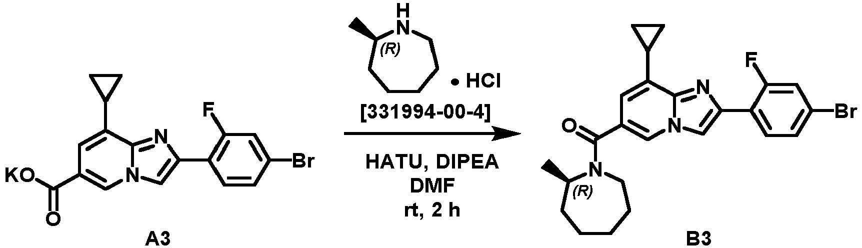

To a mixture of intermediate **A3** (1.00 g, 2.42 mmol) and (2*R*)-2-methylhexahydroazepine hydrochloride [331994-00-4] (435 mg, 2.90 mmol) in DMF (30 mL) were added DIPEA (2.11 mL, 12.1 mmol) and HATU (1.20 g, 3.15 mmol). The reaction mixture was stirred at rt for 2 h. The mixture was poured out slowly into water and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 40 g, 30 µm, mobile phase gradient: heptane / EtOAc from 100:0 to 70:30) to afford intermediate **B3** (0.62 g, 54%) as a beige solid.

### Intermediate B4

### (1R)-2-[2-(4-Bromo-2-fluorophenyl)-8-phenylimidazo[1,2-a]pyridine-6-carbonyl]-1-methyl-1,2,3,4-tetrahydroisoquinoline

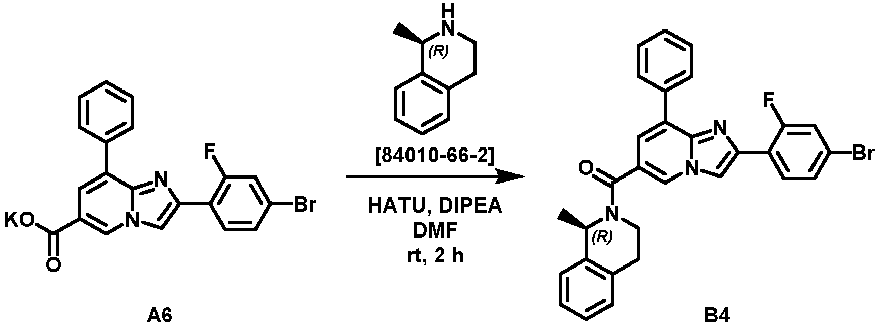

To a mixture of intermediate **A6** (0.57 g, 1.27 mmol) and (1*R*)-1-methyl-1,2,3,4-tetrahydroisoquinoline [84010-66-2] (224 mg, 1.52 mmol) in DMF (20 mL) were added DIPEA (0.67 mL, 3.81 mmol) and HATU (627 mg, 1.65 mmol). The reaction mixture was stirred at rt for 2 h. The mixture was poured out slowly into water and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 40 g, 30 µm, mobile phase gradient: heptane / EtOAc from 90:10 to 70:30) to afford intermediate **B4** (0.62 g, 90%) as a beige solid.

### Intermediate B5

### (1R)-2-[2-(4-Bromo-2-fluorophenyl)-8-(pyridin-3-yl)imidazo[1,2-a]pyridine-6-carbonyl]-1-methyl-1,2,3,4-tetrahydroisoquinoline

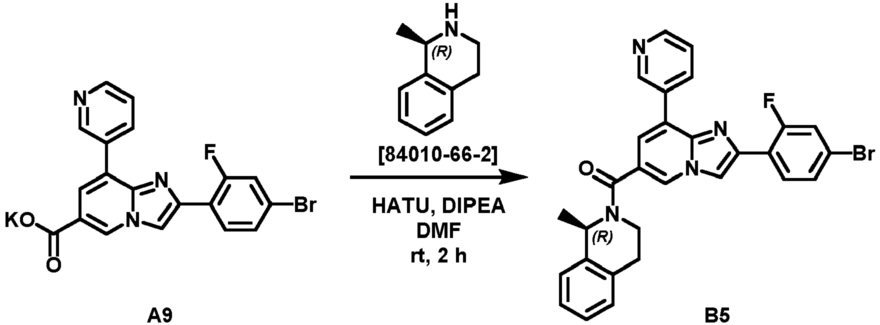

To a solution of intermediate **A9** (0.25 g, 606 µmοl) and (1*R*)-1-methyl-1,2,3,4-tetrahydroisoquinoline [84010-66-2] (107 mg, 728 µmol) in DMF (6 mL) were added DIPEA (0.42 mL, 2.43 mmol) and HATU (0.30 g, 0.79 mmol). The reaction mixture was stirred at rt for 2 h. The mixture was poured out slowly into water. The aqueous phase was extracted with DCM. The combined organic extracts were washed with brine, dried over MgSO₄, filtered and evaporated to dryness. The crude mixture was purified by flash chromatography over silica gel (Puriflash Interchim^{®} 24 g, 30 µm, mobile phase gradient: heptane / EtOAc from 100:0 to 60:40) to afford intermediate **B5** (0.27 mg, 82%) as a beige solid.

### Intermediate B6

### 3-[2-(4-Bromo-2-fluorophenyl)-6-[(4R^{∗})-4-methyl-4,5,6,7-tetrahydro-thieno[3,2-c]pyridine-5-carbonyl]imidazo[1,2-a]pyridin-8-yl]pyridine

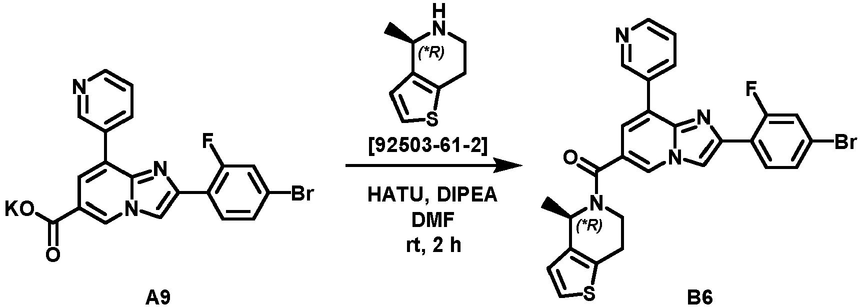

To a solution of intermediate **A9** (0.11 g, 244 µmοl) and 4-(^{∗}*R*)-methyl-4,5,6,7-tetrahydrothieno[3,2-c]pyridine [92503-61-2] (44.9 mg, 293 µmol) in DMF (2.5 mL) were added DIPEA (0.13 mL, 0.73 mmol) and HATU (111 mg, 0.29 mmol). The reaction mixture was stirred at rt for 2 h. The mixture was poured out slowly into water and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 12 g, 30 µm, liquid injection (DCM), mobile phase gradient: heptane / EtOAc from 90:10 to 50:50) to afford intermediate **B6** (0.13 g, 96%) as a beige solid.

### Synthesis of Intermediate C2

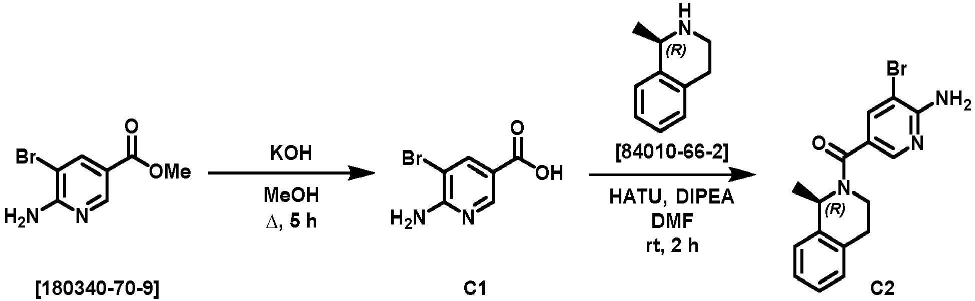

### Intermediate C1

### 6-Amino-5-bromopyridine-3-carboxylic acid

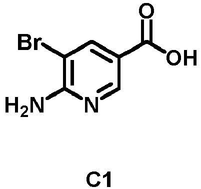

A mixture of methyl 6-amino-5-bromonicotinate [180340-70-9] (1.00 g, 4.33 mmol) and potassium hydroxide (971 mg, 17.3 mmol) in MeOH (25 mL) was stirred under reflux for 5 h. The reaction mixture was acidified with a solution of acetic acid until pH 5. The precipitate was filtered off, washed with MeOH and H₂O and dried under vacuum to afford intermediate **C1** (0.65 g, 69%) as a white solid.

### Intermediate C2

### 3-Bromo-5-[(1R)-1-methyl-1,2,3,4-tetrahydroisoquinoline-2-carbonyl]pyridin-2-amine

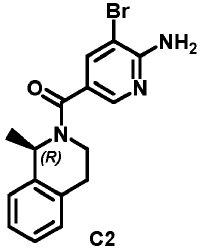

To a mixture of intermediate **C1** (0.65 g, 3.00 mmol) and (1*R*)-1-methyl-1,2,3,4-tetrahydroisoquinoline [84010-66-2] (0.53 g, 3.59 mmol) in DMF (15 mL) were added DIPEA (2.1 mL, 12.0 mmol) and HATU (1.48 g, 3.89 mmol). The reaction mixture was stirred at rt for 2 h. The mixture was poured out slowly into water and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 24 g, 30 µm, mobile phase gradient: heptane / EtOAc from 100:0 to 60:40) to afford intermediate C2 (0.46 g, 44%) as a beige solid.

### Intermediate C5

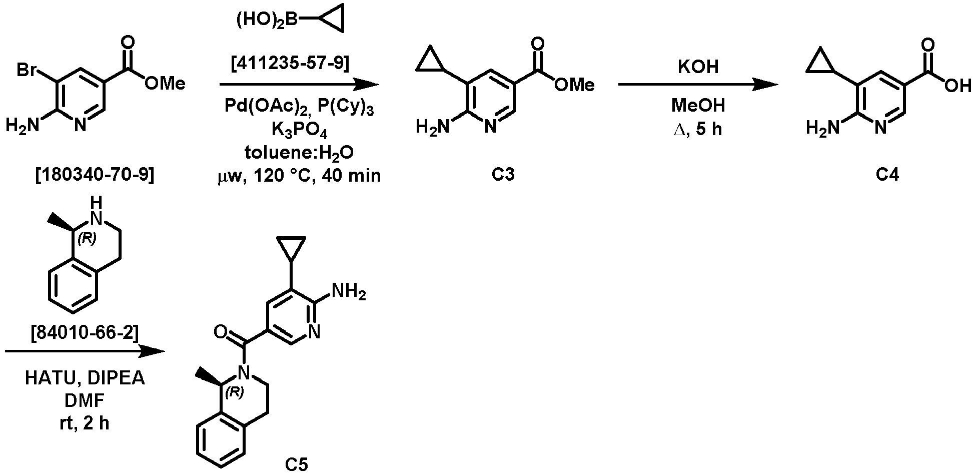

### Intermediate C3

### Methyl 6-amino-5-cyclopropylpyridine-3-carboxylate

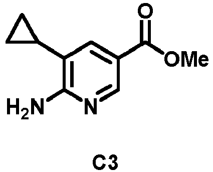

A mixture of methyl 6-amino-5-bromonicotinate [180340-70-9] (6.00 g, 26.0 mmol), cyclopropylboronic acid [411235-57-9] (3.35 g, 39.0 mmol) and potassium phosphate tribasic (18.7 g, 88.3 mmol) in toluene (56 mL) and H₂O (10 mL) was purged with nitrogen for 5 min. Tricyclohexylphosphine (728 mg, 2.60 mmol) and palladium acetate (583 mg, 2.60 mmol) were added. The reaction mixture was purged again with nitrogen for 2 min and heated at 120°C using a single mode microwave (Biotage^{®} Initiator EXP 60) with a power output ranging from 0 to 400 W for 40 min. The reaction mixture was filtered through a pad of Celite^{®} and washed with EtOAc and H₂O. The layers were separated, and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 120 g, 30 µm, mobile phase gradient: heptane / EtOAc from 90:10 to 60:40) to afford intermediate **C3** (3.35 g, 67%) as a yellow solid.

### Intermediate C4

### 6-Amino-5-cyclopropylpyridine-3-carboxylic acid

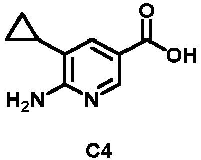

A mixture of intermediate **C3** (2.00 g, 10.4 mmol) and potassium hydroxide (2.34 g, 41.6 mmol) in MeOH (50 mL) was stirred under reflux for 5 h. The reaction mixture was acidified with a solution of acetic acid until pH 5. The precipitate was filtered off, washed with MeOH and H₂O and dried under vacuum to afford intermediate **C4** (1.5 g, 81%) as a beige solid.

### Intermediate C5

### 3-Cyclopropyl-5-[(1R)-1-methyl-1,2,3,4-tetrahydroisoquinoline-2-carbonyl]pyridin-2-amine

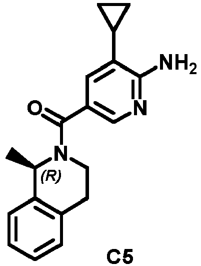

To a mixture of intermediate **C4** (1.42 g, 7.97 mmol) and (1*R*)-1-methyl-1,2,3,4-tetrahydroisoquinoline [84010-66-2] (1.41 g, 9.56 mmol) in DMF (24 mL) were added DIPEA (5.57 mL, 31.9 mmol) and HATU (3.94 g, 10.4 mmol). The reaction mixture was stirred at rt for 2 h. The mixture was poured out slowly into water and the aqueous phase was extracted with EtOAc. The combined organic extracts were washed with brine, dried over MgSO₄, filtered and evaporated to dryness. The crude mixture was purified by flash chromatography over silica gel

(Puriflash Interchim^{®} 40 g, 30 µm, mobile phase gradient: heptane / EtOAc from 100:0 to 70:30) to afford intermediate **C5** (2.2 g, 90%) as a beige solid.

### Intermediate C6

### 3-Cyclopropyl-5-[(4R^{∗})-4-methyl-4,5,6,7-tetrahydrothieno[3,2-c]pyridine-5-carbonyl]pyridin-2-amine

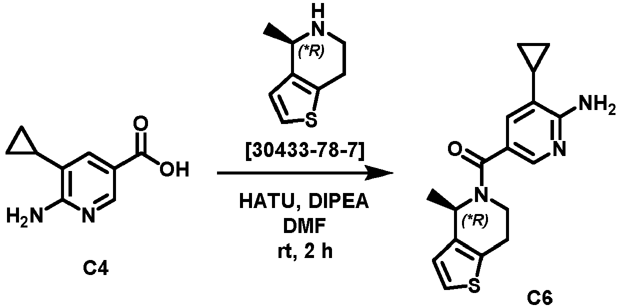

To a solution of intermediate **C4** (0.85 g, 4.74 mmol) and (4^{∗}*R*)-4-methyl-4,5,6,7-tetrahydrothieno[3,2-c]pyridine [30433-78-7] (0.87 g, 5.69 mmol) in DMF (25 mL) were added DIPEA (3.31 mL, 19.0 mmol) and HATU (2.34 g, 6.17 mmol). The reaction mixture was stirred at rt for 2 h. The mixture was poured out slowly into water and the aqueous phase was extracted with EtOAc. The combined organic extracts were was washed with brine, dried over MgSO₄, filtered and evaporated to dryness. The crude mixture was purified by flash chromatography over silica gel (Puriflash Interchim^{®} 40 g, 30 µm, mobile phase gradient: DCM / MeOH from 100:0 to 97:3) to afford intermediate **C6** (1.10 g, 74%) as a beige solid.

### Intermediate D2

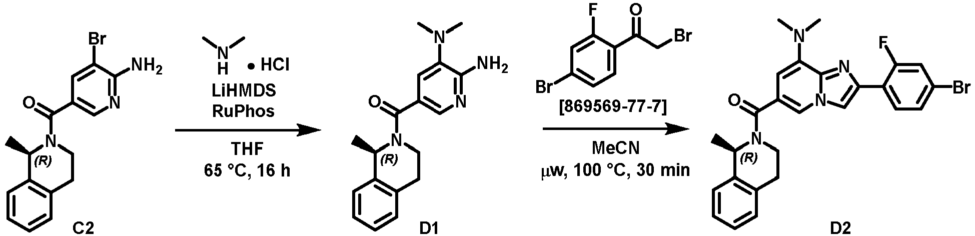

### Intermediate D1

### 3-N,N-Dimethyl-5-[(1R)-1-methyl-1,2,3,4-tetrahydroisoquinoline-2-carbonyl]pyridine-2,3-diamine

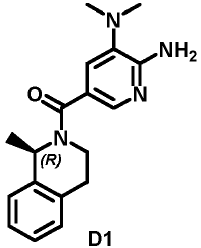

A mixture of intermediate **C2** (0.60 g, 1.73 mmol) and RuPhos (80.9 mg, 173 µmol) in THF (9 mL) was purged with nitrogen for 5 min. Dimethylamine hydrochloride [506-59-2] (0.17 g, 2.08 mmol) was added followed by LiHMDS (1.5 M in THF, 4.6 mL, 6.90 mmol). The reaction mixture was stirred at 65°C for 16 h. The reaction was quenched by the addition of a saturated aqueous solution of NH₄Cl and diluted with EtOAc. The layers were separated, and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 25 g, 30 µm, mobile phase gradient: DCM / MeOH from 100:0 to 98:2) to afford intermediate **D1** (0.22 g, 41%) as a beige solid.

### Intermediate D2

### 2-(4-Bromo-2-fluorophenyl)-N,N-dimethyl-6-[(1R)-1-methyl-1,2,3,4-tetrahydroisoquinoline-2-carbonyl]imidazo[1,2-a]pyridin-8-amine

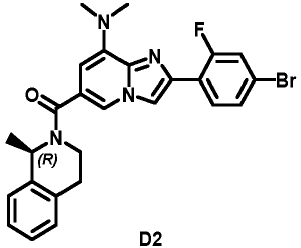

A mixture of intermediate **D1** (0.22 g, 709 µmol) and 4-bromo-2-fluorophenacyl bromide [869569-77-7] (252 mg, 851 µmol) in MeCN (4 mL) was heated at 100°C using a single mode microwave (Anton Paar Monowave^{®} 300) with a power output ranging from 0 to 850 W for 30 min. The mixture was evaporated to dryness. The crude mixture was purified by flash chromatography over silica gel (Puriflash Interchim^{®} 25 g, 30 µm, mobile phase gradient: heptane / EtOAc from 100:0 to 70:30) to afford intermediate **D2** (0.12 g, 33%) as a beige solid.

### Synthesis of Intermediate D4

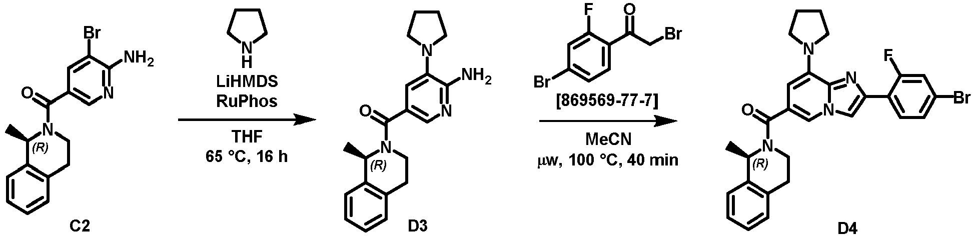

### Intermediate D3

### 5-[(1R)-1-Methyl-1,2,3,4-tetrahydroisoquinoline-2-carbonyl]-3-(pyrrolidin-1-yl)pyridin-2-amine

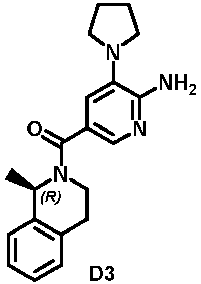

A mixture of intermediate **C2** (0.60 g, 1.73 mmol) and RuPhos (80.9 mg, 173 µmol) in THF (9 mL) was purged with nitrogen for 5 min. Pyrrolidine [123-75-1] (0.17 mL, 2.08 mmol) was added followed by LiHMDS (1.5 M in THF, 3.5 mL, 5.25 mmol). The reaction mixture was stirred at 65°C for 16 h. The reaction was quenched by the addition of H₂O and diluted with EtOAc. The layers were separated and the aqueous phase was extracted with EtOAc (twice). The combined organic extracts were washed with brine, dried over MgSO₄, filtered and the solvent was evaporated in vacuo. The residue was taken up in DCM, the mixture was cooled to 0°C and a precipitate was formed. The solid was filtered off and dried under vacuum to afford intermediate **D3** (0.43 g, 74%) as a beige solid.

### Intermediate D4

### (1R)-2-[2-(4-Bromo-2-fluorophenyl)-8-(pyrrolidin-1-yl)imidazo[1,2-a]pyridine-6-carbonyl]-1-methyl-1,2,3,4-tetrahydroisoquinoline

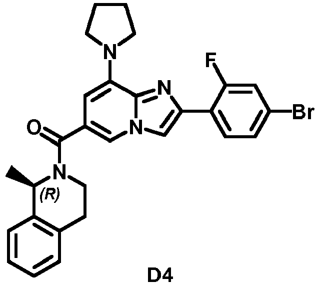

A mixture of intermediate **D3** (0.43 g, 1.28 mmol) and 4-bromo-2-fluorophenacyl bromide [869569-77-7] (454 mg, 1.53 mmol) in MeCN (6 mL) was heated at 100°C using a single mode microwave (Anton Paar Monowave^{®} 300) with a power output ranging from 0 to 850 W for 40 min. The mixture was evaporated to dryness. The crude mixture was purified by flash chromatography over silica gel (Puriflash Interchim^{®} 25 g, 30 µm, mobile phase gradient: heptane / EtOAc, from 100:0 to 70:30) to afford intermediate **D4** (0.30 g, 44%) as a green solid.

### Intermediate D5

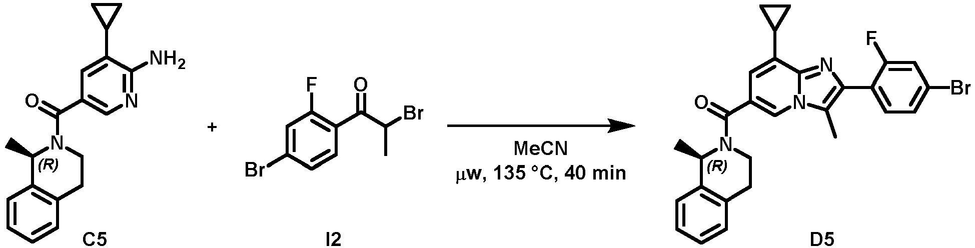

### Intermediate I2

### 2-Bromo-1 -(4-bromo-2-fluorophenyl)propan-1 -one

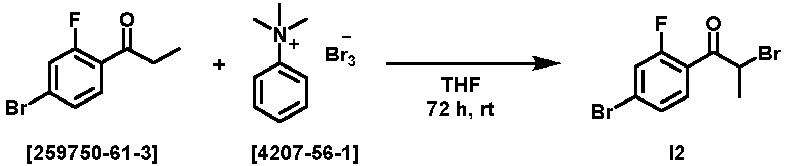

A mixture of 1-(4-bromo-2-fluorophenyl)propan-1-one [259750-61-3] (0.70 g, 3.03 mmol) and PTAT (1.14 g, 3.03 mmol) in THF (15 mL) was stirred under nitrogen atmosphere for 72 h at rt. The reaction mixture was filtered, and the filter cake was washed with THF several times. The filtrate was concentrated in vacuo. The crude mixture was purified by flash chromatography over silica gel (Puriflash Interchim^{®} 24 g, 30 µm, dry loading (Celite^{®}), mobile phase gradient: heptane / EtOAc from 100:0 to 90:10) to afford intermediate **12** (0.72 g, 77%) as a yellow oil.

### Intermediate D5

### (1R)-2-[2-(4-Bromo-2-fluorophenyl)-8-cyclopropyl-3-methylimidazo[1,2-a]pyridine-6-carbonyl]-1-methyl-1,2,3,4-tetrahydroisoquinoline

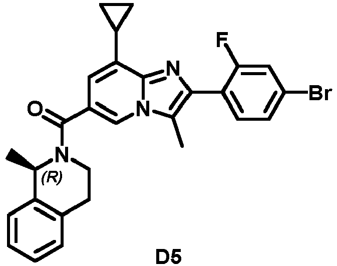

A mixture of intermediate **C5** (0.45 g, 1.46 mmol) and intermediate **12** (0.50 g, 1.61 mmol) in MeCN (9 mL) was heated at 135°C using a single mode microwave (Anton Paar Monowave^{®} 300) with a power output ranging from 0 to 850 W for 40 min. The reaction mixture was diluted with EtOAc and H₂O. The layers were separated, and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 25 g, 30 µm, dry loading (Celite^{®}), mobile phase gradient: heptane / EtOAc from 100:0 to 85:15) to afford intermediate D**5** (0.31 g, 41%) as a brown solid.

### Synthesis of Intermediate D6

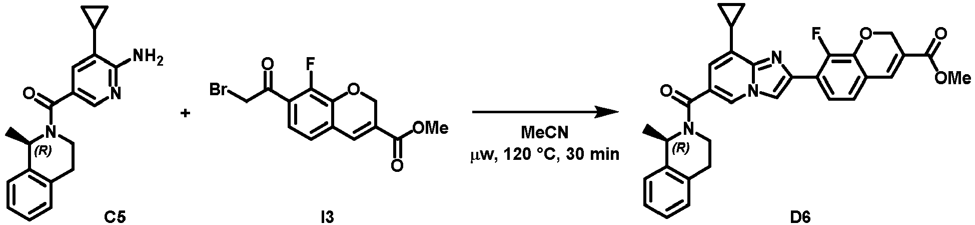

### Intermediate I3

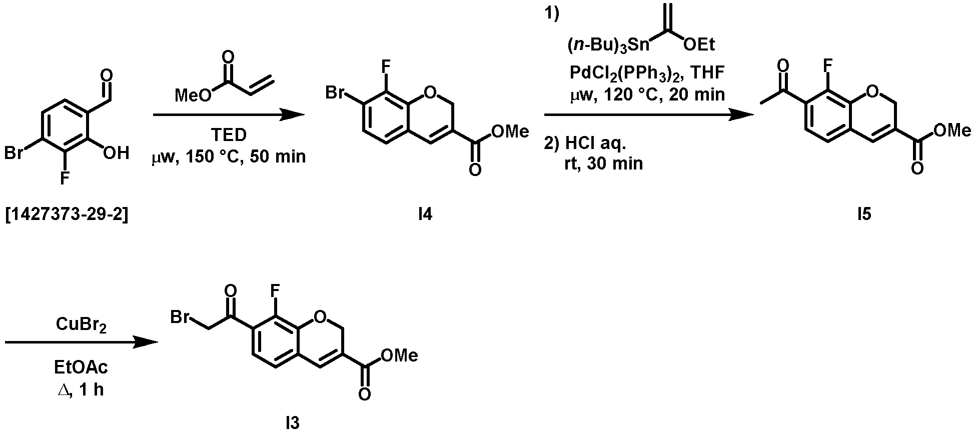

### Intermediate I4

### Methyl 7-bromo-8-fluoro-2H-chromene-3-carboxylate

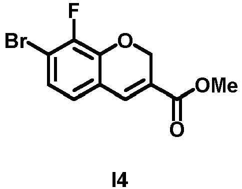

A mixture of 4-bromo-3-fluoro-2-hydroxybenzaldehyde [1427373-29-2] (1.00 g, 4.57 mmol), methyl acrylate (2.9 mL, 32.0 mmol) and triethylenediamine [280-57-9] (102 mg, 0.91 mmol) were heated at 150°C using a single mode microwave (Anton Paar Monowave^{®} 300) with a power output ranging from 0 to 850 W for 50 min. The mixture was poured out into a solution of water and DCM. The layers were separated (hydrophobic frit) and the organic phase was evaporated to dryness. The crude mixture was purified by flash chromatography over silica gel (Puriflash Interchim^{®} 40 g, 30 µm, mobile phase gradient: heptane / EtOAc from 100:0 to 80:20) to afford intermediate **14** (0.49 g, 37%) as a white solid.

### Intermediate I5

### Methyl 7-acetyl-8-fluoro-2H-chromene-3-carboxylate

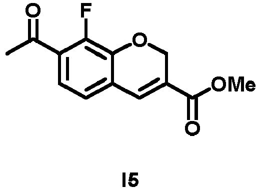

A solution of intermediate **14** (0.43 g, 1.39 mmol) in THF (1.5 mL) was purged with nitrogen for 10 min. Tributyl(1-ethoxyvinyl)tin (1.0 mL, 3.00 mmol) and bis(triphenylphosphine)palladium dichloride (105 mg, 0.15 mmol) were added. The mixture was heated at 120°C using a single mode microwave (Anton Paar Monowave^{®} 300) with a power output ranging from 0 to 850 W for 20 min. A 3N aqueous solution of HCl (1 mL) was added and the resulting mixture was stirred at rt for 30 min. The layers were separated and the aqueous phase was extracted with EtOAc. The combined organic extracts were washed with H₂O and brine and dried over MgSO₄, filtered and evaporated to dryness. The crude mixture was purified by flash chromatography over silica gel (Puriflash Interchim^{®} 25 g, 30 µm, dry loading (Celite^{®}), mobile phase gradient: heptane / EtOAc from 100:0 to 80:20). The residue (0.23 g) was hydrolysed with a 3N aqueous solution of HCl for 30 min at rt. The mixture was extracted with EtOAc. The combined organic extracts were dried over MgSO₄, filtered and evaporated to dryness to afford intermediate **15** (0.13 g, 35%) as a yellow solid.

### Intermediate I3

### Methyl 7-(2-bromoacetyl)-8-fluoro-2H-chromene-3-carboxylate

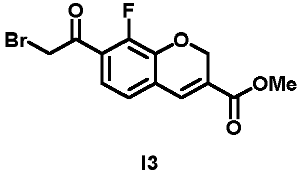

To a solution of copper bromide (1.27 g, 5.68 mmol) in EtOAc (4 mL) was added a solution of intermediate **15** (0.71 g, 2.84 mmol) in EtOAc (3 mL). The reaction mixture was stirred under reflux for 1 h. The solid was filtered off. The filtrate was washed with a saturated aqueous solution of NaHCO₃ and brine, dried over MgSO₄, filtered and concentrated in vacuo to give intermediate **13** (0.56 g, 60%) as a beige solid.

### Intermediate D6

### Methyl 6-{8-cyclopropyl-6-[(1R)-1-methyl-1,2,3,4-tetrahydroisoquinoline-2-carbonyl]imidazo[1,2-a]pyridin-2-yl}-7-fluoro-2H-chromene-3-carboxylate

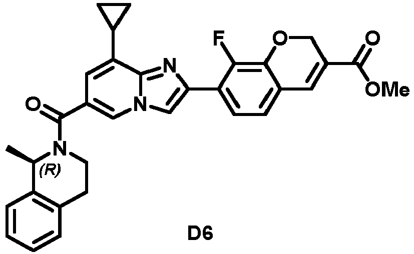

A mixture of intermediate **C5** (132 mg, 0.43 mmol) and intermediate **13** (0.17 g, 517 µmol) in MeCN (2.5 mL) was heated at 120°C using a single mode microwave (Anton Paar Monowave^{®} 300) with a power output ranging from 0 to 850 W for 30 min. The mixture was concentrated in vacuo. The crude mixture was purified by flash chromatography over silica gel (Puriflash Interchim^{®} 25 g, 30 µm, mobile phase gradient: heptane / EtOAc from 90:10 to 70:30) to afford intermediate **D6** (135 mg, 58%) as a yellow solid.

### Compound 1

### 4-{8-Cyclopropyl-6-[(1R)-1-methyl-1,2,3,4-tetrahydroisoquinoline-2-carbonyl]imidazo[1,2-a]pyridin-2-yl}-3-fluorobenzoic acid

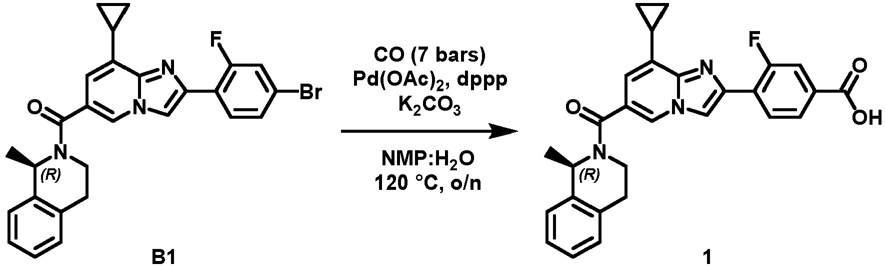

In autoclave, N₂ was bubbled into a solution of intermediate **B1** (0.50 g, 991 µmol) in H₂O (0.72 mL) and NMP (3.5 mL) for 10 min. Palladium acetate (11.1 mg, 49.6 µmοl), 1,3-bis(diphenylphosphino)propane (20.4 mg, 49.6 µmol) and potassium carbonate (164 mg, 1.19 mmol) were added. The autoclave was purged with nitrogen and with CO (3 times). The autoclave was pressurized with CO (7 bars) and heated at 120°C overnight. The reaction mixture was filtered through a pad of Celite^{®}. The filtrate was acidified with a 1N aqueous solution of HCl. The organic phase was washed with water and brine, dried over MgSO₄ and evaporated to dryness. The crude mixture was purified by flash chromatography over silica gel (Puriflash Interchim^{®} 40 g, 30 µm, mobile phase gradient: DCM / MeOH from 100:0 to 96:4) to afford **compound 1** (0.38 g, 82%) as a beige solid.

### Compound 2

### 4-{8-Cyclopropyl-6-[(1R)-1-methyl-1,2,3,4-tetrahydroisoquinoline-2-carbonyl]imidazo[1,2-a]pyridin-2-yl}-3-fluorobenzamide

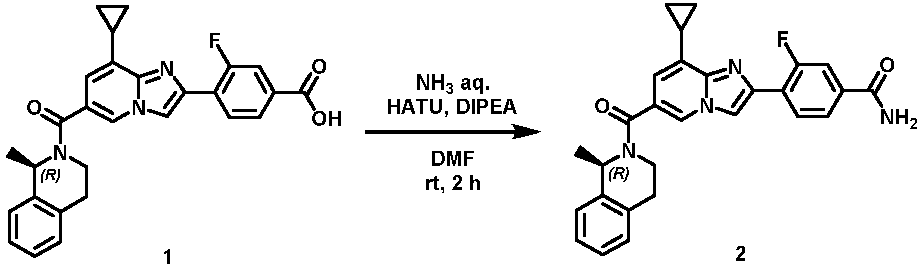

A mixture of **compound 1** (0.32 g, 0.68 mmol), HATU (311 mg, 0.82 mmol) and DIPEA (0.35 mL, 2.05 mmol) in DMF (7 mL) was stirred at rt for 15 min. Ammonia (30% in H₂O, 77 µL, 4.09 mmol) was added. The reaction mixture was stirred at rt for 2 h. The reaction mixture was diluted with H₂O and EtOAc. The layers were separated and the aqueous phase was extracted with EtOAc (twice). The combined organic extracts were washed with H₂O (3 times) and brine, dried over MgSO₄, filtered and evaporated to dryness. The crude mixture was purified by flash chromatography over silica gel (Puriflash Interchim^{®} 25 g, 30 µm, mobile phase gradient: DCM / MeOH from 100:0 to 98:2). The residue (0.11 g) was taken up in DIPE. The solid was filtered off and dried under vacuum to give **compound 2** (0.1 g, 31%) as a beige solid.

### Compound 3

### 4-{8-Cyclopropyl-6-[(1R)-1-methyl-1,2,3,4-tetrahydroisoquinoline-2-carbonyl]imidazo[1,2-a]pyridin-2-yl}-3-fluoroaniline

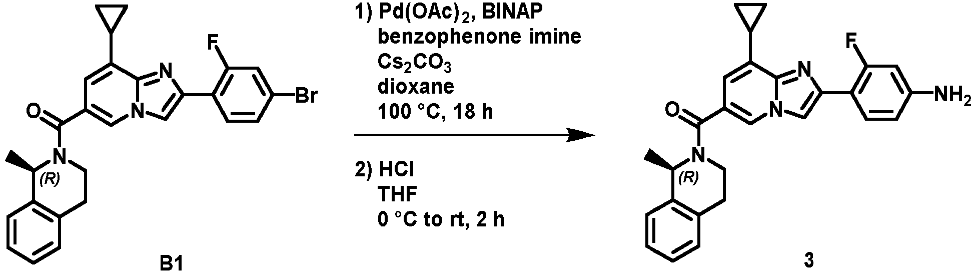

In a sealed tube a mixture of intermediate **B1** (0.50 g, 0.99 mmol), benzophenone imine (0.25 mL, 1.49 mmol) and cesium carbonate (646 mg, 1.98 mmol) in 1,4-dioxane (5 mL) was purged with nitrogen. BINAP (30.9 mg, 49.6 µmοl) and palladium acetate (11.1 mg, 49.6 µmol) were added. The reaction mixture was purged with nitrogen and stirred at 100°C for 18 h.

The reaction mixture was diluted with H₂O and EtOH. The mixture was filtered through a pad of Celite^{®} and washed with EtOAc. The layers were separated. The organic phase was washed with brine, dried over MgSO₄, filtered and evaporated to dryness. The residue was dissolved in THF (10 mL) and HCl (3.0 M in H₂O, 0.89 mL, 2.68 mmol) was added dropwise at 0°C. The mixture was stirred at rt for 2 h. A 10% aqueous solution of K₂CO₃ was added and the mixture was extracted with EtOAc (twice). The combined organic extracts were washed with brine, dried over MgSO₄, filtered and evaporated to dryness. The crude mixture was purified by flash chromatography over silica gel (Puriflash Interchim^{®} 40 g, 30µm, dry loading (Celite^{®}), mobile phase gradient: heptane / EtOAc from 100:0 to 60:40. The residue (0.28 g) was triturated in DCM and the solid was filtered off to give **compound 3** (0.25 g, 57%) as a brown powder.

### Compound 4

### (3S)-N-(4-j8-Cyclopropyl-6-[(1R)-1-methyl-1,2,3,4-tetrahydroisoquinoline-2-carbonyl]imidazo[1,2-a]pyridin-2-yl}-3-fluorophenyl)-3-hydroxypyrrolidine-1-carboxamide

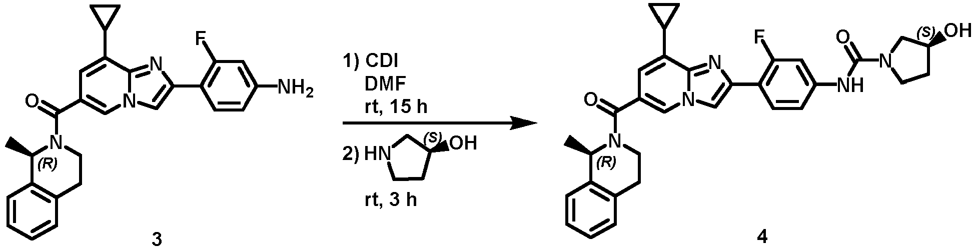

To a mixture of **compound 3** (0.19 g, 0.43 mmol) in DMF (4.0 mL) was added CDI (175 mg, 1.08 mmol). The reaction mixture was stirred at rt for 15 h and a solution of (S)-3-hydroxypyrrolidine (113 mg, 1.29 mmol) in DMF (0.6 mL) was added. The reaction mixture was stirred at rt for 3 h. The reaction mixture was diluted with H₂O and EtOAc. The layers were separated and the aqueous phase was extracted with EtOAc (twice). The combined organic extracts were washed with H₂O and brine, dried over MgSO₄, filtered and evaporated to dryness. The crude mixture was purified by flash chromatography over silica gel (Puriflash Interchim^{®} 25 g, 30 µm, mobile phase gradient: DCM / MeOH from 100:0 to 97:3). The residue (0.13 g) was taken up in Et₂O. The solid was filtered off and dried under vacuum to give **compound 4** (0.11 g 46%) as a yellow solid.

### Synthesis of Compound 5

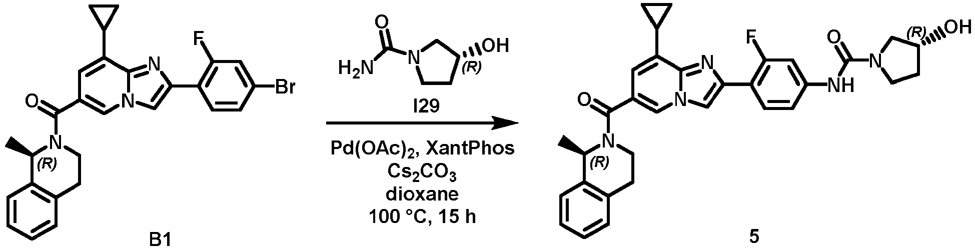

### Intermediate I29

### (3R)-3-hydroxypyrrolidine-1 -carboxamide

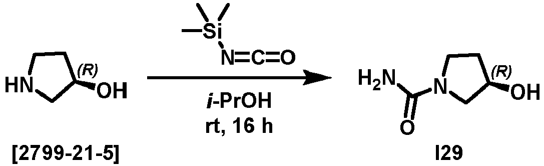

Trimethylsilyl isocyanate (8.0 mL, 64.3 mmol) was added dropwise to a solution of (*R*)-3-hydroxypyrrolidine [2799-21-5] (4.00 g, 45.9 mmol) in *i*-PrOH (110 mL). The reaction mixture was stirred at rt for 16 h. The reaction mixture was concentrate in vacuo (~ half of the solvent) until precipitation was observed. The solid was filtered off, washed with *i*-PrOH and dried to afford intermediate **129** (4.6 g, 77%) as a white solid.

### Compound 5

### (3R)-N-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)-3-hydroxypyrrolidine-1-carboxamide

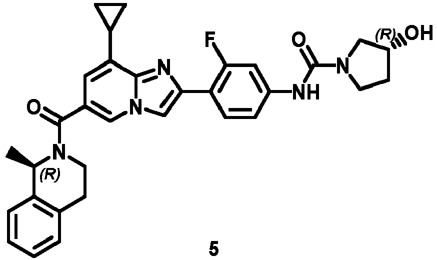

A mixture of intermediate **B1** (0.28 g, 0.56 mmol), intermediate **129** (108 mg, 0.83 mmol), cesium carbonate (0.90 g, 2.78 mmol) and XantPhos (32.1 mg, 55.5 µmol) in 1,4-dioxane (11.6 mL) was purged with nitrogen. Palladium acetate (12.5 mg, 55.5 µmol) was added and the mixture was purged again with nitrogen. The reaction mixture was stirred at 100°C for 15 h. The reaction mixture was diluted with EtOAc and H₂O. The layers were separated, and the aqueous phase was extracted with EtOAc. The combined organic extracts were washed with H₂O, dried over MgSO₄, filtered and evaporated to dryness. The crude mixture was purified by flash chromatography over silica gel (Puriflash Interchim^{®} 25 g, 30 µm, mobile phase gradient: DCM / MeOH from 100:0 to 96:4). The residue (0.21 g) was taken up in Et₂O. The solid was filtered off and dried under vacuum to give **compound 5** (0.19 g, 62%) as a brown solid.

### Compound 6

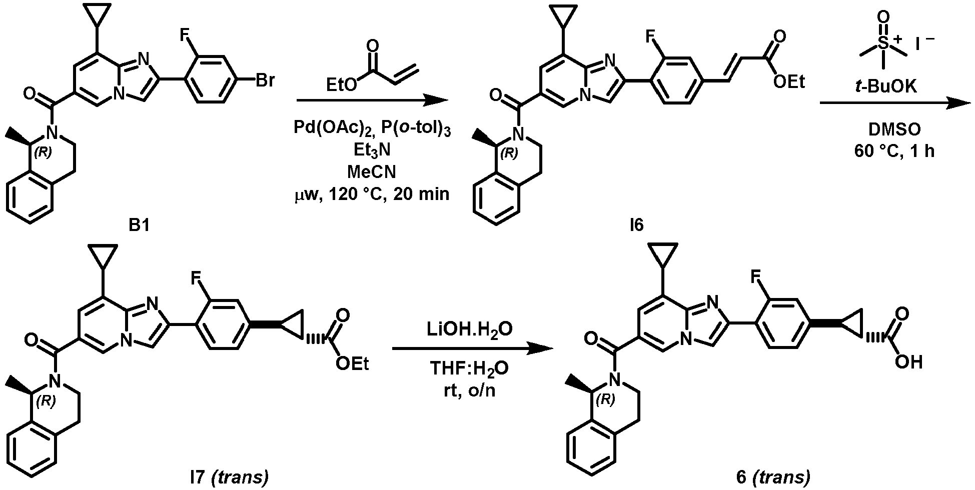

### Intermediate I6

### Ethyl (2E)-3-(4-{8-cyclopropyl-6-[(1R)-1-methyl-1,2,3,4-tetrahydroisoquinoline-2-carbonyl]imidazo[1,2-a]pyridin-2-yl}-3-fluorophenyl)prop-2-enoate

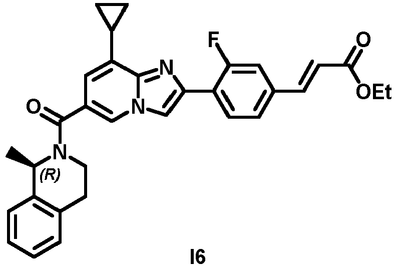

A solution of intermediate **B1** (0.43 g, 852 µmol) in MeCN (7.5 mL) was purged with nitrogen for 5 min. Ethyl acrylate (0.46 mL, 4.26 mmol), Et₃N (0.36 mL, 2.56 mmol), tri(o-tolyl)phosphine (51.9 mg, 0.17 mmol) and palladium acetate (19.1 mg, 85.3 µmol) were added. The reaction mixture was purged again with nitrogen for 2 min and heated at 120°C using a single mode microwave (Anton Paar Monowave^{®} 300) with a power output ranging from 0 to 850 W for 20 min. The mixture was poured out into a solution of water and DCM. The layers were separated (hydrophobic frit) and the organic phase was evaporated to dryness. The crude mixture was purified by flash chromatography over silica gel (Puriflash Interchim^{®} 40 g, 30 µm, mobile phase gradient: heptane / EtOAc from 90:10 to 60:40) to afford intermediate **16** (0.36 g, 81%) as a beige solid.

### Intermediate I7

### Ethyl trans -2-(4-{8-cyclopropyl-6-[(1R)-1-methyl-1,2,3,4-tetrahydroisoquinoline-2-carbonyl]imidazo[1,2-a]pyridin-2-yl}-3-fluorophenyl)cyclopropane-1-carboxylate

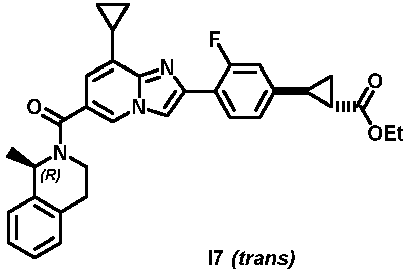

A mixture of trimethylsulfoxonium iodide (166 mg, 756 µmol) and potassium *tert*-butoxide (84.9 mg, 756 µmοl) in DMSO (4.6 mL) was stirred at rt for 1 h. A solution of intermediate **16** (0.36 g, 688 µmοl) in DMSO (3 mL) was added dropwise. The reaction mixture was stirred at 60°C for 1 h. The mixture was poured out into water and the aqueous phase was extracted with EtOAc. The combined organic extracts were washed with H₂O and brine, dried over MgSO₄, filtered and evaporated to dryness. The crude mixture was purified by flash chromatography over silica gel (Puriflash Interchim^{®} 24 g, 30 µm, mobile phase gradient: heptane / EtOAc from 90:10 to 60:40) to afford intermediate **17** (125 mg, 34%).

### Compound 6

### Trans 2-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)cyclopropane-1-carboxylic acid

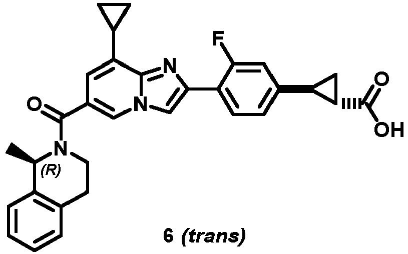

A mixture of intermediate **17** (0.12 g, 223 µmοl) and lithium hydroxide monohydrate (65.6 mg, 1.56 mmol) in THF (3.5 mL) and H₂O (1 mL) was stirred at 70°C overnight. An aqueous solution of citric acid (300 mg in 5 mL of H₂O) was added. The layers were separated, and the aqueous phase was extracted with EtOAc. The combined organic extracts were washed with brine, dried over MgSO₄, filtered and concentrated in vacuo. The residue was taken up in EtOAc and pentane (5:95). The solid was filtered off and dried under vacuum to give **compound 6** (98 mg, 86%) as a yellow solid.

### Compound 7

### Trans 2-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)cyclopropane-1-carboxamide

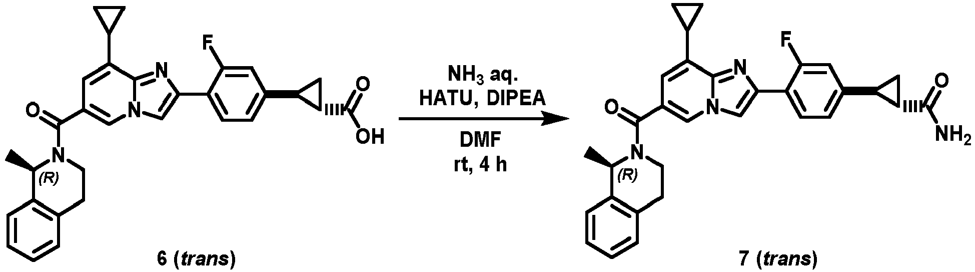

To a mixture of **compound 6** (52.0 mg, 102 µmοl) in DMF (2 mL) were added HATU (58.2 mg, 153 µmοl) and DIPEA (53.0 µL, 306 µmοl). The reaction mixture was stirred at rt for 15 min. Ammonia (30% in H₂O, 38.6 µL, 0.61 mmol) was added and the reaction mixture was stirred at rt for 4 h. The reaction mixture was diluted with H₂O and the aqueous phase was extracted with EtOAc. The combined organic extracts were washed with H₂O and brine, dried over MgSO₄, filtered and evaporated to dryness. The crude mixture was purified by flash chromatography over silica gel (Puriflash Interchim^{®} 4 g, 30 µm, mobile phase gradient: DCM / MeOH from 100:0 to 97:3). The residue (50 mg) was taken up in DIPE. The precipitate was filtered off and dried under vacuum to give **compound 7** (38 mg, 73%) as a yellow solid.

### Compound 8

### Trans 2-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)-N-methanesulfonylcyclopropane-1-carboxamide

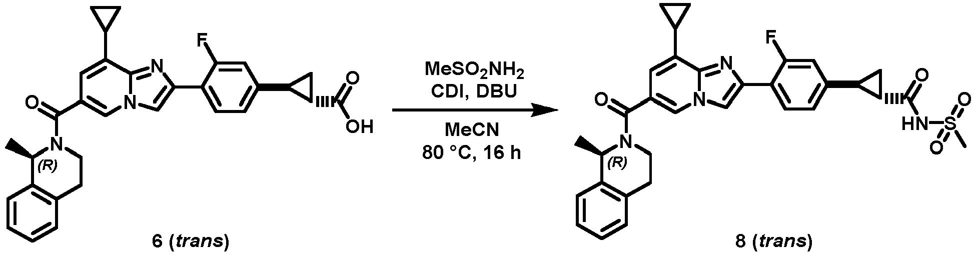

A mixture of **compound 6** (0.10 g, 196 µmοl) and CDI (38.2 mg, 0.24 mmol) in MeCN (2 mL) was stirred at rt for 2 h. Methanesulfonamide (28.0 mg, 0.29 mmol) and DBU (44.0 µL, 0.29 mmol) were added. The reaction mixture was stirred at 80°C for 16 h. A 1N aqueous solution of HCl and DCM were added. The layers were separated and the aqueous phase was extracted with DCM (twice). The combined organic extracts were washed with brine, dried over MgSO₄, filtered and evaporated in vacuo. The residue was taken up in Et₂O. The solid was filtered off and dried under vacuum to give **compound 8** (55 mg, 48%).

### Synthesis of Compound 9

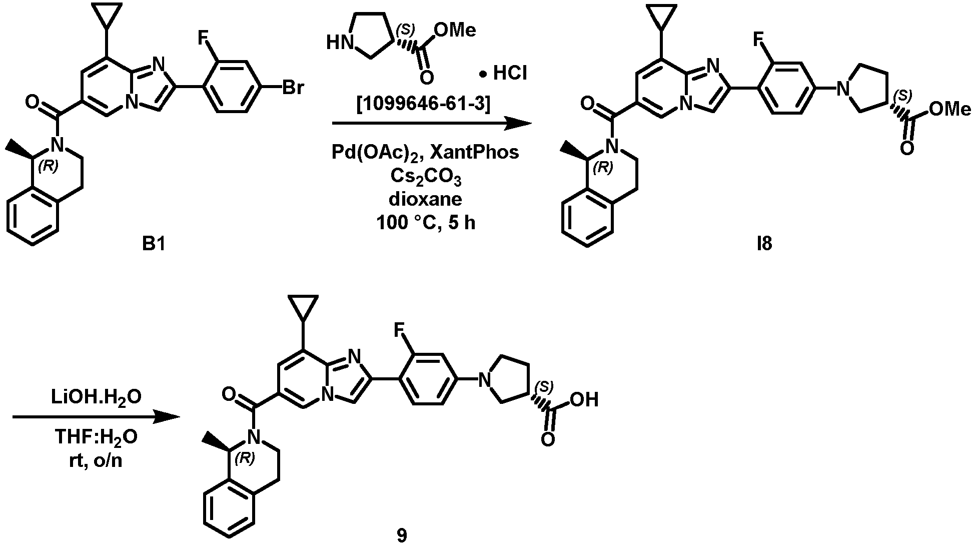

### Intermediate I8

### Methyl (3S)-1-(4-{8-cyclopropyl-6-[(1R)-1-methyl-1,2,3,4-tetrahydroisoquinoline-2-carbonyl]imidazo[1,2-a]pyridin-2-yl}-3-fluorophenyl)pyrrolidme-3-carboxylate

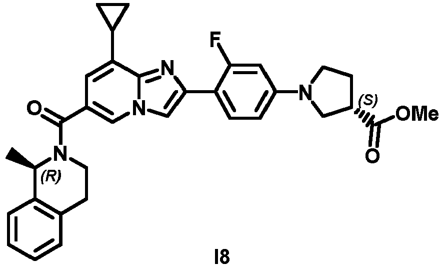

A mixture of intermediate **B1** (0.50 g, 0.99 mmol), (*S*)-methyl pyrrolidine-3-carboxylate hydrochloride [1099646-61-3] (197 mg, 1.19 mmol), cesium carbonate (969 mg, 2.97 mmol) and XantPhos (57.4 mg, 99.9 µmol) was purged with nitrogen. 1,4-Dioxane (12 mL) was added and the mixture was purged again with nitrogen. Palladium acetate (22.3 mg, 99.9 µmol) was added. The reaction mixture was purged with nitrogen and stirred at 100°C for 5 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 80 g, 30 µm, mobile phase gradient: heptane / EtOAc from 90:10 to 60:40) to afford intermediate **18** (188 mg, 34%) as a beige solid.

### Compound 9

### (3S)-1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)pyrrolidine-3-carboxylic acid

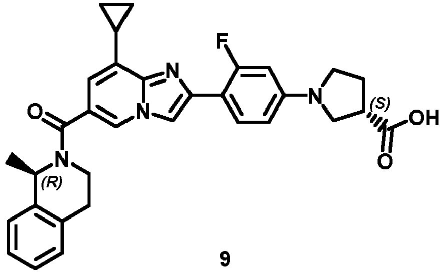

A mixture of intermediate **18** (188 mg, 0.34 mmol) and lithium hydroxide monohydrate (100 mg, 2.38 mmol) in THF (8 mL) and H₂O (2.5 mL) was stirred at rt overnight. An aqueous solution of citric acid (457 mg in 8 mL of H₂O) was added. The layers were separated, and the aqueous phase was extracted with EtOAc. The combined organic extracts were washed with brine, dried over MgSO₄, filtered and evaporated to dryness. The residue was taken up in DIPE. The solid was filtered off and dried at 25°C under vacuum to give **compound 9** (125 mg, 68%) as an orange solid.

### Compound 10

### (3S)-1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)pyrrolidine-3-carboxamide

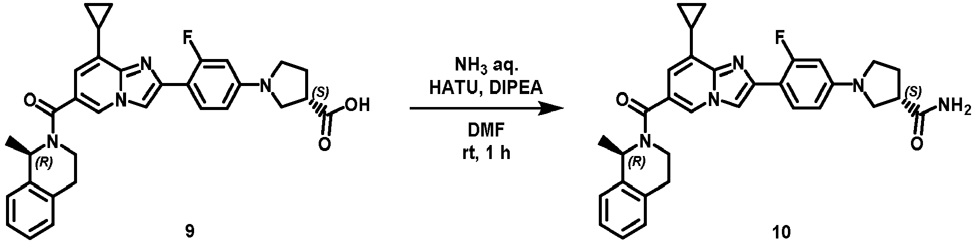

To a mixture of **compound 9** (78.0 mg, 145 µmol) in DMF (2.5 mL) were added DIPEA (75 mL, 0.43 mmol) and HATU (82.6 mg, 217 µmοl). The reaction mixture was stirred at rt for 15 min. Ammonia (30% in H₂O, 16.4 µL, 0.87 mmol) was added dropwise. The reaction mixture was stirred at rt for 1 h. The reaction mixture was diluted with H₂O and EtOAc. The layers were separated and the organic phase was washed with H₂O and brine (3 times), dried over MgSO₄, filtered and evaporated to dryness. The residue was taken up with Et₂O. The solid was filtered off and dried under vacuum to give **compound 10** (60 mg, 77%) as a beige solid.

### Compound 11

### (3S)-1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)-N-methylpyrrolidine-3-carboxamide

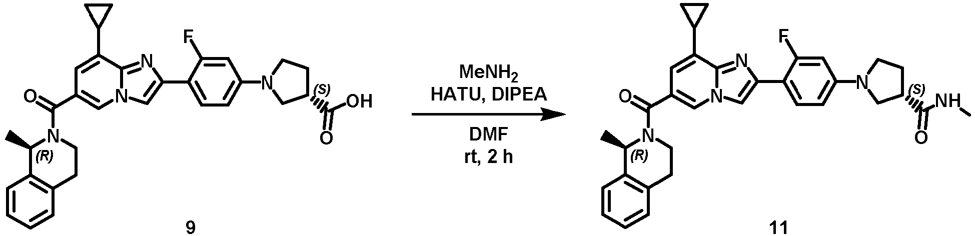

To a mixture of **compound 9** (90.0 mg, 167 µmοl) in DMF (2.5 mL) were added DIPEA (86.4 µL, 0.50 mmol) and HATU (95.3 mg, 0.25 mmol). The reaction mixture was stirred at rt for 15 min. Methylamine (47.5 µL, 1.00 mmol) was added dropwise. The reaction mixture was stirred at rt for 2 h. The reaction mixture was diluted with H₂O and EtOAc. The layers were separated and the organic phase was washed with H₂O and brine (3 times), dried over MgSO₄, filtered and evaporated to dryness. The residue was taken up in Et₂O. The solid was filtered off and dried under vacuum to give **compound 11** (60 mg, 65%) as a brown solid.

### Compound 12

### (3S)-1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)pyrrolidin-3-ol

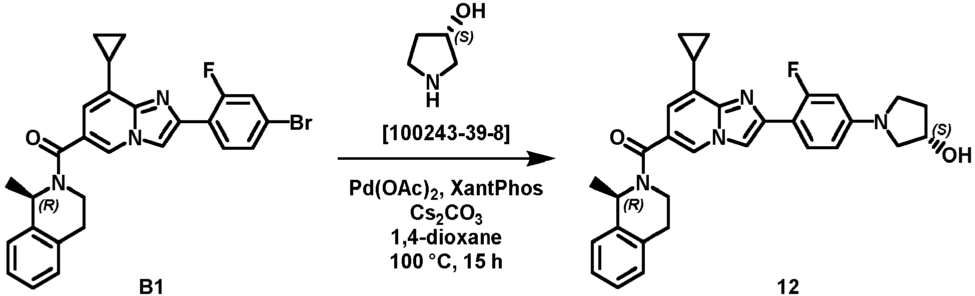

A mixture of intermediate **B1** (0.10 g, 198 µmοl), (*S*)-3-hydroxypyrrolidine [100243-39-8] (43.2 mg, 496 µmοl), cesium carbonate (258 mg, 0.79 mmol) and XantPhos (13.8 mg, 23.8 µmol) was purged with nitrogen. 1,4-Dioxane (2 mL) was added and the mixture was purged again with nitrogen. Palladium acetate (5.34 mg, 23.8 µmol) was added. The reaction mixture was purged with nitrogen and stirred at 100°C for 15 h. The reaction mixture was diluted with H₂O and EtOAc. The layers were separated and the aqueous phase was extracted with EtOAc (twice). The combined organic extracts were washed with H₂O and brine, dried over MgSO₄, filtered and evaporated to dryness. The crude mixture was purified by flash chromatography over silica gel (Puriflash Interchim^{®} 12 g, 30 µm, mobile phase gradient: DCM / MeOH from 100:0 to 97:3) to afford **compound 12** (70 mg, 69%) as a brown solid.

### Synthesis of compound 13

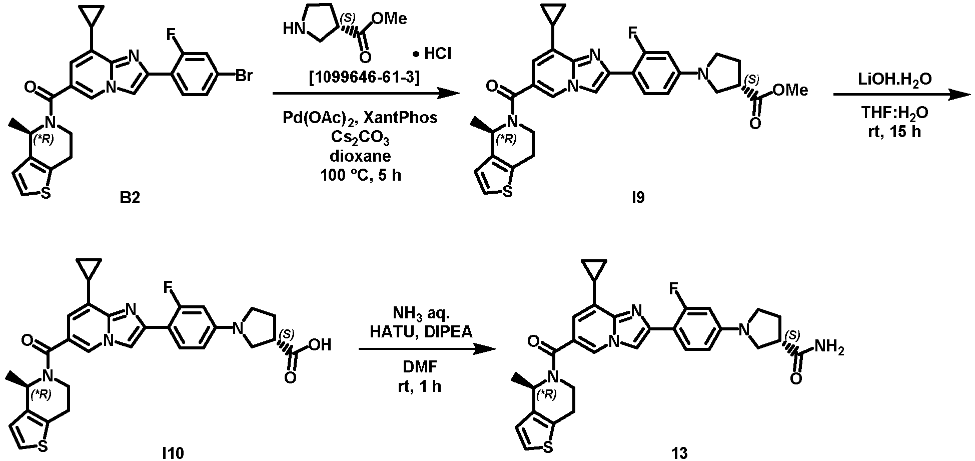

### Intermediate I9

### Methyl (3S)-1-(4-{8-cyclopropyl-6-[(4^{∗}R)-4-methyl-4,5,6,7-tetrahydro-thieno[3,2-c]pyridine-5-carbonyl]imidazo[1,2-a]pyridin-2-yl}-3-fluorophenyl)pyrrolidine-3-carboxylate

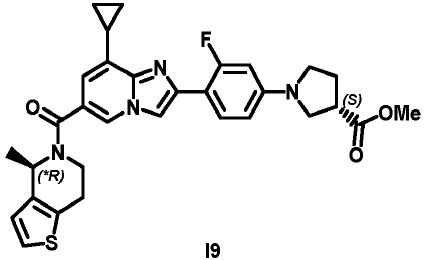

A mixture of intermediate **B2** (0.88 g, 1.72 mmol), (*S*)-methyl pyrrolidine-3-carboxylate hydrochloride [1099646-61-3] (371 mg, 2.24 mmol), cesium carbonate (1.69 g, 5.17 mmol) and XantPhos (100mg, 0.17 mmol) was purged with nitrogen. 1,4-Dioxane (15 mL) was added and the mixture was purged again with nitrogen. Palladium acetate (38.7 mg, 0.17 mmol) was added. The reaction mixture was purged with nitrogen and stirred at 100°C for 5 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 40 g, 30 µm, mobile phase gradient: heptane / EtOAc from 90:10 to 60:40) to afford intermediate **19** (384 mg, 40%) as a yellow solid.

### Intermediate I10

### (3S)-1-(4-{8-Cyclopropyl-6-[(4^{∗}R)-4-methyl-4,5,6,7-tetrahydro-thieno[3,2-c]pyridine-5-carbonyl]imidazo[1,2-a]pyridin-2-yl}-3-fluorophenyl)pyrrolidine-3-carboxylic acid

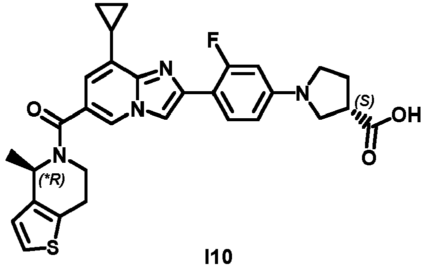

A mixture of intermediate **19** (0.38 g, 0.68 mmol) and lithium hydroxide monohydrate (171 mg, 4.08 mmol) in THF (12 mL) and H₂O (3 mL) was stirred at rt overnight. An aqueous solution of citric acid (784 mg in 20 mL of H₂O) was added. The layers were separated and the aqueous phase was extracted with EtOAc. The combined organic extracts were washed with brine, dried over MgSO₄, filtered and evaporated to dryness to afford intermediate **110** (0.3 g, 81%) as a yellow solid.

### Compound 13

### (3S)-1-(4-{8-Cyclopropyl-6-[(4^{∗}R)-4-methyl-4,5,6,7-tetrahydro-thieno[3,2-c]pyridine-5-carbonyl]imidazo[1,2-a]pyridin-2-yl}-3-fluorophenyl)pyrrolidine-3-carboxamide

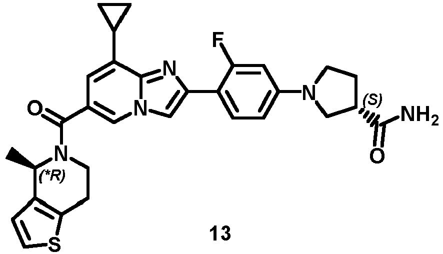

To a mixture of intermediate **110** (287 mg, 527 µmοl) in DMF (4 mL) were added DIPEA (0.27 mL, 1.58 mmol) and HATU (301 mg, 0.79 mmol). The reaction mixture was stirred at rt for 15 min. Ammonia (30% in H₂O, 60 µL, 3.16 mmol) was added and the reaction mixture was stirred at rt for 1 h. The reaction mixture was diluted with H₂O and EtOAc. The layers were separated and the organic phase was washed with H₂O and brine (3 times), dried over MgSO₄, filtered and evaporated to dryness. The crude mixture was purified by flash chromatography over silica gel

(Puriflash Interchim^{®} 4 g, 30 µm, mobile phase gradient: DCM / MeOH from 100:0 to 98:2). The residue (120 mg) was taken up in Et₂O. The solid was filtered off and dried under vacuum to give compound **13** (0.1 g, 35%) as a beige solid.

### Compound 14

### (3S)-1-(4-{8-Cyclopropyl-6-[(4^{∗}R)-4-methyl-4,5,6,7-tetrahydro-thieno[3,2-c]pyridine-5-carbonyl]imidazo[1,2-a]pyridin-2-yl}-3-fluorophenyl)-N-methylpyrrolidine-3-carboxamide

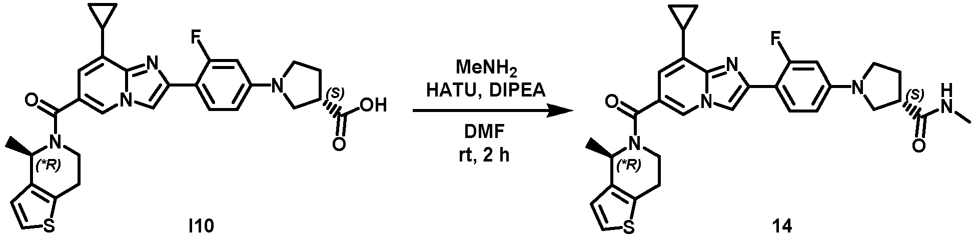

To a solution of intermediate **110** (0.15 g, 275 µmοl) in DMF (4 mL) were added DIPEA (0.14 mL, 0.83 mmol) and HATU (157 mg, 0.41 mmol). The reaction mixture was stirred at rt for 15 min. Methylamine(40% in water, 57 µL, 1.65 mmol) was added dropwise. The reaction mixture was stirred at rt for 2 h. The reaction mixture was diluted with H₂O and EtOAc. The layers were separated and the organic phase was washed with H₂O and brine (3 times), dried over MgSO₄, filtered and evaporated to dryness to give compound 14 (0.1 g, 65%) as a brown solid.

### Synthesis of compound 15

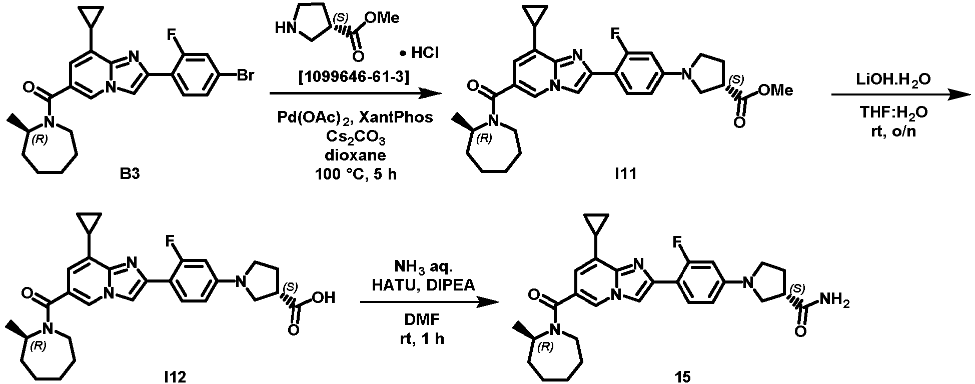

### Intermediate I11

### Methyl (3S)-1-(4-{8-cyclopropyl-6-[-carbonyl]imidazo[1,2-a]pyridin-2-yl}-3-fluorophenyl)pyrrolidine-3-carboxylate

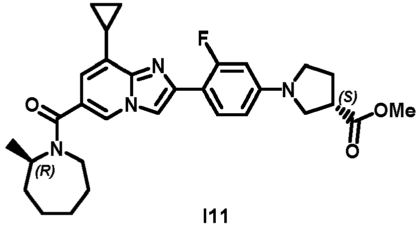

A mixture of intermediate **B3** (0.60 g, 1.28 mmol), (*S*)-methyl pyrrolidine-3-carboxylate hydrochloride [1099646-61-3] (275 mg, 1.66 mmol), cesium carbonate (1.25 g, 3.83 mmol) and XantPhos (73.8 mg, 128 µmοl) was purged with nitrogen. 1,4-Dioxane (13 mL) was added and the mixture was purged again with nitrogen. Palladium acetate (28.6 mg, 0.13 mmol) was added. The reaction mixture was purged with nitrogen and stirred at 100°C for 5 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 40 g, 30 µm, mobile phase gradient: heptane / EtOAc from 90:10 to 60:40) to afford intermediate **I11** (231 mg, 35%) as a brown solid.

### Intermediate I12

### (36)-1-(4- {8-Cyclopropyl-6-[(2R)-2-methylazepane-1-carbonyl]imidazo[1,2-a]pyridin-2-yl} -3-fluorophenyl)pyrrolidine-3-carboxylic acid

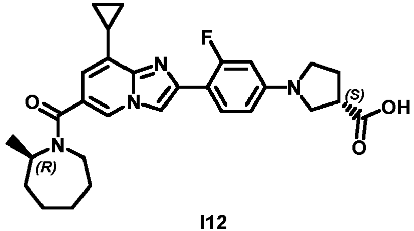

A mixture of intermediate **I11** (231 mg, 445 µmοl) and lithium hydroxide monohydrate (112 mg, 2.67 mmol) in THF (8 mL) and H₂O (2 mL) was stirred at rt overnight. An aqueous solution of citric acid (513 mg in 15 mL of H₂O) was added. The layers were separated and the aqueous phase was extracted with EtOAc. The combined organic extracts were washed with brine, dried over MgSO₄, filtered and evaporated to dryness to afford intermediate **112** (212 mg, 94%) as a yellow solid.

### Compound 15

### (3S)-1-(4-{8-Cyclopropyl-6-[(2R)-2-methylazepane-1-carbonyl]imidazo[1,2-a]pyridin-2-yl}-3-fluorophenyl)pyrrolidine-3-carboxamide

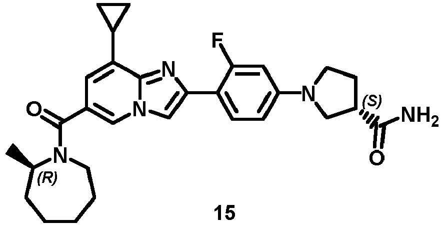

To a solution of intermediate **112** (0.20 g, 396 µmοl) in DMF (4 mL) were added DIPEA (0.21 mL, 1.19 mmol) and HATU (226 mg, 0.60 mmol). The reaction mixture was stirred at rt for 15 min. Ammonia (30% in H₂O, 45 µL, 2.38 mmol) was added and the reaction mixture was stirred at rt for 1 h. The reaction mixture was diluted with H₂O and EtOAc. The layers were separated and the organic phase was washed with H₂O and brine (3 times), dried over MgSO₄, filtered and evaporated to dryness. The crude mixture was purified by flash chromatography over silica gel (Puriflash Interchim^{®} 12 g, 30 µm, mobile phase gradient: DCM / MeOH from 100:0 to 98:2) to give compound 15 (80 mg, 40%) as a beige solid.

### Synthesis of compound 16

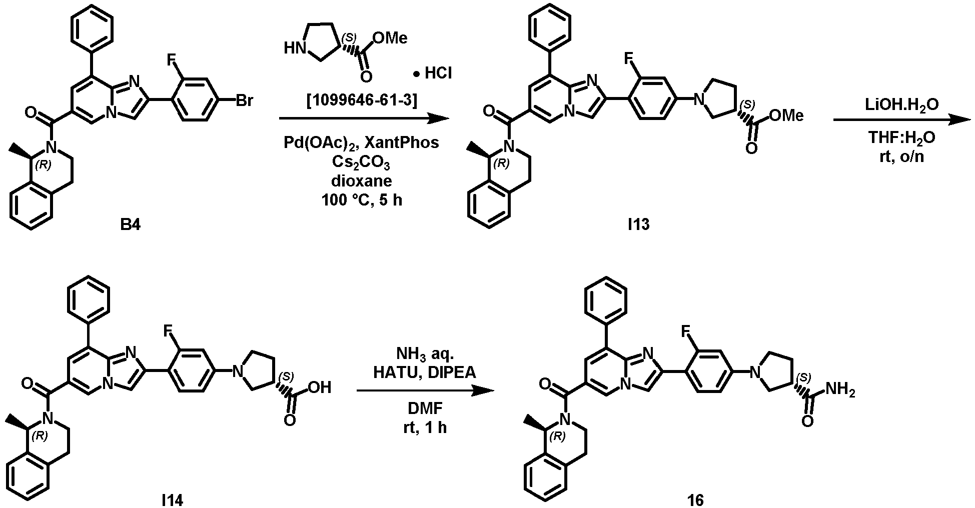

### Intermediate I13

### Methyl (3S)-1-(3-fluoro-4-{6-[(1R)-1-methyl-1,2,3,4-tetrahydroisoquinoline-2-carbonyl]-8-phenylimidazo[1,2-a]pyridin-2-yl}phenyl)pyrrolidine-3-carboxylate

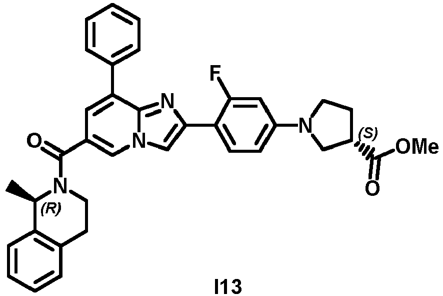

A mixture of intermediate **B4** (0.61 g, 1.13 mmol), (*S*)-methyl pyrrolidine-3-carboxylate hydrochloride [1099646-61-3] (243 mg, 1.47 mmol), cesium carbonate (1.10 g, 3.39 mmol) and XantPhos (65.3 mg, 0.11 mmol) was purged with nitrogen. 1,4-Dioxane (14 mL) was added and the mixture was purged again with nitrogen. Palladium acetate (25.3 mg, 0.11 mmol) was added. The reaction mixture was purged with nitrogen and stirred at 100°C for 5 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 40 g, 30 µm, mobile phase gradient: heptane / EtOAc from 90:10 to 60:40) to afford intermediate 113 (0.16 g, 24%) as a brown solid.

### Intermediate I14

### (3S)-1-(3-Fluoro-4-{6-[(1R)-1-methyl-1 ,2,3,4-tetrahydroisoquinoline-2-carbonyl]-8-phenylimidazo[1,2-a]pyridin-2-yl}phenyl)pyrrolidine-3-carboxylic acid

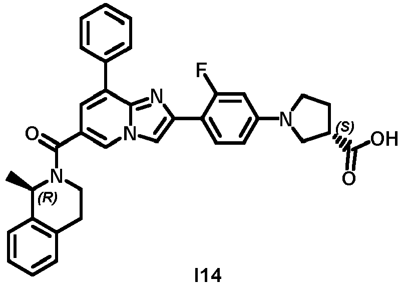

A mixture of intermediate **113** (151 mg, 257 µmοl) and lithium hydroxide monohydrate (64.6 mg, 1.54 mmol) in THF (4 mL) and H₂O (1 mL) was stirred at rt overnight. An aqueous solution of citric acid (296 mg in 10 mL of H₂O) was added. The layers were separated and the aqueous phase was extracted with EtOAc. The combined organic extracts were washed with brine, dried over MgSO₄, filtered and evaporated to dryness to afford intermediate **114** (147 mg, 99%) as a yellow solid.

### Compound 16

### (3S)-1-(3-Fluoro-4-(6-[(1R)-1-methyl-1,2,3,4-tetrahydroisoquinoline-2-carbonyl]-8-phenylimidazo[1,2-a]pyridin-2-yl}phenyl)pyrrolidine-3-carboxamide

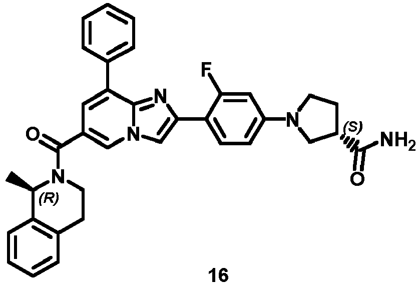

To a solution of intermediate **114** (136 mg, 237 µmοl) in DMF (3.5 mL) were added DIPEA (0.12 mL, 0.71 mmol) and HATU (135 mg, 0.36 mmol). The reaction mixture was stirred at rt for 15 min. Ammonia (30% in H₂O, 26.9 µL, 1.42 mmol) was added dropwise. The reaction mixture was stirred at rt for 1 h. The reaction mixture was diluted with H₂O and EtOAc. The layers were separated and the organic phase was washed with H₂O and brine (3 times), dried over MgSO₄, filtered and evaporated to dryness. The crude mixture was purified by flash chromatography over silica gel (Puriflash Interchim^{®} 4 g, 30 µm, mobile phase gradient: DCM / MeOH from 100:0 to 98:2) to give compound **16** (75 mg, 59%) as a beige solid.

### Synthesis of compound 17

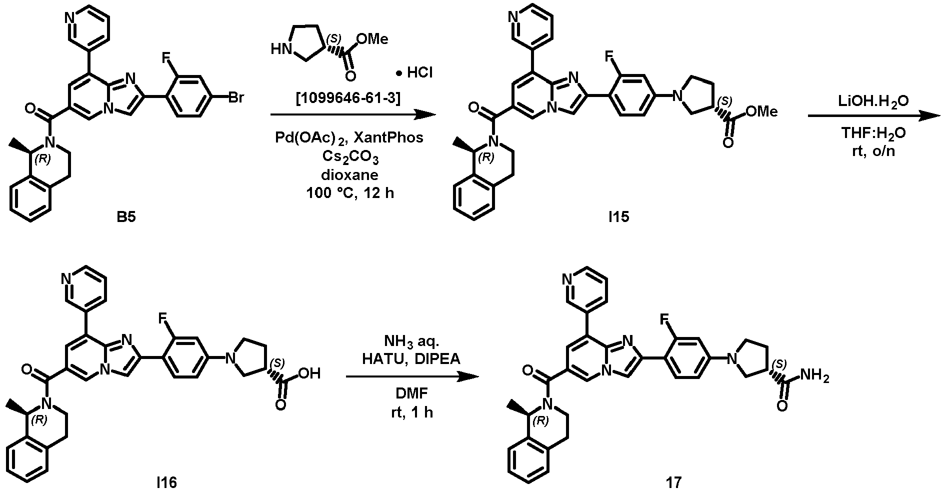

### Intermediate I15

### Methyl (3S)-1-(3-fluoro-4-{6-[(1R)-1-methyl-1,2,3,4-tetrahydroisoquinoline-2-carbonyl]-8-(pyridin-3-yl)imidazo[1,2-a]pyridin-2-yl}phenyl)pyrrolidine-3-carboxylate

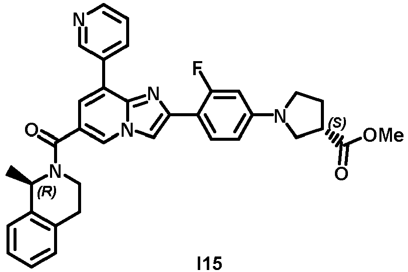

A mixture of intermediate **B5** (0.26 g, 0.48 mmol), (*S*)-methyl pyrrolidine-3-carboxylate hydrochloride (111 mg, 0.67 mmol), cesium carbonate (0.63 g, 1.92 mmol) and XantPhos (33.3 mg, 57.6 µmοl) was purged with nitrogen. 1,4-Dioxane (5 mL) was added and the mixture was purged again with nitrogen. Palladium acetate (12.9 mg, 57.6 µmοl) was added. The reaction mixture was purged with nitrogen and stirred at 100°C for 12 h. The mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 25 g, 30 µm, mobile phase gradient: DCM / MeOH from 100:0 to 98:2) to afford intermediate **115** (0.19 g, 67%) as a yellow solid.

### Intermediate I16

### (3S)-1-(3-Fluoro-4-{6-[(1R)-1-methyl-1,2,3,4-tetrahydroisoquinoline-2-carbonyl]-8-(pyridin-3-yl)imidazo[1,2-a]pyridin-2-yl}phenyl)pyrrolidine-3-carboxylic acid

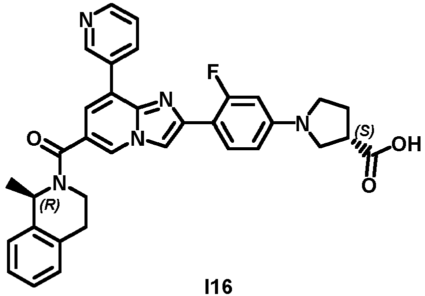

A mixture of intermediate **115** (0.18 g, 305 µmοl) and lithium hydroxide monohydrate (76.9 mg, 1.83 mmol) in THF (8 mL) and H₂O (2 mL) was stirred at rt overnight. An aqueous solution of citric acid (352 mg) was added. The layers were separated and the aqueous phase was extracted with EtOAc. The combined organic extracts were washed with brine, dried over MgSO₄, filtered and evaporated to dryness to afford intermediate **116** (0.15 g, 85%) as an orange solid.

### Compound 17

### (3S)-1-(3-Fluoro-4-(6-[(1R)-1-methyl-1,2,3,4-tetrahydroisoquinoline-2-carbonyl]-8-(pyridin-3-yl)imidazo[1,2-a]pyridin-2-yl}phenyl)pyrrolidine-3-carboxamide

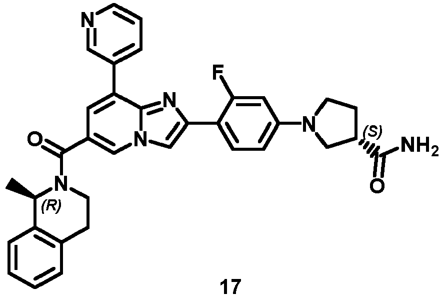

To a mixture of intermediate **I16** (0.14 g, 243 µmοl) in DMF (4 mL) were added DIPEA (0.13 mL, 0.73 mmol) and HATU (111 mg, 0.29 mmol). The reaction mixture was stirred at rt for 15 min. Ammonia (30% in H₂O, 28 µL 1.46 mmol) was added dropwise. The reaction mixture was stirred at rt for 1 h. The reaction mixture was diluted with H₂O and EtOAc. The layers were separated and the organic phase was washed with H₂O (3 times) and brine, dried over MgSO₄, filtered and evaporated to dryness. The crude mixture was purified by flash chromatography over silica gel (Puriflash Interchim^{®} 12 g, 30 µm, mobile phase gradient: DCM / MeOH from 100:0 to 98:2). The residue (0.11 g) was diluted with EtOAc and the mixture was stirred at rt for 1 h. The solid was filtered off and dried under vacuum to give compound **17** (0.06 g, 43%) as a brown solid.

### Synthesis of compound 18

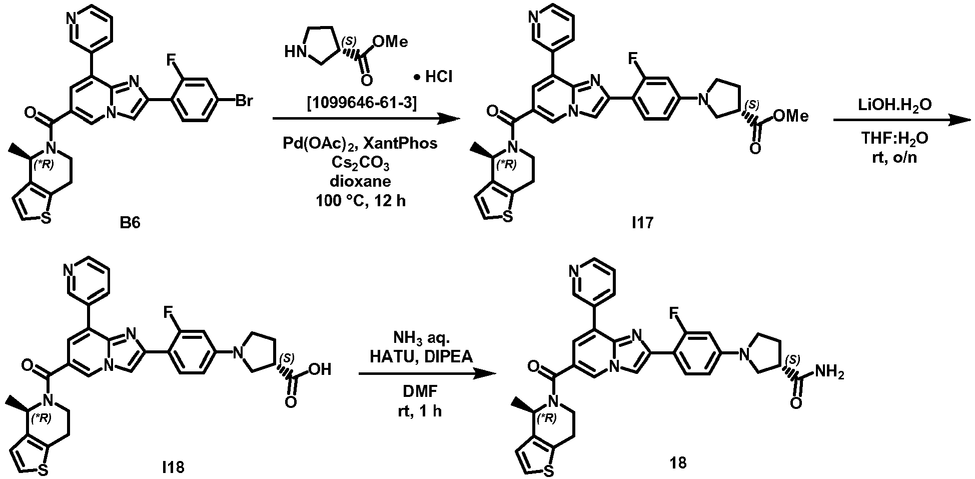

### Intermediate I17

### Methyl (3S)-1-(3-fluoro-4-{6-[(4^{∗}R)-4-methyl-4,5,6,7-tetrahydro-thieno[3,2-c]pyridine-5-carbonyl]-8-(pyridin-3-yl)imidazo[1,2-a]pyridin-2-yl}phenyl)pyrrolidine-3-carboxylate

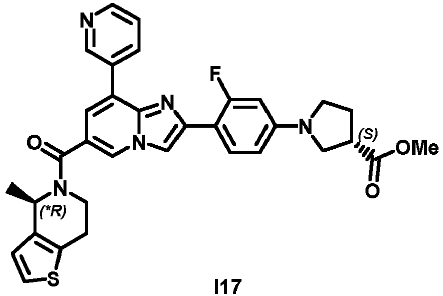

A mixture of intermediate **B6** (0.12 g, 219 µmοl), (*S*)-methyl pyrrolidine-3-carboxylate hydrochloride [1099646-61-3] (50.8 mg, 307 µmοl), cesium carbonate (286 mg, 0.88 mmol) and XantPhos (15.2 mg, 26.3 µmοl) was purged with nitrogen. 1,4-Dioxane (3 mL) was added and the mixture was purged again with nitrogen. Palladium acetate (5.91 mg, 26.3 µmοl) was added. The reaction mixture was purged with nitrogen and stirred at 100°C for 12 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 25 g, 30 µm, mobile phase gradient: DCM / MeOH from 100:0 to 98:2) to afford intermediate **117** (0.10 g, 77%) as a yellow solid.

### Intermediate I18

### (3S)-1-(3-Fluoro-4-{6-[(4^{∗}R)-4-methyl-4,5,6,7-tetrahydro-thieno[3,2-c]pyridine-5-carbonyl]-8-(pyridin-3-yl)imidazo[1,2-a]pyridin-2-yl}phenyl)pyrrolidine-3-carboxylic acid

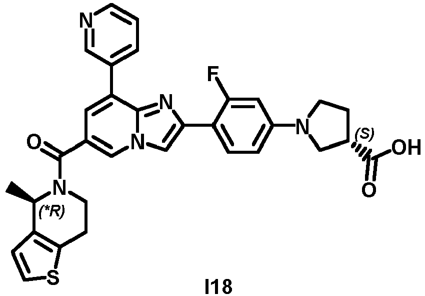

A mixture of intermediate **117** (0.1 g, 168 µmοl) and lithium hydroxide monohydrate (42.3 mg, 1.00 mmol) in THF (6 mL) and H₂O (1.5 mL) was stirred at rt overnight. An aqueous solution of citric acid (193 mg) was added. The layers were separated and the aqueous phase was extracted with EtOAc. The combined organic extracts were washed with brine, dried over MgSO₄, filtered and evaporated to dryness to afford intermediate **118** (97 mg, quant.,) as a yellow solid.

### Compound 18

### (3S)-1-(3-Fluoro-4-{6-[(4^{∗}R)-4-methyl-4,5,6,7-tetrahydro-thieno[3,2-c]pyridine-5-carbonyl]-8-(pyridin-3-yl)imidazo[1,2-a]pyridin-2-yl}phenyl)pyrrolidine-3-carboxamide

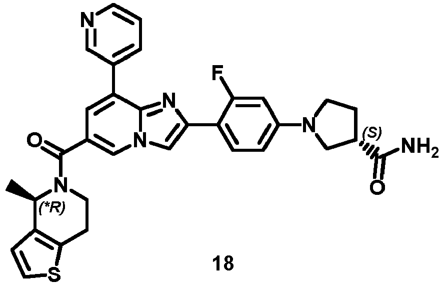

A mixture of intermediate **118** (97.0 mg, 167 µmοl) in DMF (2 mL) were added DIPEA (86.2 µL, 0.50 mmol) and HATU (76.1 mg, 0.20 mmol). The reaction mixture was stirred at rt for 15 min. Ammonia (30% in H₂O, 19 µL, 1.00 mmol) was added dropwise. The reaction mixture was stirred at rt for 1 h. The reaction mixture was diluted with H₂O and EtOAc. The layers were separated and the organic phase was washed with H₂O (3 times) and brine, dried over MgSO₄, filtered and evaporated to dryness. The crude residue was diluted with EtOAc. The solid was filtered off and dried in vacuo. The residue (0.05 g) was taken up in DIPE. The solid was filtered off and dried under vacuum to give compound **18** (25 mg, 26%, 20% over 3 steps) as a brown solid.

### Synthesis of compound 19

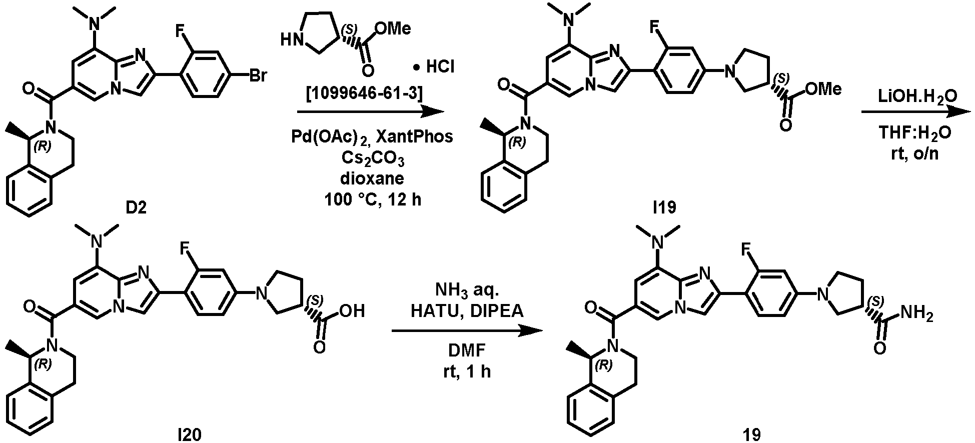

### Intermediate I19

### Methyl (3S)-1-{4-[8-(dimethylamino)-6-[(1R)-1-methyl-1,2,3,4-tetrahydroisoquinoline-2-carbonyl]imidazo[1,2-a]pyridin-2-yl]-3-fluorophenyl}pyrrolidine-3-carboxylate

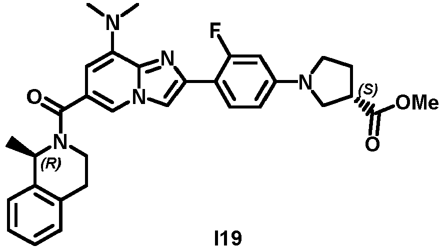

A mixture of intermediate **D2** (0.11 g, 217 µmοl), (*S*)-methyl pyrrolidine-3-carboxylate hydrochloride [1099646-61-3] (50.3 mg, 304 µmοl), cesium carbonate (283 mg, 0.87 mmol) and XantPhos (15.0 mg, 26.0 µmοl) was purged with nitrogen. 1,4-Dioxane (4 mL) was added and the mixture was purged again with nitrogen. Palladium acetate (5.84 mg, 26.0 µmοl) was added. The reaction mixture was purged with nitrogen and stirred at 100°C for 12 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 25 g, 30 µm, mobile phase gradient: heptane / EtOAc from 100:0 to 70:30) to afford intermediate **119** (80 mg, 66%) as a brown solid.

### Intermediate I20

### (3S)-1-{4-[8-(Dimethylamino)-6-[(1R)-1-methyl-1,2,3,4-tetrahydroisoquinoline-2-carbonyl]imidazo[1,2-a]pyridin-2-yl]-3-fluorophenyl}pyrrolidine-3-carboxylic acid

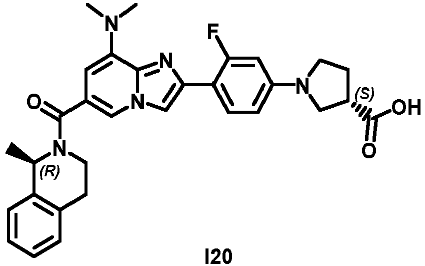

A mixture of intermediate **119** (80.0 mg, 144 µmοl) and lithium hydroxide monohydrate (36.3 mg, 0.86 mmol) in THF (5 mL) and H₂O (1 mL) was stirred at rt overnight. An aqueous solution of citric acid (166 mg) was added. The layers were separated and the aqueous phase was extracted with EtOAc. The combined organic extracts were washed with brine, dried over MgSO₄, filtered and evaporated to dryness to afford intermediate **120** (75 mg, 96%) as an orange solid.

### Compound 19

### (3S)-1-(4-[8-(Dimethylamino)-6-[(1R)-1-methyl-1,2,3,4-tetrahydroisoquinoline-2-carbonyl]imidazo[1,2-a]pyridin-2-yl]-3-fluorophenyl}pyrrolidine-3-carboxylamide

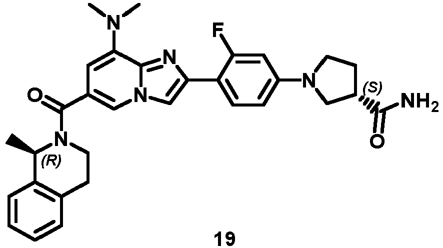

To a solution of intermediate **120** (75.0 mg, 138 µmοl) in DMF (2 mL) were added DIPEA (72 µL, 415 µmοl) and HATU (57.9 mg, 152 µmοl). The reaction mixture was stirred at rt for 15 min. Ammonia (30% in H₂O, 15.7 µL, 0.83 mmol) was added dropwise. The reaction mixture was stirred at rt for 1 h. The reaction mixture was diluted with H₂O and EtOAc. The layers were separated and the organic phase was washed with H₂O (3 times) and brine, dried over MgSO₄, filtered and evaporated to dryness. The crude mixture was purified by flash chromatography over silica gel (Puriflash Interchim^{®} 12 g, 30 µm, mobile phase gradient: DCM / MeOH from 100:0 to 98:2). The residue (45 mg) was taken up in Et₂O. The solid was filtered off and dried under vacuum to give compound **19** (34 mg, 45%) as a grey solid.

### Synthesis of Compound 20

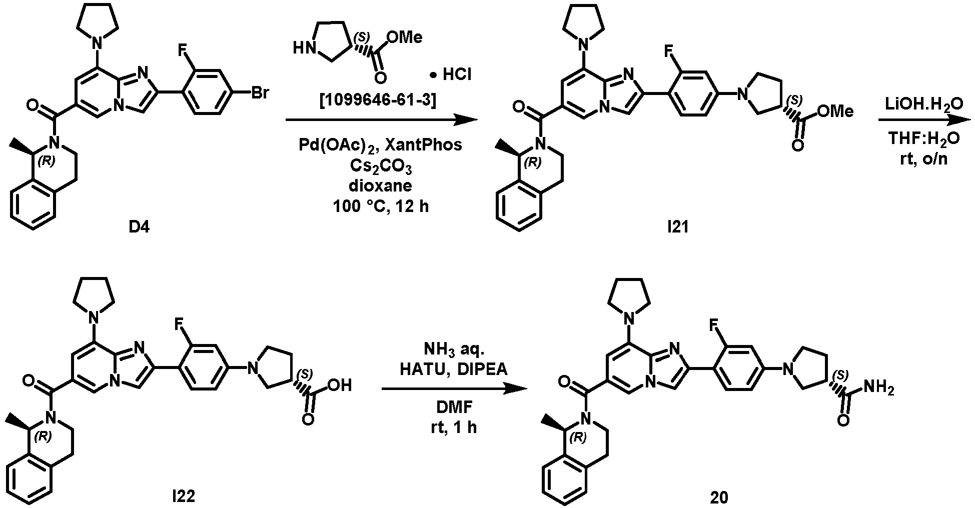

### Intermediate I21

### Methyl (3S)-1-(3-fluoro-4-{6-[(1R)-1-methyl-1,2,3,4-tetrahydroisoquinoline-2-carbonyl]-8-(pyrrolidin-1-yl)imidazo[1,2-a]pyridin-2-yl}phenyl)pyrrolidine-3-carboxylate

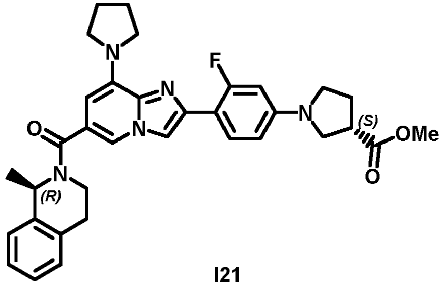

A mixture of intermediate **D4** (0.29 g, 544 µmοl), (*S*)-methyl pyrrolidine-3-carboxylate hydrochloride [1099646-61-3] (126 mg, 0.76 mmol), cesium carbonate (709 mg, 2.18 mmol) and XantPhos (37.7 mg, 65.2 µmοl) was purged with nitrogen. 1,4-Dioxane (4 mL) was added and the mixture was purged again with nitrogen. Palladium acetate (14.6 mg, 65.2 µmοl) was added. The reaction mixture was purged with nitrogen and stirred at 100°C for 12 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 25 g, 30 µm, mobile phase gradient: heptane / EtOAc from 100:0 to 70:30) to afford intermediate **121** (0.21 g, 66%) as a beige solid.

### Intermediate I22

### (3S)-1-(3-Fluoro-4-{6-[(1R)-1-methyl-1,2,3,4-tetrahydroisoquinoline-2-carbonyl]-8-(pyrrolidin-1-yl)imidazo[1,2-a]pyridin-2-yl}phenyl)pyrrolidine-3-carboxylic acid

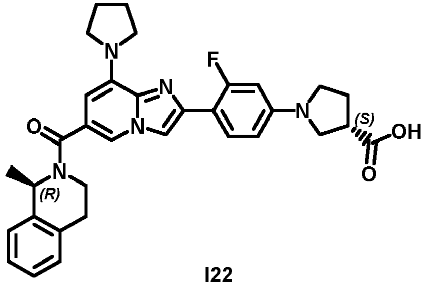

A mixture of intermediate **121** (0.19 g, 327 µmοl) and lithium hydroxide monohydrate (82.2 mg, 1.96 mmol) in THF (8 mL) and H₂O (2 mL) was stirred at rt overnight. An aqueous solution of citric acid (377 mg) was added. The layers were separated and the aqueous phase was extracted with EtOAc. The combined organic extracts were washed with brine, dried over MgSO₄, filtered and evaporated to dryness to afford intermediate **122** (188 mg, quant.) as an orange solid.

### Compound 20

### (3S)-1-(3-Fluoro-4-{6-[(1R)-1-methyl-1,2,3,4-tetrahydroisoquinoline-2-carbonyl]-8-(pyrrolidin-1-yl)imidazo[1,2-a]pyridin-2-yl}phenyl)pyrrolidine-3-carboxamide

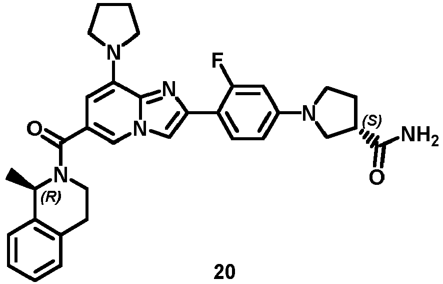

To a solution of intermediate **122** (0.15 g, 264 µmοl) in DMF (4 mL) were added DIPEA (0.14 mL, 0.79 mmol) and HATU (121 mg, 0.32 mmol). The reaction mixture was stirred at rt for 15 min. Ammonia (30% in H₂O, 30 µL, 1.59 mmol) was added dropwise. The reaction mixture was stirred at rt for 1 h. The reaction mixture was diluted with H₂O and EtOAc. The layers were separated and the organic phase was washed with H₂O (3 times) and brine, dried over MgSO₄, filtered and evaporated to dryness. The crude mixture was purified by flash chromatography over silica gel (Puriflash Interchim^{®} 12 g, 30 µm, mobile phase gradient: DCM / MeOH from 100:0 to 98:2). The residue (0.12 g) was taken up in Et₂O. The solid was filtered off and dried under vacuum to give compound 20 (94 mg, 63%) as a grey solid.

### Synthesis of compound 21

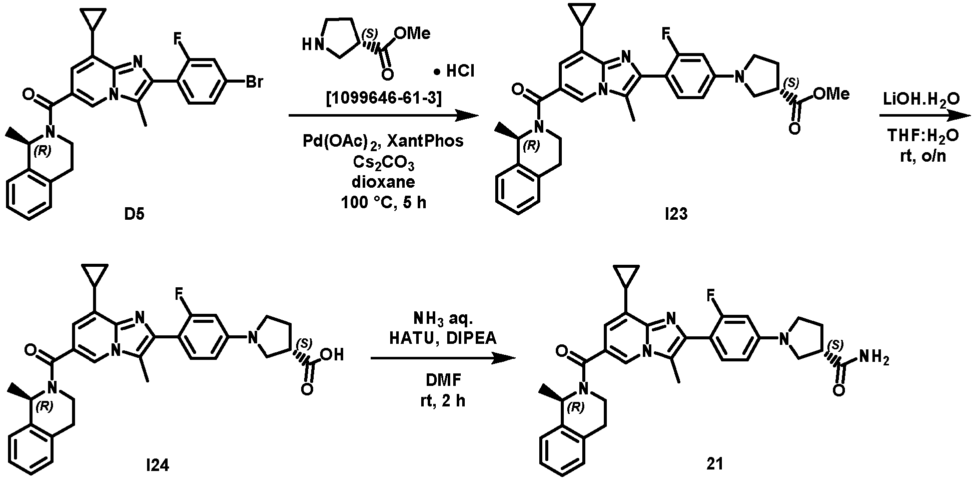

### Intermediate I23

### Methyl (3S)-1-(4-{8-cyclopropyl-3-methyl-6-[-methyl-1 ,2,3,4-tetrahydroisoquinoline-2-carbonyl]imidazo[1,2-a]pyridin-2-yl}-3-fluorophenyl)pyrrolidine-3-carboxylate

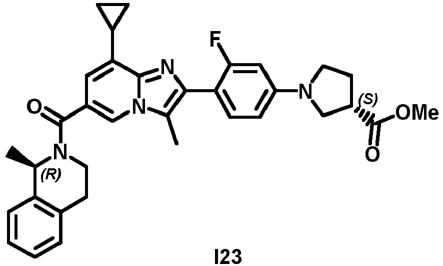

A mixture of intermediate **D5** (0.31 g,598 µmοl), (*S*)-methyl pyrrolidine-3-carboxylate hydrochloride [1099646-61-3] (139 mg, 0.84 mmol), cesium carbonate (0.78 g, 2.39 mmol) and XantPhos (41.5 mg, 71.8 µmοl) was purged with nitrogen. 1,4-Dioxane (6 mL) was added and the mixture was purged again with nitrogen. Palladium acetate (16.1 mg, 71.8 µmοl) was added. The reaction mixture was purged with nitrogen and stirred at 100°C for 5 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 25 g, 30 µm, mobile phase gradient: heptane / EtOAc from 90:10 to 50:50) to afford intermediate **123** (0.22 g, 65%) as a yellow solid.

### Intermediate I24

### (3S)-1-(4-{8-Cyclopropyl-3-methyl-6-[(1R)-1-methyl-1,2,3,4-tetrahydroisoquinoline-2-carbonyl]imidazo[1,2-a]pyridin-2-yl}-3-fluorophenyl)pyrrolidine-3-carboxylic acid

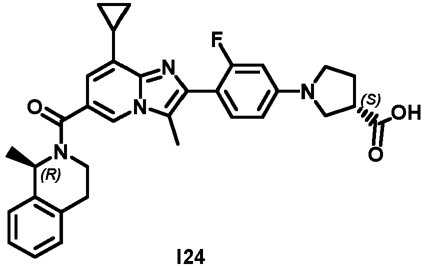

A mixture of intermediate **I23** (215 mg, 0.38 mmol) and lithium hydroxide monohydrate (95.5 mg, 2.28 mmol) in THF (6 mL) and H₂O (3 mL) was stirred at rt overnight. An aqueous solution of citric acid (438 mg in 10 mL of H₂O) was added. The layers were separated and the aqueous phase was extracted with EtOAc. The combined organic extracts were washed with brine, dried over MgSO₄, filtered and evaporated to dryness to afford intermediate **I24** (0.21 g, quant.) as an orange solid.

### Compound 21

### (3S)-1-(4-{8-Cyclopropyl-3-methyl-6-[(1R)-1-methyl-1,2,3,4-tetrahydroisoquinoline-2-carbonyl]imidazo[1,2-a]pyridin-2-yl}-3-fluorophenyl)pyrrolidine-3-carboxamide

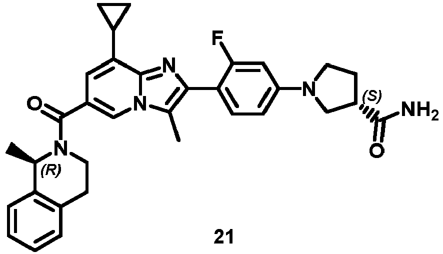

To a solution of intermediate **124** (0.21 g, 0.38 mmol) in DMF (5 mL) were added DIPEA (0.20 mL, 1.14 mmol) and HATU (217 mg, 0.57 mmol). The reaction mixture was stirred at rt for 15 min. Ammonia (30% in H₂O, 43.1 µL, 2.28 mmol) was added dropwise. The reaction mixture was stirred at rt for 1 h. The reaction mixture was diluted with H₂O and EtOAc. The layers were separated and the organic phase was washed with H₂O (3 times) and brine, dried over MgSO₄, filtered and evaporated to dryness to give compound **21** (0.15 g, 72%) as a brown solid.

### Synthesis of compound 22

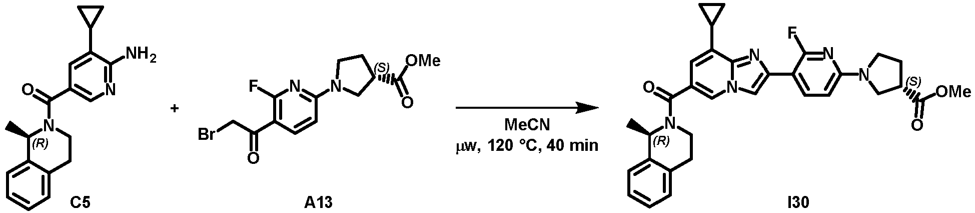

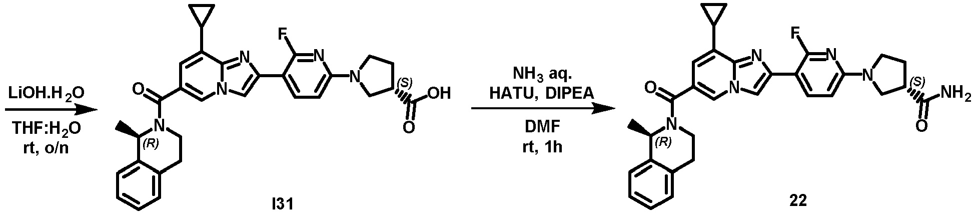

### Intermediate I30

### Methyl (3S)-1-(5-{8-cyclopropyl-6-[(1R)-1-methyl-1,2,3,4-tetrahydroisoquinoline-2-carbonyl]imidazo[1,2-a]pyridin-2-yl}-6-fluoropyridin-2-yl)pyrrolidine-3-carboxylate

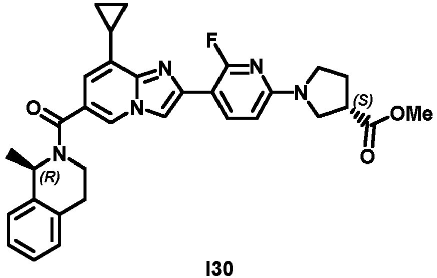

A mixture of intermediate **C5** (0.13 g, 0.42 mmol) and intermediate **A13** (175 mg, 0.51 mmol) in MeCN (2 mL) was heated at 120°C using a single mode microwave (Anton Paar Monowave^{®} 300) with a power output ranging from 0 to 850 W for 40 min. The reaction mixture was concentrated in vacuo. The crude mixture was purified by flash chromatography over silica gel (Puriflash Interchim^{®} 25 g, 30 µm, mobile phase gradient: heptane / EtOAc from 90:10 to 60:40 to afford intermediate **130** (0.173 g, 74%) as a yellow solid.

### Intermediate I30

### (3S)-1-(5-{8-Cyclopropyl-6-[(1R)-1-methyl-1,2,3,4-tetrahydroisoquinoline-2-carbonyl]imidazo[1,2-a]pyridin-2-yl}-6-fluoropyridin-2-yl)pyrrolidine-3-carboxylic acid

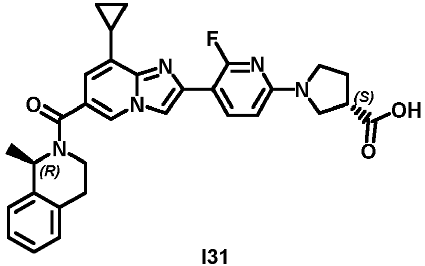

A mixture of intermediate **130** (0.17 g, 0.31 mmol) and lithium hydroxide monohydrate (91.8 mg, 2.19 mmol) in THF (6 mL) and H₂O (2.5 mL) was stirred at rt overnight. An aqueous solution of citric acid (420 mg in 10 mL of H₂O) was added. The layers were separated and the aqueous phase was extracted with EtOAc. The combined organic extracts were washed with brine, dried over MgSO₄, filtered and evaporated to dryness to afford intermediate **131** (0.168 g, quant.) as a beige solid.

### Compound 22

### (3S)-1-(5-{8-Cyclopropyl-6-[(1R)-1-methyl-1,2,3,4-tetrahydroisoquinoline-2-carbonyl]imidazo[1,2-a]pyridin-2-yl}-6-fluoropyridin-2-yl)pyrrolidine-3-carboxamide

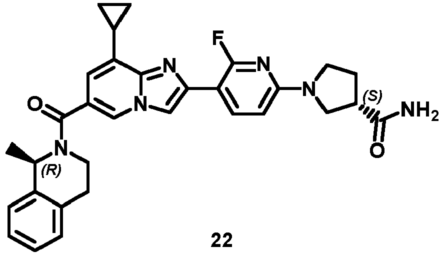

To a solution of intermediate **131** (168 mg, 0.31 mmol) in DMF (4 mL) were added DIPEA (0.16 mL, 0.93 mmol) and HATU (0.18 g, 0.47 mmol). The reaction mixture was stirred at rt for 15 min and ammonia (30% in H₂O, 35 µL, 1.87 mmol) was added dropwise. The reaction mixture was stirred at rt for 1 h. The reaction mixture was diluted with H₂O and EtOAc. The layers were separated and the organic phase was washed with water (3 times) and brine, dried over MgSO₄, filtered and evaporated to dryness. The crude mixture was purified by flash chromatography over silica gel (Puriflash Interchim^{®} 12 g, 30 µm, mobile phase gradient: DCM / MeOH from 100:0 to 95:5) to afford give compound **22** (0.085 g, 51%) as a brown solid.

### Synthesis of compound 23

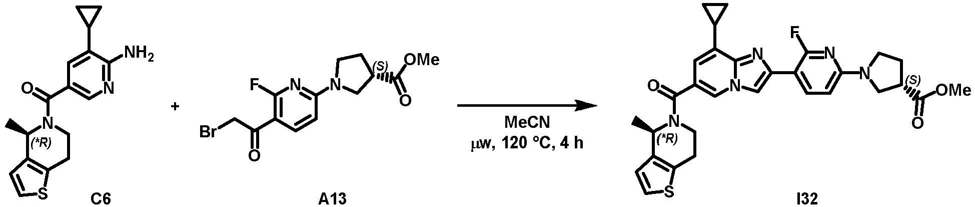

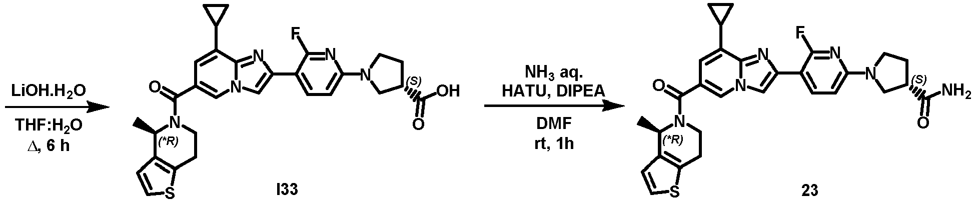

### Intermediate I32

### Methyl (3S)-1-(5-{8-Cyclopropyl-6-[(4^{∗}R)-4-methyl-4,5,6,7-tetrahydrothieno[3,2-c]pyridine-5-carbonyl]imidazo[1,2-a]pyridin-2-yl}-6-fluoropyridin-2-yl)pyrrolidine-3-carboxylate

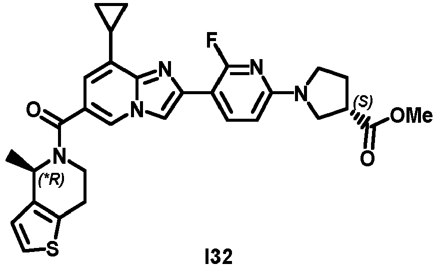

A mixture of intermediate **C6** (0.25 g, 0.80 mmol) and intermediate **A13** (0.33 g, 0.96 mmol) in MeCN (4 mL) was heated at 120°C using a single mode microwave (Anton Paar Monowave^{®} 300) with a power output ranging from 0 to 850 W for 40 min. The reaction mixture was concentrated to dryness. The crude mixture was purified by flash chromatography over silica gel (Puriflash Interchim^{®} 12 g, 30 µM, mobile phase gradient: heptane / EtOAc from 100:0 to 60:40) to afford intermediate **I32** (0.20 g, 45%) as a brown solid.

### Intermediate I33

### (3S)-1-(5-{8-Cyclopropyl-6-[(4^{∗}R)-4-methyl-4,5,6,7-tetrahydrothieno[3,2-c]pyridine-5-carbonyl]imidazo[1,2-a]pyridin-2-yl}-6-fluoropyridin-2-yl)pyrrolidine-3-carboxylic acid

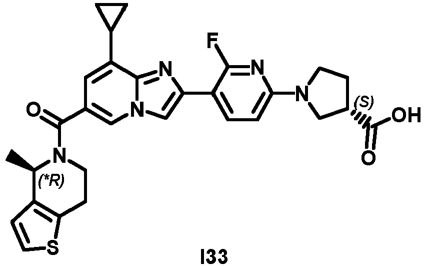

A mixture of intermediate **132** (189 mg, 0.34 mmol) and lithium hydroxide monohydrate (85 mg, 2.03 mmol) in THF (5 mL) and H₂O (1.5 mL) was stirred under reflux for 6 h. An aqueous solution of citric acid (390 mg in 5 mL of H₂O) was added. The precipitate was filtered off, washed with H₂O and Et₂O and dried under vacuum to afford intermediate **133** (0.18 g, 99%) as a yellow solid.

### Compound 23

### (3S)-1-(5-{8-cyclopropyl-6-[(4^{∗}R)-4-methyl-4,5,6,7-tetrahydrothieno[3,2-c]pyridine-5-carbonyl]imidazo[1,2-a]pyridin-2-yl}-6-fluoropyridin-2-yl)pyrrolidine-3-carboxamide

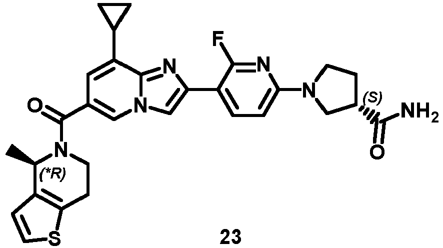

To a solution of intermediate **133** (183 mg, 0.34 mmol) in DMF (5 mL) were added DIPEA (0.17 mL, 1.01 mmol) and HATU (0.19 g, 0.50 mmol). The reaction mixture was stirred at rt for 15 min and ammonia (30% in H₂O, 38 µL, 2.01 mmol) was added dropwise. The reaction mixture was stirred at rt for 1 h. The reaction mixture was diluted with H₂O and EtOAc. The layers were separated and the organic phase was washed with H₂O (3 times) and brine, dried over MgSO₄, filtered and evaporated to dryness to give compound **23** (0.15 g, 82%) as a brown solid.

### Synthesis of compound 24

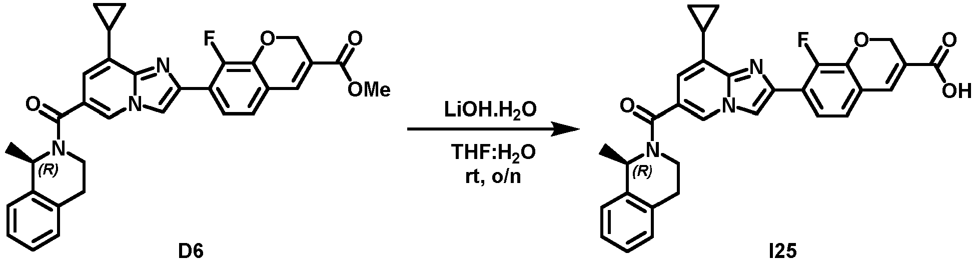

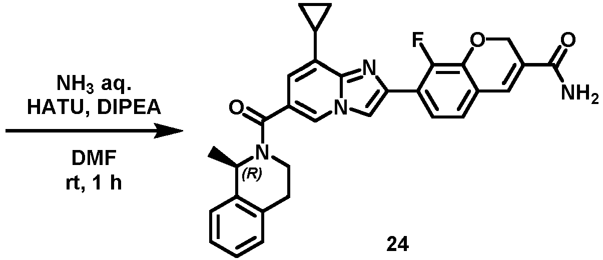

### Intermediate I25

### 6-{8-Cyclopropyl-6-[(1R)-1-methyl-1,2,3,4-tetrahydroisoquinoline-2-carbonyl]imidazo[1,2-a]pyridin-2-yl}-7-fluoro-2H-chromene-3-carboxylic acid

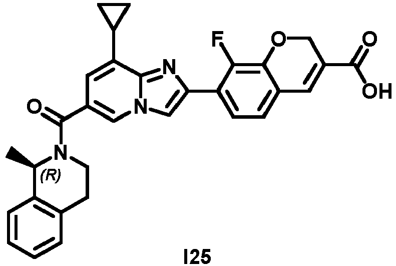

A mixture of intermediate **D6** (127 mg, 236 µmοl) and lithium hydroxide monohydrate (69.4 mg, 1.65 mmol) in THF (5 mL) and H₂O (1.5 mL) was stirred at rt for 15 h. An aqueous solution of citric acid (317 mg in 5 mL of H₂O) was added. The layers were separated and the aqueous phase was extracted with EtOAc. The combined organic extracts were washed with brine, dried over MgSO₄, filtered and evaporated to dryness to afford intermediate **125** (0.10 g, 81%) as a beige solid.

### Compound 24

### 6-{8-Cyclopropyl-6-[(1R)-1-methyl-1,2,3,4-tetrahydroisoquinoline-2-carbonyl]imidazo[1,2-a]pyridin-2-yl}-7-fluoro-2H-chromene-3-carboxamide

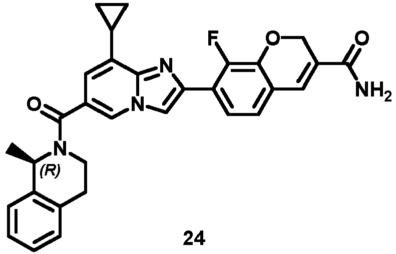

To a solution of intermediate **125** (0.16 g, 306 µmοl) in DMF (4 mL) were added DIPEA (0.16 mL, 0.92 mmol) and HATU (174 mg, 0.46 mmol). The reaction mixture was stirred at rt for 15 min. Ammonia (30% in H₂O, 35 µL, 1.83 mmol) was added dropwise. The reaction mixture was stirred at rt for 1 h. The reaction mixture was diluted with H₂O (3 times) and EtOAc. The layers were separated and the organic phase was washed with H₂O and brine (3 times), dried over MgSO₄, filtered and evaporated to dryness. The crude mixture was purified by flash chromatography over silica gel (Puriflash Interchim^{®} 12 g, 30 µm, mobile phase gradient: DCM / MeOH from 100:0 to 97:3) to give compound **24** (52 mg, 33%) as an orange solid.

### Synthesis of compound 25

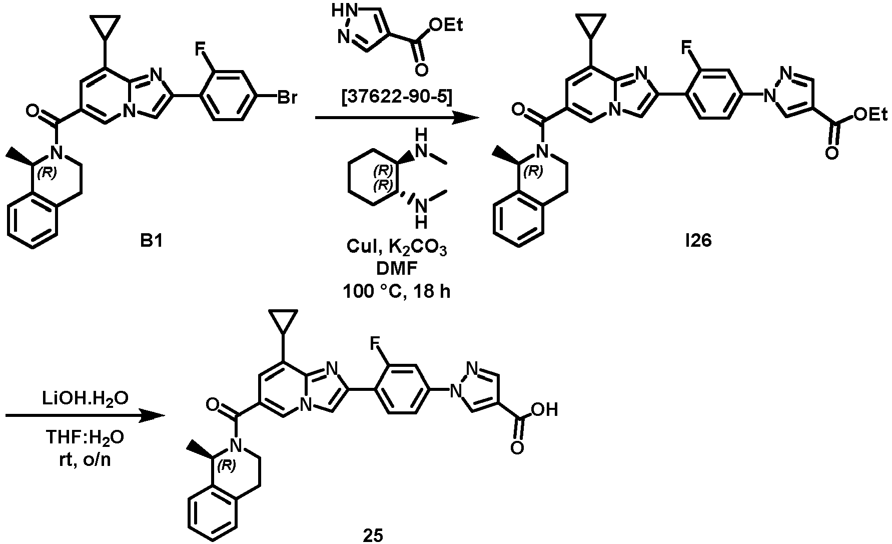

### Intermediate I26

### Ethyl 1-(4-{8-cyclopropyl-6-[(1R)-1-methyl-1,2,3,4-tetrahydroisoquinoline-2-carbonyl]imidazo[1,2-a]pyridin-2-yl}-3-fluorophenyl)-1H-pyrazole-4-carboxylate

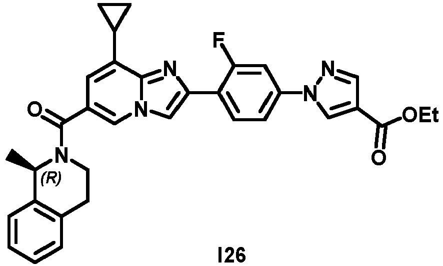

A mixture of intermediate **B1** (0.50 g, 0.99 mmol), ethyl 4-pyrazolecarboxylate [37622-90-5] (0.34 g, 2.38 mmol), copper iodide (189 mg, 0.99 mmol), (1*R*,2*R*)-N,N'-dimethylcyclohexane-1,2-diamine (0.16 mL, 0.99 mmol) and potassium carbonate (0.41 g, 2.97 mmol) was purged with nitrogen for 5 min. DMF (8.5 mL) was added and the reaction mixture was stirred at 100°C for 18 h. The mixture was poured out into a solution of EtOAc and a saturated aqueous solution of NH₄Cl. The layers were separated and the aqueous phase was extracted with EtOAc. The combined organic extracts were washed with a saturated aqueous solution of NH₄Cl, H₂O and brine, dried over MgSO₄, filtered and evaporated to dryness. The crude mixture was purified by flash chromatography over silica gel (Puriflash Interchim^{®} 40 g, 30 µm, mobile phase gradient: heptane / EtOAc from 100:0 to 60:40) to afford intermediate **126** (0.25 g, 45%) as a yellow solid.

### Compound 25

### 1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)-1H-pyrazole-4-carboxylic acid

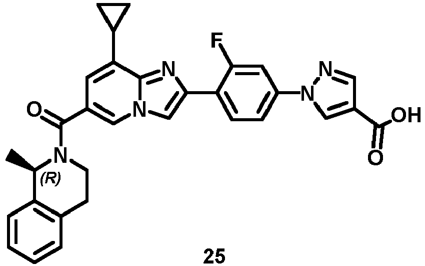

A mixture of intermediate **126** (135 mg, 0.24 mmol) and lithium hydroxide monohydrate (70.4 mg, 1.68 mmol) in THF (5 mL) and H₂O (1 mL) was stirred at rt overnight. An aqueous solution of citric acid (322 mg in 5 mL of H₂O) was added. The layers were separated and the aqueous phase was extracted with EtOAc. The combined organic extracts were washed with brine, dried over MgSO₄, filtered and evaporated to dryness. The crude mixture was purified by flash chromatography over silica gel (Puriflash Interchim^{®} 25 g, 30 µm, mobile phase gradient: DCM / MeOH from 100:0 to 98:2) to give compound **25** (51 mg, 40%) as a beige solid.

### Compound 26

### 1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)-1H-pyrazole-4-carboxamide

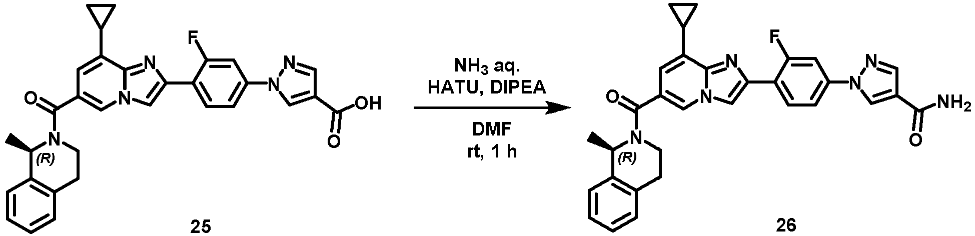

To a solution of compound **25** (61.0 mg, 114 µmοl) in DMF (2 mL) were added HATU (65.0 mg, 0.17 mmol) and DIPEA (59 µL, 0.34 mmol). The reaction mixture was stirred at rt for 15 min. Ammonia (30% in H₂O, 43 µL, 0.68 mmol) was added. The reaction mixture was stirred at rt for 1 h. The reaction mixture was diluted with H₂O and the aqueous phase was extracted with EtOAc. The combined organic extracts were washed with H₂O and brine, dried over MgSO₄, filtered and evaporated to dryness. The residue was taken up in Et₂O. The solid was filtered off and dried under vacuum. The residue was purified by flash chromatography over silica gel (Puriflash Interchim^{®} 4 g, 30 µm, mobile phase gradient: DCM / MeOH from 100:0 to 98:2) to give compound **26** (17 mg, 28%) as a beige solid.

### Compound 27

### 1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)azetidin-3-ol

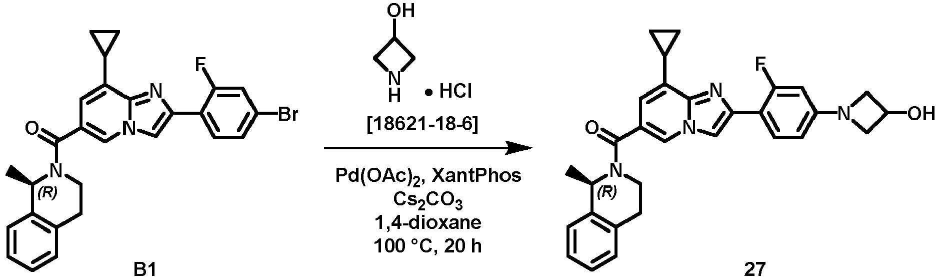

A mixture of intermediate **B1** (0.15 g, 297 µmοl), (3)-hydroxyazetidine hydrochloride [18621-18-6] (65.2 mg, 595 µmοl), cesium carbonate (388 mg, 1.19 mmol) and XantPhos (20.6 mg,35.7 µmοl) was purged with nitrogen. 1,4-Dioxane (3 mL) was added and the mixture was purged again with nitrogen. Palladium acetate (8.01 mg, 35.7 µmοl) was added. The reaction mixture was purged with nitrogen and stirred at 100°C for 20 h. The mixture was diluted with H₂O and EtOAc. The layers were separated and the aqueous phase was extracted with EtOAc (twice). The combined organic extracts were washed with H₂O and brine, dried over MgSO₄, filtered and evaporated to dryness. The crude mixture was purified by flash chromatography over silica gel (Puriflash Interchim^{®} 25 g, 30 µm, mobile phase gradient: DCM / MeOH from 100:0 to 97:3). The residue (0.11 g) was taken up in DIPE. The solid was filtered off and dried in vacuo to give compound **27** (0.09 g, 61%) as an orange solid.

### Compound 28

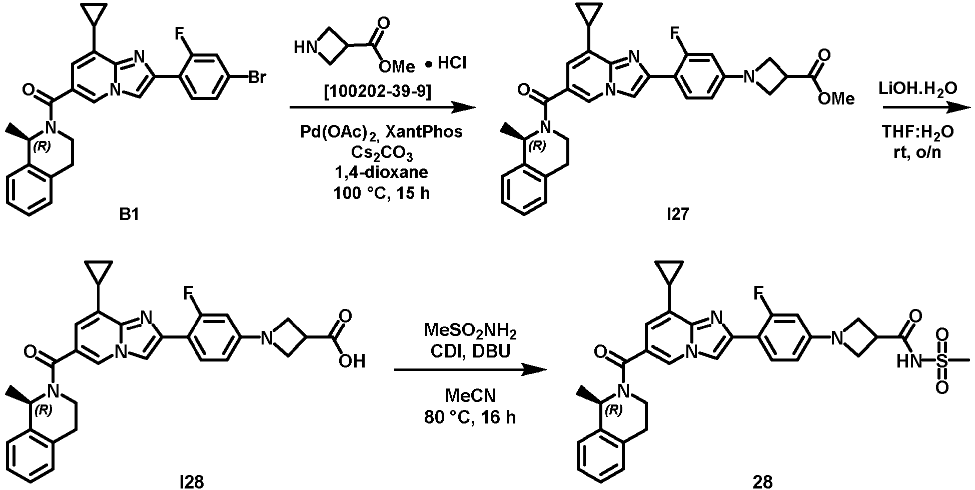

### Intermediate I27

### Methyl 1-(4- {8-cyclopropyl-6-[(1R)-1-methyl-1,2,3,4-tetrahydroisoquinoline-2-carbonyl]imidazo[1,2-a]pyridin-2-yl}-3-fluorophenyl)azetidine-3-carboxylate

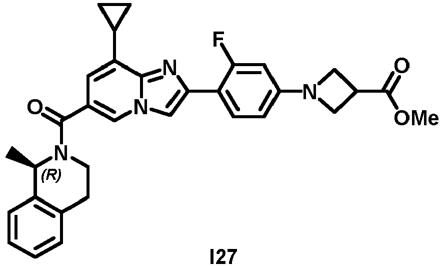

A mixture of intermediate **B1** (0.20 g, 397 µmοl), methyl azetidine-3-carboxylate hydrochloride [100202-39-9] (90.2 mg, 595 µmοl), cesium carbonate (517 mg, 1.59 mmol) and XantPhos (27.5 mg, 47.6 µmοl) was purged with nitrogen. 1,4-Dioxane (4 mL) was added and the mixture was purged again with nitrogen. Palladium acetate (10.7 mg, 47.6 µmοl) was added. The reaction mixture was purged with nitrogen and stirred at 100°C for 15 h. The mixture was diluted with H₂O and EtOAc. The layers were separated and the aqueous phase was extracted with EtOAc (twice). The combined organic extracts were washed with H₂O and brine, dried over MgSO₄, filtered and evaporated to dryness. The crude mixture was purified by flash chromatography over silica gel (Puriflash Interchim^{®} 25 g, 30 µm, mobile phase gradient: heptane / EtOAc from 100:0 to 60:40) to afford intermediate **127** (0.16 g, 75%) as a beige solid.

### Intermediate I28

### 1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)azetidine-3-carboxylic acid

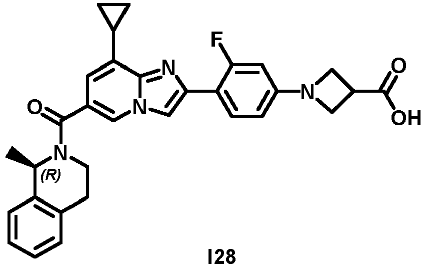

A mixture of intermediate **127** (0.15 g, 278 µmοl) and lithium hydroxide monohydrate (70.1 mg, 1.67 mmol) in THF (8 mL) and H₂O (2 mL) was stirred at rt overnight. An aqueous solution of citric acid (321 mg) was added. The layers were separated and the aqueous phase was extracted with EtOAc. The combined organic extracts were washed with brine, dried over MgSO₄, filtered and evaporated to dryness to afford intermediate **128** (0.15 g, quant.) as a yellow solid.

### Compound 28

### 1-(4-{8-Cyclopropyl-6-[(1R)-1-methyl-1,2,3,4-tetrahydroisoquinoline-2-carbonyl]imidazo[1,2-a]pyridin-2-yl}-3-fluorophenyl)-N-methanesulfonylazetidine-3-carboxamide

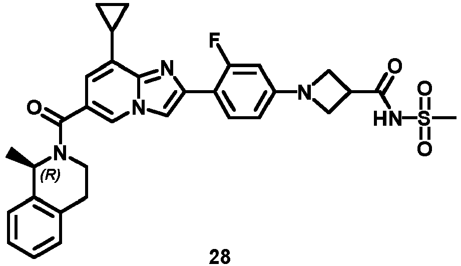

A mixture of intermediate **128** (0.14 g, 267 µmοl) and CDI (51.9 mg, 0.32 mmol) in MeCN (3 mL) was stirred at rt for 2 h. Methanesulfonamide (38.1 mg, 0.40 mmol) and DBU (59.8 µL, 0.40 mmol) were added. The reaction mixture was stirred at 80°C for 16 h. A 1N aqueous solution of HCl and DCM were added. A yellow precipitate was formed. The solid was filtered off and dried in vacuo. The yellow solid was purified by flash chromatography over silica gel (Puriflash Interchim^{®} 25 g, 30 µm, mobile phase gradient: DCM / MeOH from 100:0 to 96:4). The residue (80 mg) was taken up in DIPE. The solid was filtered off and dried in vacuo to give compound **28** (0.06 g, 37%) as a beige solid.

### Synthesis of compound 29

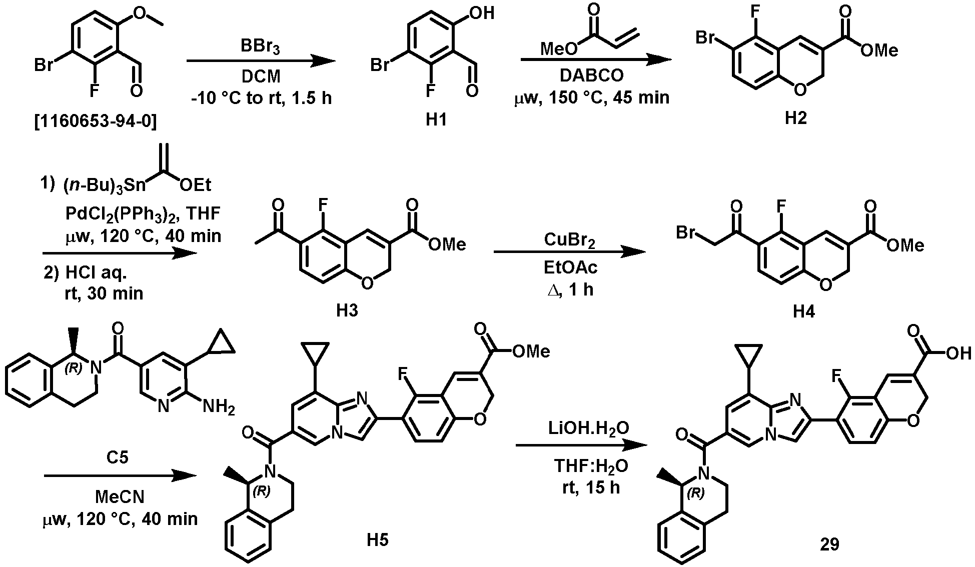

### Intermediate H1

### 3-bromo-2-fluoro-6-hydroxybenzaldehyde

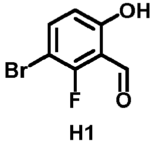

To a solution of 3-bromo-2-fluoro-6-methoxybenzaldehyde (3.0 g, 12.9 mmol) in DCM (25 mL) at -10°C was added dropwise of boron tribromide (38.6 mL, 1 M, 36.6 mmol). The resulting mixture was allowed to warm up to rt and stirred at rt for 1.5 h. The reaction mixture was quenched with ice water and extracted with DCM (twice). The combined organic extracts were washed with a saturated aqueous solution of NaHCO₃, brine and dried over MgSO₄, filtered and evaporated to dryness to afford intermediate **H1** (2.8 g, 99%) as a beige solid.

### Intermediate H2

### Methyl 6-bromo-5-fluoro-2H-chromene-3-carboxylate

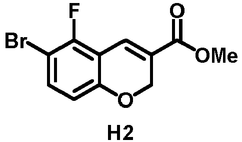

A mixture of intermediate **H1** (1.48 g, 6.76 mmol), methyl acrylate (4.48 mL, 50.00 mmol) and 1,4-diazabicyclo[2.2.2]octane (0.15 g, 1.35 mmol) were heated at 150°C using a single mode microwave (Anton Paar Monowave^{®} 300) with a power output ranging from 0 to 850 W for 45 min. The mixture was concentrated to dryness. The crude mixture was purified by flash chromatography over silica gel (Puriflash Interchim^{®} 40 g, 30 µm, dry loading (celite^{®}), mobile phase gradient: heptane / EtOAc from 100:0 to 80:20) to afford intermediate **H2** (0.60 g, 31%) as a white solid.

### Intermediate H3

### Methyl 6-acetyl-5-fluoro-2H-chromene-3-carboxylate

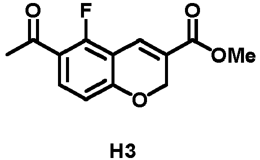

A solution of intermediate **H2** (0.38 g, 1.32 mmol) in THF (7.5 mL) was purged with nitrogen for 10 min. Tributyl(1-ethoxyvinyl)tin (0.90 mL, 2.65 mmol) and bis(triphenylphosphine)palladium dichloride (93 mg, 0.13 mmol) were added. The mixture was heated at 120°C using a single mode microwave (Anton Paar Monowave^{®} 300) with a power output ranging from 0 to 850 W for 20 min. The mixture was poured into the solution of KF (2 g in 50 mL of H₂O) and stirred for 10 min at rt then filtered through a short pad of celite^{®} and rinsed with EtOAc. The layers were separated and the aqueous phase was extracted with EtOAc. The combined organic extracts were washed with H₂O and brine and dried over MgSO₄, filtered and evaporated to dryness. The crude mixture was purified by flash chromatography over silica gel (Puriflash Interchim^{®} 40 g, 30 µm, dry loading (Celite^{®}), mobile phase gradient: heptane / EtOAc from 100:0 to 80:20) to afford intermediate **H3** (0.24 g, 72%) as a white solid.

### Intermediate H4

### Methyl 6-(2-bromoacetyl)-5-fluoro-2H-chromene-3-carboxylate

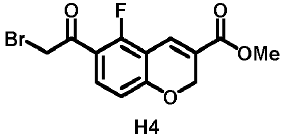

To a solution of intermediate **H3** (0.23 g, 0.92 mmol) in EtOAc (7 mL) at rt, was added copper (II) bromide (0.41 g, 1.84 mmol). The reaction mixture was stirred under reflux for 5 h. The solid was filtered off. The filtrate was washed with a saturated aqueous solution of NaHCO₃ and brine, dried over MgSO₄, filtered and concentrated in vacuo to give intermediate **H4** (0.30 g, 99%) as a white solid.

### Intermediate H5

### Methyl (R)-6-(8-cyclopropyl-6-(1 -methyl-1,2,3,4-tetrahydroisoquinoline-2-carbonyl)imidazo[1,2-a]pyridin-2-yl)-5-fluoro-2H-chromene-3-carboxylate

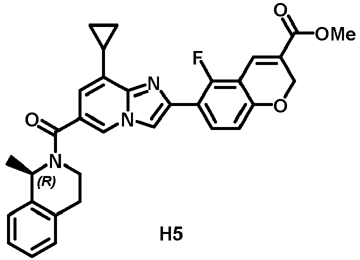

A mixture of intermediate **C5** (60 mg, 0.20 mmol) and intermediate **H4** (77 mg, 0.23 mmol) in MeCN (2.0 mL) was heated at 120°C using a single mode microwave (Anton Paar Monowave^{®} 300) with a power output ranging from 0 to 850 W for 40 min. The mixture was concentrated in vacuo. The crude mixture was purified by flash chromatography over silica gel (Puriflash Interchim^{®} 12 g, 30 µm, mobile phase gradient: heptane / EtOAc from 90:10 to 70:30) to afford intermediate **H5** (30 mg, 29%) as a yellow solid.

### Compound 29

### (R)-6-(8-cyclopropyl-6-(1-methyl-1,2,3,4-tetrahydroisoquinoline-2-carbonyl)imidazo[1,2-a]pyridin-2-yl)-5-fluoro-2H-chromene-3-carboxylic acid

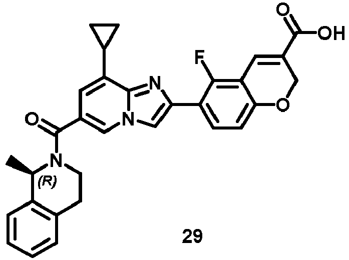

A mixture of intermediate **H5** (30 mg, 60 µmοl) and lithium hydroxide monohydrate (16 mg, 0.39 mmol) in THF (2 mL) and H₂O (0.5 mL) was stirred at rt for 15 h. An aqueous solution of citric acid (75 mg in 2 mL of H₂O) was added. The layers were separated and the aqueous phase was extracted with EtOAc. The combined organic extracts were washed with brine, dried over MgSO₄, filtered and evaporated to dryness to afford compound **29** (28 mg, 95%) as a beige foam.

### Compound 30

### (R)-N-(4-(8-cyclopropyl-6-(1-methyl-1,2,3,4-tetrahydroisoquinoline-2-carbonyl)imidazo[1,2-a]pyridin-2-yl)-3-fluorophenyl)-3-hydroxyazetidine-1-carboxamide

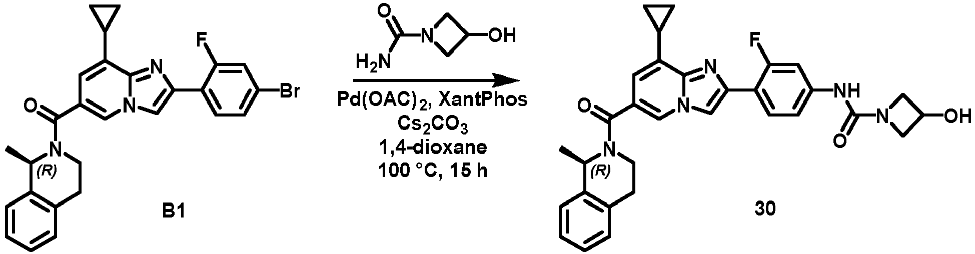

A mixture of intermediate **B1** (0.3 g, 0.60 mmol), 3-hydroxyazetidine-1-carboxamide (0.14 g, 1.19 mmol), cesium carbonate (0.78 g, 2.38 mmol) and XantPhos (34 mg, 60 µmοl) in 1,4-dioxane (6 mL) was purged with nitrogen. Palladium acetate (13 mg, 0.06 mmol) was added. The reaction mixture was purged again with nitrogen and stirred at 100°C for 15 h. The reaction mixture was diluted with EtOAc and H₂O then filtered through a short pad of celite^{®}. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 40 g, 30 µm, mobile phase gradient: DCM/MeOH, from 100:0 to 97:03) to afford compound **30** (0.11 g, yield 34%) as a yellow solid.

### Compound 31

### (R),(E)-3-(4-(8-cyclopropyl-6-(1-methyl-1,2,3,4-tetrahydroisoquinoline-2-carbonyl)imidazo[1,2-a]pyridin-2-yl)-3-fluorophenyl)acrylic acid

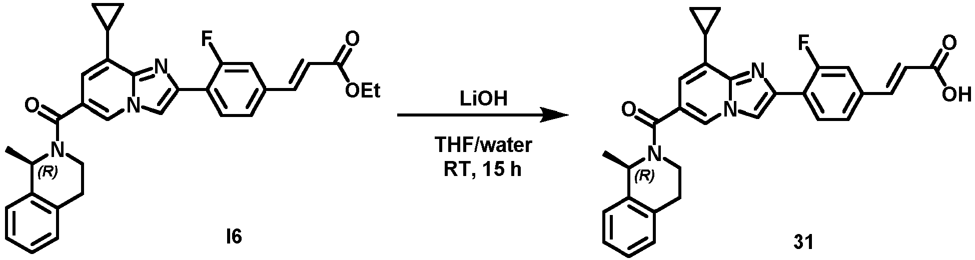

A mixture of intermediate **I6** (0.10 g, 0.19 mmol) and lithium hydroxide monohydrate (64 mg, 1.53 mmol) in THF (5.5 mL) and H₂O (1 mL) was stirred at rt for 15 h. An aqueous solution of citric acid (294 mg in 5 mL of H₂O) was added. The layers were separated and the aqueous phase was extracted with EtOAc. The combined organic extracts were washed with brine, dried over MgSO₄, filtered and concentrated in vacuo. The residue was taken up in EtOAc and stirred at rt for 5 min. The solid was filtered off and dried under vacuum to give compound **31** (65 mg, 69%) as a beige solid.

### Part 2: Imidazo[1,2-b]pyridazines

### Synthesis of Intermediate 135

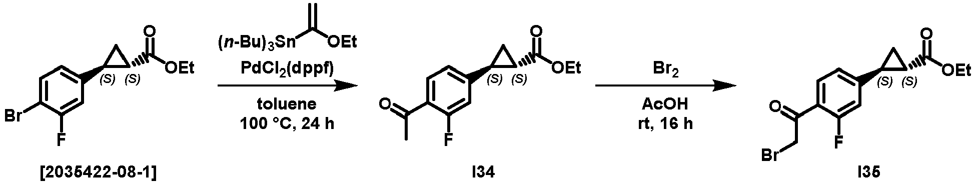

### Intermediate I34

### Ethyl (1S,2S)-2-(4-acetyl-3-fluorophenyl)cyclopropane-1-carboxylate

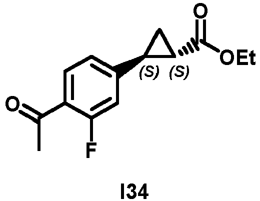

To a degassed mixture of ethyl (1S,2S)-2-(4-bromo-3-fluorophenyl)cyclopropane-1-carboxylate [2035422-08-1] (5.00 g, 17.0 mmol, 95% purity) and 1-ethoxy-1-(tributylstannyl)ethylene [97674-02-7] (6.1 mL, 18.2 mmol) in toluene (95 mL) was added [1,1'-bis(diphenylphosphino)ferrocene]dichloropalladium(II) (605 mg, 827 µmοl). The reaction mixture was stirred at 100°C for 24 h. The reaction mixture was evaporated to dryness. The crude mixture was purified by preparative LC (regular SiOH, 30 µm, 220 g Interchim^{®}, dry loading (Celite^{®}), mobile phase gradient: heptane / EtOAc from 100:0 to 80:20) to give intermediate **134** (1.3 g, 31%) and a fraction containing impurities (2.8 g). The latter was hydrolyzed with a 1N aqueous solution of HCl (50 mL) in THF (50 mL) for 1 h. The reaction mixture was diluted with H₂O and EtOAc. The layers were separated and the aqueous phase was extracted with EtOAc (twice). The combined organic extracts were dried over MgSO₄, filtered and the solvent was evaporated in vacuo. The residue was purified by preparative LC (regular SiOH, 30 µm, mobile phase gradient: heptane / EtOAc from 100:0 to 80:20) to afford intermediate **134** (1.2 g, 29%).

### Intermediate I35

### Ethyl (1S,2S)-2-[4-(2-bromoacetyl)-3-fluorophenyl]cyclopropane-1-carboxylate

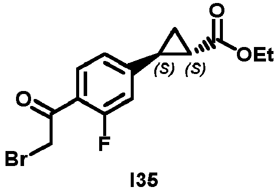

Bromine (0.27 mL, 5.19 mmol) was added dropwise to a solution of intermediate **134** (1.30 g, 5.19 mmol) in acetic acid (26 mL). The reaction mixture was stirred at rt for 16 h. The reaction mixture was evaporated to dryness and azeotroped with toluene to give intermediate **135** (1.6 g, 94%).

### Synthesis of Intermediate E3

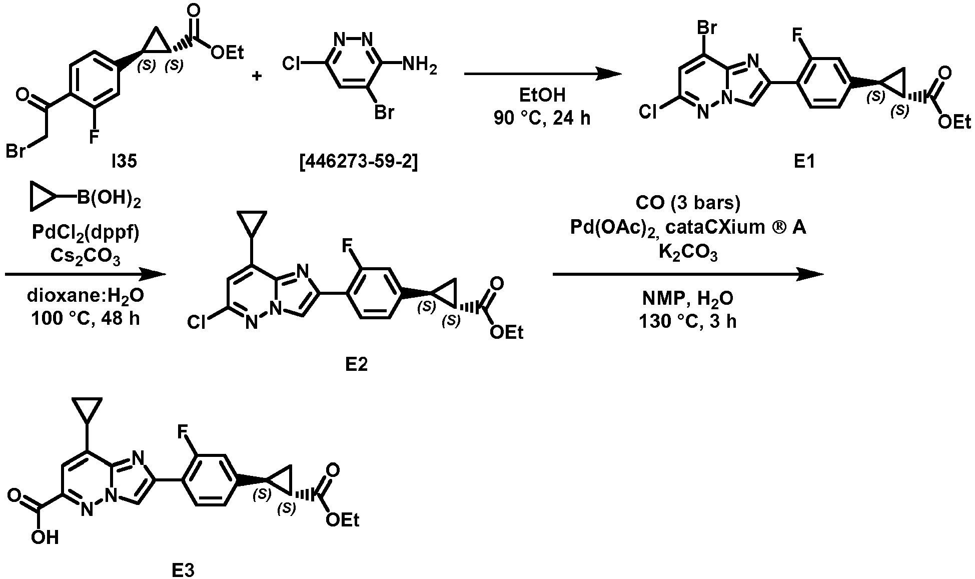

### Intermediate E1

### Ethyl (1S,2S)-2-(4-{8-bromo-6-chloroimidazo[1,2-b]pyridazin-2-yl}-3-fluorophenyl)-cyclopropane-1-carboxylate

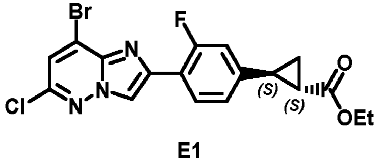

A mixture of intermediate **135** (1.20 g, 3.65 mmol) and 3-amino-4-bromo-6-chloropyridazine [446273-59-2] (912 mg, 4.38 mmol) in EtOH (34 mL) was stirred at 90°C for 24 h. The reaction mixture was cooled to 0°C. The precipitate was filtered off and dried to afford a first crop of intermediate **E1** (421 mg, 26%). Mother liquor was evaporated to dryness. The residue was taken up in EtOH. The solid was filtered off and dried. The residue, mother liquor and another batch (208 mg, 0.63 mmol) were combined and purified by preparative LC (regular SiOH, 30 µm, 80 g Interchim^{®}, dry loading (Celite^{®}), mobile phase gradient: heptane / EtOAc from 100:0 to 60:40). The residue (580 mg) was triturated in EtOH and the solid was filtered off and dried to afford a 2^{nd} crop of intermediate **E1** (157 mg).

### Intermediate E2

### Ethyl (1S,2S)-2-(4-{6-chloro-8-cyclopropylimidazo[1,2-b]pyridazin-2-yl}-3-fluorophenyl)-cyclopropane-1-carboxylate

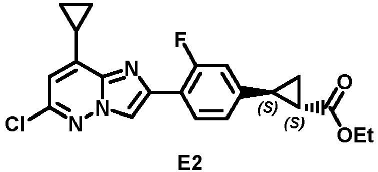

To a degassed mixture of intermediate **E1** (578 mg, 1.32 mmol), cesium carbonate (1.29 g, 3.95 mmol) and cyclopropylboronic acid [411235-57-9] (113 mg, 1.32 mmol) in H₂O (2.8 mL) and 1,4-dioxane (28 mL) was added [1,1'-bis(diphenylphosphino)ferrocene]dichloropalladium(II) (98.3 mg, 0.13 mmol). The reaction mixture was stirred at 100°C for 48 h. The reaction mixture was diluted with EtOAc and H₂O. The layers were separated and the aqueous layers was extracted with EtOAc (once). The combined organic extracts were dried over MgSO₄, filtered and evaporated in vacuo. The crude mixture was purified by preparative LC (regular SiOH, 30 µm, 40 g Interchim^{®}, mobile phase gradient: heptane / EtOAc from 100:0 to 60:40) to afford intermediate **E2** (400 mg, 76%).

### Intermediate E3

### 8-Cyclopropyl-2-{4-[(1S,2S)-2-(ethoxycarbonyl)cyclopropyl]-2-fluorophenyl[imidazo[1,2-b]pyridazine-6-carboxylic acid

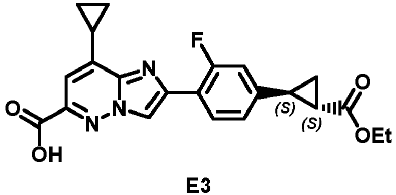

A degassed mixture of intermediate **E2** (580 mg, 1.45 mmol), potassium carbonate (241 mg, 1.74 mmol), palladium acetate (32.6 mg, 145 µmοl) and cataCXium^{®} A (104 mg, 0.29 mmol) in NMP (14.5 mL) and H₂O (1 mL) was stirred under 3 bars of carbon monoxide at 130°C for 3 h. Additional amount of palladium acetate (32.6 mg, 145 µmοl) and cataCXium^{®} A (104 mg, 0.29 mmol) were added and the reaction mixture was stirred under 3 bars of carbon monoxide at 130°C for another 24 h. The reaction mixture was diluted with H₂O and EtOAc. The layers were separated and the aqueous phase was extracted with EtOAc (once). The combined organic extracts were washed with brine (3 times), dried over MgSO₄, filtered and evaporated in vacuo.

The crude mixture was purified by preparative LC (regular SiOH, 30 µm, 80 g Interchim^{®}, mobile phase gradient: heptane / EtOAc from 100:0 to 0:100 then EtOAc / MeOH from 100:0 to 95:5 and (EtOAc / AcOH 97.5 / 2.5) / MeOH from 95:5 to 85:15) to afford intermediate **E3** (0.41 g, 40%, 58% purity).

### Synthesis of Intermediate F6

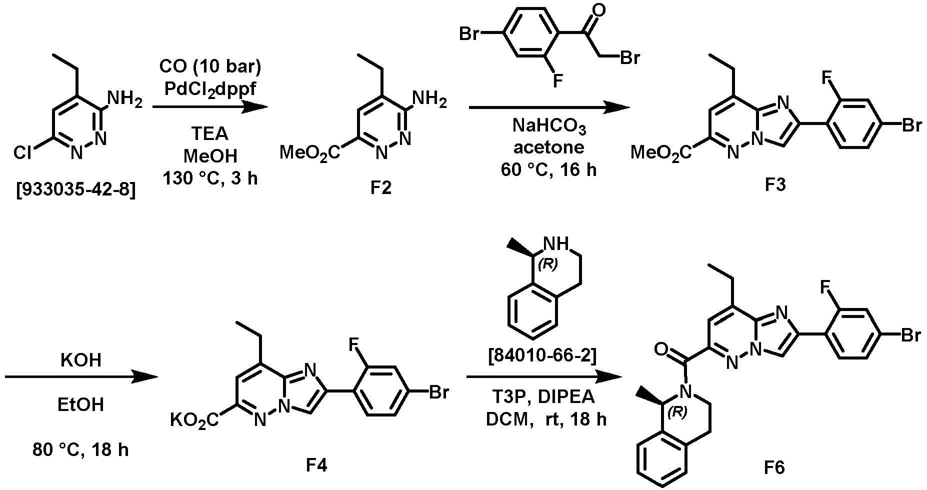

### Intermediate F2

### Methyl 6-amino-5-ethylpyridazine-3-carboxylate

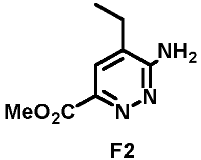

In a stainless-steel bomb, to a degassed mixture of 6-chloro-4-ethylpyridazin-3-amine [933035-42-8] (1.78 g; 11.3 mmol) and TEA (3.84 mL; 27.6 mmol) in MeOH (77 mL) was added PdCl₂(dppf) (603 mg; 0.824 mmol). The resulting mixture stirred 3 h at 130°C under 10 bars of CO. The mixture was cooled to rt, filtered and evaporated to dryness to give intermediate **F2** (3.7 g, 99%) as a brownish gum.

### Intermediate F3

### Methyl 2-(4-bromo-2-fluorophenyl)-8-ethylimidazo[1,2-b]pyridazine-6-carboxylate

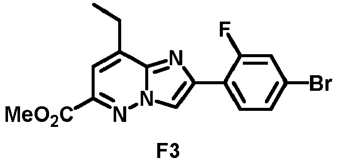

A mixture of 4-Bromo-2-fluorophenacyl bromide (3.30 g, 11.1 mmol), intermediate **F2** (3.65 g, 11.1 mmol) and NaHCO₃ (0.931 g, 11.08 mmol) in acetone (87 mL) was stirred at 60°C for 16 h. The mixture was cooled to rt. The filtrate was evaporated to dryness and the residue was taken-up with MeCN. The solid was filtered off to give intermediate **F3** (1.9 g, 47%) as a yellow solid. The filtrate was evaporated under vacuum and purified by preparative LC (irregular SiOH, 15-40 µm, 120 g Grace Resolv^{™}, solid loading (Celite^{®}), mobile phase gradient: from Heptane 100%, EtOAc 0% to heptane 70%, EtOAc 30%). The fractions containing product were evaporated under vacuum to give a residue. The residue was taken-up in MeCN and the solid was filtered to give intermediate **F3** (276 mg, 7%) as a yellow solid. (Global yield 54%)

### Intermediate F4

### Potassium 2-(4-bromo-2-fluorophenyl)-8-ethylimidazo[1 ,2-b]pyridazine-6-carboxylate

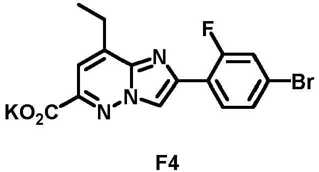

KOH (1.18 g; 17.9 mmol) was dissolved in EtOH (47 mL) then intermediate **F3** (2.26 g; 5.98 mmol) was added portionwise and the suspension was stirred at reflux for 18 h. The solid was filtered, washed with EtOH (once), then with Et₂O (4 times) and dried over frit to give intermediate **F4** (2.2 g, 92%) as a white solid.

### Intermediate F6

### (R)-(2-(4-bromo-2-fluorophenyl)-8-ethylimidazo[1,2-b]pyridazin-6-yl)(1-methyl-3,4-dihydroisoquinolin-2(1H)-yl)methanone

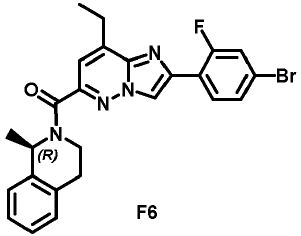

A mixture of intermediate **F4** (1.0 g; 2.49 mmol), (R)-1-methyl-1,2,3,4-tetrahydroisoquinoline [84010-66-2] (659 mg; 4.48 mmol) and DIPEA (2.14 mL; 12.4 mmol) in DCM (6.4 mL) was stirred at 0°C. 1-Propanephosphonic anhydride (50% in EtOAc; 3.7 mL; 6.2 mmol) was added slowly (over 5 min.) at 0°C. The mixture was stirred at 0°C for 10 min then at rt for 18 h. Brine, EtOAc and a sat. aq. solution of NaHCO₃ were added to the reaction mixture, the aqueous layer was extracted with EtOAc (twice). The combined organic layers were washed with a brine and a sat. aq. solution of NaHCO₃ (4 times), dried over MgSO₄ and evaporated in vacuo to give intermediate **F6** (1.31 g, 99%) as an orange foam.

### Synthesis of intermediate F20

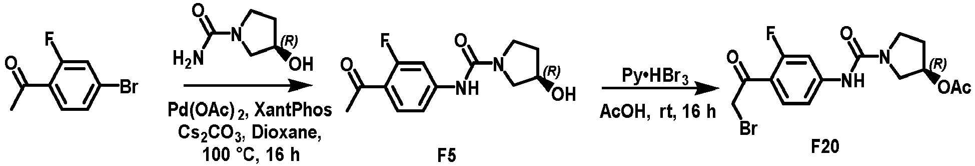

### Intermediate F5

### (R)-N-(4-acetyl-3-fluorophenyl)-3-hydroxypyrrolidine-1-carboxamide

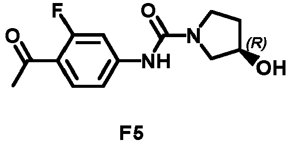

To a degassed mixture of 4-bromo-2-fluoroacetophenone (6.17 g; 28.4 mmol), (3R)-3-hydroxypyrrolidine-1-carboxamide (4.77 g; 34.1 mmol) and Cs₂CO₃ (27.8 g; 85.2 mmol) in dioxane (118 mL) was added Pd(OAc)₂ (638 mg; 2.84 mmol) and XantPhos (1.65 g; 2.84 mmol). The resulting mixture was stirred at 100°C for 16 h. Water and EtOAc were added. The layers were separated and the aqueous layer was extracted with EtOAc (once). The combined organic layers were washed with brine, dried over MgSO₄, filtered and the solvent was removed in vacuo to give 9.0 g of crude which was purified by preparative LC (regular SiOH, 30 µm, 330 g Interchim^{®}, mobile phase gradient: from heptane/EtOAc 60/40 to 0/100 then EtOAc/MeOH 100/0 to 85/15) to give intermediate **F5** (4.6 g, 61%).

### Intermediate F20

### (R)-1-((4-(2-bromoacetyl)-3-fluorophenyl)carbamoyl)pyrrolidin-3-yl acetate

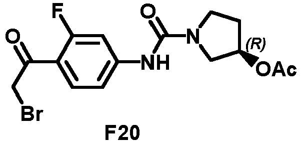

A mixture of intermediate **F5** (4.6 g; 17 mmol) and pyridine hydrobromide perbromide (5.5 g; 17 mmol) in AcOH (92 mL) was stirred at rt for 16 h. The mixture was evaporated to dryness and the residue was azeotroped with toluene (twice) then the residue was purified by preparative LC (regular SiOH, 30 µm, 220 g Interchim^{®}, mobile phase gradient: from heptane/EtOAc 70/30 to 0/100 then EtOAc/MeOH 100/0 to 90/10) to give intermediate **F20** (5.5 g, 82%).

### Synthesis of intermediate F24

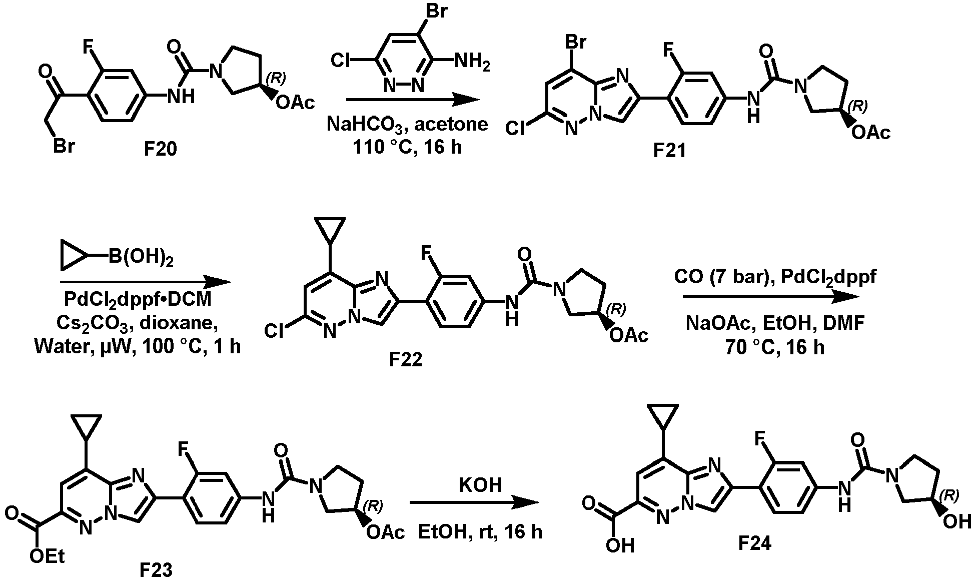

### Intermediate F21

### (R)-1-((4-(8-bromo-6-chloroimidazo[1,2-b]pyridazin-2-yl)-3-fluorophenyl)carbamoyl)-pyrrolidin-3-yl acetate

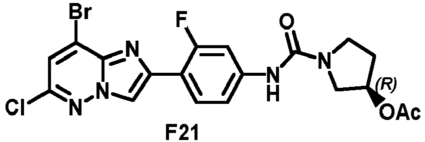

A mixture of intermediate **F20** (1.15 g; 2.97 mmol), 3-amino-4-bromo-6-chloropyridazine (619 mg; 2.97 mmol) and NaHCO₃ (249 mg; 2.97 mmol) in acetone (15 mL) was stirred at 110°C for 16 h in a pressure vessel reactor. The mixture was evaporated to dryness. The residue was taken-up with DCM and the solid was filtered and dried over frit. The solid was triturated in water, filtered and dried over frit to give intermediate **F21** (699 mg, 47%). The mother liquor was purified by preparative LC (regular SiOH, 30 µm, 40 g Interchim^{®}, dry loading (celite^{®}), mobile phase gradient: from DCM/iPrOH 100/0 to 90/10) the fraction containing product was collected and evaporated to dryness then the residue was taken-up with DCM and the solid was filtered and dried over frit to give intermediate **F21** (93 mg, 6%). The mother liquor was purified by preparative LC (regular SiOH, 30 µm, Interchim^{®} 40 g, mobile phase gradient: from heptane/EtOAc 70/30 to 0/100 then EtOAc/MeOH 100/0 to 85/15)) the fraction containing product was collected and evaporated to dryness then the residue was taken-up with DCM and the solid was filtered and dried over frit to give intermediate **F21** (76 mg, 5%). Global yield (868 mg, 58%).

### Intermediate F22

### (R)-1-((4-(6-chloro-8-cyclopropylimidazo[1,2-b]pyridazin-2-yl)-3-fluorophenyl)carbamoyl)-pyrrolidin-3-yl acetate

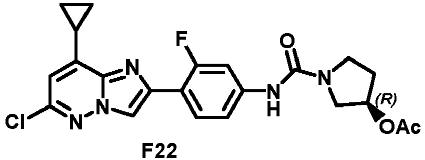

In a microwave vial, PdCl₂dppf (95 mg; 0.13 mmol) was added to a degassed solution of intermediate **21** (646 mg; 1.30 mmol), *^{c}*PrB(OH)₂ (106 mg; 1.24 mmol) and Cs₂CO₃ (1.75 g; 5.36 mmol) in dioxane (10 mL) and water (2 mL) at rt. The resulting mixture was heated at 100°C using one single mode microwave (biotage^{®}) with a power output ranging from 0 to 400 W for 1 h. Water and EtOAc were added and the layers were separated. The aqueous layer was extracted with EtOAc (once). The combined organic layer were dried over MgSO₄, filtered and the solvent was removed in vacuo. The residue was purified by preparative LC (regular SiOH, 30 µm, Interchim^{®} 40 g, mobile phase gradient: from heptane/*ⁱ*PrOH 90/10 to 40/60) to give intermediate **F22** (0.31 g, 52%).

### Intermediate F23

### Ethyl (R)-2-(4-(3-acetoxypyrrolidine-1-carboxamido)-2-fluorophenyl)-8-cyclopropyl-imidazo[1,2-b]pyridazine-6-carboxylate

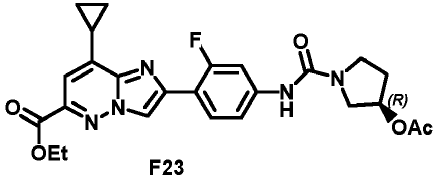

To a degassed mixture of intermediate **F22** (0.37 g; 0.808 mmol) and NaOAc (132 mg; 1.61 mmol) in EtOH (4.9 mL) and DMF (2 mL) was added PdCl₂dppf (60 mg; 81 µmοl) then the resulting mixture was stirred under 7 bar of CO in a pressure vessel reactor. The resulting mixture was stirred at 70°C for 16 h. water and EtOAc were added. The layers were separared and the aqueous layer was extracted with EtOAc (once). The combined organic layers were washed with brine, dried over MgSO₄, filtered and the solvent was removed in vacuo. The residue was purified by preparative LC (regular SiOH, 30 µm, Interchim^{®} 25 g, mobile phase gradient: from heptane/iPrOH 90/10 to 30/70) to give intermediate **F23** (263 mg, 66%).

### Intermediate F24

### (R)-8-cyclopropyl-2-(2-fluoro-4-(3-hydroxypyrrolidine-1-carboxamido)phenyl)imidazo[1,2-b]pyridazine-6-carboxylic acid

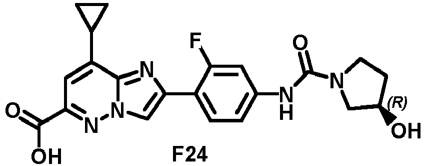

A mixture of intermediate **F23** (263 mg; 0.531 mmol) and KOH (66 mg; 1.17 mmol) in EtOH (4.6 mL) was stirred at rt for 16 h. The precipitate was filtered and dried over frit then the solid was dried by azeotrope with toluene (twice) to give 55 mg of intermediate **F24** as potassium salt. The mother liquor was evaporated to dryness and the residue was taken-up with THF and aqueous HCl 1N was added. The mixture was evaporated to dryness and the residue was dried by azeotrope with toluene (twice) to give intermediate **F24** as acid form (185 mg, purity 90%). Global yield 96%

### Synthesis of Intermediate F30

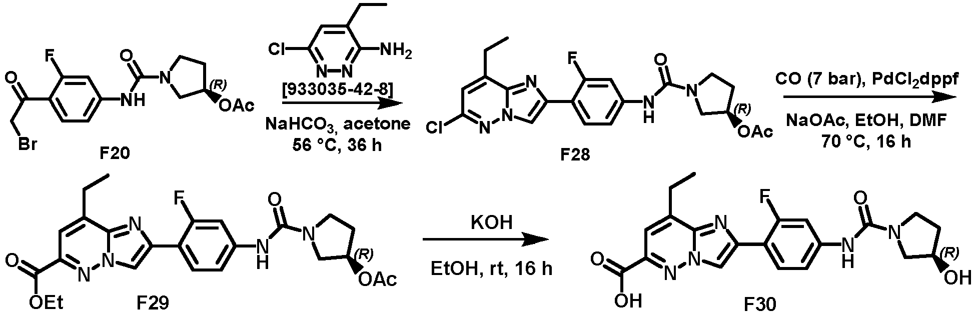

### Intermediate F28

### (R)-1-((4-(6-chloro-8-ethylimidazo[1,2-b]pyridazin-2-yl)-3-fluorophenyl)carbamoyl)pyrrolidin-3-yl acetate

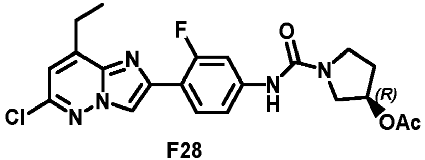

A mixture of 6-chloro-4-ethylpyridazin-3-amine [933035-42-8] (0.52 g; 3.30 mmol), intermediate **F20** (1.28 g; 3.30 mmol) and NaHCO₃ (278 mg; 3.30 mmol) in acetone (26 mL) was stirred at relux for 36 h. The mixture was evaporated to dryness and the residue was taken-up with DCM then the solid was filtered off and the mother liquor was evaporated to dryness. The residue was purified by preparative LC (regular SiOH, 30 µm, 40 g Interchim^{®}, mobile phase gradient: from heptane/*ⁱ*PrOH 98/2 to 60/40) to give intermediate **F28** (1.0 g, 68%).

### Intermediate F29

### Ethyl (R)-2-(4-(3-acetoxypyrrolidine-1-carboxamido)-2-fluorophenyl)-8-ethylimidazo[1,2-b]pyridazine-6-carboxylate

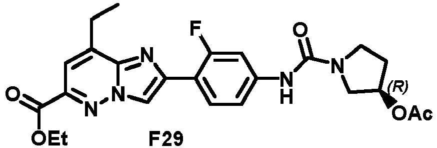

To a degassed mixture of intermediate **F28** (753 mg; 1.69 mmol) and NaOAc (202 mg; 3.38 mmol) in EtOH (10 mL) and DMF (4.4 mL) was added PdCl₂(dppf) (123 mg; 0.166 mmol) then the resulting mixture was stirred under 7 bar of CO at 70°C for 16 h. Water and EtOAc were added. The layers were separated and the aqueous layer was extracted with EtOAc (once). The combined organic layers were washed with brine, dried over MgSO₄, filtered and the solvent was removed in vacuo. The residue was purified by preparative LC (regular SiOH, 30 µm, 40 g Interchim^{®}, mobile phase gradient: from heptane/*ⁱ*PrOH 90/10 to 30/70) to give intermediate **F29** (0.50 g, 61%).

### Intermediate F30

### (R)-8-ethyl-2-(2-fluoro-4-(3-hydroxypyrrolidine-1-carboxamido)phenyl)imidazo[1,2-b]-pyridazine-6-carboxylic acid

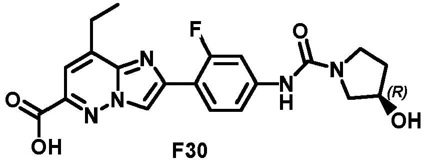

A mixture of intermediate **F29** (0.48 g; 0.993 mmol) and KOH (123 mg; 2.18 mmol) in EtOH (8.6 mL) was stirred at rt for 16 h. The mixture was evaporated to dryness. The residue was dried by azeotrope with toluene (twice) then aqueous HCl 1N and THF were added. The mixture was stirred for 5 min then was evaporated to dryness. The residue was dried by azeotrope with toluene (twice) to give 0.56 g of intermediate **F30** (0.56 g, quant, 73% purity).

### Synthesis of Intermediate F45

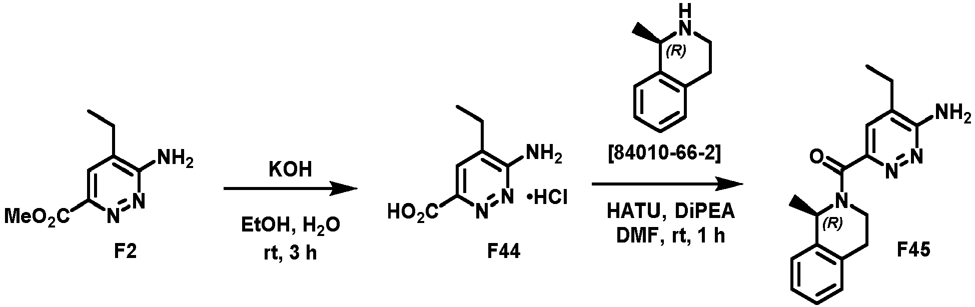

### Intermediate F44

### 6-amino-5-ethylpyridazine-3-carboxylic acid hydrochloride

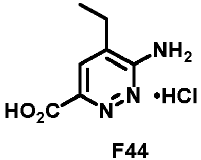

A mixture of intermediate **F2** (708 mg, 3.91 mmol) and KOH (438 mg, 7.82 mmol) in EtOH (58 mL) and water (5.8 mL) was stirred at rt for 3 h. The mixture was evaporated under vacuum then aqueous HCl 1N and THF were added. The mixture was stirred for 5 min then was evaporated to dryness to give intermediate **F44** (1.1 g, quant, 73% purity).

### Intermediate F45

### (R)-(6-amino-5-ethylpyridazin-3-yl)(1-methyl-3,4-dihydroisoquinolin-2(1H)-yl)methanone

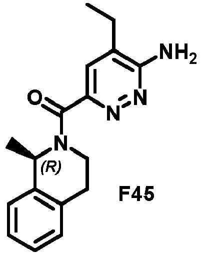

A mixture of intermediate **F44** (1.05 g, 3.77 mmol), 73% purity), (1*R*)-1-methyl-1,2,3,4-tetrahydroisoquinoline [84010-66-2] (666 mg, 4.52 mmol), HATU (2.1 g, 5.65 mmol) and DiPEA (2.0 mL) in DMF (25 mL) was stirred at rt for 1 h. Brine and EtOAc were added to the reaction mixture. The layers were separated. The aqueous layer was extracted with EtOAc (twice). The combined organic layers were washed with brine (3 times), dried over MgSO₄ and evaporated in vacuo. The residue was purified by preparative LC (irregular SiOH 15-40 µm, 80 g Grace Resolv^{™}, dry loading (celite^{®}), mobile phase gradient: from DCM/MeOH 100/00 to 95/05). The fraction containing product was collected and evaporated to dryness. The residue was triturated in MeCN, filtered and dried over frit to give intermediate **F45** (587 mg, 52%) as a white solid. The filtrate was evaporated to dryness and the residue was purified by reverse phase (spherical C18, 25 µm, 40 g YMC-ODS-25, dry loading (Celite^{®}), mobile phase gradient: from (aq. NH4HCO₃ 0.2%)/MeCN 85/15 to 45/55%). The fraction containing pure product was collected and evaporated to dryness to give intermediate **F45** (133 mg, 8%) as a white solid. Global yield (720 mg, 64%)

### Synthesis of compound 32

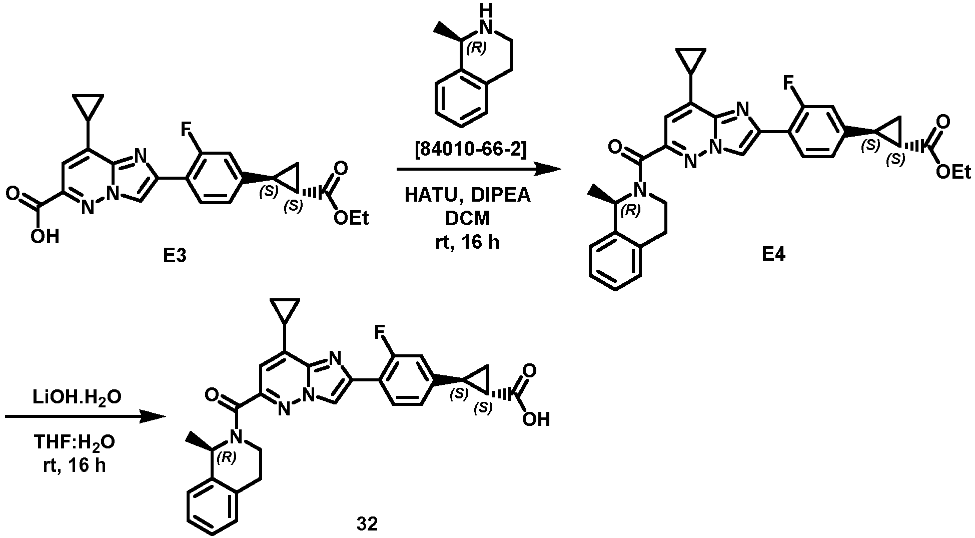

### Intermediate E4

### Ethyl (1S,2S)-2-(4-{8-cyclopropyl-6-[(1R)-1-methyl-1,2,3,4-tetrahydroisoquinoline-2-carbonyl]imidazo[1,2-b]pyridazin-2-yl}-3-fluorophenyl)cyclopropane-1-carboxylate

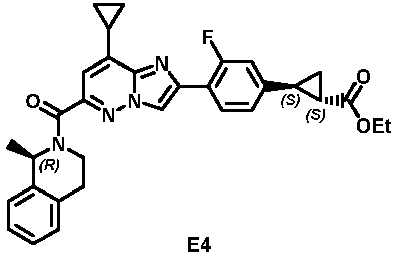

To a mixture of intermediate **E3** (0.19 g, 269 µmοl, 58% purity), DIPEA (19 µL, 1.08 mmol) and (1*R*)-1-methyl-1,2,3,4-tetrahydroisoquinoline [84010-66-2] (43.6 mg, 296 µmοl) in DCM (2.8 mL) was added HATU (153 mg, 404 µmοl). The reaction mixture was stirred at rt for 16 h. The reaction mixture was purified by preparative LC (regular SiOH, 30 µm, 12 g Interchim^{®}, mobile phase gradient: heptane / EtOAc from 100:0 to 40:60) to afford intermediate **E4** (74 mg, 51%).

### Compound 32

### (1S,2S)-2-(4-{8-Cyclopropyl-6-[(1R)-1-methyl-1,2,3,4-tetrahydroisoquinoline-2-carbonyl]imidazo[1,2-b]pyridazm-2-yl}-3-fluorophenyl)cyclopropane-1-carboxylic acid

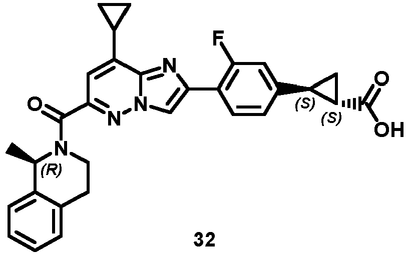

A mixture of intermediate **E4** (74.0 mg, 137 µmοl) and lithium hydroxide monohydrate (8.65 mg, 206 µmοl) in THF (1 mL) and H₂O (0.5 mL) was stirred at rt for 16 h. The reaction mixture was combined with another batch (71 mg, 132 µmοl) and diluted with EtOAc and an aqueous solution of AcOH (10% v/v). The layers were separated and the aqueous phase was extracted with EtOAc (once). The combined organic extracts were dried over MgSO₄, filtered and evaporated in vacuo. The crude mixture was purified by preparative LC (regular SiOH, 30 µm, 12 g Interchim^{®}, mobile phase gradient: heptane / (EtOAc/AcOH 97.5:2.5) from 95:5 to 50:50) to give compound **32** (52 mg, 74%). The product was combined with another fraction of compound **32** (58 mg). The residue was solubilized in EtOAc and heptane was added until precipitation. The solid was filtered off and dried under high vacuum at 60°C for 16 h to give compound **32** (70 mg).

### Synthesis of compound 33

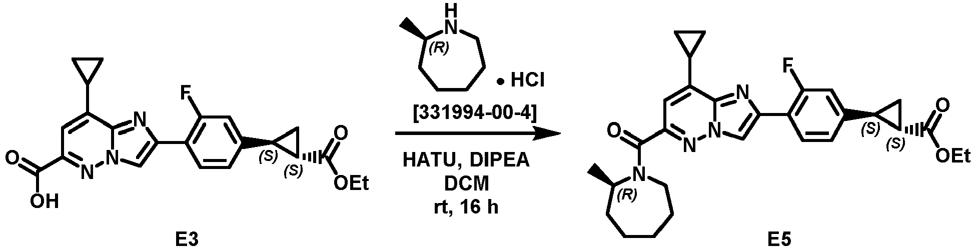

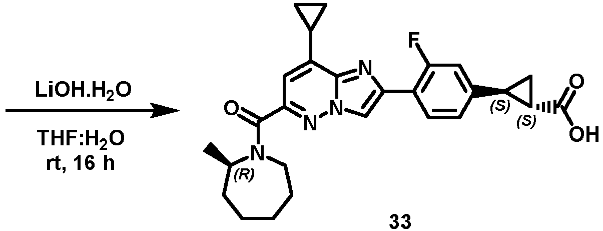

### Intermediate E5

### Ethyl (1S,2S)-2-(4-{8-cyclopropyl-6-[(2R)-2-methylazepane-1-carbonyl]imidazo[1,2-b]pyridazin-2-yl}-3-fluorophenyl)cyclopropane-1-carboxylate

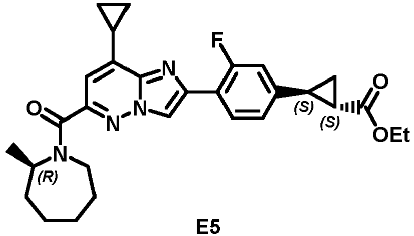

To a mixture of intermediate **E3** (170 mg, 241 µmol, 58% purity), DIPEA (0.16 mL, 0.96 mmol) and (2*R*)-2-methylazepane hydrochloride [331994-00-4] (39.6 mg, 0.27 mmol) in DCM (2.5 mL) was added HATU (137 mg, 0.36 mmol). The reaction mixture was stirred at rt for 16 h. The reaction mixture was purified by preparative LC (regular SiOH, 30 µm, 25 g Interchim^{®}, mobile phase gradient: heptane / EtOAc from 80:20 to 0:100) to afford intermediate **E5** (60 mg, 49%).

### Compound 33

### (1S,2S)-2-(4-{8-Cyclopropyl-6-[(2R)-2-methylazepane-1-carbonyl]imidazo[1 ,2-b]pyridazin-2-yl}-3-fluorophenyl)cyclopropane-1-carboxylic acid

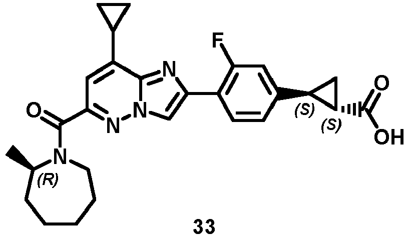

A mixture of intermediate **E5** (60.0 mg, 119 µmοl) and lithium hydroxide monohydrate (7.49 mg, 0.18 mmol) in THF (0.9 mL) and H₂O (0.5 mL) was stirred at rt for 16 h. the reaction mixture was diluted with EtOAc and an aqueous solution of AcOH (10% v/v) was added. The layers were separated and the aqueous phase was extracted with EtOAc (once). The combined organic extracts were dried over MgSO₄, filtered and evaporated in vacuo. The crude mixture was purified by preparative LC (regular SiOH, 30 µm, 12 g Interchim^{®}, mobile phase gradient: heptane / (EtOAc + 2.5% AcOH) from 100:0 to 50:50). The residue (52 mg) was solubilized in EtOAc and heptane was added until precipitation. The solid was filtered off and dried under high vacuum at 60°C for 16 h to give compound **33** (30 mg, 53%) as a white solid.

### Compound 34

### (2-(4-((3 S,4S)-3,4-dihydroxypyrrolidin-1-yl)-2-fluorophenyl)-8-ethylimidazo[1 ,2-b]pyridazin-6-yl)((R)-1-methyl-3,4-dihydroisoquinolin-2(1H)-yl)methanone

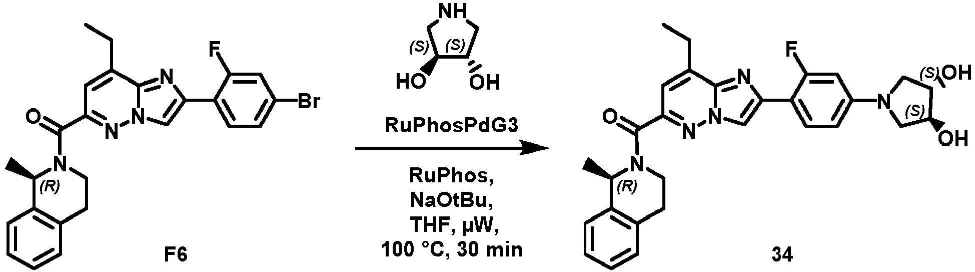

In a sealed tube, a mixture of **F6** (300 mg, 0.608 mmol), NaO*^{t}*Bu (129 mg, 1.34 mmol) and (3S, 4S)-Dihydroxypyrrolidine (75 mg, 0.73 mmol) in THF (7.2 mL) was degazed with N₂ for 10 min. RuPhos Pd G3 (51 mg, 0.061 mmol) and RuPhos (28 mg, 0.061 mmol) were added and the reaction mixture was purged with N₂. The mixture was heated at 100°C using a single mode microwave (Biotage initiator60^{®}) with a power output ranging from 0 to 400 W for 30 min. Water and EtOAc were added to the reaction mixture. The layers were separated. The aqueous layer was extracted twice with EtOAc. The combined organic layers were washed with brine, dried over MgSO₄ and evaporated in vacuo. The crude mixture was purified by preparative LC (irregular SiOH 15-40 µm, 24 g Grace Resolv^{™}, mobile phase gradient: from DCM 99%, *ⁱ*PrOH 1% to DCM 85%, *ⁱ*PrOH 15%). The fractions containing product were evaporated under vacuum to give a residue which was taken-up in MeCN and evaporated under vacuum twice, then it was suspended in MeCN (~2 mL in total) and heated at reflux until complete solubilization (oil bath 85°C). Then the heating source was stopped (the flask was kept in the oil bath during the crystallization with a gentle stirring allowing slow cooling) for 4 h. The suspension was cooled down to rt, filtered over glass frit, washed with MeCN (2 × 2 mL), dried over glass frit. The solid was dried under vacuum at 50°C for 18 h to give compound **34** (68 mg, 22%).

### Compound 35

### (R)-(8-ethyl-2-(2-fluoro-4-(3-hydroxyazetidin-1-yl)phenyl)imidazo[1,2-b]pyridazin-6-yl)(1-methyl-3,4-dihydroisoquinolin-2(1H)-yl)methanone

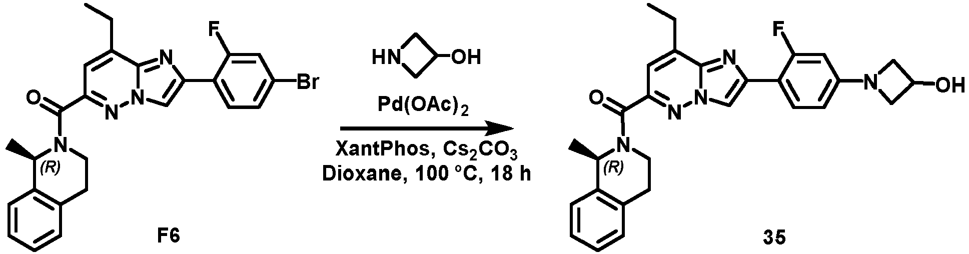

A mixture of intermediate **F6** (200 mg, 0.405 mmol) 3-Hydroxyazetidine hydrochloride (53 mg, 0.49 mmol) and Cs₂CO₃ (396 mg, 1.22 mmol) was charged in a sealed tube and purged with N₂. Dioxane (8.4 mL) was added and the mixture was degased with N₂, then Pd(OAc)₂ (9.1 mg, 0.041 mmol) and Xantphos (23 mg, 0.041 mmol) were added. The reaction mixture was stirred and heated at 100°C for 18 h. Water and EtOAc were added to the reaction mixture. The layers were separated. The aqueous layer was extracted twice with EtOAc. The combined organic layers were washed with brine, dried over MgSO₄ and evaporated in vacuo. The crude mixture was purified by preparative LC (irregular SiOH 15-40 µm, 24 g Grace Resolv^{™}, mobile phase gradient: from heptane 60%, EtOAc 40% to Heptane 0%, EtOAc 100%). The fractions containing product were evaporated under vacuum to give a residue. The residue was taken-up in MeCN and evaporated under vacuum twice and the sample was dried under vacuum at 50°C for 16 h to give compound **35** (140 mg, 71%).

### Compound 36

### (R,E)-3-(4-(8-ethyl-6-(1-methyl-1,2,3,4-tetrahydroisoquinoline-2-carbonyl)imidazo[1,2-b]pyridazin-2-yl)-3-fluorophenyl)acrylic acid

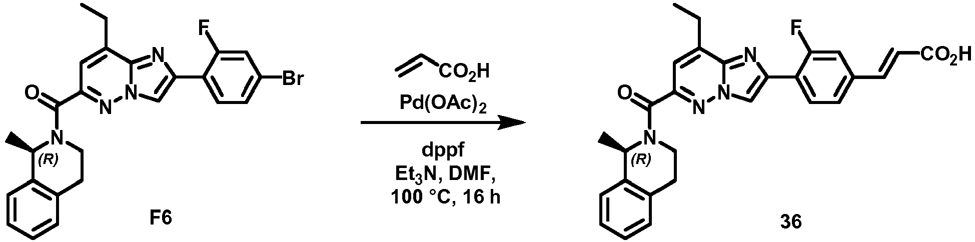

In a sealed tube, a mixture of intermediate **F6** (150 mg, 0.304 mmol), Acrylic acid (20.8 µL, 0.304 mmol) and TEA (127 µL, 0.912 mmol) in DMF (3.6 mL) was degazed with N₂ for 10 min. Pd(OAc)₂ (14 mg, 0.061 mmol) and dppf (34 mg, 0.061 mmol) were added and the reaction mixture was purged with N₂. The mixture was heated at 100°C for 16 h. Water and EtOAc were added to the reaction mixture. The layers were separated. The aqueous layer was extracted twice with EtOAc. The combined organic layers were washed with brine, dried over MgSO₄ and evaporated in vacuo. The crude mixture was purified by preparative LC (irregular SiOH 15-40 µm, 24 g Grace Resolv^{™}, mobile phase gradient: from heptane 75%, EtOAc 25% to Heptane 25%, EtOAc 75%). The fractions containing product were evaporated under vacuum. The residue was taken-up in MeCN and evaporated under vacuum twice, then it was suspended in MeCN (~2 mL in total) and heated at reflux until complete solubilization (oil bath 85°C). Then the heating source was stopped (the flask was kept in the oil bath during the crystallization with a gentle stirring allowing slow cooling) for 4 h. The suspension was cooled down to rt, filtered over glass frit, washed with MeCN (2 × 2 mL), dried over glass frit. The solid was dried under vacuum at 50°C for 18 h to give compound **36** (52 mg, 35%).

### Compound 37

### (R)-N-(4-(8-cyclopropyl-6-((R)-1-methyl-1,2,3,4-tetrahydroisoquinoline-2-carbonyl)imidazo [1 ,2-b]pyridazin-2-yl)-3-fluorophenyl)-3-hydroxypyrrolidine-1 -carboxamide

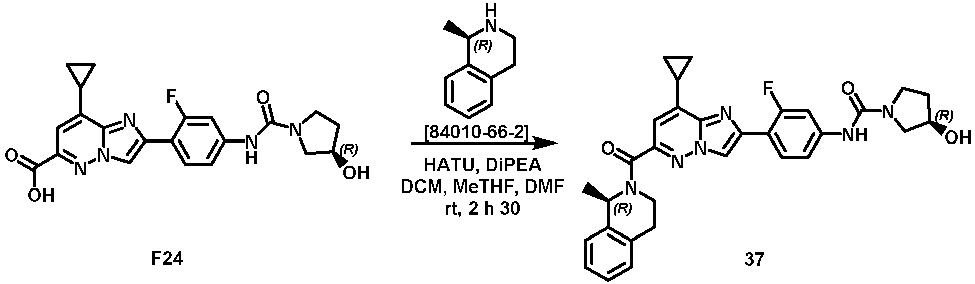

To a mixture of intermediate **F24** (149 mg; 0.315 mmol, 90% purity), (1*R*)-1-methyl-1,2,3,4-tetrahydroisoquinoline [84010-66-2] (58 µL; 0.38 mmol), DiPEA (0.27 mL; 1.6 mmol) in Me-THF (2 mL), DCM (1 mL) then DMF (1 mL) was added HATU (180 mg; 0.473 mmol) at rt. The resulting mixture was stirred at rt for 2h30. Water and EtOAc were added. The layers were separated and the aqueous layer was extracted with EtOAc (once). The combined organic layers were washed with brine, dried over MgSO₄, filtered and the solvent was removed in vacuo. The residue was purified by preparative LC (regular SiOH, 30 µm, Interchim^{®} 12 g, mobile phase gradient: from heptane/*ⁱ*PrOH 90/10 to 20/80). The pure fraction was collected and evaporated to dryness. The residue was taken-up with MeCN then the solid crystallized after few minutes. The solid was filtered and dried under high vacuum for 3 h at 50°C to give compound **37** (75 mg, 43%) as an off-white solid.

### Compound 38

### (R)-N-(4-(8-cyclopropyl-6-((R^{∗})-4-methyl-4,5,6,7-tetrahydrothieno[3,2-c]pyridine-5-carbonyl)imidazo[1,2-b]pyridazin-2-yl)-3-fluorophenyl)-3-hydroxypyrrolidine-1-carboxamide

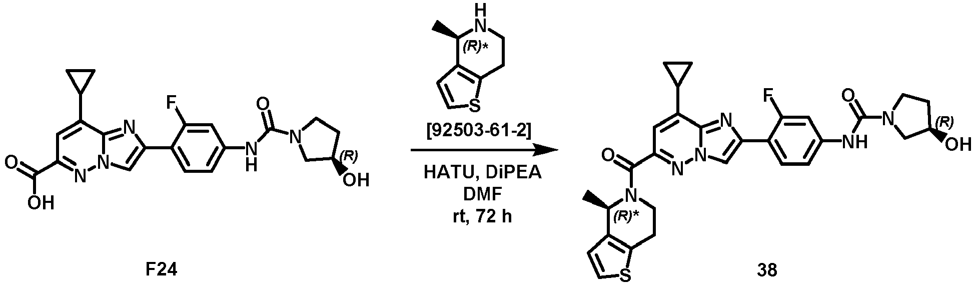

To a mixture of intermediate **F24** (48 mg; 0.102 mmol), intermediate **F24** as potassium salt (55 mg; 0.119 mmol), DiPEA (0.191 mL; 1.11 mmol), 4-(^{∗}*R*)-methyl-4,5,6,7-tetrahydrothieno[3,2-c]pyridine [92503-61-2] (41 mg; 0.27 mmol) in DMF (2 mL) was added HATU (126 mg; 0.331 mmol) at rt. The resulting mixture was stirred at rt for 72 h. Water and EtOAc were added. The layers were separated and the aqueous layer was extracted with EtOAc (once). The combined organic layers were washed with brine, dried over MgSO₄, filtered and the solvent was removed in vacuo. The residue was purified by preparative LC (regular SiOH, 30 µm, Interchim^{®} 12 g, mobile phase gradient: from heptane/*ⁱ*PrOH 75/25 to 20/80). The pure fraction was collected and evaporated to dryness. The residue was taken-up with MeCN then the solid crystallized after few minutes. The solid was filtered and dried over frit then under high vacuum for 3 h at 50°C to give compound **38** (54 mg, 44%) as an off-white solid.

### Compound 39

### (R)-N-(4-(8-ethyl-6-((R)-1-methyl-1,2,3,4-tetrahydroisoquinoline-2-carbonyl)imidazo[1,2-b]pyridazin-2-yl)-3-fluorophenyl)-3-hydroxypyrrolidine-1-carboxamide

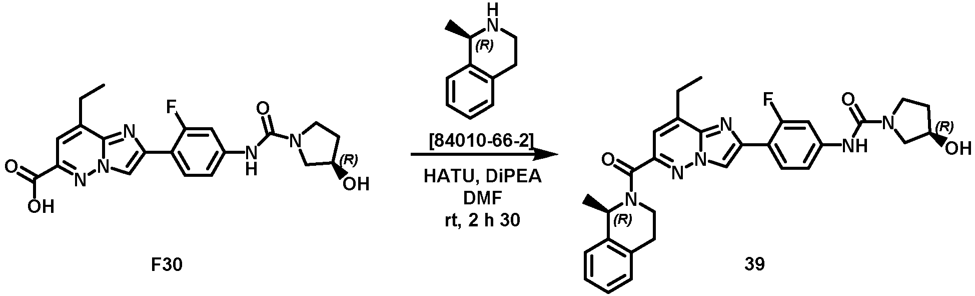

To a mixture of intermediate **F30** (100 mg; 0.177 mmol), (1*R*)-1-methyl-1,2,3,4-tetrahydroisoquinoline [84010-66-2] (32 µL; 0.21 mmol), DiPEA (152 µL; 0.883 mmol) in DMF (1 mL) was added HATU (101 mg; 0.265 mmol) at rt. The resulting mixture was stirred at rt for 2h30. Water and EtOAc were added. The layers were separated and the aqueous layer was extracted with EtOAc (once). The combined organic layers were washed with brine, dried over MgSO₄, filtered and the solvent was removed in vacuo. The residue was purified by preparative LC (regular SiOH, 30 µm, Interchim^{®} 12 g, mobile phase gradient: from heptane/*ⁱ*PrOH 90/10 to 20/80). The pure fraction was collected and evaporated to dryness. The residue was taken-up with MeCN then the solid crystallized after few minutes. The solid was filtered off and dried over frit then under high vacuum for 72 h at 50°C to give compound **39** as a yellow solid (41 mg, 43%).

### Compound 40

### (R)-N-(4-(8-ethyl-6-((R^{∗})-2-fluoro-4-methyl-4,5,6,7-tetrahydrothieno[3,2-c]pyridine-5-carbonyl)imidazo[1,2-b]pyridazin-2-yl)-3-fluorophenyl)-3-hydroxypyrrolidine-1 -carboxamide

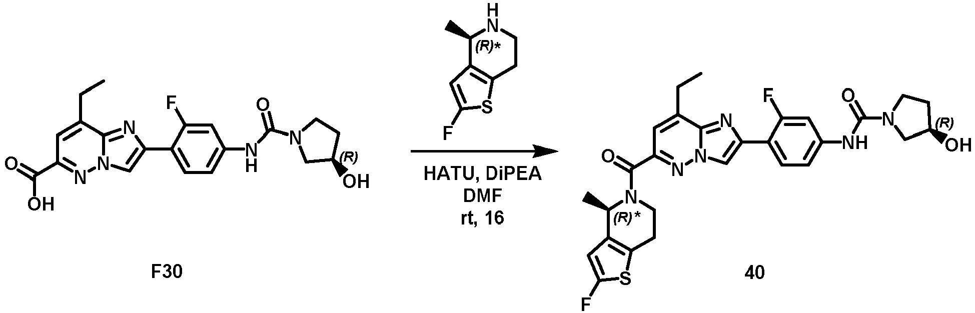

To a mixture of intermediate **F30** (110 mg; 0.194 mmol), DiPEA (0.167 mL; 0.971 mmol) and (R^{∗})-2-fluoro-4-methyl-4,5,6,7-tetrahydrothieno[3,2-c]pyridine (44 mg; 0.214 mmol) in DMF (2 mL) was added HATU (111 mg; 0.291 mmol) at rt. The resulting mixture was stirred at rt for 16 h. Water and EtOAc were added. The layers were separated and the aqueous layer was extracted with EtOAc (once). The combined organic layers were washed with brine, dried over MgSO₄, filtered and the solvent was removed in vacuo. The residue was purified by preparative LC (regular SiOH, 30 µm, Interchim^{®} 12 g, mobile phase gradient: from heptane/*ⁱ*PrOH 80/20 to 20/80) The pure fraction was collected and evaporated to dryness. The residue was taken-up with MeCN then the solid was crystallized after few minutes. The solid was filtered and dried over frit then under high vacuum for 16 h at 50°C to give compound **40** as a yellow solid (45 mg, 41%).

### Compound 41

### (R)-N-(4-(8-ethyl-6-((R^{∗})-7-fluoro-1-methyl-1,2,3,4-tetrahydroisoquinoline-2-carbonyl)-imidazo[1,2-b]pyridazin-2-yl)-3-fluorophenyl)-3-hydroxypyrrolidine-1-carboxamide

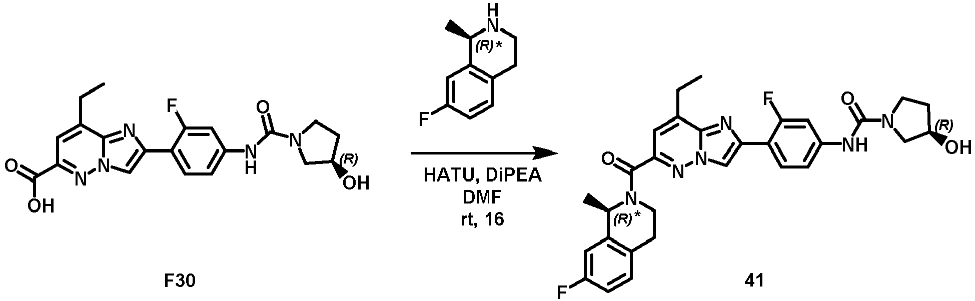

To a mixture of intermediate **F30** (103 mg; 0.182 mmol), DiPEA (157 µL; 0.909 mmol) and (R^{∗})-7-fluoro-1-methyl-1,2,3,4-tetrahydroisoquinoline (43 mg; 0.21 mmol) in DMF (2 mL) was added HATU (104 mg; 0.273 mmol) at rt. The resulting mixture was stirred at rt for 2h30. Water and EtOAc were added. The layers were separated and the aqueous layer was extracted with EtOAc (once). The combined organic layers were washed with brine, dried over MgSO₄, filtered and the solvent was removed in vacuo. The residue was purified by preparative LC (regular SiOH, 30 µm, Interchim^{®} 12 g, mobile phase gradient: from heptane/*ⁱ*PrOH 90/10 to 20/80) The pure fraction was collected and evaporated to dryness. The residue was purified via Reverse phase (Stationary phase: YMC-actus Triart C18 10µm 30^{∗} 150mm, Mobile phase: Gradient from 65% aq. NH₄HCO₃ 0.2%, 35% MeCN to 45% aq. NH₄HCO₃ 0.2%, 55% MeCN). The pure fraction was collected and evaporated to dryness. The residue was crystallized from MeCN. The solid was filtered and dried over frit then under high vacuum at 60°C for 16 h to give compound **41** (16 mg, 16%).

### Compound 42

### N,8-diethyl-2-(2-fluoro-4-((R)-3-hydroxypyrrolidine-1-carboxamido)phenyl)-N-(3-methylbutan-2-yl)imidazo[1,2-b]pyridazine-6-carboxamide

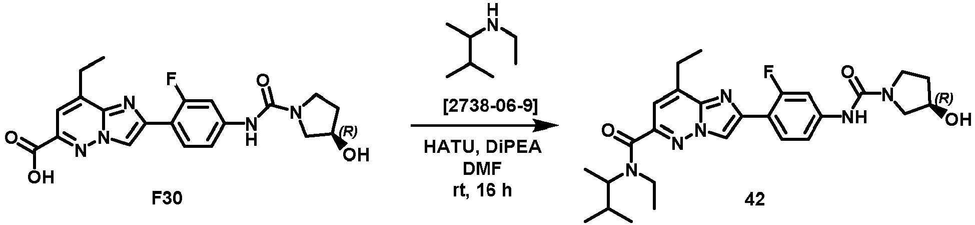

To a mixture of intermediate **F30** (105 mg; 0.185 mmol), DiPEA (0.16 mL; 0.93 mmol), N-ethyl-3-methylbutan-2-amine [2738-06-9] (31 µL; 0.20 mmol) in DMF (2 mL) was added HATU (106 mg; 0.278 mmol) at rt. The resulting mixture was stirred at rt for 16 h. Water and EtOAc were added. The layers were separated and the aqueous layer was extracted with EtOAc (once). The combined organic layers were washed with brine, dried over MgSO₄, filtered and the solvent was removed in vacuo. The residue was purified by preparative LC (regular SiOH, 30 µm, Interchim^{®} 12 g, mobile phase gradient: from heptane/*ⁱ*PrOH 80/20 to 20/80. The pure fraction was collected and evaporated to dryness. The residue was purified again by preparative LC (regular SiOH, 30 µm, 4 g Interchim^{®}, mobile phase gradient: from DCM/(DCM/*ⁱ*PrOH 60/40) 99/1 to 50/50). The pure fraction was collected and evaporated to dryness. The residue was taken-up with MeCN (twice) and evaporated to dryness to give compound **42** (25 mg, 26%).

### Synthesis of compound 43

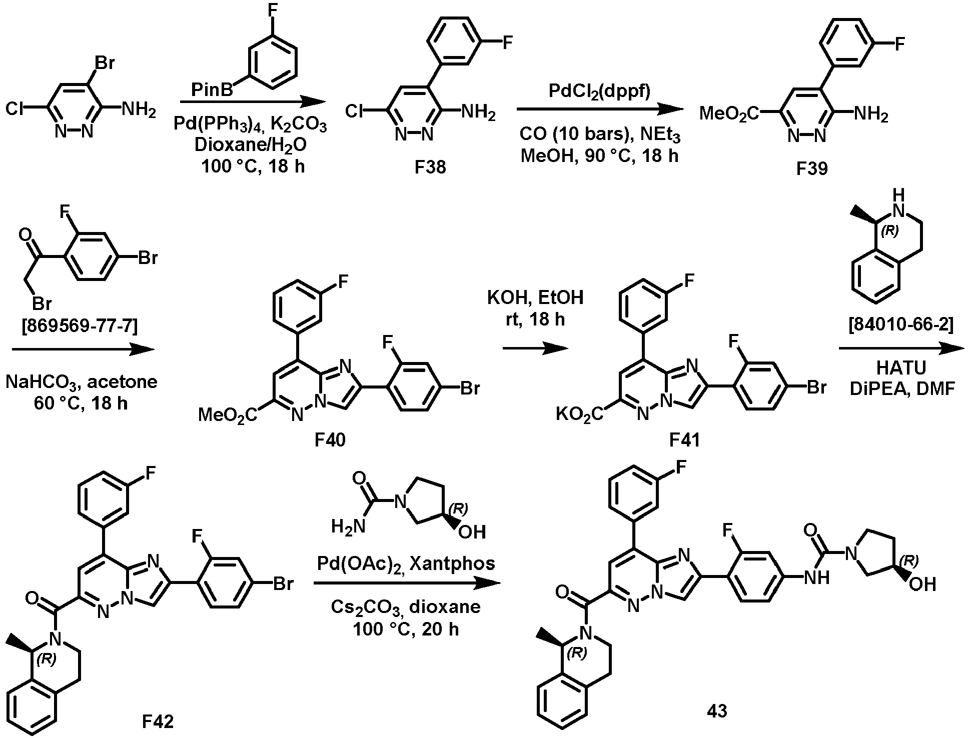

### Intermediate F38

### 6-chloro-4-(3-fluorophenyl)pyridazin-3-amine

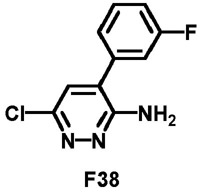

To a degassed mixture of 3-amino-4-bromo-6-chloropyridazine (500 mg, 2.40 mmol), 3-fluorophenylboronic acid pinacol ester (586 mg, 2.64 mmol) and K₂CO₃ (663 mg, 4.80 mmol) in water (3 mL) and dioxane (30 mL) was added Pd(PPh₃)₄ (277 mg, 0.240 mmol), and the mixture was stirred at 100°C for 18 h. EtOAc and water were added. The layers were separated and the aqueous layer was extracted with EtOAc (once). The combined organic layers were dried over MgSO₄, filtered and the solvent was removed in vacuo. The residue was purified by preparative LC (irregular SiOH 15-40 µm, 24 g Grace Resolv^{™}, dry loading (celite^{®}), mobile phase gradient: from Heptane/EtOAc 90/10 to 50/50). The pure fraction was collected and evaporated to dryness to give intermediate **F38** (338 mg, 63%).

### Intermediate F39

### Methyl 6-amino-5 -(3 -fluorophenyl)pyridazine-3 -carboxylate

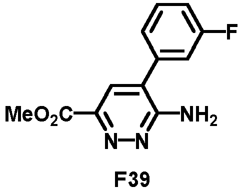

In a stainless-steel bomb, to a degassed mixture of intermediate **F38** (1.44 g; 6.44 mmol) and TEA (2.2 mL; 15.8 mmol) in MeOH (40 mL) was added PdCl₂dppf (344 mg; 0.470 mmol). The resulting mixture was stirred under 10 bars of CO for 18 h at 90°C. The mixture was cooled to rt and evaporated to dryness to give a purple residue which was triturated with MeOH, filtered off and dried on frit to afford intermediate **F39** as a grey solid (1.06 g, 67%).

### Intermediate F40

### Methyl 2-(4-bromo-2-fluorophenyl)-8-(3-fluorophenyl)imidazo[1,2-b]pyridazine-6-carboxylate

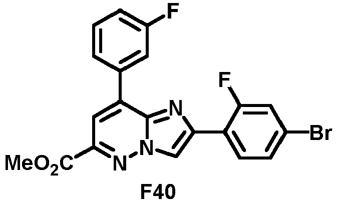

A mixture of intermediate **F39** (954 mg; 3.86 mmol), 4-bromo-2-fluorophenacyl bromide [869569-77-7] (1.14 g; 3.86 mmol) and NaHCO₃ (324 mg; 3.86 mmol) in acetone (30 mL) was stirred at 60°C for 18 h. The mixture was filtered and the resulting solid was dried on frit to give intermediate **F40** as a yellow solid (1.8 g, Quant.).

### Intermediate F41

### Potassium 2-(4-bromo-2-fluorophenyl)-8-(3-fluorophenyl)imidazo[1,2-b]pyridazine-6-carboxylate

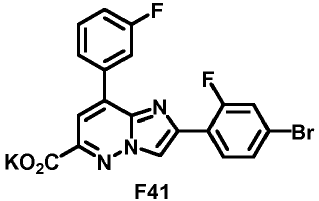

A solution of intermediate **F40** (1.8 g; 4.1 mmol) and KOH (682 mg; 12.2 mmol) in EtOH (50 mL) was stirred at rt for 2 h. The mixture was filtered, and the solid was washed with Et₂O and MeOH. The solid was dried on frit to give intermediate **F41** as a yellow solid (1.6 g, 84%).

### Intermediate F42

### (R)-(2-(4-bromo-2-fluorophenyl)-8-(3-fluorophenyl)imidazo[1,2-b]pyridazin-6-yl)(1-methyl-3,4-dihydroisoquinolin-2(1H)-yl)methanone

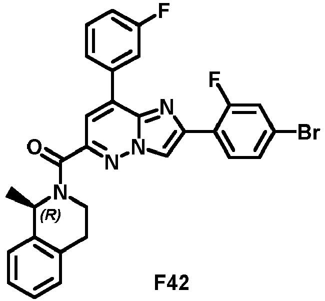

A mixture of intermediate **F41** (250 mg; 0.534 mmol), 2-bromo-1-(4-bromo-2-fluorophenyl)ethan-1-one [84010-66-2] (94 mg; 0.64 mmol), HATU (304 mg; 0.801 mmol) and DiPEA (0.28 mL; 1.6 mmol) in DMF (6.5 mL) was stirred at rt for 20 h. Brine and EtOAc were added to the reaction mixture. The layers were separated. The aqueous layer was extracted twice with EtOAc. The combined organic layers were washed with brine (3 times), dried over MgSO₄ and evaporated in vacuo. The residue was purified by preparative LC (regular SiOH, 15-40 µm, Grace Resolv^{™} 12 g, mobile phase gradient: from heptane/EtOAc 90/10 to 70/30) to give intermediate **F42** as a yellow foam (237 mg, 79%).

### Compound 43

### (R)-N-(3-fluoro-4-(8-(3-fluorophenyl)-6-((R)-1-methyl-1,2,3,4-tetrahydroisoquinoline-2-carbonyl)imidazo[1,2-b]pyridazin-2-yl)phenyl)-3-hydroxypyrrolidine-1-carboxamide

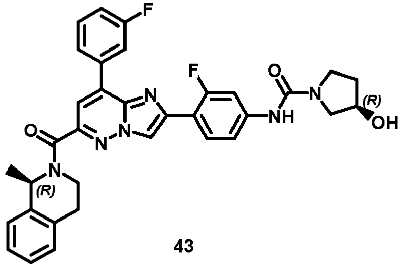

In a schlenk tube, a mixture of intermediate **F42** (217 mg; 0.388 mmol), (3R)-3-hydroxypyrrolidine-1-carboxamide (136 mg; 0.970 mmol), Cs₂CO₃ (632 mg; 1.94 mmol) was purged with N₂. 1,4 dioxane (7.9 mL) was added and the mixture was degassed with N₂, then Pd(OAc)₂ (9 mg; 0.04 mmol) and XantPhos (22 mg; 0.039 mmol) were added. The reaction mixture was stirred and heated at 100°C for 20 h. The reaction mixture was poured out into water and extracted with EtOAc (twice). The combined organic layers were dried over MgSO₄, filtered and evaporated till dryness. The residue was purified by preparative LC (irregular SiOH 15-40 µm, 12 g Grace Resolv^{™}, mobile phase gradient: from DCM/MeOH 100/0 to 95/5) to give a yellow gum which was crystallized with MeOH, filtered and dried under high vacuum at 50°C for 20 h to give compound **43** as a yellow solid (145 mg, 61%).

### Synthesis of Compound 44

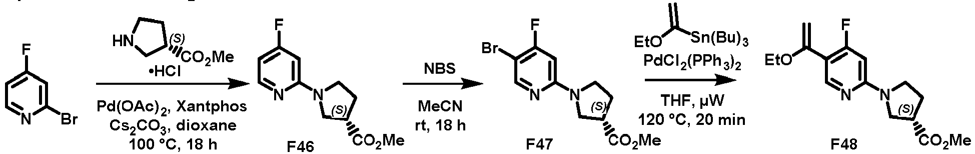

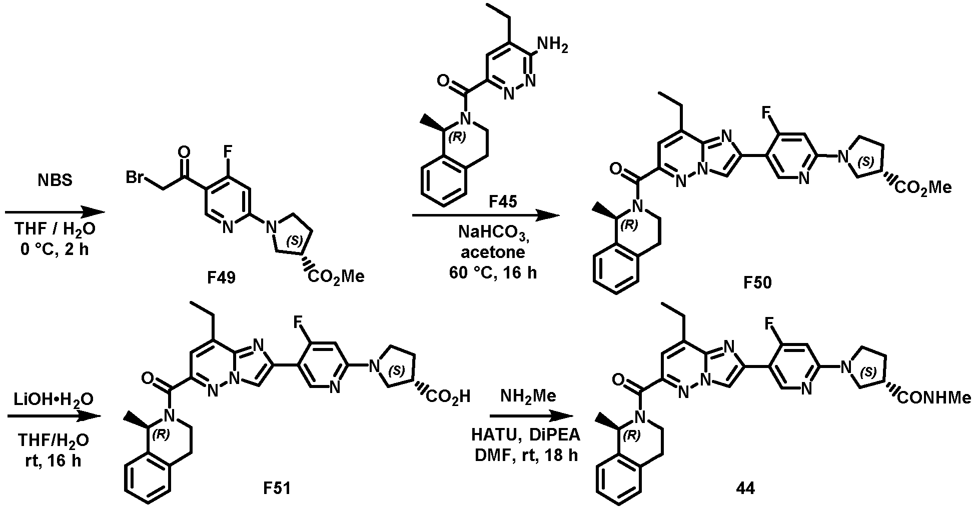

### Intermediate F46

### Methyl (S)-1-(4-fluoropyridin-2-yl)pyrrolidine-3-carboxylate

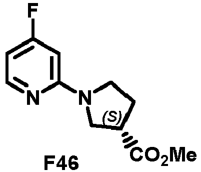

A mixture of 2-bromo-4-fluoropyridine (900 mg; 5.12 mmol), methyl (S)-pyrrodine-3-carboxylate (846 mg; 5.12 mmol) and Cs₂CO₃ (5.0 g; 15.3 mmol) was charged in a sealed tube and purged with N₂. Dioxane (36 mL) was added and the mixture was degassed with N₂, then Pd(OAc)₂ (104 mg; 0.52 mmol) and XantPhos (296 mg; 0.52 mmol) were added. The reaction mixture was stirred and heated at 100°C for 18 h. The reaction mixture was poured out into water and extracted with EtOAc. The organic layer was washed with brine, dried over MgSO₄ and evaporated till dryness. The residue was purified by preparative LC (irregular SiOH, 15-40 µm, 40 g Grace Resolv^{™}, mobile phase gradient: from heptane/EtOAc 90/10 to 40/60). The fraction containing pure product was collected and evaporated to dryness to give intermediate **F46** as a colorless oil (340 mg, 40%).

### Intermediate F47

### Methyl (S)-1-(5-bromo-4-fluoropyridin-2-yl)pyrrolidine-3-carboxylate

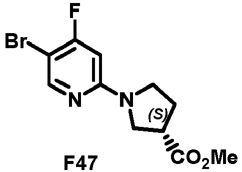

Intermediate **F46** (340 mg, 1.52 mmol) and NBS (270 mg, 1.52 mmol) were stirred in MeCN (15 mL) at rt for 18 h. The solvent was removed under vacuo then the residue was purified by preparative LC (irregular SiOH 15-40 µm, 12 g Grace Resolv^{™}, mobile phase gradient: from heptane / EtOAc from 99:1 to 60:40). The fraction containing pure product was collected and evaporated to dryness to give intermediate **F47** as a white solid (446 mg, 97%).

### Intermediate F48

### Methyl (3S)-1-[5 -(1-ethoxyethenyl)-4-fluoropyridin-2-yl]pyrrolidine-3 -carboxylate

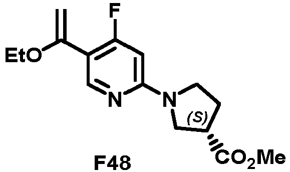

A solution of intermediate **F47** (0.45 g, 1.5 mmol) in THF (10 mL) was purged under N₂ for 10 min. To the solution tributyl(1-ethoxyvinyl)tin (1.0 mL, 2.9 mmol) and PdCl₂(PPh₃)₂ (0.10 g, 0.15 mmol) were added. The mixture was heated at 120°C using a singlemode microwave (Anton Paar Monowave^{®} 300) with a power output ranging from 0 to 850 W for 20 min. The mixture was evaporated to dryness and the residue was purified by preparative LC (irregular SiOH 15-40 µm, 24 g Grace Resolv^{™}, mobile phase gradient: from heptane / EtOAc from 99:1 to 60:40). The fraction containing pure product was collected and evaporated to dryness to give intermediate **F48** as a yellow oil (270 mg, 62%).

### Intermediate F49

### Methyl (3S)-1-[5-(bromoacetyl)-4-fluoropyridin-2-yl]pyrrolidine-3-carboxylate

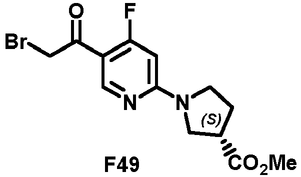

A mixture of intermediate **F48** (0.27 g; 0.92 mmol) and NBS (0.16 g; 0.92 mmol) in THF (4.4 mL) and water (0.9 mL) were stirred at 0°C for 2 h. EtOAc was added. The organic layer was washed with water and brine (twice), dried over MgSO₄, concentrated under reduce pressure. The residue was purified by preparative LC (irregular SiOH 15-40 µm, 24 g Grace Resolv^{™}, mobile phase gradient: from heptane / EtOAc from 99:1 to 60:4). The fraction containing pure product was collected and evaporated to dryness to give intermediate **F49** as a yellow oil (47 mg, 15%).

### Intermediate F50

### Methyl (3S)-1-(5-{8-ethyl-6-[(1R)-1-methyl-3,4-dihydroisoquinoline-2(1H)-carbonyl]imidazo[1,2-b]pyridazin-2-yl}-4-fluoropyridin-2-yl)pyrrolidine-3-carboxylate

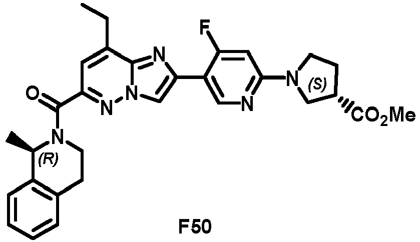

A mixture of intermediate **F45** (40 mg, 0.14 mmol), **F49** (47 mg, 0.14 mmol) and NaHCO₃ (11 mg, 0.14 mmol) in acetone (1.0 mL) was stirred at reflux for 16 h. The mixture was evaporated to dryness. The residue was purified by preparative LC (irregular SiOH, 15-40 µm, 24 g Grace Resolv^{™}, mobile phase gradient: from heptane/EtOAc 50/50 to 0/100). The fraction containing pure product was collected and evaporated to dryness to give intermediate **F50** as a yellow oil (60 mg, 81%).

### Intermediate F51

### (3S)-1-(5-{8-ethyl-6-[(1R)-1-methyl-3,4-dihydroisoquinoline-2(1H)-carbonyl]imidazo[1,2-b]pyridazin-2-yl}-4-fluoropyridin-2-yl)pyrrolidine-3-carboxylic acid

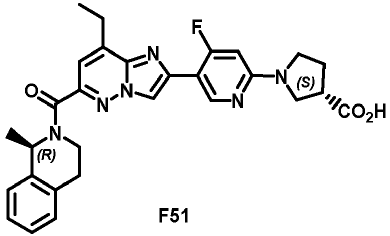

LiOH•H₂O (14 mg; 0.33 mmol) was added to a solution of intermediate **F50** (60 mg; 0.11 mmol) in THF (0.9 mL) and water (0.4 mL) and the reaction mixture was stirred at rt for 16 h. Aqueous sat. NaCl solution and an aqueous solution of KHSO₄ 10% were added. The aqueous layer was extracted (twice) with EtOAc and the combined organic layers were washed with water, dried over MgSO₄, filtered and concentrated to give intermediate **F51** as a yellowish gum (58 mg, 99%).

### Compound 44

### (3S)-1-(5-{8-ethyl-6-[(1R)-1-methyl-3,4-dihydroisoquinoline-2(1H)-carbonyl]imidazo[1,2-b]pyridazin-2-yl}-4-fluoropyridin-2-yl)-N-methylpyrrolidine-3-carboxamide

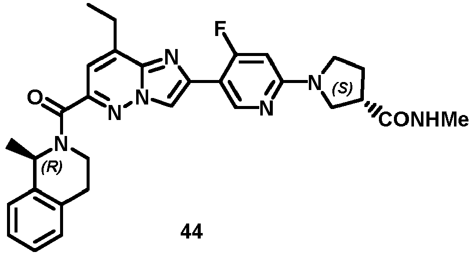

A mixture of intermediate **F51** (58 mg; 110 µmol), HATU (63 mg; 165 µmol) and DiPEA (57 µL; 0.33 mmol) in DMF (3 mL) was stirred at rt for 1 h. Methylamine 40% in water (47 µL; 0.55 mmol) was then added and the reaction mixture was stirred at rt for 18 h. Water and EtOAc were added to the reaction mixture. The layers were separated. The organic layer was washed (twice) with aq NaHCO₃ (1%), dried over MgSO₄ and evaporated in vacuo. The residue was purified by preparative LC (Irregular SiOH, 15-40 µm, 40 g Grace Resolv^{™}, mobile phase gradient: from DCM/*ⁱ*PrOH 100/0 to 75/25). The fractions containing product were evaporated under vacuum. The residue was crystallized from MeCN (2 mL) at rt and the suspension was filtered and dried over frit then under high vacuum (60°C, 16 h) to give compound **44** as yellow solid (27 mg, 45%).

### Synthesis of compound 45

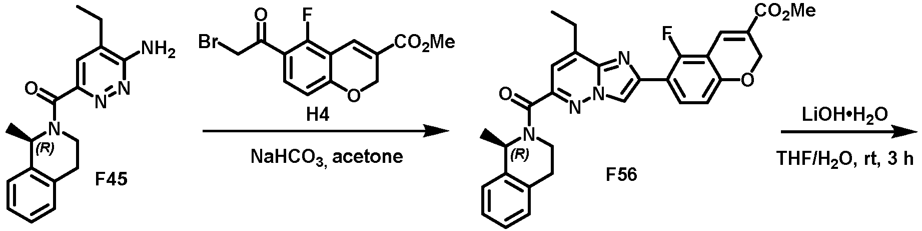

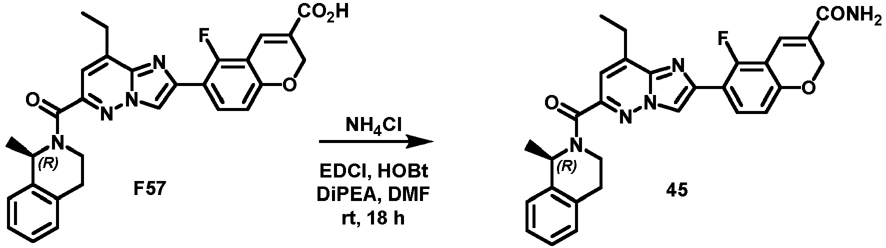

### Intermediate F56

### Methyl (R)-6-(8-ethyl-6-(1-methyl-1,2,3,4-tetrahydroisoquinoline-2-carbonyl)imidazo[1,2-b]pyridazin-2-yl)-5-fluoro-2H-chromene-3-carboxylate

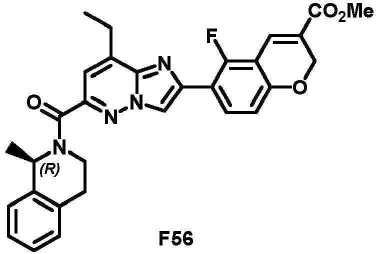

A mixture of intermediate **F45** (276 mg, 0.93 mmol), intermediate **H4** (340 mg, 0.93 mmol) and NaHCO₃ (78 mg, 0.93 mmol) in acetone (7.3 mL) was stirred at reflux for 16 h. The mixture was evaporated to dryness and the residue was taken-up with MeCN then the solid was filtered off to give intermediate **F56** (534 mg, 90%).

### Intermediate F57

### (R)-6-(8-ethyl-6-(1-methyl-1,2,3,4-tetrahydroisoquinoline-2-carbonyl)imidazo[1,2-b]pyridazin-2-yl)-5-fluoro-2H-chromene-3-carboxylic acid

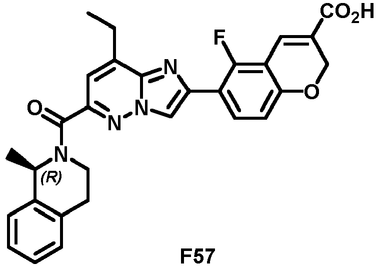

LiOH•H₂O (232 mg, 5.53 mmol) was added to a solution of intermediate **F56** (534 mg, 1.00 mmol) in THF (29 mL) and water (7.3 mL) and the reaction mixture was stirred at rt for 3 h. An aqueous solution of KHSO₄ 10% was added until pH=3 and the aqueous layer was extracted with EtOAc. The organic layer was dried over MgSO₄, filtered and evaporated to give intermediate **F57** as a yellow solid (380 mg, 72%).

### Compound 45

### (R)-6-(8-ethyl-6-(1-methyl-1,2,3,4-tetrahydroisoquinoline-2-carbonyl)imidazo[1,2-b]pyridazin-2-yl)-5-fluoro-2H-chromene-3-carboxamide

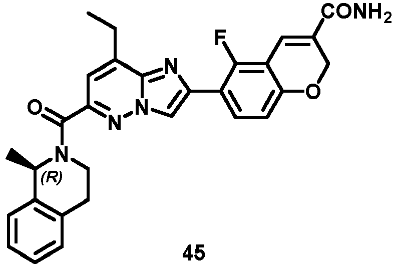

A mixture of intermediate **F57** (100 mg, 0.191 mmol), ammonium chloride (12 mg, 0.23 mmol), EDCI•HCl (36 mg, 0.19 mmol) and HOBt•H₂O (44 mg, 0.29 mmol) in DMF (9.5 mL) was stirred at 0°C. DiPEA (165 µL, 0.956 mmol) was added slowly at 0°C. The mixture was stirred at rt for 18 h. Brine and EtOAc were added. The layers were separated. The organic layer was dried over MgSO₄ and evaporated to dryness. The residue was purified by preparative LC (irregular SiOH 15-40 µm, 4 g Grace Resolv^{™}, mobile phase gradient: from DCM/MeOH 100/00 to 90/10). The fractions containing pure product were evaporated under vacuum. The residue was triturated in MeCN, filtered over frit and dried to give compound **45** (26 mg, 27%).

### Synthesis of compound 46

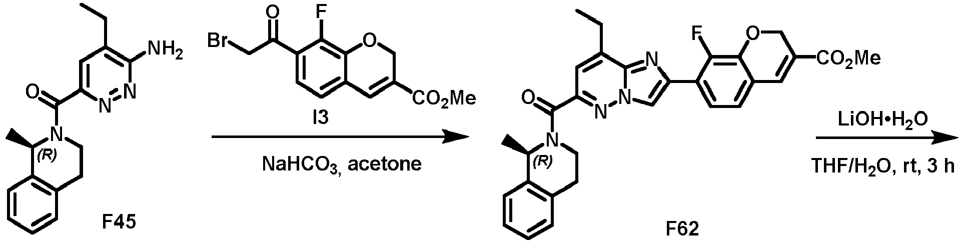

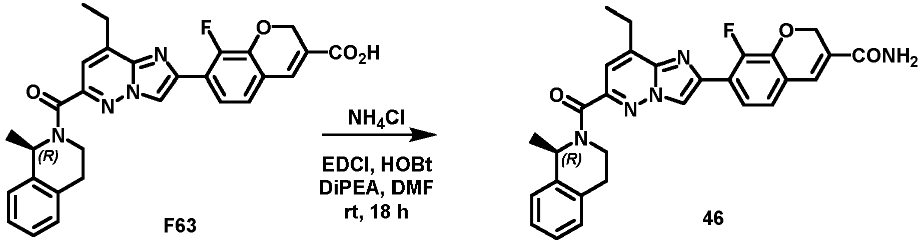

### Intermediate F62

### Methyl (R)-7-(8-ethyl-6-(1-methyl-1,2,3,4-tetrahydroisoquinoline-2-carbonyl)imidazo[1,2-b]pyridazin-2-yl)-8-fluoro-2H-chromene-3-carboxylate

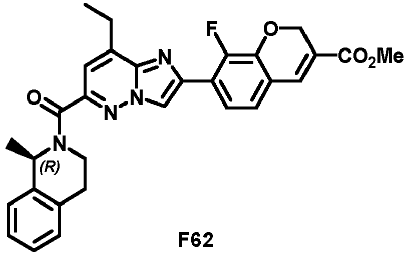

A mixture of intermediate **F45** (294 mg, 0.991 mmol), intermediate **I3** (326 mg, 0.991 mmol) and NaHCO₃ (83 mg, 0.99 mmol) in acetone (7.8 mL) was stirred at reflux for 16 h. The mixture was evaporated to dryness and the residue was taken-up with MeCN then the solid was filtered off to give intermediate **F62** (452 mg, 72%).

### Intermediate F63

### (R)-7-(8-ethyl-6-(1-methyl-1,2,3,4-tetrahydroisoquinoline-2-carbonyl)imidazo[1,2-b]pyridazin-2-yl)-8-fluoro-2H-chromene-3-carboxylic acid

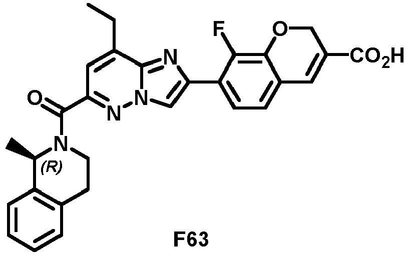

LiOH•H₂O (198 mg, 4.73 mmol) was added to a solution of intermediate **F62** (452 mg, 0.858 mmol) in THF (25 mL) and water (6.2 mL) and the reaction mixture was stirred at rt for 3 h. An aqueous solution of KHSO₄ 10% was added until pH=3 and the aqueous layer was extracted with EtOAc. The organic layer was dried over MgSO₄, filtered and evaporated to give intermediate **F63** as a yellow solid (319 mg, 71%).

### Compound 46

### (R)-7-(8-ethyl-6-(1-methyl-1,2,3,4-tetrahydroisoquinoline-2-carbonyl)imidazo[1,2-b]pyridazin-2-yl)-8-fluoro-2H-chromene-3-carboxamide

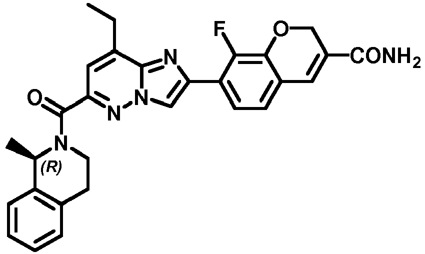

A mixture of intermediate **F63** (100 mg, 0.191 mmol), ammonium chloride (12 mg, 0.23 mmol), EDCI•HCl (36 mg, 0.19 mmol) and HOBt•H₂O (44 mg, 0.29 mmol) in DMF (9.5 mL) was stirred at 0°C. DiPEA (165 µL, 0.956 mmol) was added slowly at 0°C. The mixture was stirred at rt for 18 h. Brine and EtOAc were added. The layers were separated. The organic layer was dried over MgSO₄ and evaporated. The residue was purified by preparative LC (irregular SiOH 15-40 µm, 4 g Grace Resolv^{™}, mobile phase gradient: from DCM/MeOH 100/00 to 90/10). The fractions containing pure product were evaporated under vacuum. The residue was triturated in MeCN, the solid was filtered off and dried to give compound **46** (39 mg, 40%).

### Synthesis of compound 51

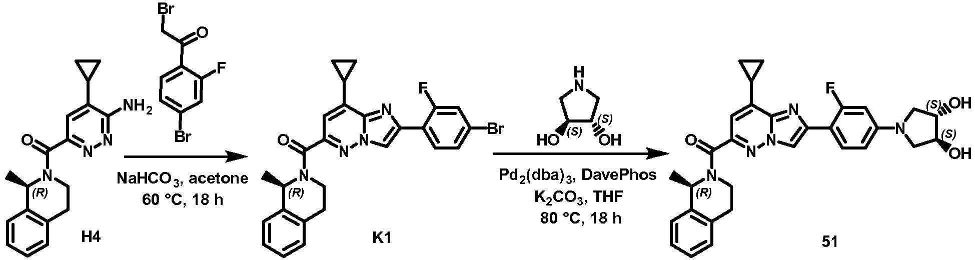

### Intermediate K1

### (R)-(2-(4-bromo-2-fluorophenyl)-8-cyclopropylimidazo[1,2-b]pyridazin-6-yl)(1-methyl-3,4-dihydroisoquinolin-2(1H)-yl)methanone

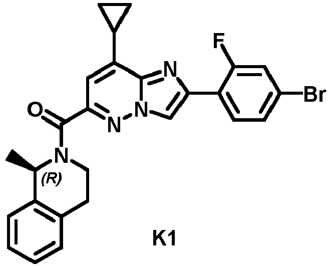

A mixture of intermediate **H4** (1.1 g, 3.57 mmol), acetone (28 mL), 4-bromo-2-fluorophenacyl bromide (1.06 g, 3.57 mmol) and NaHCO₃ (300 mg, 3.57 mmol) was stirred at 60 °C for 16 h. The mixture was evaporated under vacuum and purified by preparative LC (irregular SiOH, 15-40 µm, 80 g GraceResolv^{®}, solid loading (celite^{®}), mobile phase gradient: from Heptane / EtOAc 100:0 to 70:30) to give intermediate **K1** as a pink foam (1.53 g, 85%).

### Compound 51:

### (8-cyclopropyl-2-(4-((3S,4S)-3,4-dihydroxypyrrolidin-1-yl)-2-fluorophenyl)imidazo[1,2-b]pyridazin-6-yl)((R)-1-methyl-3,4-dihydroisoquinolin-2(1H)-yl)methanone

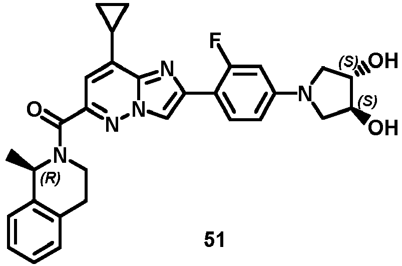

Under N₂, a mixture of intermediate **K1** (1.53 g, 3.03 mmol), (3S,4S)-pyrrolidine-3,4-diol (437 mg, 4.24 mmol) and K₂CO₃ (1.46 g, 10.6 mmol) in THF (28 mL) was degassed with N₂ for 10 min. DavePhos (119 mg, 0.303 mmol) and Pd₂dba₃ (277 mg, 0.303 mmol) were added and the reaction mixture was purged with N₂. The mixture was heated at reflux (80 °C) for 20 h. Water and EtOAc were added. The aqueous layer was extracted with EtOAc (twice), the combined organic layers were dried over MgSO₄, filtered, concentrated in vacuo and purified by preparative LC (irregular SiOH 15-40 µm, 120 g Grace Resolv^{®}, mobile phase gradient: from DCM/MeOH 100/0 to 90/10) to give 1.03 g of a solid as a yellow foam. The solid and SiliaMetS^{®} Thiol (0.27 g; 0.362 mmol) in THF (23 mL) was stirred at rt for 3 h, then filtered over celite and the filtrate was evaporated to dryness to give 1.05 g of a solid as yellow foam. The solid was purified by preparative LC (spherical C18, 25 µm, 120 g YMC-ODS-25, mobile phase gradient 0.2% aq. NH₄HCO₃ / MeCN from 65:35 to 25:75), the fraction containing product was extracted with EtOAc and water. The organic layer was dried (MgSO₄), filtered and evaporated to give 896 mg of a residue which was taken-up with MeCN and evaporated to dryness to give compound 51 as a solid (725 mg, 42%).

### Part 3 : imidazo[1,2-a]pyrazine

### Synthesis of intermediate G4

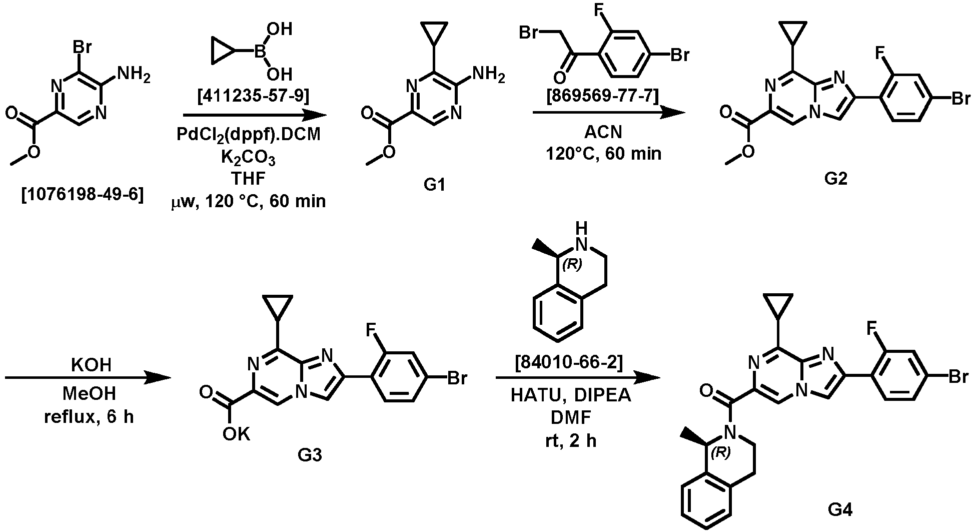

### Intermediate G1

### Methyl 5-amino-6-cyclopropylpyrazine-2-carboxylate

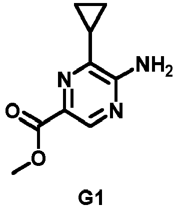

A mixture of methyl 5-amino-6-bromopyrazine-2-carboxylate (0.35 g, 1.51 mmol), cyclopropylboronic acid (0.19 g, 2.26 mmol) and potassium carbonate (0.83 g, 6.03 mmol) in THF (14 mL) was purged under nitrogen for 5 min. PdCl₂(dppf).DCM (0.12 g, 0.15 mmol) was added and the mixture was purged again under nitrogen. The mixture was heated at 120°C using a single mode microwave (Anton Paar Monowave^{®} 300) with a power output ranging from 0 to 850 W for 45 min. The reaction mixture was diluted with H₂O and EtOAc. The mixture was filtered through a pad of Celite^{®} and washed with EtOAc. The layers were separated and the organic phase was washed with H₂O, brine, dried over MgSO₄, filtered and evaporated to dryness. The crude mixture was purified by flash chromatography over silica gel (Puriflash Interchim^{®} 25 g, 30 µm, Mobile phase gradient: heptane/EtOAc from 100:0 to 40:60) to afford intermediate **G1** (0.28 g, 96%) as a yellow solid.

### Intermediate G2

### Methyl 2-(4-bromo-2-fluorophenyl)-8-cyclopropylimidazo[1,2-a]pyrazine-6-carboxylate

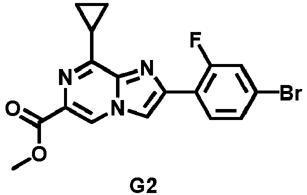

A mixture of intermediate **G1** (0.38 g, 1.97 mmol) and 4-bromo-2-fluorophenacyl bromide (0.82 g, 2.75 mmol) in ACN (17 mL) was heated at 120°C using a single mode microwave (Anton Paar Monowave^{®} 300) with a power output ranging from 0 to 850 W for 60 min. The solid was filtered off and rinsed with ACN (x3). The residue obtained was taken up in ACN (15 mL) and refluxed for 2 h. After cooled down to rt, the white precipitate was filtered off and dried in vacuum to afford intermediate **G2** (0.26 g, 33%) as a white solid.

### Intermediate G3

### Potassium 2-(4-bromo-2-fluorophenyl)-8-cyclopropylimidazo[1,2-a]pyrazine-6-carboxylate

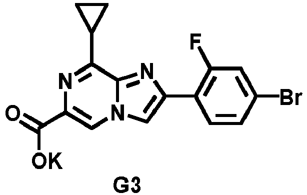

A mixture of intermediate **G2** (0.40 g, 1.03 mmol) and potassium hydroxide (0.23 g, 4.10 mmol) in MeOH (15 mL) were stirred at reflux for 6 h. The white precipitate was filtered off and dried in vacuum to afford intermediate **G3** (0.24 g, 57%) as a white solid.

### Intermediate G4

### (R)-(2-(4-bromo-2-fluorophenyl)-8-cyclopropylimidazo[1,2-a]pyrazin-6-yl)(1-methyl-3,4-dihydroisoquinolin-2(1H)-yl)methanone

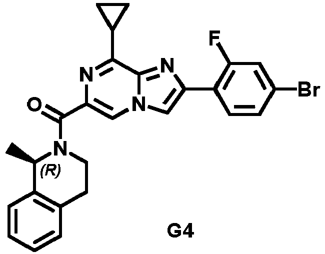

To a mixture of intermediate **G3** (0.22 g, 0.53 mmol), (1*R*)-1-methyl-1,2,3,4-tetrahydroisoquinoline (94 mg, 0.64 mmol) and DIPEA (0.37 mL, 2.12 mmol) in DMF (9 mL) was added HATU (0.26 g, 0.69 mmol). The reaction mixture was stirred at rt for 2h. The mixture was poured slowly out into water and the aqueous phase was extracted with DCM. The combined organic extracts were dried over MgSO₄, filtered and evaporated till dryness. The crude residue was taken up in EtOAc and the slurry obtained was stirred for 10 min at rt. The white precipitate was filtered off and dried in vacuum to afford intermediate G4 (0.22 g, 82%) as a white solid.

### Compound 47

### (R)-N-(4-(8-cyclopropyl-6-((R)-1-methyl-1,2,3,4-tetrahydroisoquinoline-2-carbonyl)imidazo[1,2-a]pyrazin-2-yl)-3 -fluorophenyl)-3-hydroxypyrrolidine-1-carboxamide

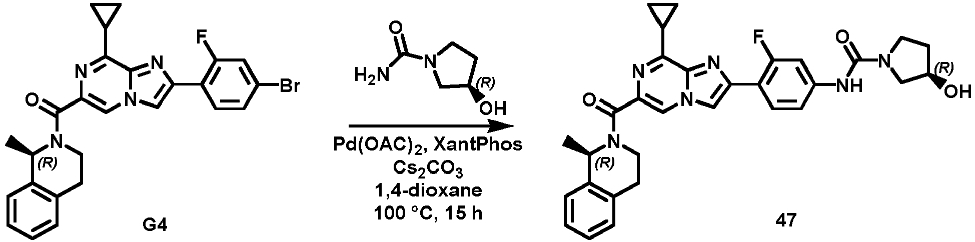

A mixture of intermediate **G4** (0.16 g, 0.32 mmol), (R)-3-hydroxypyrrolidine-1-carboxamide (82.40 mg, 0.63 mmol), cesium carbonate (0.41 g, 1.27 mmol) and XantPhos (18.3 mg, 0.03 mmol) in 1,4-dioxane (6.2 mL) was purged with nitrogen. Palladium acetate (7.1 mg, 0.03 mmol) was added. The reaction mixture was purged again with nitrogen and stirred at 100°C for 15 h. The reaction mixture was diluted with EtOAc and H₂O then filtered through a short pad of celite^{®}. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 25 g, 30 µm, mobile phase gradient: DCM/MeOH, from 100:0 to 97:03) to afford compound **47** (76 mg, yield 56%) as a brown solid.

### Compound 48

### (8-cyclopropyl-2-(4-((3S,4S)-3,4-dihydroxypyrrolidin-1-yl)-2-fluorophenyl)imidazo[1,2-a]pyrazin-6-yl)((R)-1-methyl-3,4-dihydroisoquinolin-2(1H)-yl)methanone

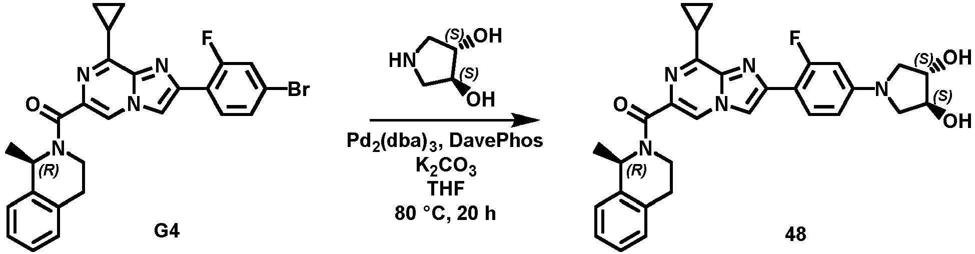

A mixture of intermediate **G4** (0.10 g, 0.20 mmol), (3S,4S)-pyrrolidine-3,4-diol (51 mg, 0.50 mmol) and potassium carbonate (0.11 g, 0.79 mmol) in THF (2.3 mL) was stirred and purged with nitrogen for 5 min. DavePhos (12.0 mg, 0.03 mmol) and Pd₂(dba)₃ (27.0 mg, 0.03 mmol) were added. The reaction mixture was purged again with nitrogen for 2 min and heated at 80°C for 20 h. The reaction mixture was diluted with EtOAc and H₂O. The layers were separated and the aqueous phase was extracted with EtOAc (twice). The combined organic extracts were washed with brine, dried over MgSO₄, filtered and the solvent was evaporated in vacuo. The crude mixture was purified by flash chromatography over silica gel (Puriflash Interchim^{®} 12 g, 30 µm, mobile phase gradient: DCM/MeOH, from 100:0 to 98:02) to afford compound **48** (67 mg, 64%) as a brown solid.

### Part 4: pyrrolo[1,2-b]pyridazine

### Synthesis of compound 49

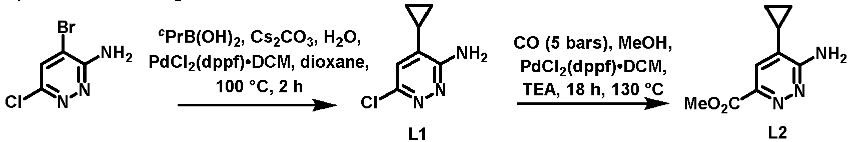

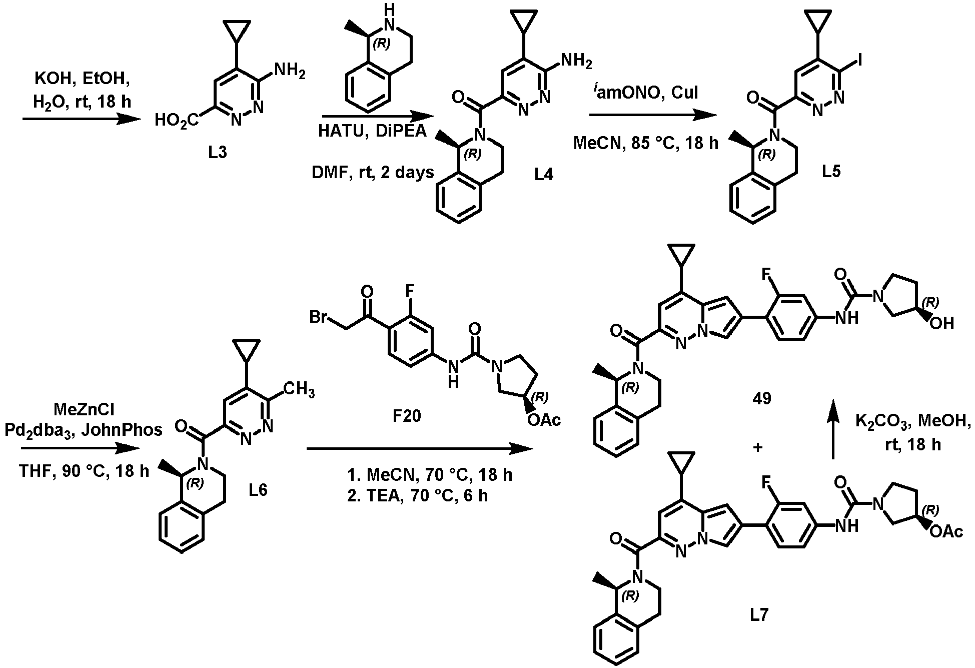

### Intermediate L1

### 6-chloro-4-cyclopropylpyridazin-3-amine

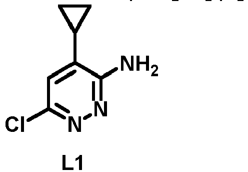

A mixture of 3-amino-4-bromo-6-chloropyridazine (10 g; 48.0 mmol), cesium carbonate (47 g; 144 mmol) and cyclopropylboronic acid (12 g; 144 mmol) in H₂O (70 mL) and 1,4-dioxane (200 mL) was purged with N₂ then PdCl₂(dppf) (3.5 g; 4.80 mmol) was added and the mixture was purged with N2 again. The resulting mixture was stirred at 100 °C for 3 h. Water and EtOAc were added. The aqueous layer was extracted with EtOAc (twice), the combined organic layers were dried over MgSO₄, filtered, concentrated in vacuo and purified by preparative LC (irregular SiOH 15-40 µm, 330 g GraceResolv^{®}, dry loading (celite^{®}), mobile phase gradient: from DCM/MeOH 100:0 to 90:10) to give intermediate **L1** as a black solid (7.24 g, 89%).

### Intermediate L2

### methyl 6-amino-5-cyclopropylpyridazine-3-carboxylate

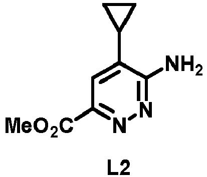

In a pressure vessel reactor, to a degassed mixture of intermediate **L1** (7.24 g; 42.7 mmol) and TEA (15.9 mL; 115 mmol) in MeOH (100 mL) was added PdCl₂(dppf) (2.66 mg; 3.63 mmol). The resulting mixture was stirred under 5 bars of CO for 18 h at 130 °C. The mixture was evaporated and purified by preparative LC (irregular SiOH 15-40 µm, 330 g GraceResolv^{®}, mobile phase gradient: DCM / MeOH from 100:0 to 90:10) to give intermediate **L2** as brown solid (1.58 g, 19%).

### Intermediate L3

### 6-amino-5-cyclopropylpyridazine-3-carboxylic acid

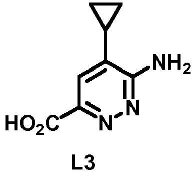

A mixture of intermediate **L2** (1.0 g; 5.18 mmol) and KOH (581 mg; 10.4 mmol) in EtOH (51 mL) and H₂O (3.6 mL) was stirred at rt for 18 h. The mixture was evaporated under vacuum and coevaporated (3 times) with THF to give crude intermediate **L3** (1.52 g, quant., estimated purity 61%).

### Intermediate L4

### (R)-(6-amino-5-cyclopropylpyridazin-3-yl)(1-methyl-3,4-dihydroisoquinolin-2(1H)-yl)methanone

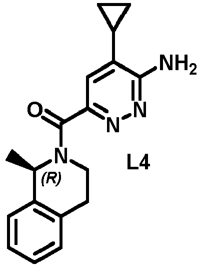

A mixture of intermediate **L3** (1.52 g; 5.18 mmol; purity 61%), 1R-methyl-1,2,3,4-tetrahydroisoquinoline (0.91 g; 6.21 mmol), HATU (2.95 g; 7.76 mmol), DiPEA (2.7 mL; 15.5 mmol) and DMF (26 mL) was stirred at rt for 2 days. EtOAc and water were added. The organic layer was separated, washed with brine, dried (MgSO₄) and purified by preparative LC (irregular SiOH 15-40 µm, 120 g GraceResolv^{®}, mobile phase gradient: DCM / MeOH from 100:0 to 95:5) to give intermediate **L4** (1.05 g, 66%).

### Intermediate L5

### (R)-(5-cyclopropyl-6-iodopyridazin-3-yl)(1-methyl-3,4-dihydroisoquinolin-2(1H)-yl)methanone

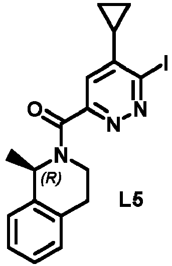

Isoamylnitrite (1.09 mL; 8.11 mmol) was added to a solution of intermediate **L4** (1 g; 3.24 mmol) and CuI (1.96 g; 10.3 mmol) in MeCN (23 mL). The solution was heated at 85°C for 24 h. EtOAc and water were added. A filtration over celite was performed. The organic layer was separated, washed with brine, dried (MgSO₄), evaporated and purified by preparative LC (irregular SiOH 15-40 µm, 80 g GraceResolv^{®}, mobile phase gradient: Heptane / EtOAc from 75:25 to 10:90) to give intermediate **L5** as yellow solid (509 mg, 37%).

### Intermediate L6

### (R)-(5-cyclopropyl-6-methylpyridazin-3-yl)(1-methyl-3,4-dihydroisoquinolin-2(1H)-yl)methanone

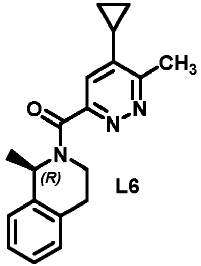

MeZnCl (1.2 mL; 2.43 mmol, 2M in THF) was added to a solution of intermediate L5 (509 mg; 1.21 mmol) in THF (10 mL). The mixture was purged with N₂. Pd₂dba₃ (111 mg; 0.121 mmol) and JohnPhos (72 mg; 0.243 mmol) was added. The mixture was purged with N₂ and stirred at 90 °C for 18 h. EtOAc and water were added. The organic layer was separated, washed with brine, dried (MgSO₄), evaporated and purified by preparative LC (irregular SiOH 15-40 µm, 40 g GraceResolv^{®}, mobile phase gradient: Heptane / EtOAc from 100:0 to 0:100) to give intermediate **L6** as brown gum (343 mg, 92%).

### Intermediate L7

### (R)-1-((4-(4-cyclopropyl-2-((R)-1-methyl-1,2,3,4-tetrahydroisoquinoline-2-carbonyl)pyrrolo[1,2-b]pyridazin-6-yl)-3-fluorophenyl)carbamoyl)pyrrolidin-3-yl acetate

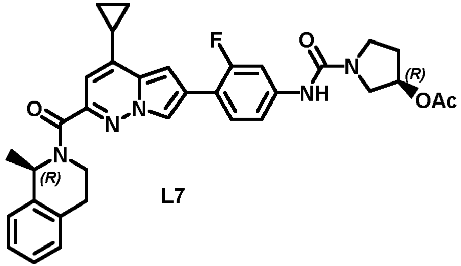

A mixture of intermediate **L6** (242 mg; 1.12 mmol), intermediate **F20** (432 mg; 1.12 mmol) and MeCN (5 mL) was stirred at 70°C for 18 h then TEA (0.465 mL; 3.35 mmol) was added and the mixture was stirred at 70 °C for 6 h. Water and EtOAc were added and an extraction was performed. The organic layer was washed with brine, dried (MgSO₄), filtered, evaporated and purified by preparative LC (spherical C18 25 µm, 40 g YMC-ODS-25, mobile phase gradient 0.2% aq. NH₄⁺HCO₃⁻ / MeCN from 70:30 to 30:70) the fractions containing product were freeze-dried to give compound **49** as white solid (67 mg, 11%, ~90% purity) and intermediate **L7** as white solid (26 mg, 4%).

### Compound 49

### (R)-N-(4-(4-cyclopropyl-2-((R)-1-methyl-1,2,3,4-tetrahydroisoquinoline-2-carbonyl)pyrrolo[1,2-b]pyridazin-6-yl)-3-fluorophenyl)-3-hydroxypyrrolidine-1-carboxamide

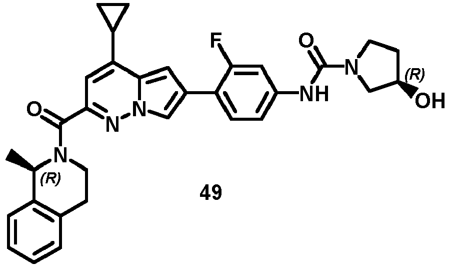

A mixture of intermediate **L7** (26 mg; 0.0436 mmol), K₂CO₃ (30.2 mg; 0.218 mmol) and MeOH (1.25 mL) was stirred at rt for 18 h. The mixture was evaporated and combined with previous isolated impure batch of compound **49.** The resulting compound was purified by preparative LC (irregular SiOH 15-40 µm, 12 g Buchi^{®}, mobile phase gradient: DCM/MeOH: 99:1 to 95:5, then DCM/MeOH:95/5) the fractions containing product were evaporated to dryness then diluted with MeCN, extended with water and freeze-dried to give compound **49** (47 mg).

### Part 5: indolizine

### Synthesis of compound 50

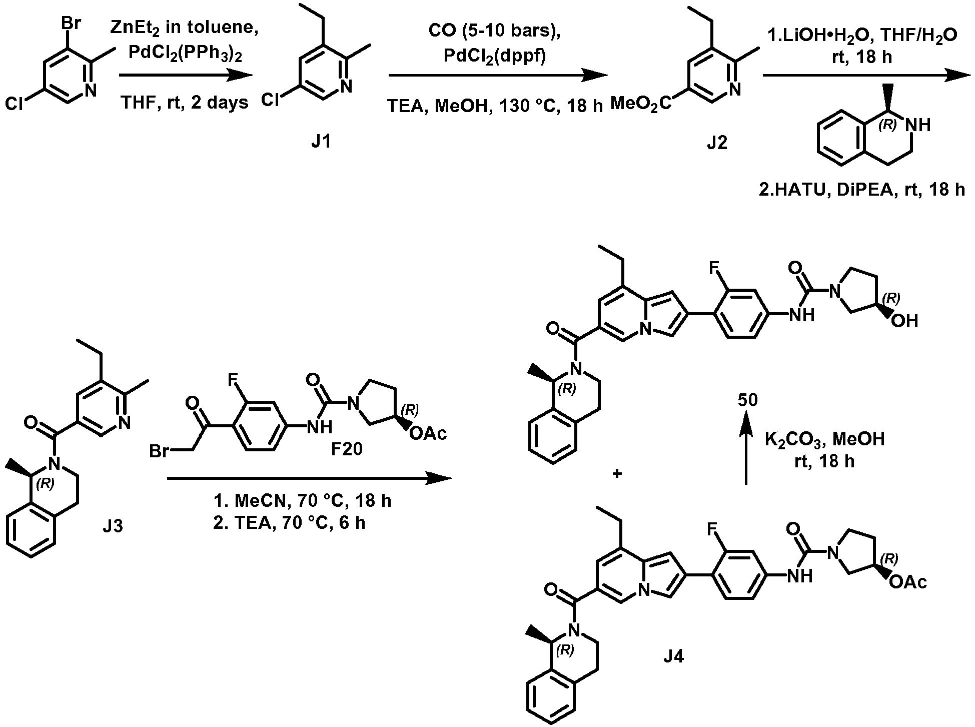

### Intermediate J1

### 5-chloro-3-ethyl-2-methylpyridine

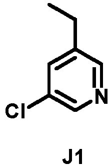

Diethylzinc (15 mL; 16.5 mmol, 1.1 M in toluene) was added to a solution of 3-bromo-5-chloro-2-methylpyridine (3.36 g; 16.3 mmol) in THF (100 mL). The mixture was purged with N₂. PdCl₂(PPh₃)₂ (1.14 g; 1.63 mmol) was added. The mixture was purged again with N₂ and stirred at rt for 2 days. EtOAc and water were added and the mixture was filtered on a celite^{®} pad then the organic layer was washed with brine, dried (MgSO₄), filtered, evaporated and purified by preparative LC (irregular SiOH 15-40 µm, 120 g GraceResolv^{®}, mobile phase gradient: heptane/EtOAc from 100:0 to 60:40) to give intermediate **J1** as a colorless oil (1.35 g, 53%).

### Intermediate J2

### methyl 5 -ethyl-6-methylnicotinate

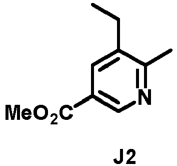

In a pressure vessel reactor, to a degassed mixture of intermediate **J1** (1.25 g; 8.03 mmol) and TEA (3 ml) in MeOH (50 mL) was added PdCl₂(dppf) (500 mg; 0.683 mmol). The resulting mixture was stirred under 5 bars of CO for 18 h at 130 °C. The mixture was evaporated and purified by preparative LC (irregular SiOH 15-40 µm, 80 g GraceResolv^{®}, mobile phase gradient: heptane / EtOAc from 100:0 to 50:50) to give intermediate **J2** as colorless oil (936 mg, 65%).

### Intermediate J3

### (R)-(5-ethyl-6-methylpyridin-3-yl)(1-methyl-3,4-dihydroisoquinolin-2(1H)-yl)methanone

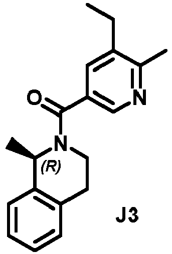

A mixture of intermediate **J2** (920 mg; 5.13 mmol), LiOH•H₂O (1.19 g; 28.3 mmol), THF (30 mL) and H₂O (5 mL) was stirred at rt for 18 h. EtOAc and 10% aq. KHSO₄ were added to the mixture and an extraction was performed. The organic layer was dried (MgSO₄), evaporated to give 50 mg of acid intermediate as white solid. The aqueous layer was freeze-dried to give 2 g of acid intermediate (~40% purity). The 50 mg batch (pure) and the 2 g batch (~40% purity) were combined and were solubilized in DMF (33 mL) then 1R-methyl-1,2,3,4-tetrahydroisoquinoline (909 mg, 6.17 mmol), HATU (2.93 g, 7.71 mmol) and DiPEA (2.70 mL; 15.4 mmol) were added and the resulting mixture was stirred at rt for 18 h. Brine and EtOAc were added to the reaction mixture. The layers were separated. The aqueous layer was extracted twice with EtOAc. The combined organic layers were washed with brine (3 times), dried over MgSO₄, evaporated and purified by preparative LC (irregular SiOH 15-40 µm, 80 g GraceResolv^{®}, mobile phase gradient: from Heptane /EtOAc 80/20 to 0/100) to give 530 mg as colorless gum. This fraction was purified by preparative LC (spherical C18 25 µm, 40 g YMC-ODS-25, mobile phase gradient 0.2% aq. NH₄HCO₃ / MeCN from 75:25 to 0:100) the fraction containing product was extracted with EtOAc and water. The organic layer was washed with brine, dried (MgSO₄), evaporated to give intermediate **J3** (265 mg, 18%).

### Intermediate J4

### (R)-1-((4-(8-ethyl-6-((R)-1-methyl-1,2,3,4-tetrahydroisoquinoline-2-carbonyl)indolizin-2-yl)-3-fluorophenyl)carbamoyl)pyrrolidin-3-yl acetate

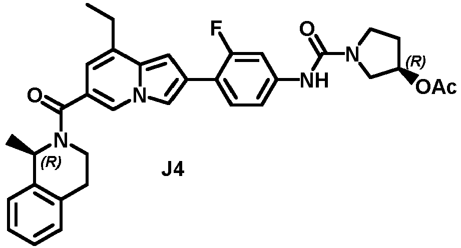

A mixture of intermediate **J3** (255 mg; 0.866 mmol), intermediate **F20** (335 mg; 0.866 mmol) and MeCN (4 mL) was stirred at 70°C for 18 h then TEA (0.361 mL; 2.60 mmol) was added and the mixture was stirred at 70 °C for 6 h. Water and EtOAc were added and an extraction was performed. The organic layer was washed with brine, dried (MgSO₄), filtered, evaporated and purified by preparative LC (irregular SiOH 15-40 µm, 40 g GraceResolv^{®}, mobile phase gradient: DCM/MeOH from 100:0 to 90:10) to give 246 mg of intermediate **J4** as pale yellow solid and 61 mg of impure compound **50** as yellow solid.

### Compound 50

### (R)-N-(4-(8-ethyl-6-((R)-1-methyl-1,2,3,4-tetrahydroisoquinoline-2-carbonyl)indolizin-2-yl)-3-fluorophenyl)-3-hydroxypyrrolidine-1-carboxamide

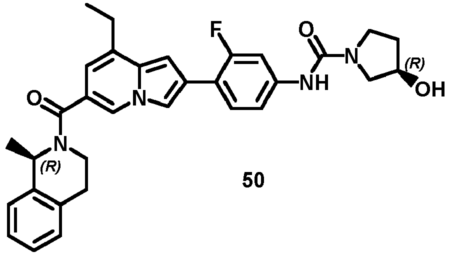

A mixture of intermediate **J4** (235 mg; 0.403 mmol), K₂CO₃ (278 mg; 2.02 mmol) and MeOH (5 mL) was stirred at rt for 18 h. The mixture was evaporated then combined with impure compound **50** (batch isolated during intermediate **J4** synthesis). The resulting mixture was purified by preparative LC (spherical C18 25 µm, 40 g YMC-ODS-25, mobile phase gradient 0.2% aq. NH₄HCO₃/MeCN from 70/30 to 30:70) the fractions containing product were extracted with EtOAc and water. The organic layer was washed with brine, dried (MgSO₄) and evaporated to give 164 mg as yellow solid which was purified again by Reverse phase (Stationary phase: YMC-actus Triart^{®} C18 10µm 30^{∗}150mm, Mobile phase gradient 0.2% aq. NH₄HCO₃ / MeCN from 55:45 to 35:65). The fractions containing product were evaporated, solubilized with MeCN and extended with water then freeze-dried to give 67 mg of compound **50** as pale yellow solid.

### C. Compound identification

### ¹H-NMR

¹H-NMR spectra were recorded on a Bruker Avance DRX 400 spectrometer using internal deuterium lock and equipped with reverse double-resonance (¹H, ¹³C, SEI) probe head with z gradients and operating at 400 MHz for proton and 100 MHz for carbon and a Bruker Avance 500 MHz spectrometer equipped with a Bruker 5mm BBFO probe head with z gradients and operating at 500 MHz for proton and 125 MHz for carbon.

NMR spectra were recorded at ambient temperature unless otherwise stated.

Data are reported as follow: chemical shift in parts per million (ppm) relative to TMS (δ = 0 ppm) which was used as internal standard, integration, multiplicity (s = singulet, d = doublet, t = triplet, q = quartet, quin = quintuplet, sex = sextuplet, m = multiplet, b = broad, or a combination of these), coupling constant(s) J in Hertz (Hz).

### Compound 1

### Major rotamer (65%)

¹H NMR (500 MHz, DMSO-*d*₆) δ ppm 13.15 (br s, 1H), 8.64 (s, 1H), 8.48 (d, *J*=4.1 Hz, 1H), 8.43 (t, *J*=7.9 Hz, 1H), 7.91 (d, *J*=7.9 Hz, 1H), 7.78 (d, *J*=12.0 Hz, 1H), 7.17 (br s, 4H), 6.88 (br s, 1H), 5.22 - 5.81 (m, 1H), 3.40 - 3.43, (m, 1H), 3.23 (br s, 1H), 3.05 - 2.99 (m, 1H), 2.78 (br d, *J*=16.4 Hz, 1H), 2.54 - 2.66 (m, 1H), 1.52 (d, *J*=6.6 Hz, 3H), 0.91 - 1.24 (m, 4H)

### Minor rotamer (35%)

¹H NMR (500 MHz, DMSO-*d₆*) δ ppm 13.15 (br s, 1H), 8.64 (s, 1H), 8.48 (d, *J*=4.1 Hz, 1H), 8.43 (t, *J*=7.9 Hz, 1H), 7.91 (d, *J*=7.9 Hz, 1H), 7.78 (d, *J*=12.0 Hz, 1H), 7.17 (br s, 4H), 6.88 (br s, 1H), 4.20 - 4.97 (m, 1H), 3.75 - 3.95 (m, 1H), 3.40 - 3.43, (m, 1H), 3.05 - 2.99 (m, 1H), 2.78 (br d, *J*=16.4 Hz, 1H), 2.54 - 2.66 (m, 1H), 1.52 (d, *J*=6.6 Hz, 3H), 0.91 - 1.24 (m, 4H)

### Compound 2

### Major rotamer (75%)

¹H NMR (500 MHz, DMSO-*d₆*) δ ppm 8.65 (s, 1H), 8.47 (d, *J*=4.1 Hz, 1H), 8.38 (t, *J*=7.9 Hz, 1H), 8.11 (s, 1H), 7.87 (dd, *J*=8.2, 1.6 Hz, 1H), 7.82 (dd, *J*=12.3, 1.3 Hz, 1H), 7.55 (s, 1H), 7.30 (br s, 1H), 7.08 - 7.25 (m, 3H), 6.89 (br s, 1H), 5.55 (br s, 1H), 3.83 (br s, 1H), 3.48 (br s, 1H), 3.05 (br s, 1H), 2.77 (br d, *J*=15.1 Hz, 1H), 2.56 - 2.63 (m, 1H), 1.99 (s, 1H), 1.52 (d, *J*=6.9 Hz, 3H), 1.06 - 1.15 (m, 3H).

### Minor rotamer (25%)

¹H NMR (500 MHz, DMSO-*d₆*) δ ppm 8.65 (s, 1H), 8.47 (d, *J*=4.1 Hz, 1H), 8.38 (t, *J*=7.9 Hz, 1H), 8.11 (s, 1H), 7.87 (dd, *J*=8.2, 1.6 Hz, 1H), 7.82 (dd, *J*=12.3, 1.3 Hz, 1H), 7.55 (s, 1H), 7.08 - 7.25 (m, 4H), 6.89 (br s, 1H), 4.96 (br s, 1H), 4.51 (br s, 1H), 3.20 - 3.30 (m, 1H), 3.05 (br s, 1H), 2.77 (br d, *J*=15.1 Hz, 1H), 2.56 - 2.63 (m, 1H), 1.91 (s, 1H), 1.52 (d, *J*=6.9 Hz, 3H), 1.06 - 1.15 (m, 3H).

### Compound 3

### Major rotamer (75%)

¹H NMR (500 MHz, DMSO-*d₆*) δ ppm 8.57 (s, 1H), 8.10 (d, *J*=4.1 Hz, 1H), 7.94 (t, *J*=8.7 Hz, 1H), 7.09 - 7.37 (m, 4H), 6.78 (br s, 1H), 6.51 (dd, *J*=8.5, 1.9 Hz, 1H), 6.42 (dd, *J*=14.0, 2.0 Hz, 1H), 5.62 (s, 2H), 5.44 - 5.58 (m, 1H), 3.81 (br d, *J*=3.8 Hz, 1H), 3.38 - 3.61 (m, 1H), 3.03 (br s, 1H), 2.77 (br d, *J*=16.1 Hz, 1H), 2.51 - 2.60 (m, 1H), 1.51 (br d, J=6.6 Hz, 3H), 1.00 - 1.13 (m, 4H)

### Minor rotamer (25%)

¹H NMR (500 MHz, DMSO-*d₆*) δ ppm 8.57 (s, 1H), 8.10 (d, *J*=4.1 Hz, 1H), 7.94 (t, *J*=8.7 Hz, 1H), 7.09 - 7.37 (m, 4H), 6.78 (br s, 1H), 6.51 (dd, *J*=8.5, 1.9 Hz, 1H), 6.42 (dd, *J*=14.0, 2.0 Hz, 1H), 5.62 (s, 2H), 4.77 - 5.07 (m, 1H), 4.28 - 4.68 (m, 1H), 3.38 - 3.61 (m, 1H), 3.03 (br s, 1H), 2.77 (br d, *J*=16.1 Hz, 1H), 2.51 - 2.60 (m, 1H), 1.51 (br d, *J*=6.6 Hz, 3H), 1.00 - 1.13 (m, 4H)

### Compound 4

### Major rotamer (70%)

¹H NMR (500 MHz, DMSO-*d₆*) δ ppm 8.61 (s, 1H), 8.47 (s, 1H), 8.26 (d, *J*=4.1 Hz, 1H), 8.14 (t, *J*=8.8 Hz, 1H), 7.70 (dd, *J*=14.5, 1.9 Hz, 1H), 7.43 (dd, *J*=8.7, 2.0 Hz, 1H), 7.05 - 7.34 (m, 4H), 6.83 (br s, 1H), 5.54 (br s, 1H), 5.00 (d, *J*=3.5 Hz, 1H), 4.31 (br s, 1H), 3.75 - 3.82 (m, 1H), 3.47 - 3.49 (m, 4H), 3.31(s, 1H), 3.04 (br s, 1H), 2.77 (br d, *J*=15.4 Hz, 1H), 2.53 - 2.61 (m, 1H), 1.73 - 1.98 (m, 2H), 1.52 (br d, *J*=6.6 Hz, 3H), 1.09 (m, 4H)

### Minor rotamer (30%)

¹H NMR (500 MHz, DMSO-*d₆*) δ ppm 8.61 (s, 1H), 8.47 (s, 1H), 8.26 (d, *J*=4.1 Hz, 1H), 8.14 (t, *J*=8.8 Hz, 1H), 7.70 (dd, *J*=14.5, 1.9 Hz, 1H), 7.43 (dd, *J*=8.7, 2.0 Hz, 1H), 7.05 - 7.34 (m, 4H), 6.83 (br s, 1H), 5.00 (d, *J*=3.5 Hz, 1H), 4.85 - 4.90 (m, 1H), 4.40 - 4.58 (m, 1H), 4.31 (br s, 1H), 3.47 - 3.49 (m, 4H), 3.31(s, 1H), 3.04 (br s, 1H), 2.77 (br d, *J*=15.4 Hz, 1H), 2.53 - 2.61 (m, 1H), 1.73 - 1.98 (m, 2H), 1.52 (br d, *J*=6.6 Hz, 3H), 1.09 (m, 4H)

### Compound 5

### Major rotamer (75%)

¹H NMR (500 MHz, DMSO-*d₆*) δ ppm 8.62 (s, 1H), 8.47 (s, 1H), 8.26 (d, *J*=4.1 Hz, 1H), 8.14 (t, *J*=8.7 Hz, 1H), 7.70 (dd, *J*=14.7, 1.7 Hz, 1H), 7.43 (dd, *J*=8.5, 1.9 Hz, 1H), 7.06 - 7.35 (m, 4H), 6.83 (br s, 1H), 5.54 (br d, *J*=1.6 Hz, 1H), 5.00 (d, *J*=3.5 Hz, 1H), 4.32 (br s, 1H), 3.71 - 3.92 (m, 1H), 3.40 - 3.55 (m, 4H), 3.32 (s, 1H), 3.04 - 3.06 (m, 1H), 2.77 (br d, *J*=15.1 Hz, 1H), 2.54 - 2.64 (m, 1H), 1.75 - 1.99 (m, 2H), 1.52 (br d, *J*=6.6 Hz, 3H), 1.08 - 1.10 (m, 4H)

### Minor rotamer (25%)

¹H NMR (500 MHz, DMSO-*d₆*) δ ppm 8.62 (s, 1H), 8.47 (s, 1H), 8.26 (d, *J*=4.1 Hz, 1H), 8.14 (t, *J*=8.7 Hz, 1H), 7.70 (dd, *J*=14.7, 1.7 Hz, 1H), 7.43 (dd, *J*=8.5, 1.9 Hz, 1H), 7.06 - 7.35 (m, 4H), 6.83 (br s, 1H), 5.00 (d, *J*=3.5 Hz, 1H), 4.81 - 4.98 (m, 1H), 4.41 - 4.58 (m, 1H), 4.32 (br s, 1H), 3.40 - 3.55 (m, 4H), 3.32 (s, 1H), 3.04 - 3.06 (m, 1H), 2.77 (br d, *J*=15.1 Hz, 1H), 2.54 - 2.64 (m, 1H), 1.75 - 1.99 (m, 2H), 1.52 (br d, *J*=6.6 Hz, 3H), 1.08 - 1.10 (m, 4H)

### Compound 6

### Major rotamer (70%)

¹H NMR (500 MHz, DMSO-*d₆*) δ ppm 12.39 (br s, 1H), 8.62 (s, 1H), 8.34 (d, *J*=4.1 Hz, 1H), 8.19 (t, *J*=8.2 Hz, 1H), 7.30 (br s, 1H), 7.05 - 7.25 (m, 5H), 6.85 (br s, 1H), 5.54 (br s, 1H), 3.82 (br s, 1H), 3.46 (br s, 1H), 3.05 (br s, 1H), 2.77 (br d, *J*=14.8 Hz, 1H), 2.53 - 2.62 (m, 1H), 2.44 - 2.48 (m, 1H), 1.86 - 1.96 (m, 1H), 1.52 (br d, *J*=6.6 Hz, 3H), 1.40 - 1.50 (m, 2H), 1.04 - 1.16 (m, 4H).

### Minor rotamer (30%)

¹H NMR (500 MHz, DMSO-*d₆*) δ ppm 12.39 (br s, 1H), 8.62 (s, 1H), 8.34 (d, *J*=4.1 Hz, 1H), 8.19 (t, *J*=8.2 Hz, 1H), 7.05 - 7.25 (m, 6H), 6.85 (br s, 1H), 4.96 (br s, 1H), 4.51 (br s, 1H), 3.46 (br s, 1H), 3.05 (br s, 1H), 2.77 (br d, *J*=14.8 Hz, 1H), 2.53 - 2.62 (m, 1H), 2.44 - 2.48 (m, 1H), 1.86 - 1.96 (m, 1H), 1.52 (br d, *J*=6.6 Hz, 3H), 1.40 - 1.50 (m, 2H), 1.04 - 1.16 (m, 4H).

### Compound 7

¹H NMR (500 MHz, DMSO-*d*₆, 77°C) δ ppm 8.55 (s, 1 H), 8.27 (d, *J*=3.8 Hz, 1H), 8.17 (br t, *J*=8.0 Hz, 1H), 7.24 - 7.57 (br s, 1H), 7.13 - 7.23 (m, 4H), 7.10 (br d, *J*=7.9 Hz, 1H), 7.06 (br d, *J*=12.9 Hz, 1H), 6.81 (s, 1H), 6.46 - 6.86 (br s, 1H), 5.36 (br s, 1H), 3.42 (br t, *J*=11.7 Hz, 1H), 2.94 - 3.04 (m, 1H), 2.78 (br d, *J*=15.8 Hz, 1H), 2.52 - 2.58 (m, 1H), 2.27 - 2.34 (m, 1H), 1.97 (s, 1H), 1.90 - 1.96 (m, 1H), 1.51 (br d, *J*=6.6 Hz, 3H), 1.39 (dt, *J*=8.7, 4.5 Hz, 1H), 1.20 - 1.26 (m, 1H), 1.10 - 1.15 (m, 2H), 1.02 - 1.10 (m, 2H).

### Compound 8

¹H NMR (500 MHz, DMSO-*d*₆, 77°C) δ ppm 11.75 (br s, 1H), 8.56 (d, *J=1.3* Hz, 1H), 8.29 (d, *J*=4.1 Hz, 1H), 8.20 (t, *J*=8.2 Hz, 1H), 7.14 - 7.23 (m, 5H), 7.13 (d, *J*=7.6 Hz, 1H), 6.82 (s, 1H), 5.36 (br s, 1H), 4.04 (br s, 1H), 3.42 - 3.47 (m, 1H), 3.24 (s, 3H), 2.95 - 3.03 (m, 1H), 2.78 (br d, *J*=16.1 Hz, 1H), 2.50 - 2.59 (m, 2H), 2.13 - 2.19 (m, 1H), 1.55 (dt, *J*=9.3, 4.8 Hz, 1H), 1.51 (d, *J*=6.9 Hz, 3H), 1.45 - 1.50 (m, 1H), 1.10 - 1.15 (m, 2H), 1.05 - 1.10 (m, 2H).

### Compound 9

### Major rotamer (75%)

¹H NMR (500 MHz, DMSO-*d₆*) δ ppm 12.51 (br s, 1H), 8.58 (br s, 1H), 8.15 (br d, *J*=3.5 Hz, 1H), 8.08 (br t, *J*=8.8 Hz, 1H), 7.29 (br s, 1H), 7.09 - 7.39 (m, 3H), 6.79 (br s, 1H), 6.53 (br d, *J*=8.2 Hz, 1H), 6.46 (br d, *J*=14.5 Hz, 1H), 5.53 (br s, 1H), 3.82 (br s, 1H), 3.42 - 3.56 (m, 3H), 3.29 - 3.42 (m, 2H), 3.17 - 3.26 (m, 1H), 3.03 (br s, 1H), 2.77 (br d, *J*=15.4 Hz, 1H), 2.54 - 2.61 (m, 1H), 2.12 - 2.29 (m, 2H), 1.51 (br d, *J*=6.3 Hz, 3H), 0.99 - 1.13 (m, 4H).

### Minor rotamer (25%)

¹H NMR (500 MHz, DMSO-*d₆*) δ ppm 12.51 (br s, 1H), 8.58 (br s, 1H), 8.15 (br d, *J*=3.5 Hz, 1H), 8.08 (br t, *J*=8.8 Hz, 1H), 7.09 - 7.39 (m, 4H), 6.79 (br s, 1H), 6.53 (br d, *J*=8.2 Hz, 1H), 6.46 (br d, *J*=14.5 Hz, 1H), 4.95 (br s, 1H), 4.50 (br s, 1H), 3.42 - 3.56 (m, 2H), 3.29 - 3.42 (m, 3H), 3.17 - 3.26 (m, 1H), 3.03 (br s, 1H), 2.77 (br d, *J*=15.4 Hz, 1H), 2.54 - 2.61 (m, 1H), 2.12-2.29 (m, 2H), 1.51 (br d, *J*=6.3 Hz, 3H), 0.99 - 1.13 (m, 4H).

### Compound 10

### Major rotamer (75%)

¹H NMR (500 MHz, DMSO-*d₆*) δ ppm 8.58 (s, 1H), 8.15 (d, *J*=4.1 Hz, 1H), 8.08 (t, *J*=8.8 Hz, 1H), 7.50 (br s, 1H), 7.29 (br s, 1H), 7.09 - 7.24 (m, 3H), 7.00 (br s, 1H), 6.78 (br s, 1H), 6.51 (dd, *J*=8.5, 1.6 Hz, 1H), 6.43 (dd, *J*=14.5, 1.3 Hz, 1H), 5.53 (br s, 1H), 3.82 (br s, 1H), 3.43 - 3.52 (m, 1H), 3.27 - 3.43 (m, 5H), 3.04 - 3.12 (m, 1H), 2.77 (br d, *J*=15.8 Hz, 1H), 2.53 - 2.60 (m, 1H), 2.15 - 2.24 (m, 1H), 2.03 - 2.14 (m, 1H), 1.51 (br d, *J*=6.3 Hz, 3H), 1.09 (br s, 4H).

### Minor rotamer (25%)

¹H NMR (500 MHz, DMSO-*d₆*) δ ppm 8.58 (s, 1H), 8.15 (d, *J*=4.1 Hz, 1H), 8.08 (t, *J*=8.8 Hz, 1H), 7.50 (br s, 1H), 7.09 - 7.24 (m, 4H), 7.00 (br s, 1H), 6.78 (br s, 1H), 6.51 (dd, *J*= 8.5, 1.6 Hz, 1H), 6.43 (dd, *J*=14.5, 1.3 Hz, 1H), 4.95 (br s, 1H), 4.49 (br s, 1H), 3.43 - 3.52 (m, 1H), 3.27 - 3.43 (m, 5H), 3.04 - 3.12 (m, 1H), 2.77 (br d, *J*=15.8 Hz, 1H), 2.53 - 2.60 (m, 1H), 2.15 - 2.24 (m, 1H), 2.03 - 2.14 (m, 1H), 1.51 (br d, *J*=6.3 Hz, 3H), 1.09 (br s, 4H).

### Compound 11

### Major rotamer (80%)

¹H NMR (400 MHz, DMSO-*d₆*) δ ppm 8.57 (s, 1H), 8.15 (d, *J*=4.0 Hz, 1H), 8.07 (t, *J*=8.8 Hz, 1H), 7.98 (br q, *J*=4.5 Hz, 1H), 7.28 (br s, 1H), 7.17 (br s, 3H), 6.78 (br s, 1H), 6.50 (br d, *J*=9.1 Hz, 1H), 6.42 (br d, *J*=14.7 Hz, 1H), 5.53 (br s, 1H), 3.82 (br s, 1H), 3.44 - 3.54 (m, 1H), 3.25 - 3.44 (m, 4H), 2.97 - 3.13 (m, 2H), 2.77 (br d, *J*=15.7 Hz, 1H), 2.62 (d, *J*=4.5 Hz, 3H), 2.56 (t, J=6.3 Hz, 1H), 2.04 - 2.23 (m, 2H), 1.51 (br d, J=6.6 Hz, 3H), 1.09 (br t, *J*=7.1 Hz, 4H).

### Minor rotamer (20%)

¹H NMR (400 MHz, DMSO-*d₆*) δ ppm 8.57 (s, 1H), 8.15 (d, *J*=4.0 Hz, 1H), 8.07 (t, *J*=8.8 Hz, 1H), 7.98 (br q, *J*=4.5 Hz, 1H), 7.17 (br s, 4H), 6.78 (br s, 1H), 6.50 (br d, *J*=9.1 Hz, 1H), 6.42 (br d, *J*=14.7 Hz, 1H), 4.97 (br s, 1H), 4.49 (br s, 1H), 3.44 - 3.54 (m, 1H), 3.25 - 3.44 (m, 4H), 2.97 - 3.13 (m, 2H), 2.77 (br d, *J*=15.7 Hz, 1H), 2.62 (d, *J*=4.5 Hz, 3H), 2.56 (t, *J*=6.3 Hz, 1H), 2.04 - 2.23 (m, 2H), 1.51 (br d, J=6.6 Hz, 3H), 1.09 (br t, *J*=7.1 Hz, 4H).

### Compound 12

¹H NMR (500 MHz, DMSO-*d₆*) δ ppm 8.57 (s, 1H), 8.14 (d, *J*=4.1 Hz, 1H), 8.07 (t, *J*=8.8 Hz, 1H), 7.27 (br s, 1H), 7.17 (br s, 3H), 6.78 (br s, 1H), 6.49 (dd, *J*=8.7, 1.7 Hz, 1H), 6.41 (br d, *J*=14.8 Hz, 1H), 5.53 (br s, 1H), 4.99 (d, *J*=3.5 Hz, 1H), 4.42 (br s, 1H), 3.83 (br s, 1H), 3.46 (br dd, *J*=10.1, 4.7 Hz, 1H), 3.32 - 3.54 (m, 3H), 3.14 (br d, *J*=9.8 Hz, 1H), 3.03 (br s, 1H), 2.77 (br d, *J*=15.8 Hz, 1H), 2.52 - 2.60 (m, 1H), 2.01 - 2.11 (m, 1H), 1.87 - 1.96 (m, 1H), 1.51 (br d, J=6.6 Hz, 3H), 1.03 - 1.15 (m, 4H).

### Compound 13

¹H NMR (500 MHz, DMSO-*d₆*) δ ppm 8.57 (d, *J=1.3* Hz, 1H), 8.15 (d, *J*=4.1 Hz, 1H), 8.07 (t, *J*=8.8 Hz, 1H), 7.49 (br s, 1H), 7.35 (br s, 1H), 6.89 - 7.10 (m, 2H), 6.78 (s, 1H), 6.51 (dd, *J*=8.7, 2.0 Hz, 1H), 6.42 (dd, *J*=14.8, 1.9 Hz, 1H), 5.45 (br s, 1H), 3.94 (br s, 1H), 3.48 (t, *J*= 8.7 Hz, 1H), 3.26 - 3.43 (m, 4H), 3.08 (quin, *J*=7.6 Hz, 1H), 3.00 (br s, 1H), 2.80 (br d, *J*=15.4 Hz, 1H), 2.53 - 2.60 (m, 1H), 2.15 - 2.25 (m, 1H), 2.04 - 2.14 (m, 1H), 1.46 (br d, *J*=6.3 Hz, 3H), 1.03 - 1.15 (m, 4H).

### Compound 14

¹H NMR (500 MHz, DMSO-*d*₆, 77°C) δ ppm 8.51 (s, 1H), 8.10 (d, *J*=4.1 Hz, 1H), 8.05 (t, *J*=8.8 Hz, 1H), 7.69 (br d, *J*=3.8 Hz, 1H), 7.29 (d, *J*=5.4 Hz, 1H), 6.91 (d, *J*=*5.0* Hz, 1H), 6.76 (s, 1H), 6.50 (dd, *J*=8.7, 2.0 Hz, 1H), 6.39 (dd, *J*=14.5, 1.9 Hz, 1H), 5.31 (br s, 1H), 4.16 (br s, 1H), 3.49 (t, *J*=8.8 Hz, 1H), 3.28 - 3.44 (m, 4H), 3.06 - 3.11 (m, 1H), 2.91 - 3.00 (m, 1H), 2.80 (dd, *J*=16.1, 3.2 Hz, 1H), 2.63 (d, *J*=4.4 Hz, 3H), 2.51 - 2.57 (m, 1H), 2.08 - 2.22 (m, 2H), 1.46 (d, *J*=6.6 Hz, 3H), 1.10 - 1.15 (m, 2H), 1.04 - 1.09 (m, 2H).

### Compound 15

### Major rotamer (65%)

¹H NMR (500 MHz, DMSO-*d₆*) δ ppm 8.44 (br s, 1H), 8.12 (br s, 1H), 8.06 (t, *J*=8.8 Hz, 1H), 7.50 (br s, 1H), 6.98 (br s, 1H), 6.68 (br s, 1H), 6.50 (dd, *J*=8.8, 1.9 Hz, 1H), 6.42 (dd, *J*=14.5, 1.6 Hz, 1H), 3.99 (br d, *J*=12.3 Hz, 1H), 3.68 (br s, 1H), 3.48 (t, *J*=8.7 Hz, 1H), 3.26 - 3.44 (m, 4H), 3.03 - 3.11 (m, 1H), 2.85 (br t, *J*=11.8 Hz, 1H), 2.52 - 2.58 (m, 1H), 2.15 - 2.23 (m, 1H), 2.05 - 2.14 (m, 1H), 1.88 - 2.04 (m, 1H), 1.51 - 1.84 (m, 4H), 1.18 - 1.33 (m, 3H), 1.10 - 1.16 (m, 2H), 1.04 - 1.10 (m, 4H).

### Minor rotamer (35%)

¹H NMR (500 MHz, DMSO-*d₆*) δ ppm 8.48 (br s, 1H), 8.12 (br s, 1H), 8.06 (t, *J*=8.8 Hz, 1H), 7.50 (br s, 1H), 6.98 (br s, 1H), 6.68 (br s, 1H), 6.50 (dd, *J*=8.8, 1.9 Hz, 1H), 6.42 (dd, *J*=14.5, 1.6 Hz, 1H), 4.43 (br s, 1H), 3.48 (t, *J*=8.7 Hz, 2H), 3.26 - 3.44 (m, 4H), 3.03 - 3.11 (m, 1H), 2.85 (br t, *J*=11.8 Hz, 1H), 2.52 - 2.58 (m, 1H), 2.15 - 2.23 (m, 1H), 2.05 - 2.14 (m, 1H), 1.88 - 2.04 (m, 1H), 1.51 - 1.84 (m, 4H), 1.18 - 1.33 (m, 3H), 1.10 - 1.16 (m, 2H), 1.04 - 1.10 (m, 4H).

### Compound 16

### Major rotamer (80%)

¹H NMR (500 MHz, DMSO-*d₆*) δ ppm 8.81 (s, 1H), 8.21 - 8.31 (m, 3H), 8.07 (t, *J*=8.8 Hz, 1H), 7.52 - 7.58 (m, 2H), 7.42 - 7.51 (m, 3H), 7.31 (br s, 1H), 7.10 - 7.26 (m, 3H), 6.99 (br s, 1H), 6.51 (dd, *J*=8.7, 2.0 Hz, 1H), 6.44 (dd, *J*=14.7, 1.7 Hz, 1H), 5.59 (br s, 1H), 3.96 (br s, 1H), 3.53 (br s, 1H), 3.49 (br t, *J*=8.7 Hz, 1H), 3.35 - 3.43 (m, 2H), 3.26 - 3.32 (m, 1H), 3.08 (br dt, *J*=15.1, 7.6 Hz, 2H), 2.79 (br d, *J*=15.4 Hz, 1H), 2.15 - 2.24 (m, 1H), 2.05 - 2.15 (m, 1H), 1.55 (br d, *J*=6.3 Hz, 3H).

### Minor rotamer (20%)

¹H NMR (500 MHz, DMSO-*d₆*) δ ppm 8.81 (s, 1H), 8.21 - 8.31 (m, 3H), 8.07 (t, *J*=8.8 Hz, 1H), 7.52 - 7.58 (m, 2H), 7.42 - 7.51 (m, 3H), 7.10 - 7.26 (m, 4H), 6.99 (br s, 1H), 6.51 (dd, *J*=8.7, 2.0 Hz, 1H), 6.44 (dd, *J*=14.7, 1.7 Hz, 1H), 5.12 (br s, 1H), 4.54 (br s, 1H), 3.49 (br t, *J*=8.7 Hz, 1H), 3.35 - 3.43 (m, 3H), 3.26 - 3.32 (m, 1H), 3.08 (br dt, *J*=15.1, 7.6 Hz, 2H), 2.79 (br d, *J*=15.4 Hz, 1H), 2.15 - 2.24 (m, 1H), 2.05 - 2.15 (m, 1H), 1.55 (br d, *J*=6.3 Hz, 3H).

### Compound 17

¹H NMR (500 MHz, DMSO-*d*₆, 37°C) δ ppm 9.37 (br s, 1H), 8.77 (s, 1H), 8.53 - 8.64 (m, 2H), 8.20 (d, *J*=3.8 Hz, 1H), 7.99 (t, *J*=8.8 Hz, 1H), 7.53 (br s, 1H), 7.50 (dd, *J*=7.7, 4.9 Hz, 1H), 7.36 (br s, 1H), 7.19 (br s, 1H), 7.11 (br s, 3H), 6.83 (br s, 1H), 6.45 (dd, *J*=8.7, 1.4 Hz, 1H), 6.36 (br d, *J*=14.5 Hz, 1H), 5.49 (br s, 1H), 3.93 (br s, 1H), 3.42 (t, *J*=8.8 Hz, 1H), 3.36 - 3.58 (m, 1H), 3.28 - 3.37 (m, 2H), 3.20 - 3.27 (m, 1H), 3.01 (br quin, *J*=7.5 Hz, 2H), 2.73 (br d, *J*=16.1 Hz, 1H), 2.08 - 2.17 (m, 1H), 1.98 - 2.08 (m, 1H), 1.49 (br d, *J*=6.6 Hz, 3H).

### Compound 18

¹H NMR (500 MHz, DMSO-*d*₆, 23°C) δ ppm 9.44 (d, *J=1.3* Hz, 1H), 8.85 (d, *J=1.3* Hz, 1H), 8.63 - 8.71 (m, 2H), 8.29 (d, *J*=4.1 Hz, 1H), 8.06 (t, *J*=8.8 Hz, 1H), 7.62 (s, 1H), 7.58 (dd, *J*=7.9, 4.7 Hz, 1H), 7.50 (br s, 1H), 7.36 (br s, 1H), 6.99 (br s, 2H), 6.52 (dd, J=8.8, 2.2 Hz, 1H), 6.44 (dd, J=14.5, 1.9 Hz, 1H), 5.52 (br s, 1H), 4.07 (br s, 1H), 3.48 (t, *J*=8.9 Hz, 1H), 3.35 - 3.43 (m, 2H), 3.34 - 3.55 (m, 1H), 3.27 - 3.30 (m, 1H), 3.08 (quin, *J*=7.6 Hz, 2H), 2.83 (br d, *J*=15.1 Hz, 1H), 2.15 - 2.24 (m, 1H), 2.05 - 2.14 (m, 1H), 1.50 (br d, *J*=6.6 Hz, 3H).

### Compound 19

### Major rotamer (75%)

¹H NMR (500 MHz, DMSO-*d₆*) δ ppm 8.25 (s, 1H), 8.07 (d, *J*=4.1 Hz, 1H), 8.04 (t, *J*=9.0 Hz, 1H), 7.50 (br s, 1H), 7.29 (br s, 1H), 7.08 - 7.25 (m, 3H), 6.99 (br s, 1H), 6.50 (dd, *J*=8.7, 2.0 Hz, 1H), 6.42 (dd, *J*=14.5, 1.9 Hz, 1H), 6.21 (br s, 1H), 5.53 (br s, 1H), 3.91 (br s, 1H), 3.48 (t, *J*=8.7 Hz, 1H), 3.35 - 3.42 (m, 2H), 3.26 - 3.35 (m, 2H), 3.24 (s, 6H), 3.03 - 3.11 (m, 1H), 2.78 (br d, *J*=16.1 Hz, 1H), 2.51 - 2.53 (m, 1H partially obscured by DMSO peak), 2.15 - 2.23 (m, 1H), 2.05 - 2.14 (m, 1H), 1.52 (br d, *J*=5.7 Hz, 3H).

### Minor rotamer (35%)

¹H NMR (500 MHz, DMSO-*d₆*) δ ppm 8.25 (s, 1H), 8.07 (d, *J*=4.1 Hz, 1H), 8.04 (t, *J*=9.0 Hz, 1H), 7.50 (br s, 1H), 7.08 - 7.25 (m, 4H), 6.99 (br s, 1H), 6.50 (dd, *J*=8.7, 2.0 Hz, 1H), 6.42 (dd, *J*=14.5, 1.9 Hz, 1H), 6.21 (br s, 1H), 5.05 (br s, 1H), 4.49 (br s, 1H), 3.48 (t, *J*=8.7 Hz, 1H), 3.35 - 3.42 (m, 2H), 3.26 - 3.35 (m, 2H), 3.24 (s, 6H), 3.03 - 3.11 (m, 1H), 2.78 (br d, *J*=16.1 Hz, 1H), 2.51 - 2.53 (m, 1H partially obscured by DMSO peak), 2.15 - 2.23 (m, 1H), 2.05 - 2.14 (m, 1H), 1.52 (br d, *J*=5.7 Hz, 3H).

### Compound 20

### Major rotamer (65%)

¹H NMR (500 MHz, DMSO-*d₆*) δ ppm 7.97 - 8.10 (m, 3H), 7.50 (br s, 1H), 7.28 (br s, 1H), 7.17 (br s, 3H), 6.99 (br s, 1H), 6.49 (dd, *J*=8.8, 1.9 Hz, 1H), 6.41 (dd, *J*=14.5, 1.6 Hz, 1H), 5.91 (br s, 1H), 5.52 (br s, 1H), 3.93 (br s, 1H), 3.80 (br d, *J*=6.0 Hz, 4H), 3.47 (t, *J*=8.7 Hz, 1H), 3.25 - 3.44 (m, 4H), 3.07 (quin, *J*=7.7 Hz, 1H), 2.94 - 3.03 (m, 1H), 2.77 (br d, *J*=15.8 Hz, 1H), 2.14 - 2.23 (m, 1H), 2.04 - 2.14 (m, 1H), 1.97 (br s, 4H), 1.52 (br s, 3H).

### Minor rotamer (35%)

¹H NMR (500 MHz, DMSO-*d₆*) δ ppm 7.97 - 8.10 (m, 3H), 7.50 (br s, 1H), 7.17 (br s, 4H), 6.99 (br s, 1H), 6.49 (dd, *J*=8.8, 1.9 Hz, 1H), 6.41 (dd, *J*=14.5, 1.6 Hz, 1H), 5.91 (br s, 1H), 5.05 (br s, 1H), 4.49 (br s, 1H), 3.80 (br d, *J*=6.0 Hz, 4H), 3.47 (t, *J*=8.7 Hz, 1H), 3.25 - 3.44 (m, 4H), 3.07 (quin, *J*=7.7 Hz, 1H), 2.94 - 3.03 (m, 1H), 2.77 (br d, *J*=15.8 Hz, 1H), 2.14 - 2.23 (m, 1H), 2.04 - 2.14 (m, 1H), 1.97 (br s, 4H), 1.52 (br s, 3H).

### Compound 21

### Major rotamer (70%)

¹H NMR (500 MHz, DMSO-*d₆*) δ ppm 8.23 (br s, 1H), 7.51 (br s, 1H), 7.45 (t, *J*=8.7 Hz, 1H), 7.31 (br s, 1H), 7.06 - 7.26 (m, 3H), 7.00 (br s, 1H), 6.79 (br s, 1H), 6.50 (dd, *J*=8.7, 2.1 Hz, 1H), 6.42 (dd, *J*=13.9, 1.9 Hz, 1H), 5.57 (br s, 1H), 3.81 (br s, 1H), 3.48 (t, *J*=8.7 Hz, 1H), 3.26 - 3.42 (m, 4H), 3.09 (quin, *J*=7.7 Hz, 1H), 3.02 (br s, 1H), 2.76 (br s, 1H), 2.52 - 2.57 (m, 1H), 2.42 (br s, 3H), 2.16 - 2.24 (m, 1H), 2.07 - 2.16 (m, 1H), 1.53 (br d, *J*=6.6 Hz, 3H), 1.00 - 1.12 (m, 4H).

### Minor rotamer (30%)

¹H NMR (500 MHz, DMSO-*d₆*) δ ppm 8.23 (br s, 1H), 7.51 (br s, 1H), 7.45 (t, *J*=8.7 Hz, 1H), 7.06 - 7.26 (m, 4H), 7.00 (br s, 1H), 6.79 (br s, 1H), 6.50 (dd, *J*=8.7, 2.1 Hz, 1H), 6.42 (dd, *J*=13.9, 1.9 Hz, 1H), 4.96 (br s, 1H), 4.53 (br s, 1H), 3.48 (t, *J*=8.7 Hz, 1H), 3.26 - 3.42 (m, 4H), 3.09 (quin, *J*=7.7 Hz, 1H), 3.02 (br s, 1H), 2.76 (br s, 1H), 2.52 - 2.57 (m, 1H), 2.42 (br s, 3H), 2.16 - 2.24 (m, 1H), 2.07 - 2.16 (m, 1H), 1.53 (br d, *J*=6.6 Hz, 3H), 1.00 - 1.12 (m, 4H).

### Compound 22

### Major rotamer (76%)

¹H NMR (500 MHz, DMSO-*d₆*) δ ppm 8.58 (s, 1H), 8.42 (dd, *J*=10.2, 8.4 Hz, 1H), 8.16 (d, *J*=4.1 Hz, 1H), 7.50 (br s, 1H), 7.28 (br s, 1H), 7.10 - 7.24 (m, 3H), 6.98 (br s, 1H), 6.80 (br s, 1H), 6.51 (dd, *J*=8.4, 2.0 Hz, 1H), 5.53 (br s, 1H), 3.82 (br s, 1H), 3.59 - 3.69 (m, 1H), 3.52 - 3.59 (m, 1H), 3.50 (dd, *J*=10.4, 6.9 Hz, 1H), 3.36 - 3.47 (m, 2H), 3.04 - 3.11 (m, 1H), 2.95 - 3.04 (m, 1H), 2.77 (br d, *J*=16.1 Hz, 1H), 2.53 - 2.59 (m, 1H), 2.15 - 2.23 (m, 1H), 2.05 - 2.15 (m, 1H), 1.51 (d, *J*=6.6 Hz, 3H), 1.03 - 1.14 (m, 4H).

### Minor rotamer (24%)

¹H NMR (500 MHz, DMSO-*d₆*) δ ppm 8.58 (s, 1H), 8.42 (dd, *J*=10.2, 8.4 Hz, 1H), 8.16 (d, *J*=4.1 Hz, 1H), 7.50 (br s, 1H), 7.10 - 7.24 (m, 4H), 6.98 (br s, 1H), 6.80 (br s, 1H), 6.51 (dd, *J*=8.4, 2.0 Hz, 1H), 4.96 (br s, 1H), 4.49 (br s, 1H), 3.59 - 3.69 (m, 1H), 3.52 - 3.59 (m, 1H), 3.50 (dd, J=10.4, 6.9 Hz, 1H), 3.36 - 3.47 (m, 2H), 3.04 - 3.11 (m, 1H), 2.95 - 3.04 (m, 1H), 2.77 (br d, *J*=16.1 Hz, 1H), 2.52 - 2.53 (m, 1H), 2.15 - 2.23 (m, 1H), 2.05 - 2.15 (m, 1H), 1.51 (d, *J*=6.6 Hz, 3H), 1.03 - 1.14 (m, 4H).

### Compound 23

### Major rotamer (84%)

¹H NMR (500 MHz, DMSO-*d₆*) δ ppm 8.58 (d, *J=1.3* Hz, 1H), 8.42 (dd, *J*=10.1, 8.5 Hz, 1H), 8.16 (d, *J*=3.8 Hz, 1H), 7.50 (br s, 1H), 7.35 (br s, 1H), 6.89 - 7.05 (m, 2H), 6.80 (s, 1H), 6.51 (dd, *J*=8.5, 1.9 Hz, 1H), 5.45 (br s, 1H), 3.93 (br s, 1H), 3.60 - 3.67 (m, 1H), 3.52 - 3.58 (m, 1H), 3.50 (dd, *J*=10.4, 6.9 Hz, 1H), 3.33 - 3.45 (m, 2H), 3.07 (quin, *J*=7.5 Hz, 1H), 2.99 (br s, 1H), 2.80 (br d, *J*=15.1 Hz, 1H), 2.53 - 2.59 (m, 1H), 2.15 - 2.23 (m, 1H), 2.05 - 2.14 (m, 1H), 1.46 (d, *J*=6.6 Hz, 3H), 1.04 - 1.14 (m, 4H).

### Minor rotamer (16%)

¹H NMR (500 MHz, DMSO-*d₆*) δ ppm 8.58 (d, *J=1.3* Hz, 1H), 8.42 (dd, *J*=10.1, 8.5 Hz, 1H), 8.16 (d, *J*=3.8 Hz, 1H), 7.50 (br s, 1H), 7.35 (br s, 1H), 6.89 - 7.05 (m, 2H), 6.80 (s, 1H), 6.51 (dd, *J*=8.5, 1.9 Hz, 1H), 4.94 (br s, 1H), 4.57 (br s, 1H), 3.60 - 3.67 (m, 1H), 3.52 - 3.58 (m, 1H), 3.50 (dd, *J*=10.4, 6.9 Hz, 1H), 3.33 - 3.45 (m, 2H), 3.07 (quin, *J*=7.5 Hz, 1H), 2.99 (br s, 1H), 2.80 (br d, *J*=15.1 Hz, 1H), 2.52 - 2.53 (m, 1H), 2.15 - 2.23 (m, 1H), 2.05 - 2.14 (m, 1H), 1.46 (d, *J*=6.6 Hz, 3H), 1.04 - 1.14 (m, 4H).

### Compound 24

### Major rotamer (70%)

¹H NMR (500 MHz, DMSO-*d₆*) δ ppm 8.64 (s, 1H), 8.41 (d, *J*=4.1 Hz, 1H), 7.86 (t, *J*=7.5 Hz, 1H), 7.78 (br s, 1H), 7.36 (s, 1H), 7.27 - 7.35 (br s, 2H), 7.10 - 7.25 (m, 4H), 6.87 (br s, 1H), 5.54 (br s, 1H), 5.03 (s, 2H), 3.83 (br s, 1H), 3.47 (br s, 1H), 3.04 (br s, 1H), 2.77 (br d, *J*=14.8 Hz, 1H), 2.53 - 2.59 (m, 1H), 1.52 (br d, *J*=6.6 Hz, 3H), 1.05 - 1.16 (m, 4H).

### Minor rotamer (30%)

¹H NMR (500 MHz, DMSO-*d₆*) δ ppm 8.64 (s, 1H), 8.41 (d, *J*=4.1 Hz, 1H), 7.86 (t, *J*=7.5 Hz, 1H), 7.78 (br s, 1H), 7.36 (s, 1H), 7.27 - 7.35 (br s, 1H), 7.10 - 7.25 (m, 5H), 6.87 (br s, 1H), 5.03 (s, 2H), 4.84 - 5.00 (m, 1H), 4.50 (br s, 1H), 3.47 (br s, 1H), 3.04 (br s, 1H), 2.77 (br d, *J*=14.8 Hz, 1H), 2.53 - 2.59 (m, 1H), 1.52 (br d, *J*=6.6 Hz, 3H), 1.05 - 1.16 (m, 4H).

### Compound 25

### Major rotamer (75%)

¹H NMR (500 MHz, DMSO-*d₆*) δ ppm 12.78 (br s, 1H), 9.14 (s, 1H), 8.65 (br s, 1H), 8.38 - 8.56 (m, 2H), 8.14 (s, 1H), 7.99 (d, *J*=14.5 Hz, 1H), 7.97 (d, *J*=9.5 Hz, 1H), 7.30 (br s, 1H), 7.06 - 7.26 (m, 3H), 6.89 (br s, 1H), 5.55 (br s, 1H), 3.83 (br s, 1H), 3.48 (br s, 1H), 3.04 (br s, 1H), 2.78 (br d, *J*=14.2 Hz, 1H), 2.55 - 2.63 (m, 1H), 1.52 (br d, *J*=6.3 Hz, 3H), 1.01 - 1.22 (m, 4H).

### Minor rotamer (25%)

¹H NMR (500 MHz, DMSO-*d₆*) δ ppm 12.78 (br s, 1H), 9.14 (s, 1H), 8.65 (br s, 1H), 8.38 - 8.56 (m, 2H), 8.14 (s, 1H), 7.99 (d, *J*=14.5 Hz, 1H), 7.97 (d, *J*=9.5 Hz, 1H), 7.06 - 7.26 (m, 4H), 6.89 (br s, 1H), 4.97 (br s, 1H), 4.52 (br s, 1H), 3.48 (br s, 1H), 3.04 (br s, 1H), 2.78 (br d, *J*=14.2 Hz, 1H), 2.55 - 2.63 (m, 1H), 1.52 (br d, *J*=6.3 Hz, 3H), 1.01 - 1.22 (m, 4H).

### Compound 26

### Major rotamer (75%)

¹H NMR (500 MHz, DMSO-*d₆*) δ ppm 9.02 (s, 1H), 8.65 (br s, 1H), 8.37 - 8.50 (m, 2H), 8.19 (s, 1H), 7.90 (d, *J*=10.7 Hz, 1H), 7.88 (d, *J*=7.3 Hz, 1H), 7.73 (br s, 1H), 7.08 - 7.41 (m, 5H), 6.89 (br s, 1H), 5.55 (br s, 1H), 3.83 (br s, 1H), 3.48 (br s, 1H), 3.05 (br s, 1H), 2.78 (br d, *J*=15.1 Hz, 1H), 2.56 - 2.62 (m, 1H), 1.52 (br d, *J*=6.6 Hz, 3H), 1.06 - 1.20 (m, 4H).

### Minor rotamer (25%)

¹H NMR (500 MHz, DMSO-*d₆*) δ ppm 9.02 (s, 1H), 8.65 (br s, 1H), 8.37 - 8.50 (m, 2H), 8.19 (s, 1H), 7.90 (d, *J*=10.7 Hz, 1H), 7.88 (d, *J*=7.3 Hz, 1H), 7.73 (br s, 1H), 7.08 - 7.41 (m, 5H), 6.89 (br s, 1H), 4.97 (br s, 1H), 4.51 (br s, 1H), 3.48 (br s, 1H), 3.05 (br s, 1H), 2.78 (br d, *J*=15.1 Hz, 1H), 2.56 - 2.62 (m, 1H), 1.52 (br d, *J*=6.6 Hz, 3H), 1.06 - 1.20 (m, 4H).

### Compound 27

### Major rotamer (80%)

¹H NMR (500 MHz, DMSO-*d₆*) δ ppm 8.58 (s, 1H), 8.16 (d, *J*=4.4 Hz, 1H), 8.07 (t, *J*=8.5 Hz, 1H), 7.29 (br s, 1H), 7.17 (br s, 3H), 6.79 (br s, 1H), 6.38 (dd, *J*=8.5, 2.2 Hz, 1H), 6.34 (dd, *J*=13.6, 2.2 Hz, 1H), 5.68 (d, *J*=6.6 Hz, 1H), 5.53 (br s, 1H), 4.55 - 4.64 (m, 1H), 4.13 (t, *J*=7.3 Hz, 2H), 3.81 (br s, 1H), 3.60 (dd, *J*=8.0, 4.9 Hz, 2H), 3.45 (br s, 1H), 3.04 (br s, 1H), 2.77 (br d, *J*=15.8 Hz, 1H), 2.56 (br quin, *J*=6.8 Hz, 1H), 1.51 (d, *J*=6.6 Hz, 3H), 1.03 - 1.14 (m, 4H).

### Minor rotamer (20%)

¹H NMR (500 MHz, DMSO-*d₆*) δ ppm 8.58 (s, 1H), 8.16 (d, *J*=4.4 Hz, 1H), 8.07 (t, *J*=8.5 Hz, 1H), 7.29 (br s, 1H), 7.17 (br s, 3H), 6.79 (br s, 1H), 6.38 (dd, *J*=8.5, 2.2 Hz, 1H), 6.34 (dd, *J*=13.6, 2.2 Hz, 1H), 5.68 (d, *J*=6.6 Hz, 1H), 4.95 (br s, 1H), 4.55 - 4.64 (m, 1H), 4.49 (br s, 1H), 4.13 (t, *J*=7.3 Hz, 2H), 3.60 (dd, *J*=8.0, 4.9 Hz, 2H), 3.45 (br s, 1H), 3.04 (br s, 1H), 2.77 (br d, *J*=15.8 Hz, 1H), 2.56 (br quin, *J*=6.8 Hz, 1H), 1.51 (d, *J*=6.6 Hz, 3H), 1.03 - 1.14 (m, 4H).

### Compound 28

### Major rotamer (75%)

¹H NMR (500 MHz, DMSO-*d₆*) δ ppm 11.95 (br s, 1H), 8.58 (s, 1H), 8.18 (d, *J*=4.1 Hz, 1H), 8.10 (t, *J*=8.7 Hz, 1H), 7.29 (br s, 1H), 7.18 (br s, 3H), 6.80 (br s, 1H), 6.37 - 6.47 (m, 2H), 5.53 (br s, 1H), 4.05 (br t, *J*=8.0 Hz, 2H), 3.96 (t, *J*=6.6 Hz, 2H), 3.81 (br s, 1H), 3.58 - 3.66 (m, 1H), 3.46 (br s, 1H), 3.27 (s, 3H), 2.94 - 3.13 (m, 1H), 2.77 (br d, *J*=15.1 Hz, 1H), 2.53 - 2.60 (m, 1H), 1.51 (br d, *J*=6.6 Hz, 3H), 1.09 (br s, 4H).

### Minor rotamer (25%)

¹H NMR (500 MHz, DMSO-*d₆*) δ ppm 11.95 (br s, 1H), 8.58 (s, 1H), 8.18 (d, *J*=4.1 Hz, 1H), 8.10 (t, *J*=8.7 Hz, 1H), 7.29 (br s, 1H), 7.18 (br s, 3H), 6.80 (br s, 1H), 6.37 - 6.47 (m, 2H), 4.96 (br s, 1H), 4.50 (br s, 1H), 4.05 (br t, *J*=8.0 Hz, 2H), 3.96 (t, *J*=6.6 Hz, 2H), 3.58 - 3.66 (m, 1H), 3.46 (br s, 1H), 3.27 (s, 3H), 2.94 - 3.13 (m, 1H), 2.77 (br d, *J*=15.1 Hz, 1H), 2.53 - 2.60 (m, 1H), 1.51 (br d, J=6.6 Hz, 3H), 1.09 (br s, 4H).

### Compound 29

### Major rotamer (70%)

¹H NMR (500 MHz, DMSO-*d₆*) δ ppm 13.11 (br s, 1H), 8.62 (s, 1H), 8.31 (d, *J*=4.1 Hz, 1H), 8.18 (t, *J*=8.7 Hz, 1H), 7.53 (s, 1H), 7.05 - 7.41 (m, 4H), 6.90 (d, *J*=8.8 Hz, 1H), 6.84 (br s, 1H), 5.39 - 5.66 (m, 1H), 5.01 (d, *J*=1.6 Hz, 2H), 3.41 - 3.55 (m, 1H), 3.03 (br d, *J*=2.2 Hz, 1H), 2.77 (br d, *J*=16.1 Hz, 1H), 2.51 - 2.59 (m, 2H), 1.52 (d, *J*=6.6 Hz, 3H), 1.01 - 1.18 (m, 4H).

### Minor rotamer (30%)

¹H NMR (500 MHz, DMSO-*d₆*) δ ppm 13.11 (br s, 1H), 8.62 (s, 1H), 8.31 (d, *J*=4.1 Hz, 1H), 8.18 (t, *J*=8.7 Hz, 1H), 7.53 (s, 1H), 7.05 - 7.41 (m, 4H), 6.90 (d, *J*=8.8 Hz, 1H), 6.84 (br s, 1H), 5.01 (d, *J*=1.6 Hz, 2H), 3.95 - 4.17 (m, 1H), 3.72 - 3.93 (m, 1H), 3.03 (br d, *J*=2.2 Hz, 1H), 2.77 (br d, *J*=16.1 Hz, 1H), 2.51 - 2.59 (m, 2H), 1.52 (d, *J*=6.6 Hz, 3H), 1.01 - 1.18 (m, 4H)

### Compound 30

### Major rotamer (80%)

¹H NMR (500 MHz, DMSO-*d₆*) δ ppm 8.73 (s, 1H), 8.61 (s, 1H), 8.25 (d, *J*=4.1 Hz, 1H), 8.14 (t, *J*=8.8 Hz, 1H), 7.67 (dd, *J*=14.5, 1.9 Hz, 1H), 7.36 (dd, *J*=8.7, 2.0 Hz, 1H), 7.03 - 7.33 (m, 4H), 6.82 (br s, 1H), 5.65 (d, *J*=6.3 Hz, 1H), 5.54 (br s, 1H), 4.42 - 4.48 (m, 1H), 4.16 (t, *J*=7.7 Hz, 2 H), 3.82 (br s, 1H), 3.74 (dd, *J*=8.8, 4.4 Hz, 2H), 3.42 - 3.53 (m, 1H), 3.03 (br s, 1H), 2.58 - 2.79 (m, 1H), 2.54 - 2.57 (m, 1H), 1.51 (d, *J*=6.6, 3H), 1.07 - 1.17 (m, 4H).

### Minor rotamer (20%)

¹H NMR (500 MHz, DMSO-*d₆*) δ ppm 8.73 (s, 1H), 8.61 (s, 1H), 8.25 (d, *J*=4.1 Hz, 1H), 8.14 (t, *J*=8.8 Hz, 1H), 7.67 (dd, *J*=14.5, 1.9 Hz, 1H), 7.36 (dd, *J*=8.7, 2.0 Hz, 1H), 7.03 - 7.33 (m, 4H), 6.82 (br s, 1H), 5.65 (d, *J*=6.3 Hz, 1H), 4.98 (br s, 1H), 4.51 (br s, 1H), 4.42 - 4.48 (m, 1H), 4.16 (t, *J*=7.7 Hz, 2 H), 3.74 (dd, *J*=8.8, 4.4 Hz, 2H), 3.42 - 3.53 (m, 1H), 3.03 (br s, 1H), 2.58 - 2.79 (m, 1H), 2.54 - 2.57 (m, 1H), 1.51 (d, *J*=6.6, 3H), 1.07 - 1.17 (m, 4H).

### Compound 31

### Major rotamer (65%)

¹H NMR (500 MHz, DMSO-*d₆*) δ ppm 12.44 (br s, 1H), 8.64 (br s, 1H), 8.28 - 8.51 (m, 2H), 7.55 - 7.82 (m, 3H), 7.06 - 7.45 (m, 4H), 6.88 (br s, 1H), 6.65 (br d, *J*=15.8 Hz, 1H), 5.55 (br s, 1H), 3.83 - 4.05 (m, 1H), 3.46 (br s, 1H), 3.04 (br s, 1H), 2.70 - 2.79 (m, 1H), 2.53 - 2.68 (m, 1H), 1.52 (br s, 3H), 1.11 - 1.13 (m, 4H).

### Minor rotamer (35%)

¹H NMR (500 MHz, DMSO-*d₆*) δ ppm 12.44 (br s, 1H), 8.64 (br s, 1H), 8.28 - 8.51 (m, 2H), 7.55 - 7.82 (m, 3H), 7.06 - 7.45 (m, 4H), 6.88 (br s, 1H), 6.65 (br d, *J*=15.8 Hz, 1H), 4.93 - 5.12 (m, 1H), 4.44 - 4.54 (m, 1H), 3.46 (br s, 1H), 3.04 (br s, 1H), 2.70 - 2.79 (m, 1H), 2.53 - 2.68 (m, 1H), 1.52 (br s, 3H), 1.11 - 1.13 (m, 4H)

### Compound 32

### Major rotamer (65%)

¹H NMR (400 MHz, DMSO-*d₆*) δ ppm 12.43 (br s, 1H), 8.52 (d, *J*=3.8 Hz, 1H), 8.18 (br t, *J*=8.1 Hz, 1H), 7.33 (br d, *J*=7.2 Hz, 1H), 7.08 - 7.27 (m, 5H), 7.04 (s, 1H), 5.59 (q, *J*=6.6 Hz, 1H), 3.84 (br dd, *J*=13.0, 4.2 Hz, 1H), 3.44 - 3.54 (m, 1H), 2.98 - 3.09 (m, 1H), 2.86 - 2.96 (m, 1H), 2.73 (br d, *J*=15.9 Hz, 1H), 2.58 - 2.66 (m, 1H), 1.89 - 1.97 (m, 1H), 1.53 (br d, *J*=6.7 Hz, 3H), 1.41 - 1.51 (m, 2H), 1.33 - 1.40 (m, 2H), 1.25 - 1.31 (m, 2H).

### Minor rotamer (35%)

¹H NMR (400 MHz, DMSO-*d₆*) δ ppm 12.43 (br s, 1H), 8.49 (d, *J*=3.7 Hz, 1H), 8.18 (br t, *J*=8.1 Hz, 1H), 7.08 - 7.27 (m, 6H), 7.00 (s, 1H), 5.00 (q, *J*=6.4 Hz, 1H), 4.56 (br d, *J*=12.6 Hz, 1H), 3.25 - 3.30 (m, 1H), 2.98 - 3.09 (m, 1H), 2.86 - 2.96 (m, 1H), 2.58 - 2.66 (m, 2H), 1.89 - 1.97 (m, 1H), 1.56 (br d, *J*=6.7 Hz, 3H), 1.41 - 1.51 (m, 2H), 1.33 - 1.40 (m, 2H), 1.25 - 1.31 (m, 2H).

### Compound 33

### Major rotamer (60%)

¹H NMR (400 MHz, DMSO-*d₆*) δ ppm 12.42 (br s, 1H), 8.45 (d, *J*=3.9 Hz, 1H), 8.16 (t, *J*=8.2 Hz, 1H), 7.15 - 7.25 (m, 2H), 6.97 (s, 1H), 3.97 (br d, *J*=13.2 Hz, 1H), 3.56 - 3.70 (m, 1H), 2.93 (br t, *J*=12.5 Hz, 1H), 2.56 - 2.65 (m, 1H), 2.43 - 2.47 (m, 1H), 1.90 - 1.97 (m, 2H), 1.53 - 1.86 (m, 4H), 1.30 - 1.52 (m, 5H), 1.20 - 1.29 (m, 4H), 1.11 (d, *J*=6.4 Hz, 3H).

### Minor rotamer (40%)

¹H NMR (400 MHz, DMSO-*d*₆) δ ppm 12.42 (br s, 1H), 8.48 (d, *J*=3.9 Hz, 1H), 8.16 (t, *J*=8.2 Hz, 1H), 7.15 - 7.25 (m, 2H), 6.97 (s, 1H), 4.41 (dt, *J*=11.9, 6.0 Hz, 1H), 3.46 (br d, *J*=15.3 Hz, 1H), 3.07 - 3.17 (m, 1H), 2.56 - 2.65 (m, 2H), 2.00 - 2.10 (m, 1H), 1.90 - 1.97 (m, 1H), 1.53 - 1.86 (m, 4H), 1.30 - 1.52 (m, 5H), 1.20 - 1.29 (m, 4H), 1.14 (d, *J*=6.5 Hz, 3H).

### Compound 34

### Major rotamer (60%)

¹H NMR (500 MHz, DMSO-*d*₆) δ ppm 12.42 (br s, 1 H) 8.45 (d, *J*= 3.8 Hz, 1 H) 8.16 (t, *J*=8.0 Hz, 1 H) 7.22 (s, 1H) 7.14 - 7.21 (m, 1 H) 6.97 (s, 1 H) 3.97 (br d, *J*=13.6 Hz, 1 H) 3.58 - 3.70 (m, 1 H) 2.93 (br t, *J*=12.6 Hz, 1 H) 2.56 - 2.62 (m, 1 H) 2.39-2.56 (m, 1H) 2.01 - 2.10 (m, 1 H) 1.89 - 2.01 (m, 1 H) 1.20 - 1.85 (m, 13 H) 1.11 (d, *J*=6.3 Hz, 3 H).

### Minor rotamer (40%)

¹H NMR (500 MHz, DMSO-*d*₆) δ ppm 12.42 (br s, 1 H) 8.48 (d, *J*= 3.8 Hz, 1 H) 8.16 (t, *J*=8.0 Hz, 1 H) 7.14 - 7.21 (m, 2 H) 6.97 (s, 1 H) 4.33 - 4.50 (m, 1 H) 3.46 (br d, *J*=15.4 Hz, 1 H) 3.05 - 3.18 (m, 1 H) 2.56 - 2.62 (m, 1 H) 2.39-2.56 (m, 1H) 1.89 - 2.01 (m, 2 H) 1.20 - 1.85 (m, 13 H) 1.13 (d, *J*=6.3 Hz, 3 H).

### Compound 35

### Major rotamer (65%)

¹H NMR (500 MHz, DMSO-*d*₆) δ ppm 8.32 (d, *J*=4.1 Hz, 1H), 8.07 (t, *J*=8.8 Hz, 1H), 7.08 - 7.36 (m, 5H), 6.49 (dd, *J*=8.8, 2.2 Hz, 1H), 6.42 (dd, *J*=14.7, 2.0 Hz, 1H), 5.58 - 5.64 (m, 1H), 5.18 (d, *J*=*3.5* Hz, 2H), 4.07 (br s, 2H), 3.86 - 3.96 (m, 1H), 3.46 - 3.57 (m, 3H), 3.16 (d, *J*=10.4 Hz, 2H), 2.71 - 3.11 (m, 4H), 1.54 (d, *J*=6.9 Hz, 3H), 1.35 - 1.41 (m, 3H).

### Minor rotamer (35%)

¹H NMR (500 MHz, DMSO-d₆) δ ppm 8.30 (d, *J*=4.1 Hz, 1H), 8.07 (t, *J*=8.8 Hz, 1H), 7.08 - 7.36 (m, 5H), 6.49 (dd, *J*=8.8, 2.2 Hz, 1H), 6.42 (dd, *J*=14.7, 2.0 Hz, 1H), 5.18 (d, *J*=3.5 Hz, 2H), 5.01 - 5.09 (m, 1H), 4.53 - 4.61 (m, 1H), 4.07 (br s, 2H), 3.52 (dd, *J*=10.6, 3.6 Hz, 2H), 3.26 - 3.35 (m, 1H), 3.16 (d, *J*=10.4 Hz, 2H), 2.71 - 3.11 (m, 4H), 1.59 (d, *J*=6.6 Hz, 3H), 1.35 - 1.41 (m, 3H).

### Compound 36

### Major rotamer (65%)

¹H NMR (500 MHz, DMSO-*d*₆) δ ppm 8.31 - 8.36 (m, 1 H) 8.07 (t, *J*=8.7 Hz, 1 H) 7.09 - 7.34 (m, 5 H) 6.33 - 6.42 (m, 2 H) 5.68 (d, *J*=6.6 Hz, 1 H) 5.58 - 5.64 (m, 1 H) 4.54 - 4.57 (m, 1 H) 4.14 (t, *J*=7.3 Hz, 2 H) 3.90 (br dd, *J*=13.6, 3.8 Hz, 1 H) 3.62 (dd, *J*=7.7, 4.9 Hz, 2 H) 3.35 - 3.56 (m, 1 H) 2.72 - 3.13 (m, 4 H) 1.54 (d, *J*=6.9 Hz, 3 H) 1.30 (t, *J*=7.3 Hz, 3 H).

### Minor rotamer (35%)

¹H NMR (500 MHz, DMSO-*d*₆) δ ppm 8.31 - 8.36 (m, 1 H) 8.07 (t, *J*=8.7 Hz, 1 H) 7.09 - 7.34 (m, 5 H) 6.33 - 6.42 (m, 2 H) 5.68 (d, *J*=6.6 Hz, 1 H) 5.02 - 5.08 (m, 1 H) 4.54 - 4.64 (m, 2 H) 4.14 (t, *J*=7.3 Hz, 2 H) 3.62 (dd, *J*=7.7, 4.9 Hz, 2 H) 3.27 - 3.31 (m, 1 H) 2.72 - 3.13 (m, 4 H) 1.58 (d, *J*=6.6 Hz, 3 H) 1.36 (t, *J*=7.6 Hz, 3 H).

### Compound 37

### Major rotamer (65%)

¹H NMR (500 MHz, DMSO-*d*₆) δ ppm 12.53 (br s, 1H), 8.64 (d, *J*=4.1 Hz, 1H), 8.32 (t, *J*=8.0 Hz, 1H), 7.79 (dd, *J*=12.3, 0.9 Hz, 1H), 7.69 (dd, *J*=8.2, 1.3 Hz, 1H), 7.63 (d, *J*=16.1 Hz, 1H), 7.07 - 7.39 (m, 5H), 6.67 (d, *J*=15.8 Hz, 1H), 5.58 - 5.66 (m, 1H), 3.87 - 3.99 (m, 1H), 3.45 - 3.62 (m, 1H), 2.70 - 3.17 (m, 4H), 1.55 (d, *J*=6.6 Hz, 3H), 1.33 - 1.48 (m, 3H).

### Minor rotamer (35%)

¹H NMR (500 MHz, DMSO-*d*₆) δ ppm 12.53 (br s, 1H), 8.62 (d, *J*=4.1 Hz, 1H), 8.32 (t, *J*=8.0 Hz, 1H), 7.79 (dd, *J*=12.3, 0.9 Hz, 1H), 7.69 (dd, *J*=8.2, 1.3 Hz, 1H), 7.63 (d, *J*=16.1 Hz, 1H), 7.07 - 7.39 (m, 5H), 6.67 (d, *J*=15.8 Hz, 1H),) 5.03 - 5.10 (m, 1H), 4.54 - 4.62 (m, 1H), 3.28 - 3.37 (m, 1H), 2.70 - 3.17 (m, 4H), 1.59 (d, *J*=6.9 Hz, 3H), 1.33 - 1.48 (m, 3H).

### Compound 38

### Major rotamer (65%)

¹H NMR (400 MHz, DMSO-*d*₆) δ ppm 8.50 (s, 1 H) 8.43 (d, *J*=3.9 Hz, 1 H) 8.13 (t, *J*=8.5 Hz, 1 H) 7.70 (br d, *J*=14.4 Hz, 1 H) 7.46 (d, *J*=8.8 Hz, 1 H) 7.32 (d, *J*=7.2 Hz, 1 H) 7.08 - 7.27 (m, 3 H) 7.01 (s, 1 H) 5.59 (q, J=7.3 Hz, 1 H) 4.99 (d, *J*=3.6 Hz, 1 H) 4.31 (br s, 1 H) 3.78 - 3.89 (m, 1 H) 3.41 - 3.55 (m, 4 H) 3.25 - 3.35 (m, 1 H) 2.82 - 3.10 (m, 2 H) 2.57 - 2.66 (m, 1 H) 1.87 - 2.00 (m, 1 H) 1.77 - 1.87 (m, 1 H) 1.53 (d, *J*=6.9 Hz, 3 H) 1.21 - 1.41 (m, 4 H).

### Minor rotamer (35%)

¹H NMR (400 MHz, DMSO-*d*₆) δ ppm 8.50 (s, 1 H) 8.41 (d, *J*=3.9 Hz, 1 H) 8.13 (t, *J*=8.5 Hz, 1 H) 7.70 (br d, *J*=14.4 Hz, 1 H) 7.46 (d, *J*=8.8 Hz, 1 H) 7.08 - 7.27 (m, 4 H) 6.97 (s, 1 H) 4.99 (d, *J*=3.6 Hz, 1 H) 4.95 - 5.04 (m, 1 H) 4.51 - 4.60 (m, 1 H) 4.31 (br s, 1 H) 3.41 - 3.55 (m, 4 H) 3.25 - 3.35 (m, 1 H) 2.74 (m, 2 H) 2.57 - 2.66 (m, 1 H) 1.87 - 2.00 (m, 1 H) 1.77 - 1.87 (m, 1 H) 1.57 (d, *J*=6.9 Hz, 3 H) 1.21 - 1.41 (m, 4 H).

### Compound 39

### Major rotamer (65%)

¹H NMR (400 MHz, DMSO-*d*₆) δ ppm 8.50 (s, 1 H) 8.44 (d, *J*=3.9 Hz, 1 H) 8.09 - 8.16 (m, 1 H) 7.70 (d, *J*=14.4 Hz, 1 H) 7.46 (d, *J*=8.7 Hz, 1 H) 7.39 (d, *J*=5.1 Hz, 1 H) 6.97 - 7.04 (m, 2 H) 5.51 - 5.57 (m, 1 H) 4.99 (d, *J*=3.6 Hz, 1 H) 4.32 (br s, 1 H) 3.95 (br dd, *J*=13.6, 4.3 Hz, 1 H) 3.39 - 3.52 (m, 4 H) 3.32 - 3.36 (m, 1 H) 2.55 - 3.06 (m, 3 H) 1.88 - 1.99 (m, 1 H) 1.78 - 1.86 (m, 1 H) 1.47 (d, *J*=6.7 Hz, 3 H) 1.21 - 1.32 (m, 2 H) 1.32 - 1.40 (m, 2 H).

### Minor rotamer (35%)

¹H NMR (400 MHz, DMSO-*d*₆) δ ppm 8.50 (s, 1 H) 8.42 (d, *J*=3.9 Hz, 1 H) 8.09 - 8.16 (m, 1 H) 7.70 (d, *J*=14.4 Hz, 1 H) 7.46 (d, *J*=8.7 Hz, 1 H) 7.30 (d, *J*=5.3 Hz, 1 H) 6.97 - 7.04 (m, 1 H) 6.80 (d, *J*=5.3 Hz, 1 H) 4.99 (d, *J*=3.6 Hz, 1 H) 4.90 - 4.97 (m, 1 H) 4.65 - 4.76 (m, 1 H) 4.32 (br s, 1 H) 3.39 - 3.52 (m, 3 H) 3.18 - 3.36 (m, 2 H) 2.55 - 3.06 (m, 3 H) 1.88 - 1.99 (m, 1 H) 1.78 - 1.86 (m, 1 H) 1.51 (d, *J*=6.5 Hz, 3 H) 1.21 - 1.32 (m, 2 H) 1.32 - 1.40 (m, 2 H).

### Compound 39

### Major rotamer (65%)

¹H NMR (500 MHz, DMSO-*d*₆) δ ppm 8.49 (s, 1 H) 8.45 (d, *J*=4.1 Hz, 1 H) 8.13 (t, *J*=8.7 Hz, 1 H) 7.71 (dd, *J*=14.7, 1.7 Hz, 1 H) 7.46 (dd, *J*=8.8, 1.9 Hz, 1 H) 7.08 - 7.35 (m, 5 H) 5.61 (m, 1 H) 4.98 (d, *J*=3.5 Hz, 1 H) 4.32 (br s, 1 H) 3.90 (br dd, *J*=13.9, 3.5 Hz, 1 H) 3.32 - 3.56 (m, 5 H) 2.73 - 3.13 (m, 4 H) 1.89 - 1.99 (m, 1 H) 1.78 - 1.86 (m, 1 H) 1.55 (d, *J*=6.9 Hz, 3 H) 1.40 (t, J=7.6 Hz, 3 H).

### Minor rotamer (35%)

¹H NMR (500 MHz, DMSO-*d*₆) δ ppm 8.49 (s, 1 H) 8.43 (d, *J*=3.8 Hz, 1 H) 8.13 (t, *J*=8.7 Hz, 1 H) 7.71 (dd, *J*=14.7, 1.7 Hz, 1 H) 7.46 (dd, *J*=8.8, 1.9 Hz, 1 H) 7.08 - 7.35 (m, 5 H) 5.06 (m, 1 H) 4.98 (d, *J*=3.5 Hz, 1 H) 4.57 (m, 1H) 4.32 (br s, 1 H) 3.32 - 3.56 (m, 5 H) 2.73 - 3.13 (m, 4 H) 1.89 - 1.99 (m, 1 H) 1.78 - 1.86 (m, 1 H) 1.59 (d, *J*=6.9 Hz, 3 H) 1.37 (t, *J*=7.6 Hz, 3 H).

### Compound 40

### Major rotamer (65%)

¹H NMR (400 MHz, DMSO-*d*₆) δ ppm 8.50 (br s, 1H), 8.42 - 8.47 (m, 1H), 8.13 (t, *J*=8.8 Hz, 1H), 7.71 (dd, *J*=14.5, 1.9 Hz, 1H), 7.46 (dd, *J*=8.7, 1.8 Hz, 1H), 7.28 (s, 1H), 6.69 (d, *J*=2.1 Hz, 1H), 5.36 - 5.43 (m, 1H), 4.99 (d, *J*=3.5 Hz, 1H), 4.31 (br s, 1H), 4.01 (br dd, *J*=13.6, 5.0 Hz, 1H), 3.42 - 3.53 (m, 4H), 3.31 - 3.36 (m, 1H), 3.03 - 3.13 (m, 2H), 2.60 - 2.99 (m, 2H), 1.88 - 1.99 (m, 1H), 1.77 - 1.86 (m, 1H), 1.46 (d, *J*=6.7 Hz, 3H), 1.35 - 1.42 (m, 3H).

### Minor rotamer (35%)

¹H NMR (400 MHz, DMSO-*d*₆) δ ppm 8.50 (br s, 1H), 8.42 - 8.47 (m, 1H), 8.13 (t, *J*=8.8 Hz, 1H), 7.71 (dd, *J*=14.5, 1.9 Hz, 1H), 7.46 (dd, *J*=8.7, 1.8 Hz, 1H), 7.25 (s, 1H), 6.44 (d, *J*=1.8 Hz, 1H), 4.99 (d, *J*=3.5 Hz, 1H), 4.85 (q, *J*=6.9 Hz, 1H), 4.63 - 4.74 (m, 1H), 4.31 (br s, 1H), 3.42 - 3.53 (m, 3H), 3.24 - 3.36 (m, 2H), 3.03 - 3.13 (m, 2H), 2.60 - 2.99 (m, 2H), 1.88 - 1.99 (m, 1H), 1.77 - 1.86 (m, 1H), 1.50 (d, *J*=6.7 Hz, 3H), 1.35 - 1.42 (m, 3H).

### Compound 41

### Major rotamer (60%)

¹H NMR (400 MHz, DMSO-*d*₆) δ ppm 8.50 (s, 1 H) 8.41 - 8.48 (m, 1 H) 8.13 (t, *J*=8.7 Hz, 1 H) 7.71 (dd, *J*=14.6, 1.7 Hz, 1 H) 7.46 (dd, *J*=8.7, 1.7 Hz, 1 H) 7.18 - 7.31 (m, 3 H) 6.98 - 7.10 (m, 1 H) 5.63 (q, *J*=6.9 Hz, 1 H) 4.99 (d, *J*=3.4 Hz, 1 H) 4.31 (br s, 1 H) 3.92 (br dd, *J*=13.2, 4.0 Hz, 1 H) 3.42 - 3.54 (m, 4 H) 3.33 - 3.35 (m, 1 H) 3.07 (q, *J*= 7.5 Hz, 2 H) 2.85 - 2.91 (m, 1 H) 2.75 (br d, *J*=16.6 Hz, 1 H) 1.90 - 1.98 (m, 1 H) 1.79 - 1.87 (m, 1 H) 1.57 (d, *J*=6.7 Hz, 1 H) 1.39 (t, *J*=7.9 Hz, 3 H).

### Minor rotamer (40%)

¹H NMR (400 MHz, DMSO-*d*₆) δ ppm 8.50 (s, 1 H) 8.41 - 8.48 (m, 1 H) 8.13 (t, *J*=8.7 Hz, 1 H) 7.71 (dd, *J*=14.6, 1.7 Hz, 1 H) 7.46 (dd, *J*=8.7, 1.7 Hz, 1 H) 7.18 - 7.31 (m, 2 H) 6.98 - 7.10 (m, 2 H) 5.09 (q, *J*=6.5 Hz, 1 H) 4.99 (d, *J*=3.4 Hz, 1 H) 4.57 (br dd, *J*=13.0, 3.4 Hz, 1 H) 4.31 (br s, 1 H) 3.42 - 3.54 (m, 4 H) 3.24 - 3.29 (m, 1 H) 3.07 (q, *J*= 7.5 Hz, 2 H) 2.95 - 3.02 (m, 1H) 2.85 - 2.91 (m, 1 H) 1.90 - 1.98 (m, 1 H) 1.79 - 1.87 (m, 1 H) 1.59 (d, *J*=6.7 Hz, 1 H) 1.39 (t, *J*=7.9 Hz, 3 H).

### Compound 42

### Major rotamer (60%)

¹H NMR (400 MHz, DMSO-*d*₆) δ ppm 8.49 (s, 1 H) 8.38 (d, *J*=3.8 Hz, 1 H) 8.12 (t, *J*=8.7 Hz, 1 H) 7.69 (dd, *J*=14.5, 1.3 Hz 1 H) 7.45 (dd, *J*=8.7, 1.7 Hz, 1 H) 7.10 (s, 1 H) 4.98 (d, *J*=3.6 Hz, 1 H) 4.31 (br s, 1 H) 3.40 - 3.60 (m, 4 H) 3.30-3.38 (m, 2 H )3.16 - 3.29 (m, 1 H) 3.07 (q, *J*=7.5 Hz, 2 H) 1.75 - 2.16 (m, 3 H) 1.37 (t, *J*=7.6 Hz, 3 H) 1.18 - 1.27 (m, 6 H) 0.81 (d, *J*=6.6 Hz, 3 H) 0.77 (d, *J*=6.5 Hz, 3 H).

### Minor rotamer (40%)

¹H NMR (400 MHz, DMSO-*d*₆) δ ppm 8.49 (s, 1 H) 8.42 (d, *J*=3.8 Hz, 1 H) 8.12 (t, *J*=8.7 Hz, 1 H) ) 7.69 (dd, J=14.5, 1.3 Hz 1 H) 7.45 (dd, *J*=8.7, 1.7 Hz, 1 H) 7.15 (s, 1 H) 4.98 (d, *J*=3.6 Hz, 1 H) 4.31 (br s, 1 H) 3.77 - 3.89 (m, 1 H) 3.30-3.38 (m, 2 H) 3.40 - 3.60 (m, 3 H) 3.16 - 3.29 (m, 1 H) 3.07 (q, *J*=7.5 Hz, 2 H) 2.46-2.58 (m, 1 H) 1.75-2.00 (m, 2 H) 1.38 (t, *J*=7.6 Hz, 3 H) 1.31 (d, *J*=6.9 Hz, 3 H) 1.07 (t, *J*=7.0 Hz, 3 H) 0.97 (d, *J*=7.0 Hz, 3 H) 0.95 (d, *J*=7.0 Hz, 3 H).

### Compound 43

### Major rotamer (60%)

¹H NMR (400 MHz, DMSO-*d*₆) δ ppm 8.60 (d, *J*=3.9 Hz, 1 H) 8.53 (s, 1 H) 8.36 - 8.46 (m, 2 H) 8.16 (t, *J*=8.7 Hz, 1 H) 7.84 (s, 1 H) 7.63 - 7.76 (m, 2 H) 7.41 - 7.51 (m, 2 H) 7.35 (d, *J*=7.2 Hz, 1 H) 7.09 - 7.27 (m, 3 H) 5.65 (q, *J*=6.6 Hz, 1 H) 4.99 (d, *J*=3.7 Hz, 1 H) 4.32 (br s, 1 H) 3.97 (br dd, *J*=13.8, 3.8 Hz, 1 H) 3.44 - 3.59 (m, 4 H) 3.33 - 3.39 (m, 1 H) 2.87 - 3.14 (m, 1 H) 2.76 (br d, *J*=16.1 Hz, 1 H) 1.88 - 2.00 (m, 1 H) 1.78 - 1.87 (m, 1 H) 1.58 (d, *J*=6.9 Hz, 3 H).

### Minor rotamer (40%)

¹H NMR (400 MHz, DMSO-*d*₆) δ ppm 8.58 (d, *J*=3.8 Hz, 1 H) 8.53 (s, 1 H) 8.36 - 8.46 (m, 2 H) 8.16 (t, *J*=8.7 Hz, 1 H) 7.79 (s, 1 H) 7.63 - 7.76 (m, 2 H) 7.41 - 7.51 (m, 2 H) 7.09 - 7.27 (m, 4 H) 5.14 (q, *J*=6.3 Hz, 1 H) 4.99 (d, *J*=3.7 Hz, 1 H) 4.58 - 4.64 (m, 1 H) 4.32 (br s, 1 H) 3.44 - 3.52 (m, 3 H) 3.33 - 3.39 (m, 1 H) 2.87 - 3.14 (m, 2 H) 2.52 - 2.56 (m, 1 H) 1.88 - 2.00 (m, 1 H) 1.78 - 1.87 (m, 1 H) 1.62 (d, *J*=6.7 Hz, 3 H).

### Compound 44

### Major rotamer (60%)

¹H NMR (500 MHz, DMSO-*d*₆) δ ppm 8.90 (d, *J*=11.4 Hz, 1 H) 8.38 (d, *J*=3.5 Hz, 1 H) 7.90-8.04 (m, 1 H) 7.33 (d, *J*=7.6 Hz, 1 H) 7.08 - 7.29 (m, 4 H) 6.43 (d, *J*=13.9 Hz, 1 H) 5.61 (q, *J*=6.9 Hz, 1 H) 3.89 (dd, *J*=13.9, 3.8 Hz, 1 H) 3.69 (t, *J*=8.8 Hz, 1 H) 3.54-3.64 (m, 1 H) 3.37 - 3.56 (m, 3 H) 3.00 - 3.14 (m, 4 H) 2.76 (m, 1 H) 2.61 (d, 3 H) 2.00 - 2.23 (m, 2 H) 1.54 (d, *J*=6.6 Hz, 3 H) 1.38 - 1.41 (m, *J*=13.2 Hz, 3 H).

### Minor rotamer (40%)

¹H NMR (500 MHz, DMSO-*d*₆) δ ppm 8.90 (d, *J*=11.4 Hz, 1 H) 8.37 (d, *J*=3.5 Hz, 1 H) 7.90-8.04 (m, 1 H) 7.33 (d, *J*=7.57 Hz, 1 H) 7.08-7.29 (m, 4 H) 6.43 (d, *J*=13.9 Hz, 1 H) 5.05 (q, *J*=6.6 Hz, 1 H) 4.56 (dd, *J*=13.2, 3.8 Hz, 1 H ) 3.66 (t, *J*=8.8 Hz, 1 H) 3.54-3.64 (m, 1 H) 3.37 - 3.56 (m, 3 H) 3.00 - 3.14 (m, 4 H) 2.89 (m, 1 H) 2.61 (d, 3 H) 2.00 - 2.23 (m, 2 H) 1.58 (d, *J*=6.6 Hz, 3 H) 1.36 (t, *J*=13.7 Hz, 3 H).

### Compound 45

### Major rotamer (60%)

¹H NMR (500 MHz, DMSO-*d*₆) δ ppm 8.55 (d, *J*=3.2 Hz, 1 H) 8.15 (t, *J*=8.4 Hz, 1 H) 7.95 (s, 1 H) 7.57 (s, 1 H) 7.28 - 7.41 (m, 2 H) 7.09-7.27 (m, 4 H) 6.90 (d, *J*=8.5 Hz, 1 H) 5.62 (q, *J*=6.3 Hz, 1 H) 4.99-5.06 (m, 2 H) 3.89-3.92 (m, 1 H) 3.50-3.55 (m, 1 H) 3.06 - 3.11 (m, 3 H) 2.75 (d, *J*= 16.1 Hz, 1 H) 1.54 (d, *J*= 6.6 Hz, 3 H) 1.40 (t, J=7.3 Hz, 3 H).

### Minor rotamer (40%)

¹H NMR (500 MHz, DMSO-*d*₆) δ ppm 8.51 (d, *J*=2.8 Hz, 1 H) 8.15 (t, *J*=8.4 Hz, 1 H) 7.95 (s, 1 H) 7.57 (s, 1 H) 7.28-7.41 (m, 2 H) 7.09-7.27 (m, 4 H) 6.90 (d, *J*=8.5 Hz, 1 H) 5.05 (q, *J*= 6.3 Hz 1 H) 4.99-5.03 (m, 2 H) 4.56-4.59 (m, 1 H) 3.06 - 3.11 (m, 3 H) 3.01-3.05 (m, 1 H) 2.88 (d, *J*= 16.4 Hz, 1 H) 1.58 (*d, J*= 6.6 Hz, 3 H) 1.35 (t, *J*=7.3 Hz, 3 H) .

### Compound 46

### Major rotamer (60%)

¹H NMR (500 MHz, DMSO-*d*₆) δ ppm 8.61-8.62 (m, *J*=3.8 Hz, 1 H) 7.84 (t, *J*=7.3 Hz, 1 H) 7.78 (br s, 1 H) 7.30-7.40 (m, 3 H) 7.09-7.27 (m, 5 H) 5.62 (q, *J*=6.5 Hz, 1 H) 5.04 (s, 2 H) 3.89-3.95 (m, 1 H) 3.49 - 3.56 (m, 1 H) 3.05-3.12 (m, 3 H) 2.75-2.78 (m, 1 H) 1.54 (d, *J*=6.9 Hz, 3 H) 1.40 (t, *J*=7.5 Hz 3 H).

### Minor rotamer (40%)

¹H NMR (500 MHz, DMSO-*d*₆) δ ppm 8.59 - 8.60 (m, *J*=3.8 Hz, 1 H) 7.84 (t, *J*=7.3 Hz, 1 H) 7.78 (br s, 1 H) 7.30 - 7.40 (m, 3 H) 7.09 - 7.27 (m, 5 H) 5.06 (q, *J*=6.6 Hz, 1 H) 5.04 (s, 2 H) 4.56-4.59 (m, 1 H) 3.05 - 3.12 (m, 3 H) 3.02-3.04 (m, 1 H) 2.85 - 2.98 (m, 1 H) 1.58 (d, *J*=6.6 Hz, 3 H) 1.36 (t, *J*=7.5 Hz 3 H).

### Compound 47

### Major rotamer (70%)

¹H NMR (500 MHz, DMSO-*d*₆) δ ppm 8.76 - 8.82 (m, 1H), 8.52 (s, 1H), 8.40 - 8.43 (m, 1H), 8.18 (br t, *J*=8.7 Hz, 1H), 7.70 - 7.74 (m, 1H), 7.48 (dd, *J*=8.7, 1.1 Hz, 1H), 7.06 - 7.29 (m, 4H), 5.51 (q, *J*=6.3 Hz, 1H), 4.99 (d, *J*=3.2 Hz, 1H), 4.32 (br s, 1H), 4.12 (br dd, *J*=13.1, 3.9 Hz, 1H), 3.39 - 3.49 (m, 4H), 2.79 - 3.37 (m, 4H), 1.83 - 1.96 (m, 2H), 1.51 (br d, *J*=6.6 Hz, 3H), 1.22 - 1.26 (m, 4 H)

### Minor rotamer (30%)

¹H NMR (500 MHz, DMSO-*d*₆) δ ppm 8.76 - 8.82 (m, 1H), 8.52 (s, 1H), 8.40 - 8.43 (m, 1 H), 8.18 (br t, *J*=8.7 Hz, 1H), 7.70 - 7.74 (m, 1H), 7.48 (dd, *J*=8.7, 1.1 Hz, 1H), 7.06 - 7.29 (m, 4 H), 5.27 (br d, *J*=6.3 Hz, 1H), 4.99 (d, *J*=3.2 Hz, 1H), 4.51 (br dd, *J*=12.0, 4.1 Hz, 1H), 4.32 (br s, 1H), 3.39 - 3.49 (m, 4 H), 2.79 - 3.37 (m, 4 H), 1.83 - 1.96 (m, 2 H), 1.61 (br d, *J*=6.6 Hz, 3 H), 1.22 - 1.26 (m, 4 H)

### Compound 48

### Major rotamer (65%)

¹H NMR (500 MHz, DMSO-*d*₆) δ ppm 8.72 - 8.77 (m, 1H), 8.29 - 8.31 (m, 1H), 8.10 (t, *J*=8.7 Hz, 1H), 7.07 - 7.30 (m, 4H), 6.41 - 6.51 (m, 2H), 5.51 (br d, *J*=6.3 Hz, 1H), 5.15 (d, *J*=3.2 Hz, 2H), 4.13 (br d, *J*=9.5 Hz, 1H), 4.07 (br s, 2 H), 3.53 (br dd, *J*=10.1, 3.2 Hz, 2H), 3.38 - 3.39 (m, 1H), 2.78 - 3.27 (m, 5H), 1.51 (br d, *J*=6.6 Hz, 3H), 1.16 - 1.33 (m, 4H)

### Minor rotamer (25%)

¹H NMR (500 MHz, DMSO-*d*₆) δ ppm 8.72 - 8.77 (m, 1H), 8.29 - 8.31 (m, 1H), 8.10 (t, *J*=8.7 Hz, 1H), 7.07 - 7.30 (m, 4H), 6.41 - 6.51 (m, 2H), 5.29 (br d, *J*=6.0 Hz, 1H), 5.15 (d, *J*=3.2 Hz, 2H), 4.51 (br d, *J*=8.5 Hz, 1H), 4.07 (br s, 2H), 3.53 (br dd, *J*=10.1, 3.2 Hz, 2H), 3.38 - 3.39 (m, 1H), 2.78 - 3.27 (m, 5H), 1.60 (br d, *J*=5.7 Hz, 3H), 1.16 - 1.33 (m, 4H).

### Compound 49

### Major rotamer (65%)

¹H NMR (500 MHz, DMSO-*d*₆) δ ppm 8.39 (br s, 1 H) 8.22 (br s, 1 H) 7.78 (br t, *J*=8.8 Hz, 1 H) 7.64 (br d, *J*=14.8 Hz, 1 H) 7.41 (br d, *J*=8.2 Hz, 1 H) 7.31 (br d, *J*=7.3 Hz, 1 H) 7.09 - 7.27 (m, 4 H) 6.40 (s, 1 H) 5.58 (br q, *J*=6.6 Hz, 1 H) 4.97 (br s, 1 H) 4.31 (br s, 1 H) 3.84 (br d, *J*=10.7 Hz, 1 H) 3.42-3.57 (m, 4 H) 3.24-3.37 (m, 1H) 2.81 - 3.10 (m, 2 H) 2.75 (br d, *J*=16.1 Hz, 1 H) 1.89-1.99 (m, 1 H) 1.78-1.86 (m, 1 H) 1.51 (d, *J*=6.6 Hz, 3 H) 0.99 - 1.21 (m, 4 H).

### Minor rotamer (35%)

¹H NMR (500 MHz, DMSO-*d*₆) δ ppm 8.39 (br s, 1 H) 8.22 (br s, 1 H) 7.78 (br t, *J*=8.8 Hz, 1 H) 7.64 (br d, *J*=14.8 Hz, 1 H) 7.41 (br d, *J*=8.2 Hz, 1 H) 7.09 - 7.27 (m, 5 H) 6.36 (s, 1 H) 5.01 (br q, *J*=7.3 Hz, 1 H) 4.97 (br s, 1 H) 4.54 (br d, *J*=10.7 Hz, 1 H) 4.31 (br s, 1 H) 3.42-3.57 (m, 4 H) 3.24-3.37 (m, 1 H) 2.81 - 3.10 (m, 2 H) 2.75 (br d, *J*=16.1 Hz, 1 H) 1.89-1.99 (m, 1 H) 1.78-1.86 (m, 1 H) 1.57 (d, *J*=6.6 Hz, 3 H) 0.99 - 1.21 (m, 4 H).

### Compound 50

¹H NMR (500 MHz, DMSO-*d*₆, 350 K) δ ppm 8.33 (s, 1 H) 8.17 (br s, 1 H) 7.94 (br s, 1 H) 7.66 (br t, *J*=8.8 Hz, 1 H) 7.58 (br d, *J*=14.5 Hz, 1 H) 7.36 (br d, *J*=8.2 Hz, 1 H) 7.12 - 7.22 (m, 4 H) 6.84 (s, 1 H) 6.54 (s, 1 H) 5.32 - 5.42 (m, 1 H) 4.76 (br s, 1 H) 4.31 (br s, 1 H) 4.04 - 4.15 (m, 1 H) 3.44 - 3.51 (m, 3 H) 3.36 - 3.44 (m, 1 H) 3.31 (br d, *J*=11.0 Hz, 1 H) 2.98-3.04 (m, 1 H) 2.73 - 2.83 (m, 3 H) 1.90 - 1.99 (m, 1 H) 1.78-1.85 (m, 1 H) 1.52 (br d, *J*=6.6 Hz, 3 H) 1.31 (t, *J*=7.6 Hz, 3H).

### Compound 51

### Major rotamer (65%)

¹H NMR (400 MHz, DMSO-*d*₆) δ ppm 8.30 (d, *J*=3.9 Hz, 1 H) 8.06 (t, *J*=8.8 Hz, 1 H) 7.32 (d, J=7.3 Hz, 1 H) 7.09 - 7.26 (m, 3 H) 6.96 (s, 1 H) 6.50 (dd, *J*=8.7, 1.6 Hz 1 H) 6.42 (dd, *J*=14.6, 1.6 Hz, 1 H) 5.59 (q, *J*=6.6 Hz, 1 H) 5.18 (d, *J*=3.2 Hz, 2 H) 4.06-4.09 (m, 2 H) 3.84 (br dd, *J*=13.8, 4.3 Hz, 1 H) 3.52 (dd, *J*=10.5, 3.7 Hz, 2 H) 3.44 - 3.50 (m, 1 H) 3.16 (br d, *J*=10.4 Hz, 2 H) 2.69 - 3.10 (m, 2 H) 2.55 - 2.66 (m, 1 H) 1.53 (d, *J*=6.9 Hz, 3 H) 1.21 - 1.38 (m, 4 H).

### Minor rotamer (35%)

¹H NMR (400 MHz, DMSO-*d*₆) δ ppm 8.28 (d, *J*=3.9 Hz, 1 H) 8.06 (t, *J*=8.8 Hz, 1 H) 7.09 - 7.26 (m, 4 H) 6.92 (s, 1 H) 6.50 (dd, *J*=8.7, 1.6 Hz 1 H) 6.42 (dd, J=14.6, 1.6 Hz, 1 H) 5.18 (d, *J*=3.2 Hz, 2 H) 5.00 (br d, *J*=6.8 Hz, 1 H) 4.55 (br dd, *J*=10.8, 3.7 Hz, 1 H) 4.06 - 4.09 (m, 2 H) 3.52 (dd, *J*=10.5, 3.7 Hz, 2 H) 3.22 - 3.31 (m, 1 H) 3.16 (br d, *J*=10.4 Hz, 2 H) 2.69-3.10 (m, 2 H) 2.55 - 2.66 (m, 1 H) 1.56 (d, *J*=6.9 Hz, 3 H) 1.21 - 1.38 (m, 4 H).

### LC-MS data

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rₜ) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc...). For molecules with multiple isotopic patterns (Br, Cl..), the reported value is the one obtained for the lowest isotope mass. All results were obtained with experimental uncertainties that are commonly associated with the method used.

Hereinafter, "SQD" means Single Quadrupole Detector, "RT" room temperature, "BEH" bridged ethylsiloxane/silica hybrid, "HSS" High Strength Silica, "DAD" Diode Array Detector.

**Table: LCMS Method codes (Flow expressed in mL/min; column temperature (T) in°C; Run time in minutes).**

| Method code | Instrument | Column | Mobile phase | gradient | Flow Column T | Run time |
|---|---|---|---|---|---|---|
| Method 1 | Waters: Acquity UPLC^{®} - DAD and Quattro Micro^{™} | Waters: BEH C18 (1.7µm, 2.1x100mm) | A: 95% CH₃COONH₄ 7mM / 5% CH₃CN, B: CH₃CN | 84.2% A for 0.49min, to 10.5% A in 2.18min, held for 1.94min, back to 84.2% A in 0.73min, held for 0.73min. | 0.343 | 6.2 |
| | | | | | 40 | |
| Method 2 | Waters: Acquity UPLC^{®} H-Class - DAD and SQD 2 | Waters: BEH C18 (1.7µm, 2.1×100mm) | A: 95% CH₃COONH₄ 7mM / 5% CH₃CN, B: CH₃CN | 84.2% A for 0.49min, to 10.5% A in 2.18min, held for 1.94min, back to 84.2% A in 0.73min, held for 0.73min. | 0.343 | 6.07 |
| | | | | | 40 | |

| **Co. No.** | **Rt** | **MW (theor)** | **BPM1 [M+H]⁺** | **LC/GC/MS Method** |
|---|---|---|---|---|
| 1 | 2.28 | 469.2 | 470.2 | Method 1 |
| 2 | 2.8 | 468.2 | 469.2 | Method 1 |
| 3 | 3.03 | 440.2 | 441.3 | Method 1 |
| 4 | 2.73 | 553.2 | 554.4 | Method 1 |
| 5 | 2.63 | 553.2 | 554.5 | Method 2 |
| 6 | 2.48 | 509.2 | 510.2 | Method 1 |
| 7 | 2.97 | 508.2 | 509.3 | Method 1 |
| 8 | 2.54 | 586.2 | 587.4 | Method 1 |
| 9 | 2.51 | 538.2 | 539.4 | Method 1 |
| 10 | 3.01 | 537.2 | 538.4 | Method 1 |
| 11 | 3.1 | 551.2 | 552.5 | Method 1 |
| 12 | 3.13 | 510.2 | 511.4 | Method 1 |
| 13 | 2.98 | 543.2 | 544.3 | Method 1 |
| 14 | 2.92 | 557.2 | 558.6 | Method 2 |
| 15 | 2.92 | 503.3 | 504.4 | Method 1 |
| 16 | 3.07 | 573.3 | 574.4 | Method 1 |
| 17 | 2.76 | 574.2 | 575.5 | Method 1 |
| 18 | 2.72 | 580.2 | 581.4 | Method 1 |
| 19 | 2.99 | 540.3 | 541.5 | Method 1 |
| 20 | 3.28 | 566.3 | 567.5 | Method 1 |
| 21 | 2.81 | 551.3 | 552.5 | Method 2 |
| 22 | 2.91 | 538.2 | 539.4 | Method 1 |
| 23 | 2.73 | 544.2 | 545.5 | Method 2 |
| 24 | 3.01 | 522.2 | 523.3 | Method 1 |
| 25 | 2.41 | 535.2 | 536.3 | Method 1 |
| 26 | 2.94 | 534.2 | 535.3 | Method 1 |
| 27 | 3.05 | 496.2 | 497.4 | Method 1 |
| 28 | 2.46 | 601.2 | 602.5 | Method 1 |
| 29 | 2.3 | 523.2 | 524.5 | Method 2 |
| 30 | 2.71 | 539.2 | 540.2 | Method 1 |
| 31 | 2.38 | 495.2 | 496.2 | Method 1 |
| 32 | 2.5 | 510.2 | 511.3 | Method 1 |
| 33 | 2.41 | 476.2 | 477.3 | Method 1 |
| 34 | 2.84 | 515.2 | 516.3 | Method 1 |
| 35 | 3.13 | 485.2 | 486.2 | Method 1 |
| 36 | 2.4 | 484.2 | 485.2 | Method 1 |
| 37 | 2.81 | 554.2 | 555.3 | Method 1 |
| 38 | 2.78 | 560.2 | 561.3 | Method 1 |
| 39 | 2.77 | 542.2 | 543.3 | Method 1 |
| 40 | 2.88 | 566.2 | 567.2 | Method 1 |
| 41 | 2.8 | 560.2 | 561.3 | Method 1 |
| 42 | 2.77 | 510.3 | 511.2 | Method 1 |
| 43 | 3.1 | 608.2 | 609.3 | Method 1 |
| 44 | 2.85 | 541.3 | 542.3 | Method 1 |
| 45 | 3.06 | 511.2 | 512.3 | Method 1 |
| 46 | 2.98 | 511.2 | 512.2 | Method 1 |
| 47 | 2.86 | 554.2 | 555.3 | Method 1 |
| 48 | 2.92 | 527.2 | 528.3 | Method 1 |
| 49 | 2.96 | 553.2 | 554.5 | Method 1 |
| 50 | 3.04 | 540.3 | 541.5 | Method 1 |
| 51 | 2.9 | 527.2 | 528.5 | Method 1 |

### Optical rotation

The optical rotation was measured using a polarimeter with light at the wavelength of the D-line of sodium (589 nm) at a temperature of 20°C in DMF as solvent. Specific optical rotation of compounds (20) and (47) was measured at 436 nm in DMF at 20°C as solvent.

| Co. No. | **[α]_{D}²⁰** | c (w/v %) | Co. No. | **[α]_{D}²⁰** | c (w/v %) |
|---|---|---|---|---|---|
| 1 | -25° | 0.28 | 29 | -21.11° | 0.18 |
| 2 | -23.32° | 0.253 | 30 | -20° | 0.25 |
| 3 | -24.62° | 0.26 | 31 | -15.85° | 0.246 |
| 4 | -23.42° | 0.269 | 32 | +149.17° | 0.24 |
| 5 | -20.61° | 0.228 | 33 | +226.55° | 0.29 |
| 9 | +22.22° | 0.27 | 34 | +52.86° | 0.28 |
| 11 | -22.89° | 0.1398 | 35 | +26.15° | 0.26 |
| 12 | -26.04° | 0.288 | 36 | +20° | 0.26 |
| 13 | -26.37° | 0.273 | 37 | +14.52° | 0.31 |
| 14 | -24.66° | 0.296 | 38 | +7.14° | 0.266 |
| 15 | -15.81° | 0.253 | 39 | +24.75° | 0.299 |
| 16 | -11.94° | 0.31 | 40 | +26.14° | 0.241 |
| 17 | -10.9° | 0.1376 | 41 | +28.46° | 0.253 |
| 18 | -18.35° | 0.1471 | 43 | +24.44° | 0.27 |
| 19 | -9.09° | 0.275 | 44 | +14.81° | 0.27 |
| 20 | +15.67° | 0.3 | 45 | +34.07° | 0.27 |
| 21 | -18.67° | 0.3 | 46 | +38.28° | 0.29 |
| 22 | -28.62° | 0.276 | 47 | +22.12° | 0.208 |
| 23 | -28.15° | 0.27 | 48 | +18.85° | 0.26 |
| 24 | -27° | 0.3 | 49 | +20.77° | 0.26 |
| 27 | -26.46° | 0.291 | 50 | -19.22° | 0.25 |
| 28 | -21.6° | 0.287 | 51 | +37.18° | 0.39 |

### E. Pharmacological examples

### E.1 Antiviral activity

Black 384-well clear-bottom microtiter plates (Corning, Amsterdam, The Netherlands) were filled via acoustic drop ejection using the echo liquid handler (Labcyte, Sunnyvale, California). 200 nL of compound stock solutions (100% DMSO) were transferred to the assay plates. 9 serial 4-fold dilutions of compound were made, creating per quadrant the same compound concentration. The assay was initiated by adding 10 µL of culture medium to each well (RPMI medium without phenol red, 10% FBS-heat inactivated, 0.04% gentamycin (50 mg/mL). All addition steps are done by using a multidrop dispenser (Thermo Scientific, Erembodegem, Belgium). Next, rgRSV224 virus (MOI = 1) diluted in culture medium was added to the plates. rgRSV224 virus is an engineered virus that includes an additional GFP gene (Hallak LK, Spillmann D, Collins PL, Peeples ME. Glycosaminoglycan sulfation requirements for respiratory syncytial virus infection; Journal of virology (2000), 74(22), 10508-13) and was in-licensed from the NIH (Bethesda, MD, USA). Finally, 20 µL of a HeLa cell suspension (3,000 cells/well) were plated. Medium, virus- and mock-infected controls were included in each test. The wells contain 0.05% DMSO per volume. Cells were incubated at 37°C in a 5% CO2 atmosphere. Three days post-virus exposure, viral replication was quantified by measuring GFP expression in the cells by an in house developed MSM laser microscope (Tibotec, Beerse, Belgium). The EC₅₀ was defined as the 50% inhibitory concentration for GFP expression. In parallel, compounds were incubated for three days in a set of white 384-well microtiter plates (Corning) and the cytotoxicity of compounds in HeLa cells was determined by measuring the ATP content of the cells using the ATPlite kit (Perkin Elmer, Zaventem, Belgium) according to the manufacturer's instructions. The CC₅₀ was defined as the 50% concentration for cytotoxicity.

**Table : antiviral data (averaged data of several repeat experiments)**

| Co. No. | RSV HELA EC₅₀ (µM) | TOX HELA CC₅₀ (µM) | Co. No. | RSV HELA EC₅₀ (µM) | TOX HELA CC₅₀ (µM) |
|---|---|---|---|---|---|
| 1 | 0.510 | 60.4 | 26 | 0.202 | 42.7 |
| 2 | 0.072 | 54 | 27 | 0.119 | 98.1 |
| 3 | 0.054 | 68 | 28 | 3.112 | >100 |
| 4 | 0.035 | 51.4 | 29 | 0.146 | 49.5 |
| 5 | 0.048 | 40.1 | 30 | 0.089 | 43.4 |
| 6 | 0.103 | 56.2 | 31 | 0.158 | 49.9 |
| 7 | 0.184 | 65.4 | 32 | 0.010 | 51.5 |
| 8 | 0.793 | 69.1 | 33 | 0.067 | 50.5 |
| 9 | 0.045 | 68.4 | 34 | 0.012 | 24.5 |
| 10 | 0.049 | 70.4 | 35 | 0.021 | 16.3 |
| 11 | 0.062 | >100 | 36 | 0.039 | 17.2 |
| 12 | 0.169 | 67.7 | 37 | 0.011 | 32.2 |
| 13 | 0.037 | 77.1 | 38 | 0.012 | 35.9 |
| 14 | 0.084 | >100 | 39 | 0.014 | 29.3 |
| 15 | 1.070 | 13.2 | 40 | 0.017 | 29.9 |
| 16 | 0.186 | 93.6 | 41 | 0.026 | 26.8 |
| 17 | 0.163 | 44.3 | 42 | 5.555 | 46.8 |
| 18 | 0.108 | 21.7 | 43 | 0.022 | >100 |
| 19 | 0.141 | 43.9 | 45 | 0.105 | >100 |
| 20 | 0.149 | >100 | 46 | 0.054 | >100 |
| 21 | 0.638 | 35 | 47 | 2.771 | >100 |
| 22 | 0.171 | 37.4 | 48 | 3.767 | 58.8 |
| 23 | 0.180 | 34.7 | 49 | 0.048 | 45.5 |
| 24 | 0.145 | 36.8 | 50 | 1.07 | 41.8 |
| 25 | 0.369 | 51.3 | 51 | 0.013 | 38 |

### F. Prophetic composition examples

"Active ingredient" as used throughout these examples relates to a final compound of Formula (I), the pharmaceutically acceptable salts thereof, the solvates and the stereochemically isomeric forms and the tautomers thereof.

Typical examples of recipes for the formulation of the invention are as follows:

### F.1. Tablets

| | |
|---|---|
| Active ingredient | 5 to 50 mg |
| Di calcium phosphate | 20 mg |
| Lactose | 30 mg |
| Talcum | 10 mg |
| Magnesium stearate | 5 mg |
| Potato starch | ad 200 mg |

In this Example, active ingredient can be replaced with the same amount of any of the compounds according to the present invention, in particular by the same amount of any of the exemplified compounds.

### F.2. Suspension

An aqueous suspension is prepared for oral administration so that each 1 milliliter contains 1 to 5 mg of one of the active compounds, 50 mg of sodium carboxymethyl cellulose, 1 mg of sodium benzoate, 500 mg of sorbitol and water ad 1 ml.

### F.3. Injectable

A parenteral composition is prepared by stirring 1.5 % by weight of active ingredient of the invention in 10% by volume propylene glycol in water.

### F.4. Ointment

| | |
|---|---|
| Active ingredient | 5 to 1000 mg |
| Stearyl alcohol | 3 g |
| Lanoline | 5 g |
| White petroleum | 15 g |
| Water | ad 100 g |

In this Example, active ingredient can be replaced with the same amount of any of the compounds according to the present invention, in particular by the same amount of any of the exemplified compounds.

## Claims

1. A compound of formula (I) wherein 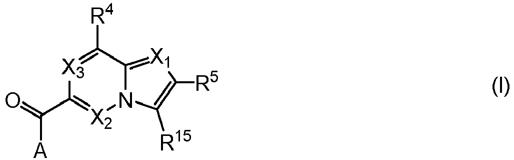 including any stereochemically isomeric form thereof, wherein
A is 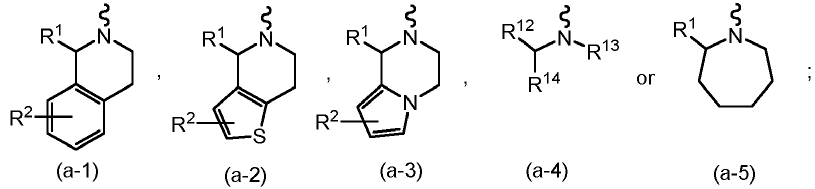 R⁵ is 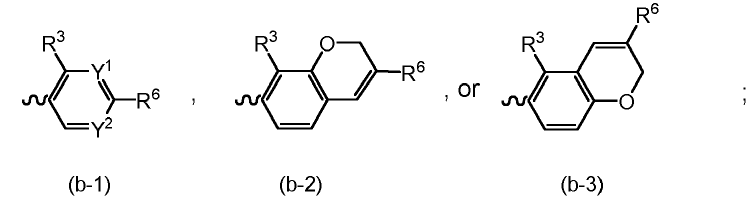
X₁, X₂, and X₃ are selected from X₁ is N, X₂ is CH, and X₃ is CH;
or X₁ is N, X₂ is N, and X₃ is CH,
or X₁ is N, X₂ is CH, and X₃ is N,
or X₁ is CH, X₂ is CH, and X₃ is CH, and
or X₁ is CH, X₂ is N, and X₃ is CH,
wherein each CH is optionally substituted with halo or C₁₋₄alkyl;
Y¹ and Y² are each independently selected from CH, CF and N;
R¹ is CH₃ or CH₂CH₃;
R² is hydrogen, halo or C₁₋₄alkyl;
R¹² is C₁₋₂alkyl;
R¹³ and R¹⁴ are each independently selected from C₁₋₆alkyl;
R¹⁵ is hydrogen or C₁₋₄alkyl;
R³ is halo;
R⁴ is C₁₋₆alkyl; C₃₋₆cycloalkyl; di(C₁₋₄alkyl)amino, pyrrolidinyl, Heteroaryl¹; phenyl; phenyl substituted with 1, 2 or 3 substituents each individually selected from halo, hydroxy, cyano, C₁₋₄alkyl, polyhaloC₁₋₄alkyl, and C₁₋₄alkyloxy;
R⁶ is a substituent selected from substituent (a), (b), (c), (d) or (e); wherein
(a) is -(CO)-OH, -(CO)-NR⁷R⁸, or -NR⁷R⁸;
(b) is Heteroaryl²;
(c) is C₂₋₆alkenyl substituted with one or two substituents selected from C₁₋₆alkyl, -(CO)-OH or -(CO)-NR⁸R⁹; or
(d) is -NR⁸-(CO)-Heterocycle wherein said Heterocycle is substituted with one, two or three substituents each independently selected from halo, hydroxy, C₁₋₄alkyl of C₁₋₄alkyloxy; or
(e) is C₃₋₆cycloalkyl or Heterocycle, wherein said C₃₋₆cycloalkyl and Heterocycle is substituted with one, two or three substituents each independently selected from
C₁₋₆alkyl;
C₁₋₆alkyl substituted with one, two or three substituents each independently selected from halo, hydroxy, hydroxycarbonyl, and aminocarbonyl;
hydroxy;
halo;
-(CO)-OH;
-(CO)-NR¹⁰R¹¹;
-(CO)-NR⁸-SO₂-R⁹;
-NR⁸R⁹;
-NR⁸-(CO)-C₁₋₄alkyl;
-NR⁸-(CO)-C₃₋₆cycloalkyl;
-NR⁸-SO₂-R⁹;
-SO₂-NR¹⁰R¹¹; or
-SO₂-NR⁸-(CO)-R⁹;
wherein
R⁷ is hydrogen, C₁₋₄alkyl, hydroxyC₁₋₄alkyl or dihydroxyC₁₋₄alkyl;
each R⁸ is independently selected from hydrogen, C₁₋₄alkyl, or hydroxyC₁₋₄alkyl;
R⁹ is C₁₋₄alkyl, polyhaloC₁₋₄alkyl, or C₃₋₆cycloalkyl;
R¹⁰ and R¹¹ are each indepently selected from hydrogen; C₁₋₄alkyl; polyhaloC₁₋₄alkyl; C₃₋₆cycloalkyl; C₃₋₆cycloalkyl substituted with C₁₋₄alkyl; or C₁₋₄alkyl substituted with hydroxy or cyano;
Heterocycle is azetidinyl, pyrrolodinyl, piperidinyl, or homopiperidinyl;
Heteroaryl¹ is thienyl, pyridinyl or pyrimidinyl, wherein each Heteroaryl¹ is optionally substituted with one or two substituents each independently selected from C₁₋₄alkyl, halo, amino, and aminocarbonyl;
Heteroaryl² is pyrrolyl, pyrazolyl or thiazolyl; wherein each Heteroaryl² is optionally substituted with one or two substituents each independently selected from C₁₋₄alkyl, halo, -(CO)-OR⁷ or -(CO)-NR⁸R⁹;
or a pharmaceutically acceptable acid addition salt thereof.

2. The compound as claimed in claim 1 wherein X₁ is N, X₂ is CH, and X₃ is CH.

3. The compound as claimed in claim 1 wherein X₁ is N, X₂ is N, and X₃ is CH.

4. The compound as claimed in claim 1 wherein X₁ is N, X₂ is CH, and X₃ is N.

5. The compound as claimed in claim 1 wherein A is a radical of formula (a-1) wherein R¹ is CH₃.

6. The compound as claimed in claim 1 wherein A is a radical of formula (a-2) wherein R¹ is CH₃.

7. The compound as claimed in claim 1 wherein A is a radical of formula (a-5) wherein R¹ is CH₃.

8. The compound as claimed in any one of claims 1 to 7 wherein R⁴ is C₃₋₆cycloalkyl.

9. The compound as claimed in any one of claims 1 to 8 wherein R⁶ is C₃₋₆cycloalkyl or pyrrolidinyl, wherein said C₃₋₆cycloalkyl or pyrrolidinyl are substituted with one or two substituents each independently selected from OH, -(CO)-OH or -(CO)-NR¹⁰R¹¹.

10. A pharmaceutical composition comprising a pharmaceutically acceptable carrier and a therapeutically active amount of a compound as claimed in any one of claims 1 to 9.

11. The pharmaceutical composition according to claim 10, which further comprises another antiviral agent.

12. The pharmaceutical composition according to claim 11, wherein the other antiviral agent is a RSV inhibiting compound.

13. A process for preparing a pharmaceutical composition as claimed in any one of claims 10 to 12 wherein a therapeutically active amount of a compound as claimed in any one of claims 1 to 9 is intimately mixed with a pharmaceutically acceptable carrier.

14. A compound as claimed in any one of claims 1 to 9 for use as a medicine.

15. A compound as claimed in any one of claims 1 to 9, or a pharmaceutical composition as claimed in any one of claims 10 to 12, for use in the treatment or prevention of a respiratory syncytial virus infection.

## Patentansprüche

1. Verbindung der Formel (I) 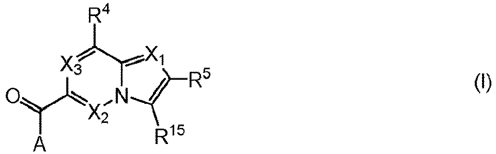 einschließlich aller stereochemisch isomeren Formen davon, wobei
A für 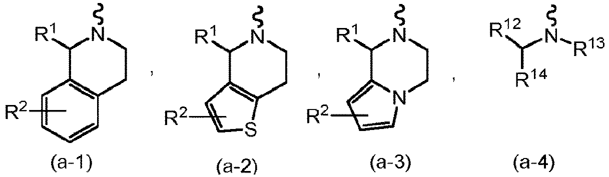 oder steht; 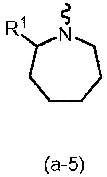
R⁵ für oder steht; 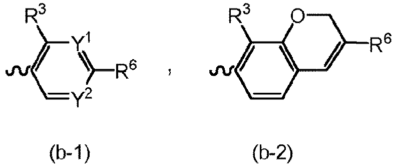 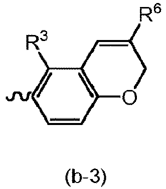
X₁, X₂ und X₃ ausgewählt sind aus
X₁ steht für N, X₂ steht für CH und X₃ steht für CH
oder X₁ steht für N, X₂ steht für N und X₃ steht für CH
oder X₁ steht für N, X₂ steht für CH und X₃ steht für N
oder X₁ steht für CH, X₂ steht für CH und X₃ steht für CH und
oder X₁ steht für CH, X₂ steht für N und X₃ steht für CH,
wobei jedes CH gegebenenfalls durch Halogen oder C₁₋₄-Alkyl substituiert ist;
Y¹ und Y² jeweils unabhängig aus CH, CF und N ausgewählt sind;
R¹ für CH₃ oder CH₂CH₃ steht;
R² für Wasserstoff, Halogen oder C₁₋₄-Alkyl steht;
R¹² für C₁₋₂-Alkyl steht;
R¹³ und R¹⁴ jeweils unabhängig aus C₁₋₆-Alkyl ausgewählt sind;
R¹⁵ für Wasserstoff oder C₁₋₄-Alkyl steht;
R³ für Halogen steht;
R⁴ für C₁₋₆-Alkyl; C₃₋₆-Cycloalkyl; Di (C₁₋₄-alkyl) - amino, Pyrrolidinyl, Heteroaryl¹; Phenyl; Phenyl, das durch 1, 2 oder 3 Substituenten, die jeweils individuell aus Halogen, Hydroxy, Cyano, C₁₋₄-Alkyl, Polyhalogen-C₁₋₄-alkyl und C₁₋₄-Alkyloxy ausgewählt sind, substituiert ist; steht;
R⁶ für einen Substituenten steht, der aus Substituent (a), (b), (c), (d) oder (e) ausgewählt ist; wobei
(a) -(CO)-OH, -(CO)-NR⁷R⁸ oder -NR⁷R⁸ ist;
(b) Heteroaryl² ist;
(c) C₂₋₆-Alkenyl, das durch einen oder zwei Substituenten, die aus C₁₋₆-Alkyl, -(CO)-OH oder -(CO)-NR⁸R⁹ ausgewählt sind, substituiert ist, ist; oder
(d) -NR⁸-C(O)-Heterocyclus ist, wobei der Heterocyclus durch einen, zwei oder drei Substituenten, die jeweils unabhängig aus Halogen, Hydroxy, C₁₋₄-Alkyl oder C₁₋₄-Alkyloxy ausgewählt sind, substituiert ist; oder
(e) C₃₋₆-Cycloalkyl oder Heterocyclus ist, wobei das C₃₋₆-Cycloalkyl und der Heterocyclus durch einen, zwei oder drei Substituenten, die jeweils unabhängig aus
C₁₋₆-Alkyl;
C₁₋₆-Alkyl, das durch einen, zwei oder drei Substituenten, die jeweils unabhängig aus Halogen, Hydroxy, Hydroxycarbonyl und Aminocarbonyl ausgewählt sind, substituiert ist;
Hydroxy;
Halogen;
-(CO)-OH;
-(CO)-NR¹⁰R¹¹;
- (CO)-NR⁸-SO₂-R⁹,
-NR⁸R⁹;
-NR⁸-(CO)-C₁₋₄-Alkyl;
-NR⁸-(CO)-C₃₋₆-Cycloalkyl;
-NR⁸-SO₂-R⁹;
-SO₂-NR¹⁰R¹¹ oder
-SO₂-NR⁸-(CO)-R⁹
ausgewählt sind, substituiert ist;
wobei
R⁷ für Wasserstoff, C₁₋₄-Alkyl, Hydroxy-C₁₋₄-alkyl oder Dihydroxy-C₁₋₄-alkyl steht;
R⁸ jeweils unabhängig aus Wasserstoff, C₁₋₄-Alkyl oder Hydroxy-C₁₋₄-alkyl ausgewählt ist;
R⁹ für C₁₋₄-Alkyl, Polyhalogen-C₁₋₄-alkyl oder C₃₋₆-Cycloalkyl steht;
R¹⁰ und R¹¹ jeweils unabhängig aus Wasserstoff; C₁₋₄-Alkyl; Polyhalogen-C₁₋₄-alkyl; C₃₋₆-Cycloalkyl; C₃₋₆-Cycloalkyl, das durch C₁₋₄-Alkyl substituiert ist; oder C₁₋₄-Alkyl, das durch Hydroxy oder Cyano substituiert ist; ausgewählt sind;
Heterocyclus Azetidinyl, Pyrrolidinyl, Piperidinyl oder Homopiperidinyl ist;
Heteroaryl¹ Thienyl, Pyridinyl oder Pyrimidinyl ist, wobei Heteroaryl¹ jeweils gegebenenfalls durch einen oder zwei Substituenten, die jeweils unabhängig aus C₁₋₄-Alkyl, Halogen, Amino und Aminocarbonyl ausgewählt sind, substituiert ist;
Heteroaryl² Pyrrolyl, Pyrazinyl oder Thiazolyl ist, wobei Heteroaryl² jeweils gegebenenfalls durch einen oder zwei Substituenten, die jeweils unabhängig aus C₁₋₄-Alkyl, Halogen, -(CO)-OR⁷ oder -(CO)-NR⁸R⁹ ausgewählt sind, substituiert ist;
oder pharmazeutisch unbedenkliches Säureadditionssalz davon.

2. Verbindung nach Anspruch 1, wobei X₁ für N steht, X₂ für CH steht und X₃ für CH steht.

3. Verbindung nach Anspruch 1, wobei X₁ für N steht, X₂ für N steht und X₃ für CH steht.

4. Verbindung nach Anspruch 1, wobei X₁ für N steht, X₂ für CH steht und X₃ für N steht.

5. Verbindung nach Anspruch 1, wobei A für einen Rest der Formel (a-1) steht, wobei R¹ für CH₃ steht.

6. Verbindung nach Anspruch 1, wobei A für einen Rest der Formel (a-2) steht, wobei R¹ für CH₃ steht.

7. Verbindung nach Anspruch 1, wobei A für einen Rest der Formel (a-5) steht, wobei R¹ für CH₃ steht.

8. Verbindung nach einem der Ansprüche 1 bis 7, wobei R⁴ für C₃₋₆-Cycloalkyl steht.

9. Verbindung nach einem der Ansprüche 1 bis 8, wobei R⁶ für C₃₋₆-Cycloalkyl oder Pyrrolidinyl steht, wobei das C₃₋₆-Cycloalkyl oder Pyrrolidinyl durch einen oder zwei Substituenten, die jeweils unabhängig aus OH, -(CO)-OH oder -(CO)-NR¹⁰R¹¹ ausgewählt sind, substituiert sind.

10. Pharmazeutische Zusammensetzung, umfassend einen pharmazeutisch unbedenklichen Träger und eine therapeutisch wirksame Menge einer Verbindung nach einem der Ansprüche 1 bis 9.

11. Pharmazeutische Zusammensetzung nach Anspruch 10, die ferner ein anderes antivirales Mittel umfasst.

12. Pharmazeutische Zusammensetzung nach Anspruch 11, wobei es sich bei dem anderen antiviralen Mittel um eine RSV-inhibierende Verbindung handelt.

13. Verfahren zur Herstellung einer pharmazeutischen Zusammensetzung nach einem der Ansprüche 10 bis 12, bei dem man eine therapeutisch wirksame Menge einer Verbindung nach einem der Ansprüche 1 bis 9 innig mit einem pharmazeutisch unbedenklichen Träger mischt.

14. Verbindung nach einem der Ansprüche 1 bis 9 zur Verwendung als Medizin.

15. Verbindung nach einem der Ansprüche 1 bis 9 oder pharmazeutische Zusammensetzung nach einem der Ansprüche 10 bis 12 zur Verwendung bei der Behandlung oder Prävention einer Infektion mit dem Respiratory Syncytial Virus.

## Revendications

1. Composé de formule (I), 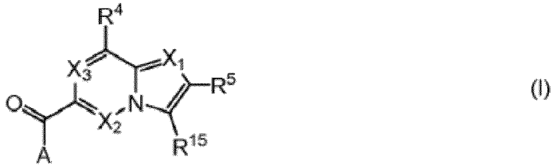
y compris une quelconque forme stéréochimiquement isomérique correspondantes,
A étant 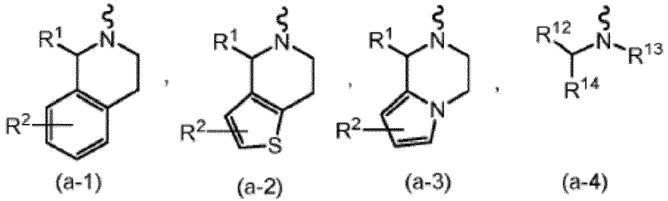 ou 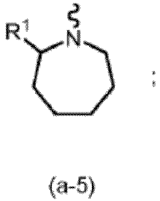
R⁵ étant 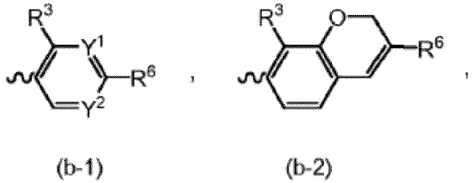 ou 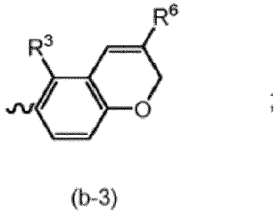
X₁, X₂, et X₃ étant choisis parmi
X₁ étant N, X₂ étant CH, et X₃ étant CH ;
ou X₁ étant N, X₂ étant N, et X₃ étant CH,
ou X₁ étant N, X₂ étant CH, et X₃ étant N,
ou X₁ étant CH, X₂ étant CH, et X₃ étant CH, et
ou X₁ étant CH, X₂ étant N, et X₃ étant CH,
chaque CH étant éventuellement substitué par halogéno ou C₁₋₄alkyle ;
Y¹ et Y² étant chacun indépendamment choisis parmi CH, CF et N ;
R¹ étant CH₃ ou CH₂CH₃ ;
R² étant hydrogène, halogéno ou C₁₋₄alkyle ;
R¹² étant C₁₋₂alkyle ;
R¹³ et R¹⁴ étant chacun indépendamment choisis parmi C₁₋₆alkyle ;
R¹⁵ étant hydrogène ou C₁₋₄alkyle ;
R³ étant halogéno ;
R⁴ étant C₁₋₆alkyle ; C₃₋₆cycloalkyle ; di(C₁₋₄alkyl)amino, pyrrolidinyle, hétéroaryle¹ ; phényle ; phényle substitué par 1, 2 ou 3 substituants chacun individuellement choisi parmi halogéno, hydroxy, cyano, C₁₋₆alkyle, polyhalogéno-C₁₋₄alkyle, et C₁₋₄alkyloxy ;
R⁶ étant un substituant choisi parmi un substituant (a), (b), (c), (d) ou (e) ;
(a) étant -(CO)-OH, -(CO)-NR⁷R⁸, ou -NR⁷R⁸ ;
(b) étant hétéroaryle² ;
(c) étant C₂₋₆alcényle substitué par un ou deux substituants choisis parmi C₁₋₆alkyle, -(CO)-OH ou-(CO)-NR⁸R⁹ ; ou
(d) étant -NR⁸-(CO)-hétérocycle, ledit hétérocycle étant substitué par un, deux ou trois substituants chacun indépendamment choisi parmi halogéno, hydroxy, C₁₋₄alkyle ou C₁₋₄alkyloxy ; ou
(e) étant C₃₋₆cycloalkyle ou hétérocycle, lesdits C₃₋₆cycloalkyle et hétérocycle étant substitués par un, deux ou trois substituants chacun indépendamment choisi parmi
C₁₋₆alkyle ;
C₁₋₆alkyle substitué par un, deux ou trois substituants chacun indépendamment choisi parmi halogéno, hydroxy, hydroxycarbonyle, et aminocarbonyle ;
hydroxy ;
halogéno ;
-(CO)-OH ;
-(CO)-NR¹⁰R¹¹ ;
-(CO)-NR⁸-SO₂-R⁹ ;
-NR⁸R⁹ ;
-NR⁸-(CO)-C₁₋₄alkyle ;
-NR⁸-(CO)-C₃₋₆cycloalkyle ;
-NR⁸-SO₂-R⁹ ;
-SO₂-NR¹⁰R¹¹ ; ou
-SO₂-NR⁸-(CO)-R⁹ ;
R⁷ étant hydrogène, C₁₋₄alkyle, hydroxy-C₁₋₄alkyle ou dihydroxy-C₁₋₄alkyle ;
chaque R⁸ étant indépendamment choisi parmi hydrogène, C₁₋₄alkyle, ou hydroxy-C₁₋₄alkyle ;
R⁹ étant C₁₋₄alkyle, polyhalogéno-C₁₋₄alkyle, ou C₃₋₆cycloalkyle ;
R¹⁰ et R¹¹ étant chacun indépendamment choisis parmi hydrogène ; C₁₋₄alkyle ; polyhalogéno-C₁₋₄alkyle ; C₃₋₆cycloalkyle ; C₃₋₆cycloalkyle substitué par C₁₋₄alkyle ; ou C₁₋₄alkyle substitué par hydroxy ou cyano ;
hétérocycle étant azétidinyle, pyrrolodinyle, pipéridinyle, ou homopipéridinyle ;
hétéroaryle¹ étant thiényle, pyridinyle ou pyrimidinyle, chaque hétéroaryle¹ étant éventuellement substitué par un ou deux substituants chacun indépendamment choisi parmi C₁₋₄alkyle, halogéno, amino, et aminocarbonyle ;
hétéroaryle² étant pyrrolyle, pyrazolyle ou thiazolyle ; chaque hétéroaryle² étant éventuellement substitué par un ou deux substituants chacun indépendamment choisi parmi C₁₋₄alkyle, halogéno, -(CO)-OR⁷ ou -(CO)-NR⁸R⁹ ;
ou sel d'addition d'acide pharmaceutiquement acceptable correspondant.

2. Composé selon la revendication 1, X₁ étant N, X₂ étant CH, et X₃ étant CH.

3. Composé selon la revendication 1, X₁ étant N, X₂ étant N, et X₃ étant CH.

4. Composé selon la revendication 1, X₁ étant N, X₂ étant CH, et X₃ étant N.

5. Composé selon la revendication 1, A étant un radical de formule (a-1), R¹ étant CH₃.

6. Composé selon la revendication 1, A étant un radical de formule (a-2), R¹ étant CH₃.

7. Composé selon la revendication 1, A étant un radical de formule (a-5), R¹ étant CH₃.

8. Composé selon l'une quelconque des revendications 1 à 7, R⁴ étant C₃₋₆cycloalkyle.

9. Composé selon l'une quelconque des revendications 1 à 8, R⁶ étant C₃₋₆cycloalkyle ou pyrrolidinyle, ledit C₃₋₆cycloalkyle ou pyrrolidinyle étant substitué par un ou deux substituants chacun indépendamment choisi parmi OH, -(CO)-OH ou -(CO)-NR¹⁰R¹¹.

10. Composition pharmaceutique comprenant un support pharmaceutiquement acceptable et une quantité thérapeutiquement active d'un composé selon l'une quelconque des revendications 1 à 9.

11. Composition pharmaceutique selon la revendication 10, qui comprend en outre un autre agent antiviral.

12. Composition pharmaceutique selon la revendication 11, l'autre agent antiviral étant un composé inhibant RSV.

13. Procédé pour la préparation d'une composition pharmaceutique selon l'une quelconque des revendications 10 à 12, une quantité thérapeutiquement active d'un composé selon l'une quelconque des revendications 1 à 9 étant intimement mélangée avec un support pharmaceutiquement acceptable.

14. Composé selon l'une quelconque des revendications 1 à 9 pour une utilisation en tant que médicament.

15. Composé selon l'une quelconque des revendications 1 à 9, ou composition pharmaceutique selon l'une quelconque des revendications 10 à 12, pour une utilisation dans le traitement ou la prévention d'une infection par un virus syncytial respiratoire.
